# EUROPEAN PATENT APPLICATION

(11) **EP 4 800 032 A1**
(43) Date of publication of application: **02.09.2026**
(21) Application number: 24881795.9
(22) Date of filing: 28.10.2024
(51) Int. Cl.: C07K 16/28, C07K 19/00, C12N 15/62, A61P 35/00

(54) **T-CELL RECEPTOR-BASED MULTI-SPECIFIC POLYPEPTIDE MOLECULE, AND COMPOSITION AND USE THEREOF**

(30) Priority: 27.10.2023 CN 202311415371
(71) Applicant: Corregene Biotechnology Co., Ltd., Beijing 102206 (CN)
(72) Inventor: JIANG, Dong, Beijing 102206 (CN); XIE, Xingwang, Beijing 102206 (CN); BI, Jinglei, Beijing 102206 (CN); YAN, Baoqi, Beijing 102206 (CN); QIAO, Xuewei, Beijing 102206 (CN); LI, Yonghong, Beijing 102206 (CN); WANG, Jianghua, Beijing 102206 (CN); WANG, Xueyan, Beijing 102206 (CN); FU, Shuangli, Beijing 102206 (CN); LI, Xian, Beijing 102206 (CN)
(74) Representative: Maiwald GmbH
(86) International application number: PCT/CN2024/127743
(87) International publication number: WO 2025/087436

(57) **Abstract**

The present invention relates to a T-cell receptor-based multi-specific polypeptide molecule. Also provided are a nucleic acid encoding the polypeptide molecule, a vector or a vector system comprising the nucleic acid, a host cell comprising the polypeptide molecule, a conjugate and a pharmaceutical composition comprising the polypeptide molecule, and a method for preventing or treating a disease in a subject by using the polypeptide molecule.

## Description

This international patent application claims the priority benefit of Chinese Patent Application CN202311415371.1 filed on October 27, 2023, the entire contents of which are incorporated herein by reference.

### FIELD OF THE INVENTION

The present disclosure relates to T cell receptor-based multi-specific polypeptide molecules and use thereof, particularly use in the treatment of cancer, infectious diseases, autoimmune diseases and/or inflammatory diseases.

### BACKGROUND

T cells are an important component of the adaptive immune system in vertebrates, and play a crucial role in viral infection, cancer and autoimmunity. TCR recognition of the major histocompatibility complex (MHC)-antigen peptide complex (pMHC) is an important step in T cell-mediated immune responses. The non-covalent binding of TCR with the CD3 signaling apparatus consisting of γ, δ, ε and ζ subunits forms the TCR-CD3 complex. The binding of pMHC to TCR activates LCK-induced phosphorylation of the intracellular immunoreceptor tyrosine-based activation motif (ITAM) in the TCR CD3ζ subunit. Then, CD3ζ phosphorylation triggers a series of phosphorylation signal transduction involving ζ-chain-associated protein kinase 70 (ZAP70) and linker for activation of T cells (LAT) in T cells, resulting in recruitment of multiple downstream adaptor and signaling molecules and formation of the LAT signalosome. The assembled LAT signalosome activates multiple signaling pathways involving transcription factors, such as activator protein 1 (AP-1), nuclear factor-κB (NF-κB) and nuclear factor of activated T cells (NFAT). Activated transcription factors can cause subsequent T cell activation, proliferation, cytokine production and effector function.

Bispecific antibodies (BsAbs) refer to antibody molecules that simultaneously target two antigens or two different antigen epitopes of a single antigen. Compared with conventional antibodies, bispecific antibodies have higher specificity, can more accurately target tumor cells, and reduce off-target toxicity. With the development of recombinant protein expression technology and antibody engineering technology, many different antibody formats have been generated. Multi-specific antibodies are used for various purposes, including (1) receptor activation, (2) blockade, (3) internalization, (4) clustering, (5) binding of membrane-associated proteins, or (6) directed targeting of cytotoxic effector cells.

T cell-redirecting bsAb (also known as a T cell engager, TcE) is an important form of cytotoxic effector cell redirection and is currently a central pillar of cancer immunotherapy. Such bsAb can recognize a target on the surface of tumor cells while recognizing a molecule on the surface of T cells (which in most cases is CD3), thereby allowing the bsAb to couple tumor cells and cytotoxic T cells together to trigger activation of downstream signaling pathways of the TCR in T cells, and kill tumor cells through the cytotoxicity of T cells. Blincyto (blinatumomab) is a CD19/CD3 bispecific antibody, which led to complete remission in 69% of relapsed/refractory B-precursor acute lymphoblastic leukemia (ALL) patients. At present, a large number of novel T cell-redirecting antibody formats have emerged, such as BiTE, BiTE-Fc, DART-Fc, TriTAC, and the like.

bsAb based on antibody recognition of molecules on the surface of target cells only recognizes tumor cell surface antigens and cannot recognize nearly 90% of intracellular proteins. In contrast, TCR can recognize antigen peptides of intracellular and cell surface tumor-specific antigens that are processed and presented on cell-surface MHC molecules, thus having a broader recognition scope. In TCR-based T cell-redirecting bispecific antibodies, the target-cell recognition region is replaced from a conventional antibody to a TCR, thereby enabling the use of the broad recognition characteristics of TCR to increase target selectivity.

For protein drugs, the glomerular filtration cutoff is generally around 60kDa. That is, low-molecular-weight antibody fragments or derivatives, such as nanobodies (15kDa) and scFv (28kDa), and even Fab (50kDa), will be cleared from the body through glomerular filtration, resulting in a short half-life. Among them, nanobodies have the smallest molecular weight among all antibody types and often have a half-life of only tens of minutes. Therefore, in order to increase the efficacy of protein drugs, it is necessary to improve protein drugs to extend their half-life.

### SUMMARY

To increase the in vivo efficacy of TcE, in addition to increasing the affinity of TCR to pMHC, the valency of the TCR of the TcE for the target-cell targets can also be increased, and the valency of the functional element of TcE for surface molecules on the effector cells can also be increased, so as to enhance T cell killing activity and proliferative capacity. Meanwhile, in order to maintain the ability of TcE to enter tumors while maintaining an appropriate half-life of TcE in vivo, it is necessary to keep the molecular size of TcE within a certain range and appropriately add other functional domains in the polypeptide molecule, such as an antibody-derived hinge region, an antibody Fc domain or a dimerization portion thereof and/or an albumin(Alb) or an albumin-binding domain thereof. The addition of these functional domains not only extends the half-life of the polypeptide molecule, but also maintains the killing activity of the polypeptide molecule. Accordingly, the present disclosure provides TCR-based multi-specific polypeptide molecules, such as TCR-based bispecific polypeptide molecules, trispecific polypeptide molecules or tetraspecific polypeptide molecules.

### Definitions

Unless otherwise stated or defined, all terms used have ordinary meanings commonly understood by those skilled in the art. For example, the definitions of terms used herein are as described in Immunobiology, fifth edition, New York: GarlandScience(2001), by Janeway CA Jr, Travers P, Walport M, et al., and "A multilingual glossary of biotechnological terms: (IUPAC Recommendations)", edited by Leuenberger, H.G.W, Nagel, B. and Kölbl, H. (1995), Helvetica Chimica Acta, CH-4010 Basel, Switzerland.

It should be noted that, as used herein and in the appended claims, the singular forms "a/an" and "the/said" include plural referents, unless the context clearly dictates otherwise. Therefore, the terms "a/an", "one", "one or more", and "at least one" may be used interchangeably. Similarly, the terms "comprising", "including" and "having" may be used interchangeably.

Unless otherwise stated or defined, the term "comprising" and variants thereof such as "including" and "containing" should be understood to mean including the stated elements or steps but not excluding any other elements or steps. For purposes of the present disclosure, the term "consisting of..." is regarded as a preferred embodiment of the term "comprising". If a group is defined below as including or containing at least a certain number of embodiments, it should also be understood that a preferred group consisting only of these embodiments is also disclosed.

As used herein, the term "T cell receptor" or "TCR" includes natural TCR as well as TCR variants, fragments and constructs. Thus, the term includes heterodimers containing TCR α chain and TCR β chain as well as multimers and single-chain constructs; optionally containing other domains and/or portions, provided that the TCR retains its ability to recognize an antigen target.

In its native form, TCR exists as a complex of several proteins on the surface of T cells. The T cell receptor consists of two (separate) protein chains, which are produced from independent T cell receptor α and β (TCRα and TCRβ) genes, and are referred to as the α chain and the β chain. Each chain of TCR has an N-terminal immunoglobulin-like (Ig)-variable (V) region/domain, an Ig-constant (C) region/domain, a transmembrane/cell membrane-spanning region anchoring the chain in the plasma membrane, and a short C-terminal cytoplasmic tail.

Antigen specificity is conferred by the variable regions (TRAV and TRBV) of the α and β chains. The two variable regions of the TCR α chain and the β chain each comprise three hypervariable or complementarity-determining regions (CDR1α/β, CDR2α/β and CDR3α/β) flanked by framework (FR) regions. CDR3 is the major determinant of antigen recognition and specificity (i.e., the ability to recognize and interact with a specific antigen), whereas CDR1 and CDR2 mainly interact with MHC molecules that present antigen peptides.

The TCR recognizes an antigenic peptide, which binds to a Major Histocompatibility Complex (MHC) molecule on the surface of an antigen-presenting cell ("presented/displayed on an MHC molecule"). Antigenic peptides presented on MHC molecules are also referred to herein as a "complex of an epitope with an MHC molecule", an "epitope-MHC complex", an "antigen-MHC complex" or a "target antigenic peptide-MHC complex". There are two different classes of MHC molecules: MHC I and MHC II, which present peptides from different cellular compartments. MHC class I molecules are expressed on the surface of all nucleated cells in the human body, and display peptides or protein fragments from intracellular compartments to cytotoxic T cells. In humans, MHC is also referred to as Human Leukocyte Antigen (HLA). There are three main types of MHC class I: HLA-A, HLA-B and HLA-C. Once the TCR binds its specific epitope-MHC complex, the T cell is activated and exerts biological effector functions.

As used herein, the term "TCR constant region" includes the TCR α chain constant region (TRAC) and the β chain constant region (TRBC), which may be a human constant region or derived from another species, such as mouse.

It has been reported that adding a disulfide bond in the constant region can promote correct pairing of the TCR α and β chains (Kuball J et al Blood.2007 Mar 15; 109(6):2331-8). Therefore, it is also contemplated herein to add one or more cysteine modifications in the constant region to form a disulfide bond between the TCR α chain and the TCR β chain.

The sequences of the wild-type TCR constant regions may be found in the public database of the International ImMunoGeneTics information system (IMGT), e.g., the constant domain sequence of the TCR molecule α chain is "TRAC*01", and the constant domain sequence of the TCR molecule β chain is "TRBC1*01" or "TRBC2*01".

In the present disclosure, the positions of the amino acid sequence of the wild-type TCR are numbered according to the nomenclature rules of the International ImMunoGeneTics information system (IMGT). E.g., for an amino acid in the TCR α chain variable region (TRAV), if the position number listed in IMGT is 104, then it is described in the present disclosure as the amino acid at position 104 of TRAV; for an amino acid in the TCR β chain variable region (TRBV), if the position number listed in IMGT is 84, then it is described in the present disclosure as the amino acid at position 84 of TRBV; and so on. E.g., for an amino acid in the TCR α chain constant region (TRAC), if the position number listed in IMGT is 48, then it is described herein as the amino acid at position 48 of TRAC; for an amino acid in the TCR β chain constant region (TRBC), if the position number listed in IMGT is 57, then it is described herein as the amino acid at position 57 of TRBC; and so on. In the present disclosure, where the sequence position numbering of other amino acids is specially described, the special description shall apply.

As used herein, the term "antibody" refers to an immunoglobulin molecule having the ability to specifically bind to a specific antigen. Such molecules usually comprise two heavy (H) chains and two light (L) chains interconnected by disulfide bonds. Each heavy chain consists of a heavy chain variable region (or domain) (abbreviated herein as VH) and a heavy chain constant region. The heavy chain constant region consists of three domains CH1, CH2 and CH3. Each light chain consists of a light chain variable region (or domain) (abbreviated herein as VL) and a light chain constant region. The light chain constant region consists of one domain CL. The variable regions of antibody heavy and light chains contain binding domains that interact with the antigen. The constant region of an antibody can mediate binding of the immunoglobulin to host tissues or factors, including various cells of the immune system (e.g., effector cells) and components of the complement system such as C1q (the first component in the classical pathway of complement activation).

The heavy chain of immunoglobulin may be divided into three functional regions: the Fd region, the hinge region and the Fc region (fragment crystallizable). The Fd region contains the VH and CH1 domains, and associates with the light chain to form Fab (antigen binding fragment). The Fc region is also referred to as the Fc domain, which contains two or three heavy chain constant domains (CH2, CH3, CH4) in each heavy chain. In IgG class antibodies, the Fc region contains the CH2 and CH3 domains. The Fc fragment is responsible for immunoglobulin effector functions, including, e.g., complement binding and binding to cognate Fc receptors on effector cells. In the native structure of immunoglobulins, the Fc domains of two heavy chains dimerize to form an Fc dimer. The portion of the Fc domain capable of facilitating formation of an Fc dimer is referred to as the "dimerization portion of the Fc domain", which typically includes a portion of the CH2 domain and the CH3 domain.

The hinge region of IgG class antibodies refers to a short amino acid sequence region between the CH1 and CH2 portions of the heavy chain, which is relatively flexible in the native state of the antibody. Hinge regions found in the IgG, IgA and IgD immunoglobulin classes act as flexible spacer regions, allowing the Fab portion to move freely in space relative to the Fc region. Hinge domains are structurally diverse, with different sequences and lengths between immunoglobulin classes and subclasses. According to crystallographic studies, the immunoglobulin hinge region may be further subdivided structurally and functionally into three regions: the upper hinge, the core hinge and the lower hinge (Shin et al., Immunological Reviews 130:87, 1992). The upper hinge includes amino acids from the carboxyl terminus of CH1 to the first residue in the hinge that restricts movement, typically the first cysteine residue that forms an interchain disulfide bond between the two heavy chains. The length of the upper hinge region is related to the fragment flexibility of the antibody. The core hinge region contains inter-heavy chain disulfide bonds. The lower hinge region connects to the amino terminus of the CH2 domain and includes residues in the CH2 domain. The core hinge region of human IgG1 contains the sequence Cys-Pro-Pro-Cys (SEQ ID NO: 1) that produces a cyclic octapeptide upon dimerization via disulfide bonds, and the cyclic octapeptide is believed to act as a pivot, thereby imparting flexibility. The structure of the polypeptide sequence of the immunoglobulin hinge region and the conformational changes permitted by its flexibility can affect effector functions of the antibody Fc portion.

A "light chain variable region (VL)" or a "heavy chain variable region (VH)" is composed of "framework" regions interspersed with three "complementarity-determining regions" or "CDRs". The framework regions are used to position the CDRs for specific binding to antigen epitopes. CDRs contain amino acid residues in an antibody that are primarily responsible for antigen binding. From the amino terminus to the carboxyl terminus, both VL and VH domains contain the following framework (FR) regions and CDR regions: FR1, CDR1, FR2, CDR2, FR3, CDR3 and FR4.

A single-domain antibody (sdAb), also known as a nanobody (nanobody), consists of a heavy chain variable domain. This heavy chain variable domain was originally found in a heavy chain antibody (HCAb) isolated from the serum of camelids and sharks, and the heavy chain variable domain therein was amplified by genetic means. This variable domain present in naturally occurring heavy chain antibodies is referred to as "VHH", in order to distinguish it from the heavy chain variable domain (VH) present in conventional 4-chain antibodies and the light chain variable domain (VL) present in conventional 4-chain antibodies. VHH cloned and expressed alone has structural stability comparable to that of the original heavy chain antibody and equivalent antigen binding activity, and VHH is currently known to be the smallest unit capable of binding a target antigen.

The VHH domain has many unique structural features and functional properties, and these structural features and functional properties make isolated VHH domains (as well as VHH-domain-based nanobodies that share these structural features and functional properties with naturally occurring VHH domains) and proteins comprising isolated VHH domains very advantageous for use as functional antigen binding domains or proteins. In particular, but not limited thereto, the VHH domain (which in essence has been "designed" to bind antigen functionally in the absence of a light chain variable domain and without any interaction with a light chain variable domain) and nanobodies mayserve as a single, relatively small, functional antigen binding structural unit, domain or protein. This distinguishes the VHH domain from the VH and VL domains of conventional 4-chain antibodies, the latter of which per se are generally not suitable for practical application as a single antigen binding protein or domain, but rather need to be combined in some form to provide a functional antigen binding unit (e.g., in conventional antibody fragments such as Fab fragments; in scFv fragments composed of a VH domain covalently connected to a VL domain).

The amino acid assignment of each VL, VH and VHH domain follows any conventional definition of CDRs. Conventional definitions include the Kabat definition (Kabat, Sequences of Proteins of Immunological Interest (National Institutes of Health, Bethesda, MD, 1987 and 1991)); the Chothia definition (Chothia & Lesk, J. Mol. Biol. 196:901-917, 1987; Chothia et al., Nature 342:878-883, 1989); a composite of Chothia Kabat CDRs, wherein CDR-H1 is a composite of Chothia and Kabat CDRs; the AbM definition used by the antibody modeling software of Oxford Molecular; and the CONTACT definition of Martin et al. (available at bioinfo.org.uk/abs). Kabat provides a widely used numbering convention (the Kabat numbering system), in which corresponding residues between different heavy chains or between different light chains are assigned the same numbering. The present disclosure may use CDRs as defined according to any one of these numbering systems, but preferred embodiments use CDRs as defined by Kabat.

As used herein, the term "antibody" should be understood in its broadest sense and includes monoclonal antibodies (including full-length monoclonal antibodies), polyclonal antibodies, antibody fragments, and multi-specific antibodies (e.g., bispecific antibodies) comprising at least two antigen binding regions. Antibodies may contain additional modifications, such as non-naturally occurring amino acids, mutations in the Fc region, and mutations at glycosylation sites. Antibodies also include post-translationally modified antibodies, fusion proteins comprising antigenic determinants of antibodies, and immunoglobulin molecules containing any other modifications to antigen recognition sites, provided that such antibodies exhibit the intended biological activity.

As used herein, an "antigen binding fragment" of an antibody refers to one or more antibody fragments that retain the ability to specifically bind an antigen. It has been shown that the antigen binding function of an antibody may be performed by fragments of a full-length antibody.

Examples of antigen binding fragments include, but are not limited to, (i) a Fab fragment, which is a monovalent fragment consisting of VL, VH, CL and CH1 domains; (ii) a F(ab')2 fragment, which is a bivalent fragment containing two Fab fragments linked by disulfide bonds in the hinge region; (iii) a Fab' fragment, which is essentially a Fab, but having a portion of the hinge region (see, FUNDAMENTAL IMMUNOLOGY (Paul ed., 3rd ed. 1993); (iv) an Fd fragment consisting of VH and CH1 domains; (v) an Fd' fragment having VH and CH1 domains and one or more cysteine residues located at the C-terminus of the CH1 domain; (vi) an Fv fragment consisting of VL and VH domains of a single arm of an antibody; (vii) a dAb fragment (Ward et al. (1989) Nature 341:544-546), which consists of a VH domain; (viii) an isolated complementarity-determining region (CDR). In addition, although the two domains VL and VH of an Fv fragment are encoded by independent genes, they may be linked by a synthetic linker using recombinant methods, which synthetic linker enables them to form a single protein chain, in which the VL and VH regions pair to form a monovalent molecule (referred to as a single-chain Fv (scFv); see, e.g., Bird et al. (1988) Science 242, 423-426; and Huston et al. (1988) Proc. Natl. Acad. Sci. USA 85, 5879-5883). Such single-chain antibodies are also intended to be included within the "antigen binding fragment" of an antibody. In addition, the term also includes "linear antibodies" containing a pair of tandem Fd fragments (VH-CH1-VH-CH1), which together with complementary light chain polypeptides and modified versions of any foregoing fragments that retain antigen binding activity form antigen binding regions.

As used herein, the term "antigen" refers to any substance capable of inducing an immune response in an organism. That is, a substance that can be specifically recognized and bound by antigen receptors (TCR/BCR) on the surface of T/B lymphocytes, activates T/B cells, causes them to proliferate and differentiate to produce immune response products (sensitized lymphocytes or antibodies), and can specifically bind to the corresponding products in vivo and in vitro.

In this document, antigens may be tumor-associated antigens (TAA), tumor-specific antigens (TSA), viral antigens, self-antigens, and immune cell surface molecules.

As used herein, the term "tumor-associated antigen (TAA)" refers to an antigen that is differentially expressed in tumor cells as compared to normal cells (e.g., markedly increased expression on tumor cells).

As used herein, the term "tumor-specific antigen (TSA)" refers to an antigen that is unique to tumor cells and is not present in normal tissues and cells.

As used herein, the term "viral antigen" refers to a substance in a virus that is capable of inducing an immune response in an organism.

As used herein, the term "self-antigen" refers to an antigenic substance that is produced when biological, physical, or chemical factors cause changes in the structure and components of self-tissue cells, thereby eliciting an immune response of the organism's immune system against these self-tissue cell components. The release of sequestered antigens can produce an immune response. Changes in self components may produce self-antigens. E.g., denatured IgG can stimulate an organism to produce anti-denatured IgG antibodies (rheumatoid factor), causing rheumatoid arthritis. After clinical use of certain drugs, the antigenicity of blood cell surfaces may be altered, causing autoimmune hemolytic anemia or granulocytopenia. Cross-reactions triggered by shared antigens can produce an immune response. Certain bacteria and viruses have epitopes similar to those on certain tissue cells of the normal human body, and autoantibodies and sensitized lymphocytes produced against the epitopes of these bacteria and viruses can cross-react with self tissue cells, causing autoimmune diseases.

"Immune cell surface molecules" may include, e.g., immune cell surface antigens (e.g., T cell antigens) and costimulatory molecules.

The site on an antigen to which a TCR or antibody binds is called an "epitope". An epitope may be formed by contiguous amino acids or by discontinuous amino acids juxtaposed by tertiary folding of one or more proteins. Epitopes formed by contiguous amino acids (also referred to as linear epitopes) are usually retained after exposure to denaturing solvents, whereas epitopes formed by tertiary folding (also referred to as conformational epitopes) are usually lost upon treatment with denaturing solvents. An epitope typically comprises at least 3 amino acids in a unique spatial conformation, more typically at least 5, or 8-10, amino acids. An epitope defines the minimal binding site of a TCR or antibody, and thus is a specific target of a TCR or antibody, or an antigen binding fragment thereof.

In this document, the term "albumin" encompasses any native albumin from any vertebrate source, the vertebrate sources including mammals such as primates (e.g., humans and monkeys) and rodents (e.g., mice and rats). Albumin also encompasses full-length, unprocessed albumin preproprotein and any form of albumin produced by processing in cells. Albumin also encompasses variants of naturally occurring albumin, such as splice variants or allelic variants. The term also encompasses any recombinant form of albumin. Albumin sequences are known in the art. For example, information on the human serum albumin gene (including the genomic DNA sequence) may be found in NCBI. An exemplary amino acid sequence of full-length human serum albumin preproprotein may be found under NCBI accession number NP_000468.1. Human serum albumin is synthesized in the form of preproalbumin, and mature albumin is a single-chain polypeptide of 585 amino acid residues, with a molecular weight of 66458, contains 17 disulfide bonds, and does not contain a carbohydrate component. In a body fluid environment of pH7.4, albumin is an anion, and each molecule may carry more than 200 negative charges. Approximately 10% of exogenously infused albumin enters the extravascular interstitial space after 2h, and equilibrium is reached in 20 days. The half-life of albumin in plasma is 15-19 days.

### >gi|4502027|ref|NP_000468.1|serum albumin preproprotein[Homo sapiens]

MKWVTFISLLFLFSSAYS**RGVFRR***DAHKSEVAHRFKDLGEENFKALVLIAFAQYLQQCPFEDHVKL VNEVTEFAKTCVADESAENCDKSLHTLFGDKLCTVATLRETYGEMADCCAKQEPERNECFLQHKDDNPN LPRLVRPEVDVMCTAFHDNEETFLKKYLYEIARRHPYFYAPELLFFAKRYKAAFTECCQAADKAACLLPKL DELRDEGKASSAKQRLKCASLQKFGERAFKAWAVARLSQRFPKAEFAEVSKLVTDLTKVHTECCHGDLLE CADDRADLAKYICENQDSISSKLKECCEKPLLEKSHCIAEVENDEMPADLPSLAADFVESKDVCKNYAEAK DVFLGMFLYEYARRHPDYSVVLLLRLAKTYETTLEKCCAAADPHECYAKVFDEFKPLVEEPQNLIKQNCEL FEQLGEYKFQNALLVRYTKKVPQVSTPTLVEVSRNLGKVGSKCCKHPEAKRMPCAEDYLSVVLNQLCVLHE KTPVSDRVTKCCTESLVNRRPCFSALEVDETYVPKEFNAETFTFHADICTLSEKERQIKKQTALVELVKHKP KATKEQLKAVMDDFAAFVEKCCKADDKETCFAEEGKKLVAASQAALGL* (the underline indicates the signal peptide, the bold indicates the propeptide, and the italics indicate the mature human serum albumin (SEQ ID NO: 2)).

As used herein, the term "albumin binding moiety" refers to a fragment that is non-covalently linked, or is covalently connected to albumin via conjugation or direct genetic fusion, and that retains the minimal functional unit for binding to albumin. In some embodiments, the albumin binding moiety includes any known moiety capable of binding to albumin, e.g., may be an endogenous or exogenous ligand that specifically binds albumin, an organic small molecule, a fatty acid, a protein, a short peptide (e.g., QRLMEDICLPRWGCLWEDDF (SEQ ID NO: 3)), an antibody, an antibody fragment and an antibody-like scaffold, streptococcal Protein G protein, and the like.

As used in the present disclosure, the term "sequence identity" refers to the degree to which two sequences (amino acids), after alignment, have identical residues at the same positions. E.g., "an amino acid sequence is X% identical to SEQ ID NO: Y" refers to the percentage identity of the amino acid sequence to SEQ ID NO: Y, and is described as X% of the residues in the amino acid sequence being identical to the sequence residues disclosed in SEQ ID NO: Y.

Such calculations are usually performed using computer programs. Exemplary programs for comparing and aligning sequence pairs include ALIGN (Myers and Miller, 1988), FASTA (Pearson and Lipman, 1988; Pearson, 1990) and gapped BLAST (Altschul et al., 1997), BLASTP, BLASTN or GCG (Devereux et al., 1984).

In addition, when determining the degree of sequence identity between two amino acid sequences, those skilled in the art may consider so-called "conservative" amino acid substitutions, which can generally be described as amino acid substitutions in which an amino acid residue is replaced by another amino acid residue having a similar chemical structure, and which have little or essentially no effect or basically no effect on the function, activity, or other biological properties of a polypeptide. Such conservative amino acid substitutions are well known in the art, e.g., WO 04/037999, GB-A-2 357 768, WO 98/49185, WO 00/46383 and WO 01/09300; and (preferably) the types and/or combinations of these substitutions may be selected according to the relevant teachings from WO 04/037999 and WO 98/49185 and additional references cited therein.

Such conservative substitutions preferably are substitutions in which one amino acid in groups (a) to (e) below is substituted with another amino acid residue in the same group: (a) small aliphatic, nonpolar or weakly polar residues: Ala, Ser, Thr, Pro and Gly; (b) polar, negatively charged residues and their (uncharged) amides: Asp, Asn, Glu and Gln; (c) polar, positively charged residues: His, Arg and Lys; (d) large aliphatic, nonpolar residues: Met, Leu, Ile, Val and Cys; and (e) aromatic residues: Phe, Tyr and Trp.

Particularly preferred conservative substitutions are as follows: Ala to Gly or to Ser; Arg to Lys; Asn to Gln or to His; Asp to Glu; Cys to Ser; Gln to Asn; Glu to Asp; Gly to Ala or to Pro; His to Asn or to Gln; Ile to Leu or to Val; Leu to Ile or to Val; Lys to Arg, to Gln or to Glu; Met to Leu, to Tyr or to Ile; Phe to Met, to Leu or to Tyr; Ser to Thr; Thr to Ser; Trp to Tyr; Tyr to Trp; and/or Phe to Val, to Ile or to Leu.

As used herein, the term "cysteine-containing peptide segment" refers to a polypeptide comprising cysteine residues that is capable of forming a disulfide bond between two polypeptide chains comprising the peptide segment, and has a length of 3-10 amino acids.

As used herein, the term "vector" refers to a nucleic acid molecule used as a vehicle for transferring (exogenous) genetic material into a host cell, in which the nucleic acid molecule serving as a vector may, e.g., replicate and/or be expressed.

As used herein, the term "host cell" refers to any type of cell into which an expression vector has been introduced so that it can express exogenous genetic material.

The term "pharmaceutically acceptable" means that the carrier or adjuvant is compatible with the other components of the composition and has no substantial toxicity to its recipient, and/or that such carrier or adjuvant is approved or may be used for inclusion in a pharmaceutical composition for parenteral administration to humans.

As used herein, the terms "treatment", "treating", and the like refer to administering an agent or performing a procedure in order to obtain an effect. These effects may be prophylactic in terms of completely or partially preventing a disease or its symptoms, and/or may be therapeutic in terms of a partial or complete cure affecting a disease and/or disease symptoms. As used in the present disclosure, "treatment" may include treatment of a disease or disorder (e.g., cancer, infectious disease, autoimmune disease or inflammatory disease) in a mammal, particularly a human, and includes: (a) preventing occurrence of the disease or disease symptoms in a subject susceptible to the disease but not yet diagnosed as having the disease (e.g., including a disease that may be related to or caused by the primary disease); (b) inhibiting the disease, i.e., preventing its development; (c) alleviating the disease, i.e., causing regression of the disease. Treatment may refer to any indication of success in treating or improving or preventing cancer, including any objective or subjective parameters, such as elimination; alleviation; reducing symptoms or making the disease condition more tolerable to the patient; slowing the rate of deterioration or decline; or reducing end-stage debilitation associated with disease progression. The treatment or improvement of symptoms is based on one or more objective or subjective parameters, including the results of a physician's examination. Therefore, the term "treatment" includes administering the multi-specific polypeptide molecule or pharmaceutical composition or conjugate disclosed in the present disclosure to prevent or delay, alleviate or stop or inhibit the development of symptoms or disorders associated with a disease (e.g., cancer). The term "therapeutic effect" refers to a reduction, elimination, or prevention of a disease, disease symptoms, or disease side effects in a subject.

As used herein, the term "effective amount" means an amount of a therapeutic agent that, when administered to a subject for treating or preventing a disease, is sufficient to achieve such treatment or prevention. An "effective amount" may vary depending on the compound, the disease and its severity, and the age, body weight, etc. of the subject to be treated. A "therapeutically effective amount" refers to an effective amount for therapeutic treatment. A "prophylactically effective amount" refers to an effective amount for prophylactic treatment.

As used herein, the term "subject" refers to any mammalian subject in need of diagnosis, cure or treatment. A "mammal" for therapeutic purposes refers to any animal classified as a mammal, including humans, livestock, and laboratory animals, zoo animals, sport animals or pet animals, such as dogs, horses, cats, cattle, sheep, goats, pigs, mice, rats, rabbits, guinea pigs, monkeys, and the like.

### PREFERRED EMBODIMENTS OF THE PRESENT DISCLOSURE

By combining the accompanying drawings with the following detailed description of the embodiments, the features and advantages of the present disclosure, as well as additional features and advantages thereof, will be understood more clearly hereinafter. The embodiments described herein with reference to the accompanying drawings are interpretive, illustrative, and are used for a general understanding of the present disclosure. The embodiments should not be construed as limiting the scope of the present disclosure. The same drawing reference numerals are used throughout the description to denote the same or similar elements and elements having the same or similar functions.

In a first aspect, the present disclosure provides a multi-specific polypeptide molecule, wherein the multi-specific polypeptide molecule comprises a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises a heavy chain variable region VH1 and a light chain variable region VL1 derived from an antibody that binds the second antigen, or a single heavy chain variable region (VHH1) derived from a single-domain antibody or a nanobody that binds the second antigen;
wherein the first antigen is selected from the group consisting of tumor-associated antigens (TAA), tumor-specific antigens (TSA), viral antigens, and self-antigens, and the second antigen is an immune cell surface molecule;
wherein the N-terminus of the antibody is connected to the C-terminus of the TCR.

In some embodiments, the multi-specific polypeptide molecule further comprises at least one functional domain capable of prolonging the half-life of the multi-specific polypeptide molecule. In some embodiments, the functional domain capable of prolonging the half-life of the multi-specific polypeptide molecule is selected from the group consisting of:
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the multi-specific polypeptide molecule further comprises at least one of the following functional domains:
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the N-terminus of the antibody is connected to the C-terminus of the TCR.

In some embodiments, the N-terminus of the amino acid chain of the second binding region is connected to the C-terminus of the amino acid chain of the first binding region.

In some embodiments, the multi-specific polypeptide molecule provided by the present disclosure does not include that the C-terminus of the amino acid chain of the second binding region is connected to the N-terminus of the amino acid chain of the first binding region.

In some embodiments, the multi-specific polypeptide molecule provided by the present disclosure does not include that the C-terminus of any one polypeptide chain of the second binding region in a single-chain format (with VH1 and VL1 distributed on one polypeptide chain, or the antibody comprising VHH1) or a double-chain format (with VH1 and VL1 distributed on two polypeptide chains) is connected to the N-terminus of one polypeptide chain or a plurality of polypeptide chains of the first binding region.

In some embodiments, the multi-specific polypeptide molecule provided by the present disclosure does not include the following situations:
VL1 and VH1 are distributed on one polypeptide chain to form a single-chain of the second binding region or the formed single-chain of the second binding region comprises VHH1, TRAV1 and TRBV1 are distributed on one polypeptide chain to form a single-chain of the first binding region, and the C-terminus of the single-chain of the second binding region is connected to the N-terminus of the single-chain of the first binding region;
VL1 and VH1 are distributed on one polypeptide chain to form a single-chain of the second binding region or the formed single-chain of the second binding region comprises VHH1, TRAV and TRBV1 are distributed on two polypeptide chains to respectively form an α chain and a β chain, and the C-terminus of the single-chain of the second binding region is connected to the N-terminus of the α chain or the β chain;
VL1 and VH1 are distributed on different polypeptide chains to respectively form an L chain and an H chain, TRAV1 and TRBV1 are distributed on one polypeptide chain to form a single-chain on the first binding region, and the C-terminus of the L chain or the H chain is connected to the N-terminus of the single-chain of the first binding region;
VL1 and VH1 are distributed on different polypeptide chains to respectively form an L chain and an H chain, TRAV1 and TRBV1 are distributed on two polypeptide chains to respectively form an α chain and a β chain, and the C-terminus of the L chain or the H chain is connected to the N-terminus of the α chain or the β chain.

In some embodiments, the multi-specific polypeptide molecule provided by the present disclosure does not include the following situations:
From N-terminus to C-terminus: scFv1-TRBV1, scFv1-TRAV1, scFv1-scTCR, VHH1-TRBV1, VHH1-TRAV1, VHH1-scTCR, VH1-TRBV1, VH1-TRAV1, VH1-scTCR, VL1-TRBV1, VL1-TRAV1, VL1-scTCR, Fab1-TRBV1, Fab1-TRAV1, Fab1-scTCR.

In some embodiments, VL1 and VH1 are distributed on one polypeptide chain, and the C-terminus of the polypeptide chain is connected to the N-terminus of the TCR.

In some embodiments, VL1 and VH1 are distributed on different polypeptide chains, and the C-terminus of the polypeptide chain on which VL1 or VH1 is located is connected to the N-terminus of the TCR.

In some embodiments, TRAV1 and TRBV1 are distributed on two different polypeptide chains (i.e., the TCR is a heterodimeric αβ TCR).

In some embodiments, VL1 and VH1 are distributed on one polypeptide chain, or VL1 and VH1 are distributed on two different polypeptide chains, and TRAV1 and TRBV1 are distributed on two different polypeptide chains.

In some embodiments, the multi-specific polypeptide molecule comprises two polypeptide chains.

In some embodiments, the multi-specific polypeptide molecule comprises two polypeptide chains, TRAV1 and TRBV1 are distributed on two different polypeptide chains, VHH1, or VH1 and VL1 are distributed on one polypeptide chain (e.g., forming scFv1), and the N-terminus thereof is connected to the C-terminus of the polypeptide chain on which TRAV1 or TRBV1 is located, or the N-terminus of the polypeptide chain on which one of VH1 and VL1 is located is connected to the C-terminus of the polypeptide chain on which TRAV1 is located, and the N-terminus of the polypeptide chain on which the other of VH1 and VL1 is located is connected to the C-terminus of the polypeptide chain on which TRBV1 is located.

In some embodiments, the multi-specific polypeptide molecule comprises three polypeptide chains.

In some embodiments, the multi-specific polypeptide molecule comprises three polypeptide chains, and VH1 and VL1 are distributed on different polypeptide chains, TRAV1 and TRBV1 are distributed on different polypeptide chains, and the N-terminus of the polypeptide chain on which VH1 or VL1 is located is connected to the C-terminus of the polypeptide chain on which TRBV1 or TRAV1 is located.

In a second aspect, the present disclosure provides a multi-specific polypeptide molecule, wherein the multi-specific polypeptide molecule comprises, on two polypeptide chains, a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises a heavy chain variable region VH1 and a light chain variable region VL1 derived from an antibody that binds the second antigen, or a single heavy chain variable region (VHH1) derived from a single-domain antibody or a nanobody that binds the second antigen;
wherein the first antigen is selected from the group consisting of tumor-associated antigens (TAA), tumor-specific antigens (TSA), viral antigens, and self-antigens, and the second antigen is an immune cell surface molecule;
wherein the N-terminus of the antibody is connected to the C-terminus of the TCR.

In some embodiments, the multi-specific polypeptide molecule further comprises at least one functional domain capable of prolonging the half-life of the multi-specific polypeptide molecule. In some embodiments, the functional domain capable of prolonging the half-life of the multi-specific polypeptide molecule is selected from the group consisting of:
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the multi-specific polypeptide molecule further comprises at least one of the following functional domains:
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the N-terminus of the antibody is connected to the C-terminus of the TCR.

In some embodiments, TRAV1 and TRBV1 are distributed on two different polypeptide chains (i.e., the TCR is a heterodimeric αβ TCR).

In some embodiments, VH1 and VL1 are distributed on one polypeptide chain, e.g., to form scFv1 or Fab1, and are connected to the C-terminus of the TCR.

In some embodiments, TRAV1 and TRBV1 are distributed on two different polypeptide chains, and VH1 and VL1 are distributed on one polypeptide chain, and the N-terminus of the polypeptide chain on which VH1 and VL1 are located is connected to the C-terminus of the polypeptide chain on which TRAV1 is located, or to the C-terminus of the polypeptide chain on which TRBV1 is located.

In some embodiments, VH1 and VL1 are linked via a linker.

In the embodiments of the multi-specific polypeptide molecule according to the second aspect of the present disclosure, the VH1 and VL1 form scFv1, and the N-terminus of scFv1 is connected to the C-terminus of the polypeptide chain comprising TRAV1 or the polypeptide chain comprising TRBV1. In some embodiments, the two polypeptide chains of the multi-specific polypeptide molecule respectively comprise: TRBV1-scFv1 and TRAV1; or TRAV1-scFv1 and TRBV1.

In some embodiments, the two polypeptide chains of the multi-specific polypeptide molecule respectively comprise: TRBV1-VL1-VH1 and TRAV1; or TRBV1-VH1-VL1 and TRAV1; or TRAV1-VL1-VH1 and TRBV1; or TRAV1-VH1-VL1 and TRBV1.

In some embodiments, the second binding region comprises a single heavy chain variable region (VHH1) derived from a single-domain antibody or a nanobody that binds the second antigen, and the VHH1 is connected to the C-terminus of the TCR. In some embodiments, the TRAV1 and TRBV1 aredistributed on two different polypeptide chains, and the second binding region comprises VHH1, wherein the N-terminus of the VHH1 is connected to the C-terminus of the polypeptide chain comprising TRAV1 or TRBV1. In some embodiments, the two polypeptide chains of the multi-specific polypeptide molecule respectively comprise: TRBV1-VHH1 and TRAV1; or TRAV1-VHH1 and TRBV1.

In some embodiments, the second binding region comprises VH1 and VL1, and the VH1 and VL1 are distributed on two different polypeptide chains. In some embodiments, the TRAV1 and TRBV1 are distributed on two different polypeptide chains, and the VH1 and VL1 are distributed on two different polypeptide chains, wherein a polypeptide chain on which one of the VH1 and VL1 is located is connected to the C-terminus of the polypeptide chain on which TRAV1 is located, and a polypeptide chain on which the other is located is connected to the C-terminus of the polypeptide chain on which TRBV1 is located.

In some embodiments, the two polypeptide chains of the multi-specific polypeptide molecule respectively comprise: TRBV1-VL1 and TRAV1-VH1; or TRAV1-VL1 and TRBV1-VH1.

In some embodiments, the TRAV1 and TRBV1 are distributed on two different polypeptide chains, and the VHH1, or the polypeptide chain where VH1 and VL1 are located (e.g., forming scFv1) is connected to the C-terminus of the TRAV1 or the TRBV1, or one of the VH1 and VL1 is connected to the C-terminus of the TRAV1 and the other is connected to the C-terminus of the TRBV1.

In some embodiments, the multi-specific polypeptide molecule further comprises a constant domain connected to the C-terminus of TRAV1 and/or TRBV1 via an optional linker, wherein the constant domain is selected from the group consisting the following (1)-(3),
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably, the CH3 in either one polypeptide chain or both polypeptide chains comprises at least one mutation capable of promoting the polypeptide molecule to form a heterodimer;
(3) CL and CH1, wherein the CL is derived from a CL domain of an antibody light chain, and the CH1 is derived from a CH1 domain of an antibody heavy chain.

In the embodiments of the multi-specific polypeptide molecule according to the second aspect of the present disclosure, the multi-specific polypeptide molecule further comprises a third binding region that binds a third antigen, wherein the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens and self-antigens, and the third antigen is the same as or different from the first antigen. In some embodiments, the TRAV2 is the same as the TRAV1. In some embodiments, the TRBV2 is the same as the TRBV1.

In some embodiments, the TRAV2 and the TRBV2 are distributed on one polypeptide chain via a linker.

In some embodiments, the VH1 and VL1 are distributed on one polypeptide chain and connected to the C-terminus of the TRAV1 or the TRBV1, and the TRAV2 and the TRBV2 are connected on the same polypeptide chain via a linker and are distributed between VH1 and VL1.

In some embodiments, the two polypeptide chains of the multi-specific polypeptide molecule respectively comprise:
TRBV1-VL1-TRAV2-TRBV2-VH1 and TRAV1; or
TRBV1-VL1-TRBV2-TRAV2-VH1 and TRAV1; or
TRBV1-VH1-TRBV2- TRAV2-VL1 and TRAV1; or
TRBV1-VH1-TRAV2- TRBV2-VL1 and TRAV1; or
TRAV1-VL1-TRBV2- TRAV2-VH1 and TRBV1; or
TRAV1-VL1-TRAV2- TRBV2-VH1 and TRBV1; or
TRAV1-VH1-TRAV2- TRBV2-VL1 and TRBV1; or
TRAV1-VH1-TRBV2- TRAV2-VL1 and TRBV1.

In some embodiments, the multi-specific polypeptide molecule further comprises a constant domain connected to the C-terminus of TRAV2 and/or TRBV2 via an optional linker, wherein the constant domain is selected from the group consisting of the following (1)-(3),
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably, the CH3 in either one polypeptide chain or both polypeptide chains comprises at least one mutation capable of promoting the polypeptide molecule to form a heterodimer;
(3) CL and CH1, wherein the CL is derived from a CL domain of an antibody light chain, and the CH1 is derived from a CH1 domain of an antibody heavy chain.

In the embodiments of the multi-specific polypeptide molecule according to the second aspect of the present disclosure, the multi-specific polypeptide molecule further comprises a fourth binding region that binds a fourth antigen, wherein the fourth binding region comprises a heavy chain variable region VH2 and a light chain variable region VL2 derived from an antibody that binds the fourth antigen, or a single heavy chain variable region (VHH2) derived from a single-domain antibody or a nanobody that binds the fourth antigen; wherein the fourth antigen is an immune cell surface molecule, and the fourth antigen is the same as or different from the second antigen. In some embodiments, the VH2 is the same as the VH1. In some embodiments, the VL2 is the same as the VL1. In some embodiments, the VHH2 is the same as the VHH1.

In some embodiments, the VHH2, or scFv2 comprising the VH2 and VL2 is connected to the N-terminus of the TRAV1 or the TRBV1, or one of the VH2 and VL2 is connected to the N-terminus of the TRAV1 and the other is connected to the N-terminus of the TRBV1.

In some embodiments, the multi-specific polypeptide molecule comprises the first binding region, the second binding region and the fourth binding region, and the VH2 and VL2 form scFv2 which is connected to the N-terminus of the TRAV1 or the TRBV1. In some embodiments, the VH2 and VL2 are linked via a linker to form scFv2.

In some embodiments, the two polypeptide chains of the multi-specific polypeptide molecule respectively comprise:
scFv2-TRBV1-scFv1 and TRAV1; or
scFv2-TRAV1-scFv1 and TRBV1; or
scFv2-TRBV1-VHH1 and TRAV1; or
scFv2-TRAV1-VHH1 and TRBV1; or
scFv2-TRBV1-VL1 and TRAV1-VH1; or
scFv2-TRAV1-VL1 and TRBV1-VH1; or
TRBV1-scFv1 and scFv2-TRAV1; or
TRAV1-scFv1 and scFv2-TRBV1; or
TRBV1-VHH1 and scFv2-TRAV1; or
TRAV1-VHH1 and scFv2-TRBV1; or
TRBV1-VL1 and scFv2-TRAV1-VH1; or
TRAV1-VL1 and scFv2-TRBV1-VH1.

In some embodiments, the multi-specific polypeptide molecule comprises the first binding region, the second binding region and the fourth binding region, and the VHH2 is connected to the N-terminus of the TRAV1 or the TRBV1. In some embodiments, the two polypeptide chains of the multi-specific polypeptide molecule respectively comprise:
VHH2-TRBV1-scFv1 and TRAV1; or
VHH2-TRAV1-scFv1 and TRBV1; or
VHH2-TRBV1-VHH1 and TRAV1; or
VHH2-TRAV1-VHH1 and TRBV1; or
VHH2-TRBV1-VL1 and TRAV1-VH1; or
VHH2-TRAV1-VL1 and TRBV1-VH1; or
TRBV1-scFv1 and VHH2-TRAV1; or
TRAV1-scFv1 and VHH2-TRBV1; or
TRBV1-VHH and VHH2-TRAV1; or
TRAV1-VHH and VHH2-TRBV1; or
TRBV1-VL1 and VHH2-TRAV1-VH1; or
TRAV1-VL1 and VHH2-TRBV1-VH1.

In some embodiments, the multi-specific polypeptide molecule comprises the first binding region, the second binding region, and the fourth binding region, and one of the VH2 and VL2 is connected to the N-terminus of the TRAV1, and the other is connected to the N-terminus of the TRBV1. In some embodiments, the two polypeptide chains of the multi-specific polypeptide molecule respectively comprise:
VL2-TRBV1-scFv1 and VH2-TRAV1; or
VH2-TRBV1-scFv1 and VL2-TRAV1; or
VL2-TRAV1-scFv1 and VH2-TRBV1; or
VH2-TRAV1-scFv1 and VL2-TRBV1; or
VL2-TRBV1-VHH and VH2-TRAV1; or
VH2-TRBV1-VHH and VL2-TRAV1; or
VL2-TRAV1-VHH and VH2-TRBV1; or
VH2-TRAV1-VHH and VL2-TRBV1; or
VL2-TRBV1-VL1 and VH2-TRAV1-VH1; or
VH2-TRBV1-VL1 and VL2-TRAV1-VH1; or
VL2-TRAV1-VL1 and VH2-TRBV1-VH1; or
VH2-TRAV1-VL1 and VL2-TRBV1-VH1.

In some embodiments, the multi-specific polypeptide molecule comprises the first binding region, the second binding region, the third binding region, and the fourth binding region, and the VH2 and VL2 form scFv2, which is connected to the N-terminus of the TRAV1 or the TRBV1. In some embodiments, the VH2 and VL2 are linked via a linker to form scFv2. In some embodiments, the two polypeptide chains of the multi-specific polypeptide molecule respectively comprise:
scFv2-TRBV1-VL1-TRAV2-TRBV2-VH1 and TRAV1; or
scFv2-TRBV1-VL1-TRBV2-TRAV2-VH1 and TRAV1; or
scFv2-TRBV1-VH1-TRBV2- TRAV2-VL1 and TRAV1; or
scFv2-TRBV1-VH1-TRAV2- TRBV2-VL1 and TRAV1; or
scFv2-TRAV1-VL1-TRBV2- TRAV2-VH1 and TRBV1; or
scFv2-TRAV1-VL1-TRAV2- TRBV2-VH1 and TRBV1; or
scFv2-TRAV1-VH1-TRAV2-TRBV2-VL1 and TRBV1; or
scFv2-TRAV1-VH1-TRBV2-TRAV2-VL1 and TRBV1; or
TRBV1-VL1-TRAV2-TRBV2-VH1 and scFv2-TRAV1; or
TRBV1-VL1-TRBV2-TRAV2-VH1 and scFv2-TRAV1; or
TRBV1-VH1-TRBV2- TRAV2-VL1 and scFv2-TRAV1; or
TRBV1-VH1-TRAV2- TRBV2-VL1 and scFv2-TRAV1; or
TRAV1-VL1-TRBV2- TRAV2-VH1 and scFv2-TRBV1; or
TRAV1-VL1-TRAV2- TRBV2-VH1 and scFv2-TRBV1; or
TRAV1-VH1-TRAV2-TRBV2-VL1 and scFv2-TRBV1; or
TRAV1-VH1-TRBV2-TRAV2-VL1 and scFv2-TRBV1.

In some embodiments, the multi-specific polypeptide molecule comprises the first binding region, the second binding region, the third binding region, and the fourth binding region, and the VHH2 is connected to the N-terminus of the TRAV1 or the TRBV1. In some embodiments, the two polypeptide chains of the multi-specific polypeptide molecule respectively comprise:
VHH2-TRBV1-VL1-TRAV2-TRBV2-VH1 and TRAV1; or
VHH2-TRBV1-VL1-TRBV2-TRAV2-VH1 and TRAV1; or
VHH2-TRBV1-VH1-TRBV2- TRAV2-VL1 and TRAV1; or
VHH2-TRBV1-VH1-TRAV2- TRBV2-VL1 and TRAV1; or
VHH2-TRAV1-VL1-TRBV2- TRAV2-VH1 and TRBV1; or
VHH2-TRAV1-VL1-TRAV2- TRBV2-VH1 and TRBV1; or
VHH2-TRAV1-VH1-TRAV2-TRBV2-VL1 and TRBV1; or
VHH2-TRAV1-VH1-TRBV2-TRAV2-VL1 and TRBV1; or
TRBV1-VL1-TRAV2-TRBV2-VH1 and VHH2-TRAV1; or
TRBV1-VL1-TRBV2-TRAV2-VH1 and VHH2-TRAV1; or
TRBV1-VH1-TRBV2- TRAV2-VL1 and VHH2-TRAV1; or
TRBV1-VH1-TRAV2- TRBV2-VL1 and VHH2-TRAV1; or
TRAV1-VL1-TRBV2- TRAV2-VH1 and VHH2-TRBV1; or
TRAV1-VL1-TRAV2- TRBV2-VH1 and VHH2-TRBV1; or
TRAV1-VH1-TRAV2-TRBV2-VL1 and VHH2-TRBV1; or
TRAV1-VH1-TRBV2-TRAV2-VL1 and VHH2-TRBV1.

In some embodiments, the multi-specific polypeptide molecule comprises the first binding region, the second binding region, the third binding region, and the fourth binding region, and one of the VH2 and VL2 is connected to the N-terminus of the TRAV1, and the other is connected to the N-terminus of the TRBV1. In some embodiments, the two polypeptide chains of the multi-specific polypeptide molecule respectively comprise:
VL2-TRBV1-VL1-TRAV2-TRBV2-VH1 and VH2-TRAV1; or
VL2-TRBV1-VL1-TRBV2-TRAV2-VH1 and VH2-TRAV1; or
VH2-TRBV1-VL1-TRAV2-TRBV2-VH1 and VL2-TRAV1; or
VH2-TRBV1-VL1-TRBV2-TRAV2-VH1 and VL2-TRAV1; or
VL2-TRBV1-VH1-TRBV2- TRAV2-VL1 and VH2-TRAV1; or
VL2-TRBV1-VH1-TRAV2- TRBV2-VL1 and VH2-TRAV1; or
VH2-TRBV1-VH1-TRBV2- TRAV2-VL1 and VL2-TRAV1; or
VH2-TRBV1-VH1-TRAV2- TRBV2-VL1 and VL2-TRAV1; or
VL2-TRAV1-VL1-TRBV2- TRAV2-VH1 and VH2-TRBV1; or
VL2-TRAV1-VL1-TRAV2- TRBV2-VH1 and VH2-TRBV1; or
VH2-TRAV1-VL1-TRBV2- TRAV2-VH1 and VL2-TRBV1; or
VH2-TRAV1-VL1-TRAV2- TRBV2-VH1 and VL2-TRBV1; or
VL2-TRAV1-VH1-TRAV2-TRBV2-VL1 and VH2-TRBV1; or
VL2-TRAV1-VH1-TRBV2-TRAV2-VL1 and VH2-TRBV1; or
VH2-TRAV1-VH1-TRAV2-TRBV2-VL1 and VL2-TRBV1; or
VH2-TRAV1-VH1-TRBV2-TRAV2-VL1 and VL2-TRBV1.

In the embodiments of the multi-specific polypeptide molecule of the second aspect of the present disclosure, the functional domain is derived from albumin or a binding domain thereof and an optional antibody hinge region; and the functional domain is connected to the C-terminus or N-terminus of any one polypeptide chain of the multi-specific polypeptide molecule, preferably the C-terminus, via an optional linker.

In some embodiments, the functional domain is derived from albumin or a binding domain thereof, and the albumin or a binding domain thereof is connected to the C-terminus of any one polypeptide chain of the multi-specific polypeptide molecule via a linker.

In some embodiments, the functional domain is derived from an albumin or a binding domain thereof and an antibody hinge region, the functional domain is connected to the C-terminus of any one polypeptide chain of the multi-specific polypeptide molecule via an optional linker, and the albumin or a binding domain thereof is connected to the C-terminus of any one chain of the antibody hinge region via an optional linker.

In some embodiments, the functional domain comprises two chains, and the two chains of the multi-specific polypeptide molecule are connected via covalent bonds or non-covalent bonds between the two chains of the functional domain.

In some embodiments, the functional domain comprises two chains, and the first binding region and the second binding region are connected via covalent bonds or non-covalent bonds between the two chains of the functional domain.

In some embodiments, the functional domain comprises two chains, which are respectively connected to the C-terminus or N-terminus of the two polypeptide chains of the multi-specific polypeptide molecule, preferably the C-terminus.

In some embodiments, one chain of the functional domain is connected to the C-terminus or N-terminus of the TCR, preferably the C-terminus, via an optional linker, and the other chain is connected to the C-terminus or N-terminus of the antibody, preferably the C-terminus.

In some embodiments, one chain of the functional domain is connected to the C-terminus or N-terminus of the chain on which TRAV1 or TRBV1 is located, preferably the C-terminus, and the other chain is connected to the C-terminus or N-terminus of the chain on which the antibody is located, preferably the C-terminus.

In some embodiments, TRAV1 and TRBV1 are located on two different polypeptide chains, one chain of the functional domain is connected to the C-terminus or N-terminus of the chain on which TRAV1 or TRBV1 is located, preferably the C-terminus, and the other chain is connected to the C-terminus or N-terminus of the chain on which the antibody is located, preferably the C-terminus.

In some embodiments, TRAV1 and TRBV1 are located on two different polypeptide chains, the second binding region comprises VHH1, or comprises VH1 and VL1 and the VH1 and VL1 are distributed on one polypeptide chain, one chain of the functional domain is connected to the C-terminus or N-terminus of the chain on which TRAV1 or TRBV1 is located, preferably the C-terminus, and the other chain is connected to the C-terminus or N-terminus of the chain on which the antibody is located, preferably the C-terminus.

In some embodiments, TRAV1 and TRBV1 are located on two different polypeptide chains, and one of VH1 and VL1 is connected to the C-terminus of the chain on which the TRAV1 is located, and the other is connected to the C-terminus of the chain on which the TRBV1 is located, wherein the two chains of the functional domain are respectively connected to the C-terminus or N-terminus of the two polypeptide chains of the multi-specific polypeptide molecule, preferably the C-terminus.

In some embodiments, TRAV1 and TRBV1 are distributed on two different polypeptide chains, VH1 and VL1 are distributed on one polypeptide chain and the N-terminus thereof is connected to the C-terminus of the polypeptide chain on which TRAV1 or TRBV1 is located, the C-terminus thereof is connected to one chain of the functional domain, and the other chain of the functional domain is connected to the C-terminus of the polypeptide chain on which TRBV1 or TRAV1 is located.

In some embodiments, TRAV1 and TRBV1 are distributed on two different polypeptide chains, VH1 and VL1 are distributed on one polypeptide chain which is connected to the C-terminus of the polypeptide chain on which TRAV1 or TRBV1 is located, one chain of the functional domain is connected to the N-terminus of the chain on which TRAV1 is located, and the other chain is connected to the N-terminus of the chain on which TRBV1 is located.

In embodiments of the multi-specific polypeptide molecule of the second aspect of the present disclosure, the functional domain is derived from an antibody hinge region, and/or an antibody Fc domain or a dimerization portion thereof, and/or an antibody CH3 domain; and the functional domain is connected to the C-terminus or N-terminus of the two polypeptide chains of the multi-specific polypeptide molecule, preferably the C-terminus, via an optional linker. In some embodiments, the functional domain is derived from an antibody hinge region, and/or an antibody Fc domain or a dimerization portion thereof, and/or an antibody CH3 domain; and the two chains of the functional domain are respectively connected to the C-terminus or N-terminus of the two polypeptide chains of the multi-specific polypeptide molecule, preferably the C-terminus, via an optional linker.

In some embodiments, the functional domain is derived from an antibody hinge region, and/or an antibody Fc domain or a dimerization portion thereof, and/or an antibody CH3 domain, and the two chains of the multi-specific polypeptide molecule are connected via covalent bonds or non-covalent bonds between the two chains of the functional domain.

In some embodiments, the functional domain is derived from an antibody Fc domain or a dimerization portion thereof and an optional antibody hinge region. In some embodiments, the functional domain is derived from an antibody hinge region and an antibody Fc domain or a dimerization portion thereof. In some embodiments, the functional domain is derived from an antibody Fc domain. In some embodiments, the functional domain is an antibody Fc domain. In some embodiments, the functional domain is respectively connected to the C-terminus or N-terminus of the two polypeptide chains of the multi-specific polypeptide molecule, preferably the C-terminus, via an optional linker.

In some embodiments, the functional domain is derived from an antibody CH3 domain and an optional antibody hinge region. In some embodiments, the functional domain is derived from an antibody hinge region and an antibody CH3 domain. In some embodiments, the functional domain is respectively connected to the C-terminus or N-terminus of the two polypeptide chains of the multi-specific polypeptide molecule, preferably the C-terminus, via an optional linker.

In some embodiments, the functional domain is derived from a constant region of a TCR or a fragment thereof and an optional antibody hinge region. In some embodiments, the functional domain is derived from an antibody hinge region and a constant region of a TCR or a fragment thereof. In some embodiments, the functional domain is respectively connected to the C-terminus or N-terminus of the two polypeptide chains of the multi-specific polypeptide molecule, preferably the C-terminus, via an optional linker. In some embodiments, the constant region or fragment thereof comprises a TCR α chain constant region (TRAC) and a TCR β chain constant region (TRBC), and the TRAC and TRBC are respectively connected to the C-terminus or N-terminus of the two polypeptide chains of the multi-specific polypeptide molecule, preferably the C-terminus, via an optional linker.

In embodiments of the multi-specific polypeptide molecule of the second aspect of the present disclosure, the multi-specific polypeptide molecule further comprises a constant domain connected to the C-terminus of TRAV1 and/or TRBV1 via an optional linker.

In one embodiment, the constant domain is a TCR constant region or a fragment thereof, and the TCR constant region, e.g., comprises a TCR α chain constant region (TRAC) and/or a β chain constant region (TRBC).

In one embodiment, the constant domain is CH3, the CH3 is derived from an antibody Fc domain, preferably the CH3 in any one polypeptide chain or in both polypeptide chains comprises at least one mutation capable of promoting the polypeptide molecule to form a heterodimer.

In one embodiment, the constant domain is CL and CH1, the CL is derived from an antibody light chain CL domain, and the CH1 is derived from an antibody heavy chain CH1 domain.

In some embodiments, the multi-specific polypeptide molecule comprises a TCR constant region or a fragment thereof connected to the C-terminus of TRAV1 and TRBV1 via an optional linker. In some embodiments, the multi-specific polypeptide molecule comprises TRAC and TRBC respectively connected to the C-terminus of TRAV1 and TRBV1, via an optional linker.

In some embodiments, the multi-specific polypeptide molecule comprises a first polypeptide chain and a second polypeptide chain, and comprises a first binding region, a second binding region, and a functional domain comprising an albumin or a binding domain thereof and an optional antibody hinge region that is connected to the C-terminus of any one polypeptide chain; wherein:
the first polypeptide chain comprises, from N-terminus to C-terminus: TRBV1- optional linker -TRBC- linker -scFv1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: TRBV1- optional linker -TRBC- linker -scFv1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: TRAV1- optional linker -TRAC- linker -scFv1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: TRAV1- optional linker -TRAC- linker -scFv1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: TRBV1- optional linker -TRBC- linker -VHH1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: TRBV1- optional linker -TRBC- linker -VHH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: TRAV1- optional linker -TRAC- linker -VHH1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: TRAV1- optional linker -TRAC- linker -VHH1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: TRBV1- optional linker -TRBC- linker -VL1- optional linker - optional hinge region -Alb, and the second polypeptide chain comprises, from N-terminus to C-terminus: TRAV1- optional linker -TRAC- linker -VH1- optional linker - optional hinge region; or
the first polypeptide chain comprises, from N-terminus to C-terminus: TRBV1- optional linker -TRBC- linker -VL1- optional linker - optional hinge region, and the second polypeptide chain comprises, from N-terminus to C-terminus: TRAV1- optional linker -TRAC- linker -VH1- optional linker - optional hinge region -Alb; or
the first polypeptide chain comprises, from N-terminus to C-terminus: TRAV1- optional linker -TRAC- linker -VL1- optional linker - optional hinge region -Alb, and the second polypeptide chain comprises, from N-terminus to C-terminus: TRBV1- optional linker -TRBC- linker -VH1- optional linker - optional hinge region; or
the first polypeptide chain comprises, from N-terminus to C-terminus: TRAV1- optional linker -TRAC- linker -VL1- optional linker - optional hinge region, and the second polypeptide chain comprises, from N-terminus to C-terminus: TRBV1- optional linker -TRBC- linker -VH1- optional linker - optional hinge region - Alb; or
the first polypeptide chain comprises, from N-terminus to C-terminus: TRBV1- optional linker -TRBC- linker -VL1- linker -VH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: TRBV1- optional linker -TRBC- linker -VH1- linker -VL1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: TRBV1- optional linker -TRBC- linker -VL1- linker -VH1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: TRBV1- optional linker -TRBC- linker -VH1- linker -VL1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: TRAV1- optional linker -TRAC- linker -VL1- linker -VH1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: TRAV1- optional linker -TRAC- linker -VH1- linker -VL1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: TRAV1- optional linker -TRAC- linker -VL1- linker -VH1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: TRAV1- optional linker -TRAC- linker -VH1- linker -VL1- optional linker - optional hinge region -Alb, and the second polypeptide chain comprises, from N-terminus to C-terminus: TRBV1- optional linker -TRBC- optional linker - optional hinge region.

In some embodiments, the multi-specific polypeptide molecule comprises a first polypeptide chain and a second polypeptide chain, and comprises a first binding region and a second binding region, and a functional domain comprising an antibody-derived Fc domain or a dimerizing portion thereof and an optional antibody hinge region that is respectively connected to the C-terminus of the first polypeptide chain and the second polypeptide chain; wherein:
the first polypeptide chain comprises, from N-terminus to C-terminus: TRBV1- optional linker -TRBC- linker -scFv1- optional linker - optional hinge region -CH2-CH3, and the second polypeptide chain comprises, from N-terminus to C-terminus: TRAV1- optional linker -TRAC- optional linker - optional hinge region -CH2-CH3; or
the first polypeptide chain comprises, from N-terminus to C-terminus: TRAV1- optional linker -TRAC- linker -scFv1- optional linker - optional hinge region -CH2-CH3, and the second polypeptide chain comprises, from N-terminus to C-terminus: TRBV1- optional linker -TRBC- optional linker - optional hinge region -CH2-CH3; or
the first polypeptide chain comprises, from N-terminus to C-terminus: TRBV1- optional linker -TRBC- linker -VHH1- optional linker - optional hinge region -CH2-CH3, and the second polypeptide chain comprises, from N-terminus to C-terminus: TRAV1- optional linker -TRAC- optional linker - optional hinge region -CH2-CH3; or
the first polypeptide chain comprises, from N-terminus to C-terminus: TRAV1- optional linker -TRAC- linker -VHH1- optional linker - optional hinge region -CH2-CH3, and the second polypeptide chain comprises, from N-terminus to C-terminus: TRBV1- optional linker -TRBC- optional linker - optional hinge region -CH2-CH3; or
the first polypeptide chain comprises, from N-terminus to C-terminus: TRBV1- optional linker -TRBC- linker -VL1- optional linker - optional hinge region -CH2-CH3, and the second polypeptide chain comprises, from N-terminus to C-terminus: TRAV1- optional linker -TRAC- linker -VH1- optional linker - optional hinge region -CH2-CH3; or
the first polypeptide chain comprises, from N-terminus to C-terminus: TRAV1- optional linker -TRAC- linker -VL1- optional linker - optional hinge region -CH2-CH3, and the second polypeptide chain comprises, from N-terminus to C-terminus: TRBV1- optional linker -TRBC- linker -VH1- optional linker - optional hinge region -CH2-CH3; or
the first polypeptide chain comprises, from N-terminus to C-terminus: TRAV1- optional linker -TRAC- linker -VL1- linker -VH1- optional linker - optional hinge region -CH2-CH3, and the second polypeptide chain comprises, from N-terminus to C-terminus: TRBV1- optional linker -TRBC- optional linker - optional hinge region -CH2-CH3; or
the first polypeptide chain comprises, from N-terminus to C-terminus: TRAV1- optional linker -TRAC- linker -VH1- linker -VL1- optional linker - optional hinge region -CH2-CH3, and the second polypeptide chain comprises, from N-terminus to C-terminus: TRBV1- optional linker -TRBC- optional linker - optional hinge region -CH2-CH3; or
the first polypeptide chain comprises, from N-terminus to C-terminus: TRBV1- optional linker -TRBC- linker -VL1- linker -VH1- optional linker - optional hinge region -CH2-CH3, and the second polypeptide chain comprises, from N-terminus to C-terminus: TRAV1- optional linker -TRAC- optional linker - optional hinge region -CH2-CH3; or
the first polypeptide chain comprises, from N-terminus to C-terminus: TRBV1- optional linker -TRBC- linker -VH1- linker -VL1- optional linker - optional hinge region -CH2-CH3, and the second polypeptide chain comprises, from N-terminus to C-terminus: TRAV1- optional linker -TRAC- optional linker - optional hinge region -CH2-CH3.

In some embodiments, the multi-specific polypeptide molecule comprises a first polypeptide chain and a second polypeptide chain, and comprises a first binding region, a second binding region and a third binding region, and a functional domain comprising an from albumin or a binding domain thereof and an optional antibody hinge region that is connected to the C-terminus of any chain of the polypeptides, and the VH1 and VL1 distributed on one polypeptide chain are connected to the C-terminus of the TRAV1 or the TRBV1, via a linker, and the TRAV2 and the TRBV2 are distributed on the same polypeptide chain and are located between VH1 and VL1; wherein:
the first polypeptide chain comprises, from N-terminus to C-terminus: TRBV1- optional linker -TRBC- linker -VL1- linker -TRAV2- linker -TRBV2- linker -VH1- optional linker - optional hinge region -Alb, and the second polypeptide chain comprises, from N-terminus to C-terminus: TRAV1- optional linker -TRAC-optional linker - optional hinge region; or
the first polypeptide chain comprises, from N-terminus to C-terminus: TRBV1- optional linker -TRBC- linker -VL1- linker -TRAV2- linker -TRBV2- linker -VH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: TRBV1- optional linker -TRBC- linker -VL1- linker -TRBV2- linker -TRAV2- linker -VH1- optional linker - optional hinge region -Alb, and the second polypeptide chain comprises, from N-terminus to C-terminus: TRAV1- optional linker -TRAC-optional linker - optional hinge region; or
the first polypeptide chain comprises, from N-terminus to C-terminus: TRBV1- optional linker -TRBC- linker -VL1- linker -TRBV2- linker -TRAV2- linker -VH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: TRBV1- optional linker -TRBC- linker -VH1- linker -TRBV2- linker - TRAV2- linker -VL1- optional linker - optional hinge region -Alb, and the second polypeptide chain comprises, from N-terminus to C-terminus: TRAV1- optional linker -TRAC-optional linker - optional hinge region; or
the first polypeptide chain comprises, from N-terminus to C-terminus: TRBV1- optional linker -TRBC- linker -VH1- linker -TRBV2- linker - TRAV2- linker -VL1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: TRBV1- optional linker -TRBC- linker -VH1- linker -TRAV2- linker - TRBV2- linker -VL1- optional linker - optional hinge region -Alb, and the second polypeptide chain comprises, from N-terminus to C-terminus: TRAV1- optional linker -TRAC-optional linker - optional hinge region; or
the first polypeptide chain comprises, from N-terminus to C-terminus: TRBV1- optional linker -TRBC- linker -VH1- linker -TRAV2- linker - TRBV2- linker -VL1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: TRAV1- optional linker -TRAC- linker -VL1- linker -TRBV2- linker - TRAV2- linker -VH1- optional linker - optional hinge region -Alb, and the second polypeptide chain comprises, from N-terminus to C-terminus: TRBV1- optional linker -TRBC-optional linker - optional hinge region; or
the first polypeptide chain comprises, from N-terminus to C-terminus: TRAV1- optional linker -TRAC- linker -VL1- linker -TRBV2- linker - TRAV2- linker -VH1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: TRAV1- optional linker -TRAC- linker -VL1- linker -TRAV2- linker - TRBV2- linker -VH1- optional linker - optional hinge region -Alb, and the second polypeptide chain comprises, from N-terminus to C-terminus: TRBV1- optional linker -TRBC-optional linker - optional hinge region; or
the first polypeptide chain comprises, from N-terminus to C-terminus: TRAV1- optional linker -TRAC- linker -VL1- linker -TRAV2- linker - TRBV2- linker -VH1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: TRAV1- optional linker -TRAC- linker -VH1- linker -TRAV2- linker - TRBV2- linker -VL1- optional linker - optional hinge region -Alb, and the second polypeptide chain comprises, from N-terminus to C-terminus: TRBV1- optional linker -TRBC-optional linker - optional hinge region; or
the first polypeptide chain comprises, from N-terminus to C-terminus: TRAV1- optional linker -TRAC- linker -VH1- linker -TRAV2- linker - TRBV2- linker -VL1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: TRAV1- optional linker -TRAC- linker -VH1- linker -TRBV2- linker - TRAV2- linker -VL1- optional linker - optional hinge region -Alb, and the second polypeptide chain comprises, from N-terminus to C-terminus: TRBV1- optional linker -TRBC-optional linker - optional hinge region; or
the first polypeptide chain comprises, from N-terminus to C-terminus: TRAV1- optional linker -TRAC- linker -VH1- linker -TRBV2- linker - TRAV2- linker -VL1- optional linker - optional hinge region, and the second polypeptide chain comprises, from N-terminus to C-terminus: TRBV1- optional linker -TRBC- optional linker - optional hinge region -Alb.

In some embodiments, the multi-specific polypeptide molecule comprises the first binding region, the second binding region, and the third binding region, as well as a functional domain comprising an antibody Fc domain or a dimerization portion thereof and optionally an antibody hinge region, and the multi-specific polypeptide molecule comprises a first polypeptide chain and a second polypeptide chain; wherein
the first polypeptide chain comprises, from N-terminus to C-terminus: TRBV1- optional linker -TRBC- linker -VL1- linker -TRAV2- linker -TRBV2- linker -VH1- optional linker - optional hinge region -CH2-CH3, and the second polypeptide chain comprises, from N-terminus to C-terminus: TRAV1- optional linker -TRAC-optional linker - optional hinge region -CH2-CH3; or
the first polypeptide chain comprises, from N-terminus to C-terminus: TRBV1- optional linker -TRBC- linker -VL1- linker -TRBV2- linker -TRAV2- linker -VH1- optional linker - optional hinge region -CH2-CH3, and the second polypeptide chain comprises, from N-terminus to C-terminus: TRAV1- optional linker -TRAC-optional linker - optional hinge region -CH2-CH3; or
the first polypeptide chain comprises, from N-terminus to C-terminus: TRBV1- optional linker -TRBC- linker -VH1- linker -TRBV2- linker - TRAV2- linker -VL1- optional linker - optional hinge region -CH2-CH3, and the second polypeptide chain comprises, from N-terminus to C-terminus: TRAV1- optional linker -TRAC-optional linker - optional hinge region -CH2-CH3; or
the first polypeptide chain comprises, from N-terminus to C-terminus: TRBV1- optional linker -TRBC- linker -VH1- linker -TRAV2- linker - TRBV2- linker -VL1- optional linker - optional hinge region -CH2-CH3, and the second polypeptide chain comprises, from N-terminus to C-terminus: TRAV1- optional linker -TRAC-optional linker - optional hinge region -CH2-CH3; or
the first polypeptide chain comprises, from N-terminus to C-terminus: TRAV1- optional linker -TRAC- linker -VL1- linker -TRBV2- linker - TRAV2- linker -VH1- optional linker - optional hinge region -CH2-CH3, and the second polypeptide chain comprises, from N-terminus to C-terminus: TRBV1- optional linker -TRBC-optional linker - optional hinge region -CH2-CH3; or
the first polypeptide chain comprises, from N-terminus to C-terminus: TRAV1- optional linker -TRAC- linker -VL1- linker -TRAV2- linker - TRBV2- linker -VH1- optional linker - optional hinge region -CH2-CH3, and the second polypeptide chain comprises, from N-terminus to C-terminus: TRBV1- optional linker -TRBC-optional linker - optional hinge region -CH2-CH3; or
the first polypeptide chain comprises, from N-terminus to C-terminus: TRAV1- optional linker -TRAC- linker -VH1- linker -TRAV2- linker - TRBV2- linker -VL1- optional linker - optional hinge region -CH2-CH3, and the second polypeptide chain comprises, from N-terminus to C-terminus: TRBV1- optional linker -TRBC-optional linker - optional hinge region -CH2-CH3; or
the first polypeptide chain comprises, from N-terminus to C-terminus: TRAV1- optional linker -TRAC- linker -VH1- linker -TRBV2- linker - TRAV2- linker -VL1- optional linker - optional hinge region -CH2-CH3, and the second polypeptide chain comprises, from N-terminus to C-terminus: TRBV1- optional linker -TRBC-optional linker - optional hinge region -CH2-CH3.

In some embodiments, the multi-specific polypeptide molecule comprises a first binding region, a second binding region, a third binding region, and a functional domain comprising an antibody Fc domain or a dimerization portion thereof and an optional antibody hinge region, and the multi-specific polypeptide molecule comprises a first polypeptide chain and a second polypeptide chain; wherein:
the first polypeptide chain comprises, from N-terminus to C-terminus: TRBV1- optional linker -TRBC- linker -VL1- linker -TRAV2- linker -TRBV2- linker -VH1- optional linker - optional hinge region -CH2-CH3, and the second polypeptide chain comprises, from N-terminus to C-terminus: TRAV1- optional linker -TRAC-optional linker - optional hinge region -CH2-CH3; or
the first polypeptide chain comprises, from N-terminus to C-terminus: TRBV1- optional linker -TRBC- linker -VL1- linker -TRBV2- linker -TRAV2- linker -VH1- optional linker - optional hinge region -CH2-CH3, and the second polypeptide chain comprises, from N-terminus to C-terminus: TRAV1- optional linker -TRAC-optional linker - optional hinge region -CH2-CH3; or
the first polypeptide chain comprises, from N-terminus to C-terminus: TRBV1- optional linker -TRBC- linker -VH1- linker -TRBV2- linker - TRAV2- linker -VL1- optional linker - optional hinge region -CH2-CH3, and the second polypeptide chain comprises, from N-terminus to C-terminus: TRAV1- optional linker -TRAC-optional linker - optional hinge region -CH2-CH3; or
the first polypeptide chain comprises, from N-terminus to C-terminus: TRBV1- optional linker -TRBC- linker -VH1- linker -TRAV2- linker - TRBV2- linker -VL1- optional linker - optional hinge region -CH2-CH3, and the second polypeptide chain comprises, from N-terminus to C-terminus: TRAV1- optional linker -TRAC-optional linker - optional hinge region -CH2-CH3; or
the first polypeptide chain comprises, from N-terminus to C-terminus: TRAV1- optional linker -TRAC- linker -VL1- linker -TRBV2- linker - TRAV2- linker -VH1- optional linker - optional hinge region -CH2-CH3, and the second polypeptide chain comprises, from N-terminus to C-terminus: TRBV1- optional linker -TRBC-optional linker - optional hinge region -CH2-CH3; or
the first polypeptide chain comprises, from N-terminus to C-terminus: TRAV1- optional linker -TRAC- linker -VL1- linker -TRAV2- linker - TRBV2- linker -VH1- optional linker - optional hinge region -CH2-CH3, and the second polypeptide chain comprises, from N-terminus to C-terminus: TRBV1- optional linker -TRBC-optional linker - optional hinge region -CH2-CH3; or
the first polypeptide chain comprises, from N-terminus to C-terminus: TRAV1- optional linker -TRAC- linker -VH1- linker -TRAV2- linker - TRBV2- linker -VL1- optional linker - optional hinge region -CH2-CH3, and the second polypeptide chain comprises, from N-terminus to C-terminus: TRBV1- optional linker -TRBC-optional linker - optional hinge region -CH2-CH3; or
the first polypeptide chain comprises, from N-terminus to C-terminus: TRAV1- optional linker -TRAC- linker -VH1- linker -TRBV2- linker - TRAV2- linker -VL1- optional linker - optional hinge region -CH2-CH3, and the second polypeptide chain comprises, from N-terminus to C-terminus: TRBV1- optional linker -TRBC-optional linker - optional hinge region -CH2-CH3.

In some embodiments, the multi-specific polypeptide molecule comprises a first binding region, a second binding region and a fourth binding region as well as a functional domain comprising an albumin or a binding domain thereof and an optional antibody hinge region, and the multi-specific polypeptide molecule comprises a first polypeptide chain and a second polypeptide chain; wherein:
the first polypeptide chain comprises, from N-terminus to C-terminus: scFv2- linker -TRBV1- optional linker -TRBC- linker -scFv1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: scFv2- linker -TRBV1- optional linker -TRBC- linker -scFv1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: scFv2- linker -TRAV1- optional linker -TRAC- linker -scFv1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: scFv2- linker -TRAV1- optional linker -TRAC- linker -scFv1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: scFv2- linker -TRBV1- optional linker -TRBC- linker -VHH1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: scFv2- linker -TRBV1- optional linker -TRBC- linker -VHH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: scFv2- linker -TRAV1- optional linker -TRAC- linker -VHH1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: scFv2- linker -TRAV1- optional linker -TRAC- linker -VHH1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: scFv2- linker -TRBV1- optional linker -TRBC- linker -VL1- optional linker - optional hinge region -Alb, and the second polypeptide chain comprises, from N-terminus to C-terminus: TRAV1- optional linker -TRAC- linker -VH1- optional linker - optional hinge region; or
the first polypeptide chain comprises, from N-terminus to C-terminus: scFv2- linker -TRBV1- optional linker -TRBC- linker -VL1- optional linker - optional hinge region, and the second polypeptide chain comprises, from N-terminus to C-terminus: TRAV1- optional linker -TRAC- linker -VH1- optional linker - optional hinge region -Alb; or
the first polypeptide chain comprises, from N-terminus to C-terminus: scFv2- linker -TRAV1- optional linker -TRAC- linker -VL1- optional linker - optional hinge region -Alb, and the second polypeptide chain comprises, from N-terminus to C-terminus: TRBV1- optional linker -TRBC- linker -VH1- optional linker - optional hinge region; or
the first polypeptide chain comprises, from N-terminus to C-terminus: scFv2- linker -TRAV1- optional linker -TRAC- linker -VL1- optional linker - optional hinge region, and the second polypeptide chain comprises, from N-terminus to C-terminus: TRBV1- optional linker -TRBC- linker -VH1- optional linker - optional hinge region -Alb; or
the first polypeptide chain comprises, from N-terminus to C-terminus: TRBV1- optional linker -TRBC- linker -scFv1- optional linker - optional hinge region -Alb, and the second polypeptide chain comprises, from N-terminus to C-terminus: scFv2- linker -TRAV1- optional linker -TRAC- optional linker - optional hinge region; or
the first polypeptide chain comprises, from N-terminus to C-terminus: TRBV1- optional linker -TRBC- linker -scFv1- optional linker - optional hinge region, and the second polypeptide chain comprises, from N-terminus to C-terminus: scFv2- linker -TRAV1- optional linker -TRAC- optional linker - optional hinge region -Alb; or
the first polypeptide chain comprises, from N-terminus to C-terminus: TRAV1- optional linker -TRAC- linker -scFv1- optional linker - optional hinge region -Alb, and the second polypeptide chain comprises, from N-terminus to C-terminus: scFv2- linker -TRBV1- optional linker -TRBC- optional linker - optional hinge region; or
the first polypeptide chain comprises, from N-terminus to C-terminus: TRAV1- optional linker -TRAC- linker -scFv1- optional linker - optional hinge region, and the second polypeptide chain comprises, from N-terminus to C-terminus: scFv2- linker -TRBV1- optional linker -TRBC- optional linker - optional hinge region -Alb; or
the first polypeptide chain comprises, from N-terminus to C-terminus: TRBV1- optional linker -TRBC- linker -VHH1- optional linker - optional hinge region -Alb, and the second polypeptide chain comprises, from N-terminus to C-terminus: scFv2- linker -TRAV1- optional linker -TRAC- optional linker - optional hinge region; or
the first polypeptide chain comprises, from N-terminus to C-terminus: TRBV1- optional linker -TRBC- linker -VHH1- optional linker - optional hinge region, and the second polypeptide chain comprises, from N-terminus to C-terminus: scFv2- linker -TRAV1- optional linker -TRAC- optional linker - optional hinge region -Alb; or
the first polypeptide chain comprises, from N-terminus to C-terminus: TRAV1- optional linker -TRAC- linker -VHH1- optional linker - optional hinge region -Alb, and the second polypeptide chain comprises, from N-terminus to C-terminus: scFv2- linker -TRBV1- optional linker -TRBC- optional linker - optional hinge region; or
the first polypeptide chain comprises, from N-terminus to C-terminus: TRAV1- optional linker -TRAC- linker -VHH1- optional linker - optional hinge region, and the second polypeptide chain comprises, from N-terminus to C-terminus: scFv2- linker -TRBV1- optional linker -TRBC- optional linker - optional hinge region -Alb; or
the first polypeptide chain comprises, from N-terminus to C-terminus: TRBV1- optional linker -TRBC- linker -VL1- optional linker - optional hinge region -Alb, and the second polypeptide chain comprises, from N-terminus to C-terminus: scFv2- linker -TRAV1- optional linker -TRAC- linker -VH1- optional linker - optional hinge region; or
the first polypeptide chain comprises, from N-terminus to C-terminus: TRBV1- optional linker -TRBC- linker -VL1- optional linker - optional hinge region, and the second polypeptide chain comprises, from N-terminus to C-terminus: scFv2- linker -TRAV1- optional linker -TRAC- linker -VH1- optional linker - optional hinge region -Alb; or
the first polypeptide chain comprises, from N-terminus to C-terminus: TRAV1- optional linker -TRAC- linker -VL1- optional linker - optional hinge region -Alb, and the second polypeptide chain comprises, from N-terminus to C-terminus: scFv2- linker -TRBV1- optional linker -TRBC- linker -VH1- optional linker - optional hinge region; or
the first polypeptide chain comprises, from N-terminus to C-terminus: TRAV1- optional linker -TRAC- linker -VL1- optional linker - optional hinge region, and the second polypeptide chain comprises, from N-terminus to C-terminus: scFv2- linker -TRBV1- optional linker -TRBC- linker -VH1- optional linker - optional hinge region -Alb; or
the first polypeptide chain comprises, from N-terminus to C-terminus: VHH2- linker -TRBV1- optional linker -TRBC- linker -scFv1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: VHH2- linker -TRBV1- optional linker -TRBC- linker -scFv1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: VHH2- linker -TRAV1- optional linker -TRAC- linker -scFv1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: VHH2- linker -TRAV1- optional linker -TRAC- linker -scFv1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: VHH2- linker -TRBV1- optional linker -TRBC- linker -VHH1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: VHH2- linker -TRBV1- optional linker -TRBC- linker -VHH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: VHH2- linker -TRAV1- optional linker -TRAC- linker -VHH1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: VHH2- linker -TRAV1- optional linker -TRAC- linker -VHH1- optional linker - optional hinge region, and the second polypeptide chain comprises, from N-terminus to C-terminus: TRBV1- optional linker -TRBC- optional linker - optional hinge region -Alb; or
the first polypeptide chain comprises, from N-terminus to C-terminus: VHH2- linker -TRBV1- optional linker -TRBC- linker -VL1- optional linker - optional hinge region -Alb, and the second polypeptide chain comprises, from N-terminus to C-terminus: TRAV1- optional linker -TRAC- linker -VH1- optional linker - optional hinge region; or
the first polypeptide chain comprises, from N-terminus to C-terminus: VHH2- linker -TRBV1- optional linker -TRBC- linker -VL1- optional linker - optional hinge region, and the second polypeptide chain comprises, from N-terminus to C-terminus: TRAV1- optional linker -TRAC- linker -VH1- optional linker - optional hinge region -Alb; or
the first polypeptide chain comprises, from N-terminus to C-terminus: VHH2- linker -TRAV1- optional linker -TRAC- linker -VL1- optional linker - optional hinge region -Alb, and the second polypeptide chain comprises, from N-terminus to C-terminus: TRBV1- optional linker -TRBC- linker -VH1- optional linker - optional hinge region; or
the first polypeptide chain comprises, from N-terminus to C-terminus: VHH2- linker -TRAV1- optional linker -TRAC- linker -VL1- optional linker - optional hinge region, and the second polypeptide chain comprises, from N-terminus to C-terminus: TRBV1- optional linker -TRBC- linker -VH1- optional linker - optional hinge region -Alb; or
the first polypeptide chain comprises, from N-terminus to C-terminus: TRBV1- optional linker -TRBC- linker -scFv1- optional linker - optional hinge region -Alb, and the second polypeptide chain comprises, from N-terminus to C-terminus: VHH2- linker -TRAV1- optional linker -TRAC- optional linker - optional hinge region; or
the first polypeptide chain comprises, from N-terminus to C-terminus: TRBV1- optional linker -TRBC- linker -scFv1- optional linker - optional hinge region, and the second polypeptide chain comprises, from N-terminus to C-terminus: VHH2- linker -TRAV1- optional linker -TRAC- optional linker - optional hinge region -Alb; or
the first polypeptide chain comprises, from N-terminus to C-terminus: TRAV1- optional linker -TRAC- linker -scFv1- optional linker - optional hinge region -Alb, and the second polypeptide chain comprises, from N-terminus to C-terminus: VHH2- linker -TRBV1- optional linker -TRBC- optional linker - optional hinge region; or
the first polypeptide chain comprises, from N-terminus to C-terminus: TRAV1- optional linker -TRAC- linker -scFv1- optional linker - optional hinge region, and the second polypeptide chain comprises, from N-terminus to C-terminus: VHH2- linker -TRBV1- optional linker -TRBC- optional linker - optional hinge region -Alb; or
the first polypeptide chain comprises, from N-terminus to C-terminus: TRBV1- optional linker -TRBC- linker -VHH1- optional linker - optional hinge region -Alb, and the second polypeptide chain comprises, from N-terminus to C-terminus: VHH2- linker -TRAV1- optional linker -TRAC- optional linker - optional hinge region; or
the first polypeptide chain comprises, from N-terminus to C-terminus: TRBV1- optional linker -TRBC- linker -VHH1- optional linker - optional hinge region, and the second polypeptide chain comprises, from N-terminus to C-terminus: VHH2- linker -TRAV1- optional linker -TRAC- optional linker - optional hinge region -Alb; or
the first polypeptide chain comprises, from N-terminus to C-terminus: TRAV1- optional linker -TRAC- linker -VHH1- optional linker - optional hinge region -Alb, and the second polypeptide chain comprises, from N-terminus to C-terminus: VHH2- linker -TRBV1- optional linker -TRBC- optional linker - optional hinge region; or
the first polypeptide chain comprises, from N-terminus to C-terminus: TRAV1- optional linker -TRAC- linker -VHH1- optional linker - optional hinge region, and the second polypeptide chain comprises, from N-terminus to C-terminus: VHH2- linker -TRBV1- optional linker -TRBC- optional linker - optional hinge region -Alb; or
the first polypeptide chain comprises, from N-terminus to C-terminus: TRBV1- optional linker -TRBC- linker -VL1- optional linker - optional hinge region -Alb, and the second polypeptide chain comprises, from N-terminus to C-terminus: VHH2- linker -TRAV1- optional linker -TRAC- linker -VH1- optional linker - optional hinge region; or
the first polypeptide chain comprises, from N-terminus to C-terminus: TRBV1- optional linker -TRBC- linker -VL1- optional linker - optional hinge region, and the second polypeptide chain comprises, from N-terminus to C-terminus: VHH2- linker -TRAV1- optional linker -TRAC- linker -VH1- optional linker - optional hinge region -Alb; or
the first polypeptide chain comprises, from N-terminus to C-terminus: TRAV1- optional linker -TRAC- linker -VL1- optional linker - optional hinge region -Alb, and the second polypeptide chain comprises, from N-terminus to C-terminus: VHH2- linker -TRBV1- optional linker -TRBC- linker -VH1- optional linker - optional hinge region; or
the first polypeptide chain comprises, from N-terminus to C-terminus: TRAV1- optional linker -TRAC- linker -VL1- optional linker - optional hinge region, and the second polypeptide chain comprises, from N-terminus to C-terminus: VHH2- linker -TRBV1- optional linker -TRBC- linker -VH1- optional linker - optional hinge region -Alb; or
the first polypeptide chain comprises, from N-terminus to C-terminus: VL2- linker -TRBV1- optional linker -TRBC- linker -scFv1- optional linker - optional hinge region -Alb, and the second polypeptide chain comprises, from N-terminus to C-terminus: VH2- linker -TRAV1- optional linker -TRAC- optional linker - optional hinge region; or
the first polypeptide chain comprises, from N-terminus to C-terminus: VL2- linker -TRBV1- optional linker -TRBC- linker -scFv1- optional linker - optional hinge region, and the second polypeptide chain comprises, from N-terminus to C-terminus: VH2- linker -TRAV1- optional linker -TRAC- optional linker - optional hinge region -Alb; or
the first polypeptide chain comprises, from N-terminus to C-terminus: VH2- linker -TRBV1- optional linker -TRBC- linker -scFv1- optional linker - optional hinge region -Alb, and the second polypeptide chain comprises, from N-terminus to C-terminus: VL2- linker -TRAV1- optional linker -TRAC- optional linker - optional hinge region; or
the first polypeptide chain comprises, from N-terminus to C-terminus: VH2- linker -TRBV1- optional linker -TRBC- linker -scFv1- optional linker - optional hinge region, and the second polypeptide chain comprises, from N-terminus to C-terminus: VL2- linker -TRAV1- optional linker -TRAC- optional linker - optional hinge region -Alb; or
the first polypeptide chain comprises, from N-terminus to C-terminus: VL2- linker -TRAV1- optional linker -TRAC- linker -scFv1- optional linker - optional hinge region -Alb, and the second polypeptide chain comprises, from N-terminus to C-terminus: VH2- linker -TRBV1- optional linker -TRBC- optional linker - optional hinge region; or
the first polypeptide chain comprises, from N-terminus to C-terminus: VL2- linker -TRAV1- optional linker -TRAC- linker -scFv1- optional linker - optional hinge region, and the second polypeptide chain comprises, from N-terminus to C-terminus: VH2- linker -TRBV1- optional linker -TRBC- optional linker - optional hinge region -Alb; or
the first polypeptide chain comprises, from N-terminus to C-terminus: VH2- linker -TRAV1- optional linker -TRAC- linker -scFv1- optional linker - optional hinge region -Alb, and the second polypeptide chain comprises, from N-terminus to C-terminus: VL2- linker -TRBV1- optional linker -TRBC- optional linker - optional hinge region; or
the first polypeptide chain comprises, from N-terminus to C-terminus: VH2- linker -TRAV1- optional linker -TRAC- linker -scFv1- optional linker - optional hinge region, and the second polypeptide chain comprises, from N-terminus to C-terminus: VL2- linker -TRBV1- optional linker -TRBC- optional linker - optional hinge region -Alb; or
the first polypeptide chain comprises, from N-terminus to C-terminus: VL2- linker -TRBV1- optional linker -TRBC- linker -VHH1- optional linker - optional hinge region -Alb, and the second polypeptide chain comprises, from N-terminus to C-terminus: VH2- linker -TRAV1- optional linker -TRAC- optional linker - optional hinge region; or
the first polypeptide chain comprises, from N-terminus to C-terminus: VL2- linker -TRBV1- optional linker -TRBC- linker -VHH1- optional linker - optional hinge region, and the second polypeptide chain comprises, from N-terminus to C-terminus: VH2- linker -TRAV1- optional linker -TRAC- optional linker - optional hinge region -Alb; or
the first polypeptide chain comprises, from N-terminus to C-terminus: VH2- linker -TRBV1- optional linker -TRBC- linker -VHH1- optional linker - optional hinge region -Alb, and the second polypeptide chain comprises, from N-terminus to C-terminus: VL2- linker -TRAV1- optional linker -TRAC- optional linker - optional hinge region; or
the first polypeptide chain comprises, from N-terminus to C-terminus: VH2- linker -TRBV1- optional linker -TRBC- linker -VHH1- optional linker - optional hinge region, and the second polypeptide chain comprises, from N-terminus to C-terminus: VL2- linker -TRAV1- optional linker -TRAC- optional linker - optional hinge region -Alb; or
the first polypeptide chain comprises, from N-terminus to C-terminus: VL2- linker -TRAV1- optional linker -TRAC- linker -VHH1- optional linker - optional hinge region -Alb, and the second polypeptide chain comprises, from N-terminus to C-terminus: VH2- linker -TRBV1- optional linker -TRBC- optional linker - optional hinge region; or
the first polypeptide chain comprises, from N-terminus to C-terminus: VL2- linker -TRAV1- optional linker -TRAC- linker -VHH1- optional linker - optional hinge region, and the second polypeptide chain comprises, from N-terminus to C-terminus: VH2- linker -TRBV1- optional linker -TRBC- optional linker - optional hinge region -Alb; or
the first polypeptide chain comprises, from N-terminus to C-terminus: VH2- linker -TRAV1- optional linker -TRAC- linker -VHH1- optional linker - optional hinge region -Alb, and the second polypeptide chain comprises, from N-terminus to C-terminus: VL2- linker -TRBV1- optional linker -TRBC- optional linker - optional hinge region; or
the first polypeptide chain comprises, from N-terminus to C-terminus: VH2- linker -TRAV1- optional linker -TRAC- linker -VHH1- optional linker - optional hinge region, and the second polypeptide chain comprises, from N-terminus to C-terminus: VL2- linker -TRBV1- optional linker -TRBC- optional linker - optional hinge region -Alb; or
the first polypeptide chain comprises, from N-terminus to C-terminus: VL2- linker -TRBV1- optional linker -TRBC- linker -VL1- optional linker - optional hinge region -Alb, and the second polypeptide chain comprises, from N-terminus to C-terminus: VH2- linker -TRAV1- optional linker -TRAC- linker -VH1-optional linker - optional hinge region; or
the first polypeptide chain comprises, from N-terminus to C-terminus: VL2- linker -TRBV1- optional linker -TRBC- linker -VL1- optional linker - optional hinge region, and the second polypeptide chain comprises, from N-terminus to C-terminus: VH2- linker -TRAV1- optional linker -TRAC- linker -VH1- optional linker - optional hinge region -Alb; or
the first polypeptide chain comprises, from N-terminus to C-terminus: VH2- linker -TRBV1- optional linker -TRBC- linker -VL1- optional linker - optional hinge region -Alb, and the second polypeptide chain comprises, from N-terminus to C-terminus: VL2- linker -TRAV1- optional linker -TRAC- linker -VH1-optional linker - optional hinge region; or
the first polypeptide chain comprises, from N-terminus to C-terminus: VH2- linker -TRBV1- optional linker -TRBC- linker -VL1- optional linker - optional hinge region, and the second polypeptide chain comprises, from N-terminus to C-terminus: VL2- linker -TRAV1- optional linker -TRAC- linker -VH1- optional linker - optional hinge region -Alb; or
the first polypeptide chain comprises, from N-terminus to C-terminus: VL2- linker -TRAV1- optional linker -TRAC- linker -VL1- optional linker - optional hinge region -Alb, and the second polypeptide chain comprises, from N-terminus to C-terminus: VH2- linker -TRBV1- optional linker -TRBC- linker -VH1-optional linker - optional hinge region; or
the first polypeptide chain comprises, from N-terminus to C-terminus: VL2- linker -TRAV1- optional linker -TRAC- linker -VL1- optional linker - optional hinge region, and the second polypeptide chain comprises, from N-terminus to C-terminus: VH2- linker -TRBV1- optional linker -TRBC- linker -VH1- optional linker - optional hinge region -Alb; or
the first polypeptide chain comprises, from N-terminus to C-terminus: VH2- linker -TRAV1- optional linker -TRAC- linker -VL1- optional linker - optional hinge region -Alb, and the second polypeptide chain comprises, from N-terminus to C-terminus: VL2- linker -TRBV1- optional linker -TRBC- linker -VH1- optional linker - optional hinge region; or
the first polypeptide chain comprises, from N-terminus to C-terminus: VH2- linker -TRAV1- optional linker -TRAC- linker -VL1- optional linker - optional hinge region, and the second polypeptide chain comprises, from N-terminus to C-terminus: VL2- linker -TRBV1- optional linker -TRBC- linker -VH1- optional linker - optional hinge region -Alb.

In some embodiments, the multi-specific polypeptide molecule comprises a first binding region, a second binding region, a fourth binding region, and a functional domain comprising an antibody-derived Fc domain or a dimerization portion thereof and an optional antibody hinge region, and the multi-specific polypeptide molecule comprises a first polypeptide chain and a second polypeptide chain; wherein:
the first polypeptide chain comprises, from N-terminus to C-terminus: scFv2- linker -TRBV1- optional linker -TRBC- linker -scFv1- optional linker - optional hinge region -CH2-CH3, and the second polypeptide chain comprises, from N-terminus to C-terminus: TRAV1- optional linker -TRAC- optional linker - optional hinge region -CH2-CH3; or
the first polypeptide chain comprises, from N-terminus to C-terminus: scFv2- linker -TRAV1- optional linker -TRAC- linker -scFv1- optional linker - optional hinge region -CH2-CH3, and the second polypeptide chain comprises, from N-terminus to C-terminus: TRBV1- optional linker -TRBC- optional linker - optional hinge region -CH2-CH3; or
the first polypeptide chain comprises, from N-terminus to C-terminus: scFv2- linker -TRBV1- optional linker -TRBC- linker -VHH1- optional linker - optional hinge region -CH2-CH3, and the second polypeptide chain comprises, from N-terminus to C-terminus: TRAV1- optional linker -TRAC- optional linker - optional hinge region -CH2-CH3; or
the first polypeptide chain comprises, from N-terminus to C-terminus: scFv2- linker -TRAV1- optional linker -TRAC- linker -VHH1- optional linker - optional hinge region -CH2-CH3, and the second polypeptide chain comprises, from N-terminus to C-terminus: TRBV1- optional linker -TRBC- optional linker - optional hinge region -CH2-CH3; or
the first polypeptide chain comprises, from N-terminus to C-terminus: scFv2- linker -TRBV1- optional linker -TRBC- linker -VL1- optional linker - optional hinge region -CH2-CH3, and the second polypeptide chain comprises, from N-terminus to C-terminus: TRAV1- optional linker -TRAC- linker -VH1- optional linker - optional hinge region -CH2-CH3; or
the first polypeptide chain comprises, from N-terminus to C-terminus: scFv2- linker -TRAV1- optional linker -TRAC- linker -VL1- optional linker - optional hinge region -CH2-CH3, and the second polypeptide chain comprises, from N-terminus to C-terminus: TRBV1- optional linker -TRBC- linker -VH1- optional linker - optional hinge region -CH2-CH3; or
the first polypeptide chain comprises, from N-terminus to C-terminus: TRBV1- optional linker -TRBC- linker -scFv1- optional linker - optional hinge region -CH2-CH3, and the second polypeptide chain comprises, from N-terminus to C-terminus: scFv2- linker -TRAV1- optional linker -TRAC- optional linker - optional hinge region -CH2-CH3; or
the first polypeptide chain comprises, from N-terminus to C-terminus: TRAV1- optional linker -TRAC- linker -scFv1- optional linker - optional hinge region -CH2-CH3, and the second polypeptide chain comprises, from N-terminus to C-terminus: scFv2- linker -TRBV1- optional linker -TRBC- optional linker - optional hinge region -CH2-CH3; or
the first polypeptide chain comprises, from N-terminus to C-terminus: TRBV1- optional linker -TRBC- linker -VHH1- optional linker - optional hinge region -CH2-CH3, and the second polypeptide chain comprises, from N-terminus to C-terminus: scFv2- linker -TRAV1- optional linker -TRAC- optional linker - optional hinge region -CH2-CH3; or
the first polypeptide chain comprises, from N-terminus to C-terminus: TRAV1- optional linker -TRAC- linker -VHH1- optional linker - optional hinge region -CH2-CH3, and the second polypeptide chain comprises, from N-terminus to C-terminus: scFv2- linker -TRBV1- optional linker -TRBC- optional linker - optional hinge region -CH2-CH3; or
the first polypeptide chain comprises, from N-terminus to C-terminus: TRBV1- optional linker -TRBC- linker -VL1- optional linker - optional hinge region -CH2-CH3, and the second polypeptide chain comprises, from N-terminus to C-terminus: scFv2- linker -TRAV1- optional linker -TRAC- linker -VH1- optional linker - optional hinge region -CH2-CH3; or
the first polypeptide chain comprises, from N-terminus to C-terminus: TRAV1- optional linker -TRAC- linker -VL1- optional linker - optional hinge region -CH2-CH3, and the second polypeptide chain comprises, from N-terminus to C-terminus: scFv2- linker -TRBV1- optional linker -TRBC- linker -VH1- optional linker - optional hinge region -CH2-CH3; or
the first polypeptide chain comprises, from N-terminus to C-terminus: VHH2- linker -TRBV1- optional linker -TRBC- linker -scFv1- optional linker - optional hinge region -CH2-CH3, and the second polypeptide chain comprises, from N-terminus to C-terminus: TRAV1- optional linker -TRAC- optional linker - optional hinge region -CH2-CH3; or
the first polypeptide chain comprises, from N-terminus to C-terminus: VHH2- linker -TRAV1- optional linker -TRAC- linker -scFv1- optional linker - optional hinge region -CH2-CH3, and the second polypeptide chain comprises, from N-terminus to C-terminus: TRBV1- optional linker -TRBC- optional linker - optional hinge region -CH2-CH3; or
the first polypeptide chain comprises, from N-terminus to C-terminus: VHH2- linker -TRBV1- optional linker -TRBC- linker -VHH1- optional linker - optional hinge region -CH2-CH3, and the second polypeptide chain comprises, from N-terminus to C-terminus: TRAV1- optional linker -TRAC- optional linker - optional hinge region -CH2-CH3; or
the first polypeptide chain comprises, from N-terminus to C-terminus: VHH2- linker -TRAV1- optional linker -TRAC- linker -VHH1- optional linker - optional hinge region -CH2-CH3, and the second polypeptide chain comprises, from N-terminus to C-terminus: TRBV1- optional linker -TRBC- optional linker - optional hinge region -CH2-CH3; or
the first polypeptide chain comprises, from N-terminus to C-terminus: VHH2- linker -TRBV1- optional linker -TRBC- linker -VL1- optional linker - optional hinge region -CH2-CH3, and the second polypeptide chain comprises, from N-terminus to C-terminus: TRAV1- optional linker -TRAC- linker -VH1- optional linker - optional hinge region -CH2-CH3; or
the first polypeptide chain comprises, from N-terminus to C-terminus: VHH2- linker -TRAV1- optional linker -TRAC- linker -VL1- optional linker - optional hinge region -CH2-CH3, and the second polypeptide chain comprises, from N-terminus to C-terminus: TRBV1- optional linker -TRBC- linker -VH1- optional linker - optional hinge region -CH2-CH3; or
the first polypeptide chain comprises, from N-terminus to C-terminus: TRBV1- optional linker -TRBC- linker -scFv1- optional linker - optional hinge region -CH2-CH3, and the second polypeptide chain comprises, from N-terminus to C-terminus: VHH2- linker -TRAV1- optional linker -TRAC- optional linker - optional hinge region -CH2-CH3; or
the first polypeptide chain comprises, from N-terminus to C-terminus: TRAV1- optional linker -TRAC- linker -scFv1- optional linker - optional hinge region -CH2-CH3, and the second polypeptide chain comprises, from N-terminus to C-terminus: VHH2- linker -TRBV1- optional linker -TRBC- optional linker - optional hinge region -CH2-CH3; or
the first polypeptide chain comprises, from N-terminus to C-terminus: TRBV1- optional linker -TRBC- linker -VHH1- optional linker - optional hinge region -CH2-CH3, and the second polypeptide chain comprises, from N-terminus to C-terminus: VHH2- linker -TRAV1- optional linker -TRAC- optional linker - optional hinge region -CH2-CH3; or
the first polypeptide chain comprises, from N-terminus to C-terminus: TRAV1- optional linker -TRAC- linker -VHH1- optional linker - optional hinge region -CH2-CH3, and the second polypeptide chain comprises, from N-terminus to C-terminus: VHH2- linker -TRBV1- optional linker -TRBC- optional linker - optional hinge region -CH2-CH3; or
the first polypeptide chain comprises, from N-terminus to C-terminus: TRBV1- optional linker -TRBC- linker -VL1- optional linker - optional hinge region -CH2-CH3, and the second polypeptide chain comprises, from N-terminus to C-terminus: VHH2- linker -TRAV1- optional linker -TRAC- linker -VH1- optional linker - optional hinge region -CH2-CH3; or
the first polypeptide chain comprises, from N-terminus to C-terminus: TRAV1- optional linker -TRAC- linker -VL1- optional linker - optional hinge region -CH2-CH3, and the second polypeptide chain comprises, from N-terminus to C-terminus: VHH2- linker -TRBV1- optional linker -TRBC- linker -VH1- optional linker - optional hinge region -CH2-CH3; or
the first polypeptide chain comprises, from N-terminus to C-terminus: VL2- linker -TRBV1- optional linker -TRBC- linker -scFv1- optional linker - optional hinge region -CH2-CH3, and the second polypeptide chain comprises, from N-terminus to C-terminus: VH2- linker -TRAV1- optional linker -TRAC- optional linker - optional hinge region -CH2-CH3; or
the first polypeptide chain comprises, from N-terminus to C-terminus: VH2- linker -TRBV1- optional linker -TRBC- linker -scFv1- optional linker - optional hinge region -CH2-CH3, and the second polypeptide chain comprises, from N-terminus to C-terminus: VL2- linker -TRAV1- optional linker -TRAC- optional linker - optional hinge region -CH2-CH3; or
the first polypeptide chain comprises, from N-terminus to C-terminus: VL2- linker -TRAV1- optional linker -TRAC- linker -scFv1- optional linker - optional hinge region -CH2-CH3, and the second polypeptide chain comprises, from N-terminus to C-terminus: VH2- linker -TRBV1- optional linker -TRBC- optional linker - optional hinge region -CH2-CH3; or
the first polypeptide chain comprises, from N-terminus to C-terminus: VH2- linker -TRAV1- optional linker -TRAC- linker -scFv1- optional linker - optional hinge region -CH2-CH3, and the second polypeptide chain comprises, from N-terminus to C-terminus: VL2- linker -TRBV1- optional linker -TRBC- optional linker - optional hinge region -CH2-CH3; or
the first polypeptide chain comprises, from N-terminus to C-terminus: VL2- linker -TRBV1- optional linker -TRBC- linker -VHH1- optional linker - optional hinge region -CH2-CH3, and the second polypeptide chain comprises, from N-terminus to C-terminus: VH2- linker -TRAV1- optional linker -TRAC- optional linker - optional hinge region -CH2-CH3; or
the first polypeptide chain comprises, from N-terminus to C-terminus: VH2- linker -TRBV1- optional linker -TRBC- linker -VHH1- optional linker - optional hinge region -CH2-CH3, and the second polypeptide chain comprises, from N-terminus to C-terminus: VL2- linker -TRAV1- optional linker -TRAC- optional linker - optional hinge region -CH2-CH3; or
the first polypeptide chain comprises, from N-terminus to C-terminus: VL2- linker -TRAV1- optional linker -TRAC- linker -VHH1- optional linker - optional hinge region -CH2-CH3, and the second polypeptide chain comprises, from N-terminus to C-terminus: VH2- linker -TRBV1- optional linker -TRBC- optional linker - optional hinge region -CH2-CH3; or
the first polypeptide chain comprises, from N-terminus to C-terminus: VH2- linker -TRAV1- optional linker -TRAC- linker -VHH1- optional linker - optional hinge region -CH2-CH3, and the second polypeptide chain comprises, from N-terminus to C-terminus: VL2- linker -TRBV1- optional linker -TRBC- optional linker - optional hinge region -CH2-CH3; or
the first polypeptide chain comprises, from N-terminus to C-terminus: VL2- linker -TRBV1- optional linker -TRBC- linker -VL1- optional linker - optional hinge region -CH2-CH3, and the second polypeptide chain comprises, from N-terminus to C-terminus: VH2- linker -TRAV1- optional linker -TRAC- linker -VH1-optional linker - optional hinge region -CH2-CH3; or
the first polypeptide chain comprises, from N-terminus to C-terminus: VH2- linker -TRBV1- optional linker -TRBC- linker -VL1- optional linker - optional hinge region -CH2-CH3, and the second polypeptide chain comprises, from N-terminus to C-terminus: VL2- linker -TRAV1- optional linker -TRAC- linker -VH1-optional linker - optional hinge region -CH2-CH3; or
the first polypeptide chain comprises, from N-terminus to C-terminus: VL2- linker -TRAV1- optional linker -TRAC- linker -VL1- optional linker - optional hinge region -CH2-CH3, and the second polypeptide chain comprises, from N-terminus to C-terminus: VH2- linker -TRBV1- optional linker -TRBC- linker -VH1-optional linker - optional hinge region -CH2-CH3; or
the first polypeptide chain comprises, from N-terminus to C-terminus: VH2- linker -TRAV1- optional linker -TRAC- linker -VL1- optional linker - optional hinge region -CH2-CH3, and the second polypeptide chain comprises, from N-terminus to C-terminus: VL2- linker -TRBV1- optional linker -TRBC- linker -VH1-optional linker - optional hinge region -CH2-CH3.

In some embodiments, the multi-specific polypeptide molecule comprises a first binding region, a second binding region, a third binding region, a fourth binding region, and a functional domain comprising an albumin or a binding domain thereof and an optional antibody hinge region, and the multi-specific polypeptide molecule comprises a first polypeptide chain and a second polypeptide chain; wherein:
the first polypeptide chain comprises, from N-terminus to C-terminus: scFv2- linker -TRBV1- optional linker -TRBC- linker -VL1- linker -TRAV2- linker -TRBV2- linker -VH1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: scFv2- linker -TRBV1- optional linker -TRBC- linker -VL1- linker -TRAV2- linker -TRBV2- linker -VH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: scFv2- linker -TRBV1- optional linker -TRBC- linker -VL1- linker -TRBV2- linker -TRAV2- linker -VH1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: scFv2- linker -TRBV1- optional linker -TRBC- linker -VL1- linker -TRBV2- linker -TRAV2- linker -VH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: scFv2- linker -TRBV1- optional linker -TRBC- linker -VH1- linker -TRBV2- linker -TRAV2- linker -VL1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: scFv2- linker -TRBV1- optional linker -TRBC- linker -VH1- linker -TRBV2- linker -TRAV2- linker -VL1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: scFv2- linker -TRBV1- optional linker -TRBC- linker -VH1- linker -TRAV2- linker -TRBV2- linker -VL1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: scFv2- linker -TRBV1- optional linker -TRBC- linker -VH1- linker -TRAV2- linker -TRBV2- linker -VL1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: scFv2- linker -TRAV1- optional linker -TRAC- linker -VL1- linker -TRBV2- linker -TRAV2- linker -VH1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: scFv2- linker -TRAV1- optional linker -TRAC- linker -VL1- linker -TRBV2- linker -TRAV2- linker -VH1- optional linker - optional hinge region, and the second polypeptide chain comprises, from N-terminus to C-terminus: TRBV1- optional linker - TRBC- optional linker - optional hinge region -Alb; or
the first polypeptide chain comprises, from N-terminus to C-terminus: scFv2- linker -TRAV1- optional linker -TRAC- linker -VL1- linker -TRAV2- linker -TRBV2- linker -VH1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: scFv2- linker -TRAV1- optional linker -TRAC- linker -VL1- linker -TRAV2- linker -TRBV2- linker -VH1- optional linker - optional hinge region, and the second polypeptide chain comprises, from N-terminus to C-terminus: TRBV1- optional linker - TRBC- optional linker - optional hinge region -Alb; or
the first polypeptide chain comprises, from N-terminus to C-terminus: scFv2- linker -TRAV1- optional linker -TRAC- linker -VH1- linker -TRAV2- linker -TRBV2- linker -VL1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: scFv2- linker -TRAV1- optional linker -TRAC- linker -VH1- linker -TRAV2- linker -TRBV2- linker -VL1- optional linker - optional hinge region, and the second polypeptide chain comprises, from N-terminus to C-terminus: TRBV1- optional linker - TRBC- optional linker - optional hinge region -Alb; or
the first polypeptide chain comprises, from N-terminus to C-terminus: scFv2- linker -TRAV1- optional linker -TRAC- linker -VH1- linker -TRBV2- linker -TRAV2- linker -VL1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: scFv2- linker -TRAV1- optional linker -TRAC- linker -VH1- linker -TRBV2- linker -TRAV2- linker -VL1- optional linker - optional hinge region, and the second polypeptide chain comprises, from N-terminus to C-terminus: TRBV1- optional linker - TRBC- optional linker - optional hinge region -Alb; or
the first polypeptide chain comprises, from N-terminus to C-terminus: TRBV1- optional linker -TRBC- linker -VL1- linker -TRAV2- linker -TRBV2- linker -VH1- optional linker - optional hinge region -Alb, and the second polypeptide chain comprises, from N-terminus to C-terminus: scFv2- linker -TRAV1- optional linker -TRAC- optional linker - optional hinge region; or
the first polypeptide chain comprises, from N-terminus to C-terminus: TRBV1- optional linker -TRBC- linker -VL1- linker -TRAV2- linker -TRBV2- linker -VH1- optional linker - optional hinge region, and the second polypeptide chain comprises, from N-terminus to C-terminus: scFv2- linker -TRAV1- optional linker - TRAC- optional linker - optional hinge region -Alb; or
the first polypeptide chain comprises, from N-terminus to C-terminus: TRBV1- optional linker -TRBC- linker -VL1- linker -TRBV2- linker -TRAV2- linker -VH1- optional linker - optional hinge region -Alb, and the second polypeptide chain comprises, from N-terminus to C-terminus: scFv2- linker -TRAV1- optional linker -TRAC- optional linker - optional hinge region; or
the first polypeptide chain comprises, from N-terminus to C-terminus: TRBV1- optional linker -TRBC- linker -VL1- linker -TRBV2- linker -TRAV2- linker -VH1- optional linker - optional hinge region, and the second polypeptide chain comprises, from N-terminus to C-terminus: scFv2- linker -TRAV1- optional linker - TRAC- optional linker - optional hinge region -Alb; or
the first polypeptide chain comprises, from N-terminus to C-terminus: TRBV1- optional linker -TRBC- linker -VH1- linker -TRBV2- linker -TRAV2- linker -VL1- optional linker - optional hinge region -Alb, and the second polypeptide chain comprises, from N-terminus to C-terminus: scFv2- linker -TRAV1- optional linker -TRAC- optional linker - optional hinge region; or
the first polypeptide chain comprises, from N-terminus to C-terminus: TRBV1- optional linker -TRBC- linker -VH1- linker -TRBV2- linker -TRAV2- linker -VL1- optional linker - optional hinge region, and the second polypeptide chain comprises, from N-terminus to C-terminus: scFv2- linker -TRAV1- optional linker - TRAC- optional linker - optional hinge region -Alb; or
the first polypeptide chain comprises, from N-terminus to C-terminus: TRBV1- optional linker -TRBC- linker -VH1- linker -TRAV2- linker -TRBV2- linker -VL1- optional linker - optional hinge region -Alb, and the second polypeptide chain comprises, from N-terminus to C-terminus: scFv2- linker -TRAV1- optional linker -TRAC- optional linker - optional hinge region; or
the first polypeptide chain comprises, from N-terminus to C-terminus: TRBV1- optional linker -TRBC- linker -VH1- linker -TRAV2- linker -TRBV2- linker -VL1- optional linker - optional hinge region, and the second polypeptide chain comprises, from N-terminus to C-terminus: scFv2- linker -TRAV1- optional linker - TRAC- optional linker - optional hinge region -Alb; or
the first polypeptide chain comprises, from N-terminus to C-terminus: TRAV1- optional linker -TRAC- linker -VL1- linker -TRBV2- linker -TRAV2- linker -VH1- optional linker - optional hinge region -Alb, and the second polypeptide chain comprises, from N-terminus to C-terminus: scFv2- linker -TRBV1- optional linker -TRBC- optional linker - optional hinge region; or
the first polypeptide chain comprises, from N-terminus to C-terminus: TRAV1- optional linker -TRAC- linker -VL1- linker -TRBV2- linker -TRAV2- linker -VH1- optional linker - optional hinge region, and the second polypeptide chain comprises, from N-terminus to C-terminus: scFv2- linker -TRBV1- optional linker - TRBC- optional linker - optional hinge region -Alb; or
the first polypeptide chain comprises, from N-terminus to C-terminus: TRAV1- optional linker -TRAC- linker -VL1- linker -TRAV2- linker -TRBV2- linker -VH1- optional linker - optional hinge region -Alb, and the second polypeptide chain comprises, from N-terminus to C-terminus: scFv2- linker -TRBV1- optional linker -TRBC- optional linker - optional hinge region; or
the first polypeptide chain comprises, from N-terminus to C-terminus: TRAV1- optional linker -TRAC- linker -VL1- linker -TRAV2- linker -TRBV2- linker -VH1- optional linker - optional hinge region, and the second polypeptide chain comprises, from N-terminus to C-terminus: scFv2- linker -TRBV1- optional linker - TRBC- optional linker - optional hinge region -Alb; or
the first polypeptide chain comprises, from N-terminus to C-terminus: TRAV1- optional linker -TRAC- linker -VH1- linker -TRAV2- linker -TRBV2- linker -VL1- optional linker - optional hinge region -Alb, and the second polypeptide chain comprises, from N-terminus to C-terminus: scFv2- linker -TRBV1- optional linker -TRBC- optional linker - optional hinge region; or
the first polypeptide chain comprises, from N-terminus to C-terminus: TRAV1- optional linker -TRAC- linker -VH1- linker -TRAV2- linker -TRBV2- linker -VL1- optional linker - optional hinge region, and the second polypeptide chain comprises, from N-terminus to C-terminus: scFv2- linker -TRBV1- optional linker - TRBC- optional linker - optional hinge region -Alb; or
the first polypeptide chain comprises, from N-terminus to C-terminus: TRAV1- optional linker -TRAC- linker -VH1- linker -TRBV2- linker -TRAV2- linker -VL1- optional linker - optional hinge region -Alb, and the second polypeptide chain comprises, from N-terminus to C-terminus: scFv2- linker -TRBV1- optional linker -TRBC- optional linker - optional hinge region; or
the first polypeptide chain comprises, from N-terminus to C-terminus: TRAV1- optional linker -TRAC- linker -VH1- linker -TRBV2- linker -TRAV2- linker -VL1- optional linker - optional hinge region, and the second polypeptide chain comprises, from N-terminus to C-terminus: scFv2- linker -TRBV1- optional linker - TRBC- optional linker - optional hinge region -Alb; or
the first polypeptide chain comprises, from N-terminus to C-terminus: VHH2- linker -TRBV1- optional linker -TRBC- linker -VL1- linker -TRAV2- linker -TRBV2- linker -VH1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: VHH2- linker -TRBV1- optional linker -TRBC- linker -VL1- linker -TRAV2- linker -TRBV2- linker -VH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: VHH2- linker -TRBV1- optional linker -TRBC- linker -VL1- linker -TRBV2- linker -TRAV2- linker -VH1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: VHH2- linker -TRBV1- optional linker -TRBC- linker -VL1- linker -TRBV2- linker -TRAV2- linker -VH1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: VHH2- linker -TRBV1- optional linker -TRBC- linker -VH1- linker -TRBV2- linker -TRAV2- linker -VL1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: VHH2- linker -TRBV1- optional linker -TRBC- linker -VH1- linker -TRBV2- linker -TRAV2- linker -VL1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: VHH2- linker -TRBV1- optional linker -TRBC- linker -VH1- linker -TRAV2- linker -TRBV2- linker -VL1- optional linker - optional hinge region -Alb, and the second polypeptide chain comprises, from N-terminus to C-terminus: TRAV1- optional linker -TRAC- optional linker - optional hinge region; or
the first polypeptide chain comprises, from N-terminus to C-terminus: VHH2- linker -TRBV1- optional linker -TRBC- linker -VH1- linker -TRAV2- linker -TRBV2- linker -VL1- optional linker - optional hinge region, and the second polypeptide chain comprises, from N-terminus to C-terminus: TRAV1- optional linker -TRAC- optional linker - optional hinge region -Alb; or
the first polypeptide chain comprises, from N-terminus to C-terminus: VHH2- linker -TRAV1- optional linker -TRAC- linker -VL1- linker -TRBV2- linker -TRAV2- linker -VH1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: VHH2- linker -TRAV1- optional linker -TRAC- linker -VL1- linker -TRBV2- linker -TRAV2- linker -VH1- optional linker - optional hinge region, and the second polypeptide chain comprises, from N-terminus to C-terminus: TRBV1- optional linker - TRBC- optional linker - optional hinge region -Alb; or
the first polypeptide chain comprises, from N-terminus to C-terminus: VHH2- linker -TRAV1- optional linker -TRAC- linker -VL1- linker -TRAV2- linker -TRBV2- linker -VH1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: VHH2- linker -TRAV1- optional linker -TRAC- linker -VL1- linker -TRAV2- linker -TRBV2- linker -VH1- optional linker - optional hinge region, and the second polypeptide chain comprises, from N-terminus to C-terminus: TRBV1- optional linker - TRBC- optional linker - optional hinge region -Alb; or
the first polypeptide chain comprises, from N-terminus to C-terminus: VHH2- linker -TRAV1- optional linker -TRAC- linker -VH1- linker -TRAV2- linker -TRBV2- linker -VL1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: VHH2- linker -TRAV1- optional linker -TRAC- linker -VH1- linker -TRAV2- linker -TRBV2- linker -VL1- optional linker - optional hinge region, and the second polypeptide chain comprises, from N-terminus to C-terminus: TRBV1- optional linker - TRBC- optional linker - optional hinge region -Alb; or
the first polypeptide chain comprises, from N-terminus to C-terminus: VHH2- linker -TRAV1- optional linker -TRAC- linker -VH1- linker -TRBV2- linker -TRAV2- linker -VL1- optional linker - optional hinge region -Alb, and the second polypeptide chain comprises, from N-terminus to C-terminus: TRBV1- optional linker -TRBC- optional linker - optional hinge region; or
the first polypeptide chain comprises, from N-terminus to C-terminus: VHH2- linker -TRAV1- optional linker -TRAC- linker -VH1- linker -TRBV2- linker -TRAV2- linker -VL1- optional linker - optional hinge region, and the second polypeptide chain comprises, from N-terminus to C-terminus: TRBV1- optional linker - TRBC- optional linker - optional hinge region -Alb; or
the first polypeptide chain comprises, from N-terminus to C-terminus: TRBV1- optional linker -TRBC- linker -VL1- linker -TRAV2- linker -TRBV2- linker -VH1- optional linker - optional hinge region -Alb, and the second polypeptide chain comprises, from N-terminus to C-terminus: VHH2- linker -TRAV1- optional linker -TRAC- optional linker - optional hinge region; or
the first polypeptide chain comprises, from N-terminus to C-terminus: TRBV1- optional linker -TRBC- linker -VL1- linker -TRAV2- linker -TRBV2- linker -VH1- optional linker - optional hinge region, and the second polypeptide chain comprises, from N-terminus to C-terminus: VHH2- linker -TRAV1- optional linker - TRAC- optional linker - optional hinge region -Alb; or
the first polypeptide chain comprises, from N-terminus to C-terminus: TRBV1- optional linker -TRBC- linker -VL1- linker -TRBV2- linker -TRAV2- linker -VH1- optional linker - optional hinge region -Alb, and the second polypeptide chain comprises, from N-terminus to C-terminus: VHH2- linker -TRAV1- optional linker -TRAC- optional linker - optional hinge region; or
the first polypeptide chain comprises, from N-terminus to C-terminus: TRBV1- optional linker -TRBC- linker -VL1- linker -TRBV2- linker -TRAV2- linker -VH1- optional linker - optional hinge region, and the second polypeptide chain comprises, from N-terminus to C-terminus: VHH2- linker -TRAV1- optional linker - TRAC- optional linker - optional hinge region -Alb; or
the first polypeptide chain comprises, from N-terminus to C-terminus: TRBV1- optional linker -TRBC- linker -VH1- linker -TRBV2- linker -TRAV2- linker -VL1- optional linker - optional hinge region -Alb, and the second polypeptide chain comprises, from N-terminus to C-terminus: VHH2- linker -TRAV1- optional linker -TRAC- optional linker - optional hinge region; or
the first polypeptide chain comprises, from N-terminus to C-terminus: TRBV1- optional linker -TRBC- linker -VH1- linker -TRBV2- linker -TRAV2- linker -VL1- optional linker - optional hinge region, and the second polypeptide chain comprises, from N-terminus to C-terminus: VHH2- linker -TRAV1- optional linker - TRAC- optional linker - optional hinge region -Alb; or
the first polypeptide chain comprises, from N-terminus to C-terminus: TRBV1- optional linker -TRBC- linker -VH1- linker -TRAV2- linker -TRBV2- linker -VL1- optional linker - optional hinge region -Alb, and the second polypeptide chain comprises, from N-terminus to C-terminus: VHH2- linker -TRAV1- optional linker -TRAC- optional linker - optional hinge region; or
the first polypeptide chain comprises, from N-terminus to C-terminus: TRBV1- optional linker -TRBC- linker -VH1- linker -TRAV2- linker -TRBV2- linker -VL1- optional linker - optional hinge region, and the second polypeptide chain comprises, from N-terminus to C-terminus: VHH2- linker -TRAV1- optional linker - TRAC- optional linker - optional hinge region -Alb; or
the first polypeptide chain comprises, from N-terminus to C-terminus: TRAV1- optional linker -TRAC- linker -VL1- linker -TRBV2- linker -TRAV2- linker -VH1- optional linker - optional hinge region -Alb, and the second polypeptide chain comprises, from N-terminus to C-terminus: VHH2- linker -TRBV1- optional linker -TRBC- optional linker - optional hinge region; or
the first polypeptide chain comprises, from N-terminus to C-terminus: TRAV1- optional linker -TRAC- linker -VL1- linker -TRBV2- linker -TRAV2- linker -VH1- optional linker - optional hinge region, and the second polypeptide chain comprises, from N-terminus to C-terminus: VHH2- linker -TRBV1- optional linker - TRBC- optional linker - optional hinge region -Alb; or
the first polypeptide chain comprises, from N-terminus to C-terminus: TRAV1- optional linker -TRAC- linker -VL1- linker -TRAV2- linker -TRBV2- linker -VH1- optional linker - optional hinge region -Alb, and the second polypeptide chain comprises, from N-terminus to C-terminus: VHH2- linker -TRBV1- optional linker -TRBC- optional linker - optional hinge region; or
the first polypeptide chain comprises, from N-terminus to C-terminus: TRAV1- optional linker -TRAC- linker -VL1- linker -TRAV2- linker -TRBV2- linker -VH1- optional linker - optional hinge region, and the second polypeptide chain comprises, from N-terminus to C-terminus: VHH2- linker -TRBV1- optional linker - TRBC- optional linker - optional hinge region -Alb; or
the first polypeptide chain comprises, from N-terminus to C-terminus: TRAV1- optional linker -TRAC- linker -VH1- linker -TRAV2- linker -TRBV2- linker -VL1- optional linker - optional hinge region -Alb, and the second polypeptide chain comprises, from N-terminus to C-terminus: VHH2- linker -TRBV1- optional linker -TRBC- optional linker - optional hinge region; or
the first polypeptide chain comprises, from N-terminus to C-terminus: TRAV1- optional linker -TRAC- linker -VH1- linker -TRAV2- linker -TRBV2- linker -VL1- optional linker - optional hinge region, and the second polypeptide chain comprises, from N-terminus to C-terminus: VHH2- linker -TRBV1- optional linker - TRBC- optional linker - optional hinge region -Alb; or
the first polypeptide chain comprises, from N-terminus to C-terminus: TRAV1- optional linker -TRAC- linker -VH1- linker -TRBV2- linker -TRAV2- linker -VL1- optional linker - optional hinge region -Alb, and the second polypeptide chain comprises, from N-terminus to C-terminus: VHH2- linker -TRBV1- optional linker -TRBC- optional linker - optional hinge region; or
the first polypeptide chain comprises, from N-terminus to C-terminus: TRAV1- optional linker -TRAC- linker -VH1- linker -TRBV2- linker -TRAV2- linker -VL1- optional linker - optional hinge region, and the second polypeptide chain comprises, from N-terminus to C-terminus: VHH2- linker -TRBV1- optional linker - TRBC- optional linker - optional hinge region -Alb; or
the first polypeptide chain comprises, from N-terminus to C-terminus: VL2- linker -TRBV1- optional linker -TRBC- linker -VL1- linker -TRAV2- linker -TRBV2- linker -VH1- optional linker - optional hinge region -Alb, and the second polypeptide chain comprises, from N-terminus to C-terminus: VH2- linker - TRAV1- optional linker -TRAC- optional linker - optional hinge region; or
the first polypeptide chain comprises, from N-terminus to C-terminus: VL2- linker -TRBV1- optional linker -TRBC- linker -VL1- linker -TRAV2- linker -TRBV2- linker -VH1- optional linker - optional hinge region, and the second polypeptide chain comprises, from N-terminus to C-terminus: VH2- linker -TRAV1-optional linker -TRAC- optional linker - optional hinge region -Alb; or
the first polypeptide chain comprises, from N-terminus to C-terminus: VL2- linker -TRBV1- optional linker -TRBC- linker -VL1- linker -TRBV2- linker -TRAV2- linker -VH1- optional linker - optional hinge region -Alb, and the second polypeptide chain comprises, from N-terminus to C-terminus: VH2- linker - TRAV1- optional linker -TRAC- optional linker - optional hinge region; or
the first polypeptide chain comprises, from N-terminus to C-terminus: VL2- linker -TRBV1- optional linker -TRBC- linker -VL1- linker -TRBV2- linker -TRAV2- linker -VH1- optional linker - optional hinge region, and the second polypeptide chain comprises, from N-terminus to C-terminus: VH2- linker -TRAV1-optional linker -TRAC- optional linker - optional hinge region -Alb; or
the first polypeptide chain comprises, from N-terminus to C-terminus: VH2- linker -TRBV1- optional linker -TRBC- linker -VL1- linker -TRAV2- linker -TRBV2- linker -VH1- optional linker - optional hinge region -Alb, and the second polypeptide chain comprises, from N-terminus to C-terminus: VL2- linker - TRAV1- optional linker -TRAC- optional linker - optional hinge region; or
the first polypeptide chain comprises, from N-terminus to C-terminus: VH2- linker -TRBV1- optional linker -TRBC- linker -VL1- linker -TRAV2- linker -TRBV2- linker -VH1- optional linker - optional hinge region, and the second polypeptide chain comprises, from N-terminus to C-terminus: VL2- linker -TRAV1-optional linker -TRAC- optional linker - optional hinge region -Alb; or
the first polypeptide chain comprises, from N-terminus to C-terminus: VH2- linker -TRBV1- optional linker -TRBC- linker -VL1- linker -TRBV2- linker -TRAV2- linker -VH1- optional linker - optional hinge region -Alb, and the second polypeptide chain comprises, from N-terminus to C-terminus: VL2- linker - TRAV1- optional linker -TRAC- optional linker - optional hinge region; or
the first polypeptide chain comprises, from N-terminus to C-terminus: VH2- linker -TRBV1- optional linker -TRBC- linker -VL1- linker -TRBV2- linker -TRAV2- linker -VH1- optional linker - optional hinge region, and the second polypeptide chain comprises, from N-terminus to C-terminus: VL2- linker -TRAV1-optional linker -TRAC- optional linker - optional hinge region -Alb; or
the first polypeptide chain comprises, from N-terminus to C-terminus: VL2- linker -TRBV1- optional linker -TRBC- linker -VH1- linker -TRBV2- linker -TRAV2- linker -VL1- optional linker - optional hinge region -Alb, and the second polypeptide chain comprises, from N-terminus to C-terminus: VH2- linker - TRAV1- optional linker -TRAC- optional linker - optional hinge region; or
the first polypeptide chain comprises, from N-terminus to C-terminus: VL2- linker -TRBV1- optional linker -TRBC- linker -VH1- linker -TRBV2- linker -TRAV2- linker -VL1- optional linker - optional hinge region, and the second polypeptide chain comprises, from N-terminus to C-terminus: VH2- linker -TRAV1-optional linker -TRAC- optional linker - optional hinge region -Alb; or
the first polypeptide chain comprises, from N-terminus to C-terminus: VL2- linker -TRBV1- optional linker -TRBC- linker -VH1- linker -TRAV2- linker -TRBV2- linker -VL1- optional linker - optional hinge region -Alb, and the second polypeptide chain comprises, from N-terminus to C-terminus: VH2- linker - TRAV1- optional linker -TRAC- optional linker - optional hinge region; or
the first polypeptide chain comprises, from N-terminus to C-terminus: VL2- linker -TRBV1- optional linker -TRBC- linker -VH1- linker -TRAV2- linker -TRBV2- linker -VL1- optional linker - optional hinge region, and the second polypeptide chain comprises, from N-terminus to C-terminus: VH2- linker -TRAV1-optional linker -TRAC- optional linker - optional hinge region -Alb; or
the first polypeptide chain comprises, from N-terminus to C-terminus: VH2- linker -TRBV1- optional linker -TRBC- linker -VH1- linker -TRBV2- linker -TRAV2- linker -VL1- optional linker - optional hinge region -Alb, and the second polypeptide chain comprises, from N-terminus to C-terminus: VL2- linker - TRAV1- optional linker -TRAC- optional linker - optional hinge region; or
the first polypeptide chain comprises, from N-terminus to C-terminus: VH2- linker -TRBV1- optional linker -TRBC- linker -VH1- linker -TRBV2- linker -TRAV2- linker -VL1- optional linker - optional hinge region, and the second polypeptide chain comprises, from N-terminus to C-terminus: VL2- linker -TRAV1-optional linker -TRAC- optional linker - optional hinge region -Alb; or
the first polypeptide chain comprises, from N-terminus to C-terminus: VH2- linker -TRBV1- linker - optional linker -TRBC- linker -VH1- linker -TRAV2- linker -TRBV2- linker -VL1- optional linker - optional hinge region -Alb, and the second polypeptide chain comprises, from N-terminus to C-terminus: VL2- linker - TRAV1- linker - optional linker -TRAC- optional linker - optional hinge region; or
the first polypeptide chain comprises, from N-terminus to C-terminus: VH2- linker -TRBV1- linker - optional linker -TRBC- linker -VH1- linker -TRAV2- linker -TRBV2- linker -VL1- optional linker - optional hinge region, and the second polypeptide chain comprises, from N-terminus to C-terminus: VL2- linker - TRAV1- linker - optional linker -TRAC- optional linker - optional hinge region -Alb; or
the first polypeptide chain comprises, from N-terminus to C-terminus: VL2- linker -TRAV1- optional linker -TRAC- linker -VL1- linker -TRBV2- linker -TRAV2- linker -VH1- optional linker - optional hinge region -Alb, and the second polypeptide chain comprises, from N-terminus to C-terminus: VH2- linker - TRBV1- optional linker -TRBC- optional linker - optional hinge region; or
the first polypeptide chain comprises, from N-terminus to C-terminus: VL2- linker -TRAV1- optional linker -TRAC- linker -VL1- linker -TRBV2- linker -TRAV2- linker -VH1- optional linker - optional hinge region, and the second polypeptide chain comprises, from N-terminus to C-terminus: VH2- linker -TRBV1-optional linker -TRBC- optional linker - optional hinge region -Alb; or
the first polypeptide chain comprises, from N-terminus to C-terminus: VL2- linker -TRAV1- optional linker -TRAC- linker -VL1- linker -TRAV2- linker -TRBV2- linker -VH1- optional linker - optional hinge region -Alb, and the second polypeptide chain comprises, from N-terminus to C-terminus: VH2- linker - TRBV1- optional linker -TRBC- optional linker - optional hinge region; or
the first polypeptide chain comprises, from N-terminus to C-terminus: VL2- linker -TRAV1- optional linker -TRAC- linker -VL1- linker -TRAV2- linker -TRBV2- linker -VH1- optional linker - optional hinge region, and the second polypeptide chain comprises, from N-terminus to C-terminus: VH2- linker -TRBV1-optional linker -TRBC- optional linker - optional hinge region -Alb; or
the first polypeptide chain comprises, from N-terminus to C-terminus: VH2- linker -TRAV1- optional linker -TRAC- linker -VL1- linker -TRBV2- linker -TRAV2- linker -VH1- optional linker - optional hinge region -Alb, and the second polypeptide chain comprises, from N-terminus to C-terminus: VL2- linker - TRBV1- optional linker -TRBC- optional linker - optional hinge region; or
the first polypeptide chain comprises, from N-terminus to C-terminus: VH2- linker -TRAV1- optional linker -TRAC- linker -VL1- linker -TRBV2- linker -TRAV2- linker -VH1- optional linker - optional hinge region, and the second polypeptide chain comprises, from N-terminus to C-terminus: VL2- linker -TRBV1-optional linker -TRBC- optional linker - optional hinge region -Alb; or
the first polypeptide chain comprises, from N-terminus to C-terminus: VH2- linker -TRAV1- optional linker -TRAC- linker -VL1- linker -TRAV2- linker -TRBV2- linker -VH1- optional linker - optional hinge region -Alb, and the second polypeptide chain comprises, from N-terminus to C-terminus: VL2- linker - TRBV1- optional linker -TRBC- optional linker - optional hinge region; or
the first polypeptide chain comprises, from N-terminus to C-terminus: VH2- linker -TRAV1- optional linker -TRAC- linker -VL1- linker -TRAV2- linker -TRBV2- linker -VH1- optional linker - optional hinge region, and the second polypeptide chain comprises, from N-terminus to C-terminus: VL2- linker -TRBV1-optional linker -TRBC- optional linker - optional hinge region -Alb; or
the first polypeptide chain comprises, from N-terminus to C-terminus: VL2- linker -TRAV1- optional linker -TRAC- linker -VH1- linker -TRAV2- linker -TRBV2- linker -VL1- optional linker - optional hinge region -Alb, and the second polypeptide chain comprises, from N-terminus to C-terminus: VH2- linker - TRBV1- optional linker -TRBC- optional linker - optional hinge region; or
the first polypeptide chain comprises, from N-terminus to C-terminus: VL2- linker -TRAV1- optional linker -TRAC- linker -VH1- linker -TRAV2- linker -TRBV2- linker -VL1- optional linker - optional hinge region, and the second polypeptide chain comprises, from N-terminus to C-terminus: VH2- linker -TRBV1-optional linker -TRBC- optional linker - optional hinge region -Alb; or
the first polypeptide chain comprises, from N-terminus to C-terminus: VL2- linker -TRAV1- optional linker -TRAC- linker -VH1- linker -TRBV2- linker -TRAV2- linker -VL1- optional linker - optional hinge region -Alb, and the second polypeptide chain comprises, from N-terminus to C-terminus: VH2- linker - TRBV1- optional linker -TRBC- optional linker - optional hinge region; or
the first polypeptide chain comprises, from N-terminus to C-terminus: VL2- linker -TRAV1- optional linker -TRAC- linker -VH1- linker -TRBV2- linker -TRAV2- linker -VL1- optional linker - optional hinge region, and the second polypeptide chain comprises, from N-terminus to C-terminus: VH2- linker -TRBV1-optional linker -TRBC- optional linker - optional hinge region -Alb; or
the first polypeptide chain comprises, from N-terminus to C-terminus: VH2- linker -TRAV1- optional linker -TRAC- linker -VH1- linker -TRAV2- linker -TRBV2- linker -VL1- optional linker - optional hinge region -Alb, and the second polypeptide chain comprises, from N-terminus to C-terminus: VL2- linker - TRBV1- optional linker -TRBC- optional linker - optional hinge region; or
the first polypeptide chain comprises, from N-terminus to C-terminus: VH2- linker -TRAV1- optional linker -TRAC- linker -VH1- linker -TRAV2- linker -TRBV2- linker -VL1- optional linker - optional hinge region, and the second polypeptide chain comprises, from N-terminus to C-terminus: VL2- linker -TRBV1-optional linker -TRBC- optional linker - optional hinge region -Alb; or
the first polypeptide chain comprises, from N-terminus to C-terminus: VH2- linker -TRAV1- optional linker -TRAC- linker -VH1- linker -TRBV2- linker -TRAV2- linker -VL1- optional linker - optional hinge region -Alb, and the second polypeptide chain comprises, from N-terminus to C-terminus: VL2- linker - TRBV1- optional linker -TRBC- optional linker - optional hinge region; or
the first polypeptide chain comprises, from N-terminus to C-terminus: VH2- linker -TRAV1- optional linker -TRAC- linker -VH1- linker -TRBV2- linker -TRAV2- linker -VL1- optional linker - optional hinge region, and the second polypeptide chain comprises, from N-terminus to C-terminus: VL2- linker -TRBV1-optional linker -TRBC- optional linker - optional hinge region -Alb.

In some embodiments, the multi-specific polypeptide molecule comprises a first binding region, a second binding region, a third binding region, a fourth binding region and a functional domain comprising an antibody-derived Fc domain or a dimerizing portion thereof and an optional antibody hinge region, and the multi-specific polypeptide molecule comprises a first polypeptide chain and a second polypeptide chain; wherein:
the first polypeptide chain comprises, from N-terminus to C-terminus: scFv2- linker -TRBV1- optional linker -TRBC- linker -VL1- linker -TRAV2- linker -TRBV2- linker -VH1- optional linker - optional hinge region -CH2-CH3, and the second polypeptide chain comprises, from N-terminus to C-terminus: TRAV1-optional linker -TRAC- optional linker - optional hinge region -CH2-CH3; or
the first polypeptide chain comprises, from N-terminus to C-terminus: scFv2- linker -TRBV1- optional linker -TRBC- linker -VL1- linker -TRBV2- linker -TRAV2- linker -VH1- optional linker - optional hinge region -CH2-CH3, and the second polypeptide chain comprises, from N-terminus to C-terminus: TRAV1-optional linker -TRAC- optional linker - optional hinge region -CH2-CH3; or
the first polypeptide chain comprises, from N-terminus to C-terminus: scFv2- linker -TRBV1- optional linker -TRBC- linker -VH1- linker -TRBV2- linker -TRAV2- linker -VL1- optional linker - optional hinge region -CH2-CH3, and the second polypeptide chain comprises, from N-terminus to C-terminus: TRAV1-optional linker -TRAC- optional linker - optional hinge region -CH2-CH3; or
the first polypeptide chain comprises, from N-terminus to C-terminus: scFv2- linker -TRBV1- optional linker -TRBC- linker -VH1- linker -TRAV2- linker -TRBV2- linker -VL1- optional linker - optional hinge region -CH2-CH3, and the second polypeptide chain comprises, from N-terminus to C-terminus: TRAV1-optional linker -TRAC- optional linker - optional hinge region -CH2-CH3; or
the first polypeptide chain comprises, from N-terminus to C-terminus: scFv2- linker -TRAV1- optional linker -TRAC- linker -VL1- linker -TRBV2- linker -TRAV2- linker -VH1- optional linker - optional hinge region -CH2-CH3, and the second polypeptide chain comprises, from N-terminus to C-terminus: TRBV1-optional linker -TRBC- optional linker - optional hinge region -CH2-CH3; or
the first polypeptide chain comprises, from N-terminus to C-terminus: scFv2- linker -TRAV1- optional linker -TRAC- linker -VL1- linker -TRAV2- linker -TRBV2- linker -VH1- optional linker - optional hinge region -CH2-CH3, and the second polypeptide chain comprises, from N-terminus to C-terminus: TRBV1-optional linker -TRBC- optional linker - optional hinge region -CH2-CH3; or
the first polypeptide chain comprises, from N-terminus to C-terminus: scFv2- linker -TRAV1- optional linker -TRAC- linker -VH1- linker -TRAV2- linker -TRBV2- linker -VL1- optional linker - optional hinge region -CH2-CH3, and the second polypeptide chain comprises, from N-terminus to C-terminus: TRBV1-optional linker -TRBC- optional linker - optional hinge region -CH2-CH3; or
the first polypeptide chain comprises, from N-terminus to C-terminus: scFv2- linker -TRAV1- optional linker -TRAC- linker -VH1- linker -TRBV2- linker -TRAV2- linker -VL1- optional linker - optional hinge region -CH2-CH3, and the second polypeptide chain comprises, from N-terminus to C-terminus: TRBV1-optional linker -TRBC- optional linker - optional hinge region -CH2-CH3; or
the first polypeptide chain comprises, from N-terminus to C-terminus: TRBV1- optional linker -TRBC- linker -VL1- linker -TRAV2- linker -TRBV2- linker -VH1- optional linker - optional hinge region -CH2-CH3, and the second polypeptide chain comprises, from N-terminus to C-terminus: scFv2- linker -TRAV1- optional linker -TRAC- optional linker - optional hinge region -CH2-CH3; or
the first polypeptide chain comprises, from N-terminus to C-terminus: TRBV1- optional linker -TRBC- linker -VL1- linker -TRBV2- linker -TRAV2- linker -VH1- optional linker - optional hinge region -CH2-CH3, and the second polypeptide chain comprises, from N-terminus to C-terminus: scFv2- linker -TRAV1- optional linker -TRAC- optional linker - optional hinge region -CH2-CH3; or
the first polypeptide chain comprises, from N-terminus to C-terminus: TRBV1- optional linker -TRBC- linker -VH1- linker -TRBV2- linker -TRAV2- linker -VL1- optional linker - optional hinge region -CH2-CH3, and the second polypeptide chain comprises, from N-terminus to C-terminus: scFv2- linker -TRAV1- optional linker -TRAC- optional linker - optional hinge region -CH2-CH3; or
the first polypeptide chain comprises, from N-terminus to C-terminus: TRBV1- optional linker -TRBC- linker -VH1- linker -TRAV2- linker -TRBV2- linker -VL1- optional linker - optional hinge region -CH2-CH3, and the second polypeptide chain comprises, from N-terminus to C-terminus: scFv2- linker -TRAV1- optional linker -TRAC- optional linker - optional hinge region -CH2-CH3; or
the first polypeptide chain comprises, from N-terminus to C-terminus: TRAV1- optional linker -TRAC- linker -VL1- linker -TRBV2- linker -TRAV2- linker -VH1- optional linker - optional hinge region -CH2-CH3, and the second polypeptide chain comprises, from N-terminus to C-terminus: scFv2- linker -TRBV1- optional linker -TRBC- optional linker - optional hinge region -CH2-CH3; or
the first polypeptide chain comprises, from N-terminus to C-terminus: TRAV1- optional linker -TRAC- linker -VL1- linker -TRAV2- linker -TRBV2- linker -VH1- optional linker - optional hinge region -CH2-CH3, and the second polypeptide chain comprises, from N-terminus to C-terminus: scFv2- linker -TRBV1- optional linker -TRBC- optional linker - optional hinge region -CH2-CH3; or
the first polypeptide chain comprises, from N-terminus to C-terminus: TRAV1- optional linker -TRAC- linker -VH1- linker -TRAV2- linker -TRBV2- linker -VL1- optional linker - optional hinge region -CH2-CH3, and the second polypeptide chain comprises, from N-terminus to C-terminus: scFv2- linker -TRBV1- optional linker -TRBC- optional linker - optional hinge region -CH2-CH3; or
the first polypeptide chain comprises, from N-terminus to C-terminus: TRAV1- optional linker -TRAC- linker -VH1- linker -TRBV2- linker -TRAV2- linker -VL1- optional linker - optional hinge region -CH2-CH3, and the second polypeptide chain comprises, from N-terminus to C-terminus: scFv2- linker -TRBV1- optional linker -TRBC- optional linker - optional hinge region -CH2-CH3; or
the first polypeptide chain comprises, from N-terminus to C-terminus: VHH2- linker -TRBV1- optional linker -TRBC- linker -VL1- linker -TRAV2- linker -TRBV2- linker -VH1- optional linker - optional hinge region -CH2-CH3, and the second polypeptide chain comprises, from N-terminus to C-terminus: TRAV1-optional linker -TRAC- optional linker - optional hinge region -CH2-CH3; or
the first polypeptide chain comprises, from N-terminus to C-terminus: VHH2- linker -TRBV1- optional linker -TRBC- linker -VL1- linker -TRBV2- linker -TRAV2- linker -VH1- optional linker - optional hinge region -CH2-CH3, and the second polypeptide chain comprises, from N-terminus to C-terminus: TRAV1-optional linker -TRAC- optional linker - optional hinge region -CH2-CH3; or
the first polypeptide chain comprises, from N-terminus to C-terminus: VHH2- linker -TRBV1- optional linker -TRBC- linker -VH1- linker -TRBV2- linker -TRAV2- linker -VL1- optional linker - optional hinge region -CH2-CH3, and the second polypeptide chain comprises, from N-terminus to C-terminus: TRAV1-optional linker -TRAC- optional linker - optional hinge region -CH2-CH3; or
the first polypeptide chain comprises, from N-terminus to C-terminus: VHH2- linker -TRBV1- optional linker -TRBC- linker -VH1- linker -TRAV2- linker -TRBV2- linker -VL1- optional linker - optional hinge region -CH2-CH3, and the second polypeptide chain comprises, from N-terminus to C-terminus: TRAV1-optional linker -TRAC- optional linker - optional hinge region -CH2-CH3; or
the first polypeptide chain comprises, from N-terminus to C-terminus: VHH2- linker -TRAV1- optional linker -TRAC- linker -VL1- linker -TRBV2- linker -TRAV2- linker -VH1- optional linker - optional hinge region -CH2-CH3, and the second polypeptide chain comprises, from N-terminus to C-terminus: TRBV1-optional linker -TRBC- optional linker - optional hinge region -CH2-CH3; or
the first polypeptide chain comprises, from N-terminus to C-terminus: VHH2- linker -TRAV1- optional linker -TRAC- linker -VL1- linker -TRAV2- linker -TRBV2- linker -VH1- optional linker - optional hinge region -CH2-CH3, and the second polypeptide chain comprises, from N-terminus to C-terminus: TRBV1-optional linker -TRBC- optional linker - optional hinge region -CH2-CH3; or
the first polypeptide chain comprises, from N-terminus to C-terminus: VHH2- linker -TRAV1- optional linker -TRAC- linker -VH1- linker -TRAV2- linker -TRBV2- linker -VL1- optional linker - optional hinge region -CH2-CH3, and the second polypeptide chain comprises, from N-terminus to C-terminus: TRBV1-optional linker -TRBC- optional linker - optional hinge region -CH2-CH3; or
the first polypeptide chain comprises, from N-terminus to C-terminus: VHH2- linker -TRAV1- optional linker -TRAC- linker -VH1- linker -TRBV2- linker -TRAV2- linker -VL1- optional linker - optional hinge region -CH2-CH3, and the second polypeptide chain comprises, from N-terminus to C-terminus: TRBV1-optional linker -TRBC- optional linker - optional hinge region -CH2-CH3; or
the first polypeptide chain comprises, from N-terminus to C-terminus: TRBV1- optional linker -TRBC- linker -VL1- linker -TRAV2- linker -TRBV2- linker -VH1- optional linker - optional hinge region -CH2-CH3, and the second polypeptide chain comprises, from N-terminus to C-terminus: VHH2- linker -TRAV1- optional linker -TRAC- optional linker - optional hinge region -CH2-CH3; or
the first polypeptide chain comprises, from N-terminus to C-terminus: TRBV1- optional linker -TRBC- linker -VL1- linker -TRBV2- linker -TRAV2- linker -VH1- optional linker - optional hinge region -CH2-CH3, and the second polypeptide chain comprises, from N-terminus to C-terminus: VHH2- linker -TRAV1- optional linker -TRAC- optional linker - optional hinge region -CH2-CH3; or
the first polypeptide chain comprises, from N-terminus to C-terminus: TRBV1- optional linker -TRBC- linker -VH1- linker -TRBV2- linker -TRAV2- linker -VL1- optional linker - optional hinge region -CH2-CH3, and the second polypeptide chain comprises, from N-terminus to C-terminus: VHH2- linker -TRAV1- optional linker -TRAC- optional linker - optional hinge region -CH2-CH3; or
the first polypeptide chain comprises, from N-terminus to C-terminus: TRBV1- optional linker -TRBC- linker -VH1- linker -TRAV2- linker -TRBV2- linker -VL1- optional linker - optional hinge region -CH2-CH3, and the second polypeptide chain comprises, from N-terminus to C-terminus: VHH2- linker -TRAV1- optional linker -TRAC- optional linker - optional hinge region -CH2-CH3; or
the first polypeptide chain comprises, from N-terminus to C-terminus: TRAV1- optional linker -TRAC- linker -VL1- linker -TRBV2- linker -TRAV2- linker -VH1- optional linker - optional hinge region -CH2-CH3, and the second polypeptide chain comprises, from N-terminus to C-terminus: VHH2- linker -TRBV1- optional linker -TRBC- optional linker - optional hinge region -CH2-CH3; or
the first polypeptide chain comprises, from N-terminus to C-terminus: TRAV1- optional linker -TRAC- linker -VL1- linker -TRAV2- linker -TRBV2- linker -VH1- optional linker - optional hinge region -CH2-CH3, and the second polypeptide chain comprises, from N-terminus to C-terminus: VHH2- linker -TRBV1- optional linker -TRBC- optional linker - optional hinge region -CH2-CH3; or
the first polypeptide chain comprises, from N-terminus to C-terminus: TRAV1- optional linker -TRAC- linker -VH1- linker -TRAV2- linker -TRBV2- linker -VL1- optional linker - optional hinge region -CH2-CH3, and the second polypeptide chain comprises, from N-terminus to C-terminus: VHH2- linker -TRBV1- optional linker -TRBC- optional linker - optional hinge region -CH2-CH3; or
the first polypeptide chain comprises, from N-terminus to C-terminus: TRAV1- optional linker -TRAC- linker -VH1- linker -TRBV2- linker -TRAV2- linker -VL1- optional linker - optional hinge region -CH2-CH3, and the second polypeptide chain comprises, from N-terminus to C-terminus: VHH2- linker -TRBV1- optional linker -TRBC- optional linker - optional hinge region -CH2-CH3; or
the first polypeptide chain comprises, from N-terminus to C-terminus: VL2- linker -TRBV1- optional linker -TRBC- linker -VL1- linker -TRAV2- linker -TRBV2- linker -VH1- optional linker - optional hinge region -CH2-CH3, and the second polypeptide chain comprises, from N-terminus to C-terminus: VH2- linker - TRAV1- optional linker -TRAC- optional linker - optional hinge region -CH2-CH3; or
the first polypeptide chain comprises, from N-terminus to C-terminus: VL2- linker -TRBV1- optional linker -TRBC- linker -VL1- linker -TRBV2- linker -TRAV2- linker -VH1- optional linker - optional hinge region -CH2-CH3, and the second polypeptide chain comprises, from N-terminus to C-terminus: VH2- linker - TRAV1- optional linker -TRAC- optional linker - optional hinge region -CH2-CH3; or
the first polypeptide chain comprises, from N-terminus to C-terminus: VH2- linker -TRBV1- optional linker -TRBC- linker -VL1- linker -TRAV2- linker -TRBV2- linker -VH1- optional linker - optional hinge region -CH2-CH3, and the second polypeptide chain comprises, from N-terminus to C-terminus: VL2- linker - TRAV1- optional linker -TRAC- optional linker - optional hinge region -CH2-CH3; or
the first polypeptide chain comprises, from N-terminus to C-terminus: VH2- linker -TRBV1- optional linker -TRBC- linker -VL1- linker -TRBV2- linker -TRAV2- linker -VH1- optional linker - optional hinge region -CH2-CH3, and the second polypeptide chain comprises, from N-terminus to C-terminus: VL2- linker - TRAV1- optional linker -TRAC- optional linker - optional hinge region -CH2-CH3; or
the first polypeptide chain comprises, from N-terminus to C-terminus: VL2- linker -TRBV1- optional linker -TRBC- linker -VH1- linker -TRBV2- linker -TRAV2- linker -VL1- optional linker - optional hinge region -CH2-CH3, and the second polypeptide chain comprises, from N-terminus to C-terminus: VH2- linker - TRAV1- optional linker -TRAC- optional linker - optional hinge region -CH2-CH3; or
the first polypeptide chain comprises, from N-terminus to C-terminus: VL2- linker -TRBV1- optional linker -TRBC- linker -VH1- linker -TRAV2- linker -TRBV2- linker -VL1- optional linker - optional hinge region -CH2-CH3, and the second polypeptide chain comprises, from N-terminus to C-terminus: VH2- linker - TRAV1- optional linker -TRAC- optional linker - optional hinge region -CH2-CH3; or
the first polypeptide chain comprises, from N-terminus to C-terminus: VH2- linker -TRBV1- optional linker -TRBC- linker -VH1- linker -TRBV2- linker -TRAV2- linker -VL1- optional linker - optional hinge region -CH2-CH3, and the second polypeptide chain comprises, from N-terminus to C-terminus: VL2- linker - TRAV1- optional linker -TRAC- optional linker - optional hinge region -CH2-CH3; or
the first polypeptide chain comprises, from N-terminus to C-terminus: VH2- linker -TRBV1- linker - optional linker -TRBC- linker -VH1- linker -TRAV2- linker -TRBV2- linker -VL1- optional linker - optional hinge region -CH2-CH3, and the second polypeptide chain comprises, from N-terminus to C-terminus: VL2-linker -TRAV1- linker - optional linker -TRAC- optional linker - optional hinge region -CH2-CH3; or
the first polypeptide chain comprises, from N-terminus to C-terminus: VL2- linker -TRAV1- optional linker -TRAC- linker -VL1- linker -TRBV2- linker -TRAV2- linker -VH1- optional linker - optional hinge region -CH2-CH3, and the second polypeptide chain comprises, from N-terminus to C-terminus: VH2- linker - TRBV1- optional linker -TRBC- optional linker - optional hinge region -CH2-CH3; or
the first polypeptide chain comprises, from N-terminus to C-terminus: VL2- linker -TRAV1- optional linker -TRAC- linker -VL1- linker -TRAV2- linker -TRBV2- linker -VH1- optional linker - optional hinge region -CH2-CH3, and the second polypeptide chain comprises, from N-terminus to C-terminus: VH2- linker - TRBV1- optional linker -TRBC- optional linker - optional hinge region -CH2-CH3; or
the first polypeptide chain comprises, from N-terminus to C-terminus: VH2- linker -TRAV1- optional linker -TRAC- linker -VL1- linker -TRBV2- linker -TRAV2- linker -VH1- optional linker - optional hinge region -CH2-CH3, and the second polypeptide chain comprises, from N-terminus to C-terminus: VL2- linker - TRBV1- optional linker -TRBC- optional linker - optional hinge region -CH2-CH3; or
the first polypeptide chain comprises, from N-terminus to C-terminus: VH2- linker -TRAV1- optional linker -TRAC- linker -VL1- linker -TRAV2- linker -TRBV2- linker -VH1- optional linker - optional hinge region -CH2-CH3, and the second polypeptide chain comprises, from N-terminus to C-terminus: VL2- linker - TRBV1- optional linker -TRBC- optional linker - optional hinge region -CH2-CH3; or
the first polypeptide chain comprises, from N-terminus to C-terminus: VL2- linker -TRAV1- optional linker -TRAC- linker -VH1- linker -TRAV2- linker -TRBV2- linker -VL1- optional linker - optional hinge region -CH2-CH3, and the second polypeptide chain comprises, from N-terminus to C-terminus: VH2- linker - TRBV1- optional linker -TRBC- optional linker - optional hinge region -CH2-CH3; or
the first polypeptide chain comprises, from N-terminus to C-terminus: VL2- linker -TRAV1- optional linker -TRAC- linker -VH1- linker -TRBV2- linker -TRAV2- linker -VL1- optional linker - optional hinge region -CH2-CH3, and the second polypeptide chain comprises, from N-terminus to C-terminus: VH2- linker - TRBV1- optional linker -TRBC- optional linker - optional hinge region -CH2-CH3; or
the first polypeptide chain comprises, from N-terminus to C-terminus: VH2- linker -TRAV1- optional linker -TRAC- linker -VH1- linker -TRAV2- linker -TRBV2- linker -VL1- optional linker - optional hinge region -CH2-CH3, and the second polypeptide chain comprises, from N-terminus to C-terminus: VL2- linker - TRBV1- optional linker -TRBC- optional linker - optional hinge region -CH2-CH3; or
the first polypeptide chain comprises, from N-terminus to C-terminus: VH2- linker -TRAV1- optional linker -TRAC- linker -VH1- linker -TRBV2- linker -TRAV2- linker -VL1- optional linker - optional hinge region -CH2-CH3, and the second polypeptide chain comprises, from N-terminus to C-terminus: VL2- linker - TRBV1- optional linker -TRBC- optional linker - optional hinge region -CH2-CH3.

In some embodiments, the scFv1 consists of, from the N-terminus to the C-terminus: VH1-linker-VL1 or VL1-linker-VH1.

In some embodiments, the scFv2 consists of, from the N-terminus to the C-terminus: VH2-linker-VL2 or VL2-linker-VH2.

In some embodiments, the multi-specific polypeptide molecule comprises CH3 domains connected to the C-terminus of TRAV1 and TRBV1, respectively, via an optional linker. In such case, TRAC and TRBC connected to the C- terminus of TRAV1 and TRBV1, respectively, in the first polypeptide chain and the second polypeptide chain of the above multi-specific polypeptide molecule are both replaced with CH3.

In some embodiments, the multi-specific polypeptide molecule comprises CL and CH1 connected to the C-terminus of TRAV1 and TRBV1 via an optional linker. In some embodiments, CL is connected to the C-terminus of TRAV1, and CH1 is connected to the C-terminus of TRBV1. In some embodiments, CH1 is connected to the C-terminus of TRAV1, and CL is connected to the C-terminus of TRBV1. In such case, TRAC and TRBC connected to the C-terminus of TRAV1 and TRBV1, respectively, in the first polypeptide chain and the second polypeptide chain of the above multi-specific polypeptide molecule are replaced with CL and CH1, respectively, or replaced with CH1 and CL, respectively.

In some embodiments, the multi-specific polypeptide molecule does not comprise a constant region connected to the C-terminus of TRAV1 and/or TRBV1 via an optional linker, e.g., a TCR constant region or a fragment thereof, CH3, or CL and CH1. In such case, TRAC and TRBC connected to the C-terminus of TRAV1 and TRBV1, respectively, in the first polypeptide chain and the second polypeptide chain of the above multi-specific polypeptide molecule are removed, respectively. For example, "the first polypeptide chain comprises, from the N-terminus to the C-terminus: TRBV1-optional linker-TRBC-linker-scFv1-optional linker-optional hinge region-CH2-CH3, and the second polypeptide chain comprises, from the N-terminus to the C-terminus: TRAV1-optional linker-TRAC-optional linker-optional hinge region-CH2-CH3" is adjusted to "the first polypeptide chain comprises, from the N-terminus to the C-terminus: TRBV1-linker-scFv1-optional linker-optional hinge region-CH2-CH3, and the second polypeptide chain comprises, from the N-terminus to the C-terminus: TRAV1-optional linker-optional hinge region-CH2-CH3".

In embodiments of the multi-specific polypeptide molecule of the second aspect of the present disclosure, the multi-specific polypeptide molecule further comprises antibody-derived CH1 and antibody-derived CL, which are respectively connected to the VH1 and VL1, wherein the VH1, VL1, CH1 and CL form a single-chain Fab and are connected to the C-terminus of the TRAV1 or the TRBV1, via a linker. In some embodiments, the two polypeptide chains of the multi-specific polypeptide molecule respectively comprise:
TRBV1-VL1-CL-VH1-CH1 and TRAV1; or
TRBV1-VH1-CH1-VL1-CL and TRAV1; or
TRAV1-VL1-CL-VH1-CH1 and TRBV1; or
TRAV1-VH1-CH1-VL1-CL and TRBV1.

In embodiments of the multi-specific polypeptide molecule of the second aspect of the present disclosure, the multi-specific polypeptide molecule further comprises a fourth binding region that binds a fourth antigen, wherein the fourth binding region comprises a heavy chain variable region VH2 and a light chain variable region VL2 derived from an antibody that binds the fourth antigen, and antibody-derived CH1 and antibody-derived CL.

In some embodiments, the VH2, VL2, CH1 and CL form a single-chain Fab2 which is connected to the N-terminus of the TRAV1 or the TRBV1. In some embodiments, the two polypeptide chains of the multi-specific polypeptide molecule respectively comprise:
Fab2-TRBV1-scFv1 and TRAV1; or
Fab2-TRAV1-scFv1 and TRBV1; or
Fab2-TRBV1-VHH1 and TRAV1; or
Fab2-TRAV1-VHH1 and TRBV1; or
Fab2-TRBV1-VL1 and TRAV1-VH1; or
Fab2-TRAV1-VL1 and TRBV1-VH1; or
TRBV1-scFv1 and Fab2-TRAV1; or
TRAV1-scFv1 and Fab2-TRBV1; or
TRBV1-VHH1 and Fab2-TRAV1; or
TRAV1-VHH1 and Fab2-TRBV1; or
TRBV1-VL1 and Fab2-TRAV1-VH1; or
TRAV1-VL1 and Fab2-TRBV1-VH1.

In some embodiments, the multi-specific polypeptide molecule comprises a first binding region, a second binding region, a third binding region and a fourth binding region, wherein the fourth binding region comprises a heavy chain variable region VH2 and a light chain variable region VL2 derived from an antibody that binds the fourth antigen, and antibody-derived CH1 and antibody-derived CL, wherein the VH2, VL2, CH1 and CL form a single-chain Fab2 which is connected to the N-terminus of the TRAV1 or the TRBV1.

In some embodiments, the two polypeptide chains of the multi-specific polypeptide molecule respectively comprise:
Fab2-TRBV1-VL1-TRAV2-TRBV2-VH1 and TRAV1; or
Fab2-TRBV1-VL1-TRBV2-TRAV2-VH1 and TRAV1; or
Fab2-TRBV1-VH1-TRBV2- TRAV2-VL1 and TRAV1; or
Fab2-TRBV1-VH1-TRAV2- TRBV2-VL1 and TRAV1; or
Fab2-TRAV1-VL1-TRBV2- TRAV2-VH1 and TRBV1; or
Fab2-TRAV1-VL1-TRAV2- TRBV2-VH1 and TRBV1; or
Fab2-TRAV1-VH1-TRAV2-TRBV2-VL1 and TRBV1; or
Fab2-TRAV1-VH1-TRBV2-TRAV2-VL1 and TRBV1; or
TRBV1-VL1-TRAV2-TRBV2-VH1 and Fab2-TRAV1; or
TRBV1-VL1-TRBV2-TRAV2-VH1 and Fab2-TRAV1; or
TRBV1-VH1-TRBV2- TRAV2-VL1 and Fab2-TRAV1; or
TRBV1-VH1-TRAV2- TRBV2-VL1 and Fab2-TRAV1; or
TRAV1-VL1-TRBV2- TRAV2-VH1 and Fab2-TRBV1; or
TRAV1-VL1-TRAV2- TRBV2-VH1 and Fab2-TRBV1; or
TRAV1-VH1-TRAV2-TRBV2-VL1 and Fab2-TRBV1; or
TRAV1-VH1-TRBV2-TRAV2-VL1 and Fab2-TRBV1.

In some embodiments, the Fab2 is selected from the group consisting of, from the N-terminus to the C-terminus, VL2-CL-VH2-CH1, VH2-CH1-VL2-CL, VL2-CL-VH2-CH1 and VH2-CH1-VL2-CL.

In preferred embodiments of the multi-specific polypeptide molecule in the second aspect of the present disclosure, the polypeptide molecule comprises a first polypeptide chain and a second polypeptide chain; wherein:
the first polypeptide chain comprises, from N-terminus to C-terminus: TRBV1- optional linker -TRBC- linker -VL1- linker -VH1- optional linker -hinge region -CH2-CH3, and the second polypeptide chain comprises, from N-terminus to C-terminus: TRAV1- optional linker -TRAC- optional linker -hinge region -CH2-CH3 (format 62); or
the first polypeptide chain comprises, from N-terminus to C-terminus: TRAV1- optional linker -TRAC- linker -VL1- linker -VH1- optional linker -hinge region -CH2-CH3, and the second polypeptide chain comprises, from N-terminus to C-terminus: TRBV1- optional linker -TRBC- optional linker -hinge region -CH2-CH3 (format 62-LJH1); or
the first polypeptide chain comprises, from N-terminus to C-terminus: TRBV1- optional linker -TRBC- linker -VH1- linker -VL1- optional linker -hinge region -CH2-CH3, and the second polypeptide chain comprises, from N-terminus to C-terminus: TRAV1- optional linker -TRAC- optional linker -hinge region -CH2-CH3 (format 62-LJH2); or
the first polypeptide chain comprises, from N-terminus to C-terminus: TRAV1- optional linker -TRAC- linker -VH1- linker -VL1- optional linker -hinge region -CH2-CH3, and the second polypeptide chain comprises, from N-terminus to C-terminus: TRBV1- optional linker -TRBC- optional linker -hinge region -CH2-CH3 (format 62-LJH3); or
the first polypeptide chain comprises, from N-terminus to C-terminus: TRBV1- optional linker -TRBC- linker -VL1- linker -VH1- linker -CH2-CH3, and the second polypeptide chain comprises, from N-terminus to C-terminus: TRAV1- optional linker -TRAC- linker -CH2-CH3 (format 62-3); or
the first polypeptide chain comprises, from N-terminus to C-terminus: TRBV1- optional linker -TRBC- linker -VL1- linker -VH1- optional linker -hinge region -CH3, and the second polypeptide chain comprises, from N-terminus to C-terminus: TRAV1- optional linker -TRAC- optional linker -hinge region -CH3 (format 62-4); or
the first polypeptide chain comprises, from N-terminus to C-terminus: TRBV1- optional linker -TRBC- linker -VL1- linker -VH1- optional linker -hinge region -TRBC, and the second polypeptide chain comprises, from N-terminus to C-terminus: TRAV1- optional linker -TRAC- optional linker -hinge region -TRAC (format 62-5); or
the first polypeptide chain comprises, from N-terminus to C-terminus: TRBV1- optional linker -TRBC- linker -VL1- linker -VH1- optional linker -hinge region, and the second polypeptide chain comprises, from N-terminus to C-terminus: TRAV1- optional linker -TRAC- optional linker -hinge region -Alb (format 62-6); or
the first polypeptide chain comprises, from N-terminus to C-terminus: TRBV1- optional linker -TRBC- linker -VL1- linker -VH1- linker -Alb, and the second polypeptide chain comprises, from N-terminus to C-terminus: TRAV1- optional linker -TRAC (format 62-7); or
the first polypeptide chain comprises, from N-terminus to C-terminus: TRAV1- optional linker -CH3-linker -VL1- linker -VH1- optional linker - optional hinge region -CH2-CH3, and the second polypeptide chain comprises, from N-terminus to C-terminus: TRBV1- optional linker -CH3- optional linker - optional hinge region -CH2-CH3 (format 62-8); or
the first polypeptide chain comprises, from N-terminus to C-terminus: TRAV1- optional linker -CH1-linker -VL1- linker -VH1- optional linker - optional hinge region -CH2-CH3, and the second polypeptide chain comprises, from N-terminus to C-terminus: TRBV1- optional linker -CL- optional linker - optional hinge region -CH2-CH3; or
the first polypeptide chain comprises, from N-terminus to C-terminus: TRAV1- optional linker -CL- linker -VL1- linker -VH1- optional linker - optional hinge region -CH2-CH3, and the second polypeptide chain comprises, from N-terminus to C-terminus: TRBV1- optional linker -CH1- optional linker - optional hinge region -CH2-CH3; or
the first polypeptide chain comprises, from N-terminus to C-terminus: TRBV1- optional linker -TRBC- linker -VHH1- optional linker -hinge region -CH2-CH3, and the second polypeptide chain comprises, from N-terminus to C-terminus: TRAV1- optional linker -TRAC- optional linker -hinge region -CH2-CH3 (format 62-10) ; or
the antibody is a Fab, and the first polypeptide chain comprises, from N-terminus to C-terminus: TRBV1-optional linker -TRBC- linker -VL1-CL- linker -VH1-CH1- optional linker -hinge region -CH2-CH3, and the second polypeptide chain comprises, from N-terminus to C-terminus: TRAV1- optional linker -TRAC- optional linker -hinge region -CH2-CH3 (format 62-12); or
the first polypeptide chain comprises, from N-terminus to C-terminus: TRBV1- optional linker -TRBC- linker -VL1- linker -TRAV2- linker -TRBV2- linker -VH1- optional linker -hinge region -CH2-CH3, and the second polypeptide chain comprises, from N-terminus to C-terminus: TRAV1- optional linker -TRAC- optional linker -hinge region -CH2-CH3 (format 58-2); or
the first polypeptide chain comprises, from N-terminus to C-terminus: VL2- linker -VH2- linker -TRBV1-optional linker -TRBC- linker -VL1- linker -VH1- optional linker -hinge region -CH2-CH3, and the second polypeptide chain comprises, from N-terminus to C-terminus: TRAV1- optional linker -TRAC- optional linker -hinge region -CH2-CH3 (format 72); or
the first polypeptide chain comprises, from N-terminus to C-terminus: VL2- linker -TRBV1- linker -VL1-linker -VH1- optional linker -hinge region -CH2-CH3, and the second polypeptide chain comprises, from N-terminus to C-terminus: VH2- linker -TRAV1- optional linker -hinge region -CH2-CH3 (format 72-2); or
the first polypeptide chain comprises, from N-terminus to C-terminus: VL2- linker -TRBV1- optional linker -TRBC- linker -VL1- linker -VH1- optional linker -hinge region -CH2-CH3, and the second polypeptide chain comprises, from N-terminus to C-terminus: VH2- linker -TRAV1- optional linker -TRAC- optional linker -hinge region -CH2-CH3 (format 72-3); or
the first polypeptide chain comprises, from N-terminus to C-terminus: VL2- linker -VH2- linker -TRBV1-optional linker -TRBC- linker -VL1- linker -TRAV2- linker -TRBV2- linker -VH1- optional linker -hinge region -CH2-CH3, and the second polypeptide chain comprises, from N-terminus to C-terminus: TRAV1-optional linker -TRAC- optional linker -hinge region -CH2-CH3 (format 50-2).

In a third aspect, the present disclosure provides a multi-specific polypeptide molecule, wherein the multi-specific polypeptide molecule comprises, on three polypeptide chains, a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises a heavy chain variable region VH1 and a light chain variable region VL1 derived from an antibody that binds the second antigen, or a single heavy chain variable region (VHH1) derived from a single-domain antibody or a nanobody that binds the second antigen;
wherein the first antigen is selected from the group consisting of tumor-associated antigens (TAA), tumor-specific antigens (TSA), viral antigens and self-antigens, and the second antigen is an immune cell surface molecule;
the second binding region is not connected to the first binding region.

In some embodiments, the multi-specific polypeptide molecule further comprises at least one functional domain capable of extending the half-life of the multi-specific polypeptide molecule. In some embodiments, the functional domain capable of prolonging the half-life of the multi-specific polypeptide molecule is selected from the group consisting of the following 1)-5):
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the multi-specific polypeptide molecule further comprises at least one of the following functional domains:
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the second binding region and the first binding region are not directly connected. In some embodiments, the second binding region and the first binding region are not connected via a linker. The term "not directly linked" means that all or part of the amino acid sequence of the second binding region is not connected, in any form, to all or part of the amino acid sequence of the first binding region on a single polypeptide chain.

In some embodiments, VH1 and VL1 are linked in tandem on a single polypeptide chain (e.g., forming scFv1 or Fab1), and TRAV1 and TRBV1 are distributed on different polypeptide chains. In some embodiments, VH1 and VL1 are linked in tandem on a single polypeptide chain (e.g., forming scFv1 or Fab1), and TRAV1 and TRBV1 are respectively distributed on two other polypeptide chains. In some embodiments, VH1 and VL1 are linked in tandem on a single polypeptide chain (e.g., forming scFv1 or Fab1), and are not directly connected to the polypeptide chains on which TRAV1 and TRBV1 are located.

In some embodiments, the three polypeptide chains of the multi-specific polypeptide molecule respectively comprise:
TRBV1, VL1-VH1, and TRAV1; or
TRBV1, VH1-VL1, and TRAV1; or
TRBV1, VHH1, and TRAV1.

In some embodiments of the multi-specific polypeptide molecule according to the third aspect of the present disclosure, the multi-specific polypeptide molecule further comprises a constant domain selected from the group consisting of the following (1)-(3), which is connected to the C-terminus of TRAV1 and/or TRBV1 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments, the multi-specific polypeptide molecule comprises a TCR constant region or a fragment thereof, which is connected to the C-terminus of TRAV1 and TRBV1 via an optional linker. In some embodiments, the multi-specific polypeptide molecule comprises TRAC and TRBC that are respectively connected to the C-terminus of TRAV1 and to the C-terminus of TRBV1 via an optional linker. In some embodiments, the three polypeptide chains of the multi-specific polypeptide molecule respectively comprise:
TRBV1-TRBC, VL1-VH1, and TRAV1-TRAC; or
TRBV1-TRBC, VH1-VL1, and TRAV1-TRAC; or
TRBV1-TRBC, scFv1, and TRAV1-TRAC; or
TRBV1-TRBC, VHH1, and TRAV1-TRAC.

In some embodiments of the multi-specific polypeptide molecule according to the third aspect of the present disclosure, the multi-specific polypeptide molecule further comprises antibody-derived CH1 and CL that are respectively connected to VH1 and VL1, wherein VH1, VL1, CH1 and CL form a single-chain Fab1 and are connected to the N-terminus of the functional domain via an optional linker. In some embodiments, the three polypeptide chains of the multi-specific polypeptide molecule respectively comprise: TRBV1-TRBC, Fab1, and TRAV1-TRAC.

In some embodiments of the multi-specific polypeptide molecule according to the third aspect of the present disclosure, the multi-specific polypeptide molecule further comprises a third binding region binding a third antigen, wherein the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens and self-antigens, and the third antigen is the same as or different from the first antigen. In some embodiments, TRAV2 is the same as TRAV1, and/or TRBV2 is the same as TRBV1.

In some embodiments, TRAV2 and TRBV2 are distributed on the same polypeptide chain via a linker.

In some embodiments, TRAV2 and TRBV2 are distributed on the same polypeptide chain via a linker and are connected between VH1 and VL1.

In some embodiments of the multi-specific polypeptide molecule according to the third aspect of the present disclosure, the multi-specific polypeptide molecule further comprises a constant domain selected from the group consisting of the following (1)-(3), which is connected to the C-terminus of TRAV2 and/or TRBV2 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments, the multi-specific polypeptide molecule according to the third aspect of the present disclosure comprises a first binding region, a second binding region and a third binding region, and the three polypeptide chains of the multi-specific polypeptide molecule respectively comprise:
TRBV1-TRBC, VL1-TRBV2-TRAV2-VH1, and TRAV1-TRAC; or
TRBV1-TRBC, VL1-TRAV2-TRBV2-VH1, and TRAV1-TRAC; or
TRBV1-TRBC, VH1-TRBV2-TRAV2-VL1, and TRAV1-TRAC; or
TRBV1-TRBC, VH1-TRAV2-TRBV2-VL1, and TRAV1-TRAC.

In some embodiments of the multi-specific polypeptide molecule according to the third aspect of the present disclosure, the multi-specific polypeptide molecule further comprises a fourth binding region binding a fourth antigen, wherein the fourth binding region comprises a heavy chain variable region VH2 and a light chain variable region VL2 derived from an antibody that binds the fourth antigen, or a single heavy chain variable region (VHH2) derived from a single-domain antibody or nanobody that binds the fourth antigen; wherein the fourth antigen is an immune cell surface molecule, and the fourth antigen is the same as or different from the second antigen. In some embodiments, VH2 is the same as VH1. In some embodiments, VL2 is the same as VL1. In some embodiments, VHH2 is the same as VHH1.

In some embodiments, VHH2 or scFv2 formed by VH2 and VL2 is connected to the N-terminus or C-terminus of TRAV1 or TRBV1, or one of VH2 and VL2 is connected to the N-terminus or C-terminus of TRAV1 and the other is connected to the N-terminus or C-terminus of TRBV1. In some embodiments, VHH2 or scFv2 formed by VH2 and VL2 is connected to the N-terminus of TRAV1 or TRBV1, or one of VH2 and VL2 is connected to the N-terminus of TRAV1 and the other is connected to the N-terminus of TRBV1. In some embodiments, scFv2 comprises VH2-VL2 or VL2-VH2.

In some embodiments, in the multi-specific polypeptide molecule according to the third aspect of the present disclosure, the three polypeptide chains of the multi-specific polypeptide molecule respectively comprise:
scFv2-TRBV1-TRBC, VL1-VH1, and TRAV1-TRAC; or
TRBV1-TRBC, VL1-VH1, and scFv2-TRAV1-TRAC; or
scFv2-TRBV1-TRBC, VH1-VL1, and TRAV1-TRAC; or
TRBV1-TRBC, VH1-VL1, and scFv2-TRAV1-TRAC; or
scFv2-TRBV1-TRBC, scFv1, and TRAV1-TRAC; or
TRBV1-TRBC, scFv1, and scFv2-TRAV1-TRAC.

In some embodiments of the multi-specific polypeptide molecule of the third aspect of the present disclosure, the multi-specific polypeptide molecule further comprises CH1 and CL derived from an antibody, which are respectively connected to the VH2 and VL2. In some embodiments, the VH2, VL2, CH1, and CL form Fab2 which is connected to the N-terminus of the TRBV1 or TRAV1 via an optional linker. In some embodiments, Fab2 comprises VL1-CL-VH1-CH1, VL1-CH1-VH1-CL, VH1-CH1-VL1-CL, or VH1-CL-VL1-CH1.

In some embodiments, the multi-specific polypeptide molecule of the third aspect of the present disclosure comprises a first binding region, a second binding region, a third binding region, and a fourth binding region, and the three polypeptide chains of the polypeptide molecule respectively comprise:
scFv2-TRBV1-TRBC, VL1-TRBV2-TRAV2-VH1, and TRAV1-TRAC; or
scFv2-TRBV1-TRBC, VH1-TRBV2-TRAV2-VL1, and TRAV1-TRAC; or
scFv2-TRBV1-TRBC, VL1-TRAV2-TRBV2-VH1, and TRAV1-TRAC; or
scFv2-TRBV1-TRBC, VH1-TRAV2-TRBV2-VL1, and TRAV1-TRAC; or
TRBV1-TRBC, VL1-TRBV2-TRAV2-VH1, and scFv2-TRAV1-TRAC; or
TRBV1-TRBC, VH1-TRBV2-TRAV2-VL1, and scFv2-TRAV1-TRAC; or
TRBV1-TRBC, VL1-TRAV2-TRBV2-VH1, and scFv2-TRAV1-TRAC; or
TRBV1-TRBC, VH1-TRAV2-TRBV2-VL1, and scFv2-TRAV1-TRAC; or
scFv2-TRBV1-TRBC, VH1-TRBV2-TRAV2-VL1, and TRAV1-TRAC; or
scFv2-TRBV1-TRBC, VL1-TRBV2-TRAV2-VH1, and TRAV1-TRAC; or
scFv2-TRBV1-TRBC, VH1-TRAV2-TRBV2-VL1, and TRAV1-TRAC; or
scFv2-TRBV1-TRBC, VL1-TRAV2-TRBV2-VH1, and TRAV1-TRAC; or
TRBV1-TRBC, VH1-TRBV2-TRAV2-VL1, and scFv2-TRAV1-TRAC; or
TRBV1-TRBC, VL1-TRBV2-TRAV2-VH1, and scFv2-TRAV1-TRAC; or
TRBV1-TRBC, VH1-TRAV2-TRBV2-VL1, and scFv2-TRAV1-TRAC; or
TRBV1-TRBC, VL1-TRAV2-TRBV2-VH1, and scFv2-TRAV1-TRAC.

In some embodiments, the functional domain comprises two chains, which are respectively connected to the C-terminus or N-terminus (preferably the C-terminus) of any two polypeptide chains of the multi-specific polypeptide molecule of the third aspect of the present disclosure.

In some embodiments, the functional domain comprises two chains, and the first binding region and the second binding region of the multi-specific polypeptide molecule are connected via covalent bonds or non-covalent bonds between the two chains of the functional domain.

In some embodiments, the functional domain comprises two chains, which are respectively connected to the C-terminus or N-terminus (preferably the C-terminus) of any two polypeptide chains of the multi-specific polypeptide molecule of the third aspect of the present disclosure, and the first binding region and the second binding region of the multi-specific polypeptide molecule are connected via covalent bonds or non-covalent bonds between the two chains of the functional domain.

In some embodiments, the first binding region and the second binding region of the multi-specific polypeptide molecule are connected via covalent bonds or non-covalent bonds between the two chains of the functional domain, and one chain of the functional domain is connected to the C-terminus or N-terminus(preferably the C-terminus) of the polypeptide chain containing the second binding region, and the other chain is connected to the C-terminus or N-terminus (preferably the C-terminus) of the polypeptide chain containing TRAV1 or TRBV1 of the first binding region.

In some embodiments of the multi-specific polypeptide molecule of the third aspect of the present disclosure, the functional domain is derived from an antibody hinge region and/or an antibody Fc domain or a dimerization portion thereof; and the functional domain is connected to the C-terminus or N-terminus (preferably the C-terminus) of any two polypeptide chains of the multi-specific polypeptide molecule via an optional linker.

In some embodiments of the multi-specific polypeptide molecule of the third aspect of the present disclosure, the functional domain is derived from an antibody CH3 domain and an optional antibody hinge region; and the functional domain is connected to the C-terminus or N-terminus (preferably the C-terminus) of any two polypeptide chains of the multi-specific polypeptide molecule via an optional linker.

In some embodiments of the multi-specific polypeptide molecule of the third aspect of the present disclosure, the functional domain is derived from a TCR constant region or a fragment thereof and an optional antibody hinge region; and the functional domain is connected to the C-terminus or N-terminus (preferably the C-terminus) of any two polypeptide chains of the multi-specific polypeptide molecule via an optional linker.

In some embodiments, the constant region of the TCR or a fragment thereof is selected from the group consisting of a TCR α chain constant region (TRAC) and a TCR β chain constant region (TRBC).

In some embodiments of the multi-specific polypeptide molecule of the third aspect of the present disclosure, the TCR is an αβ TCR, distributed on two polypeptide chains, and VH1 and VL1 are linked in tandem to form an scFv, distributed on a third polypeptide chain; and the functional domain is derived from an antibody hinge region and an antibody Fc domain or a dimerization portion thereof; wherein one chain of the functional domain is connected to the C-terminus of the scFv via an optional linker, and the other chain is connected to the C-terminus of the α chain or β chain of the TCR. In some embodiments, the α chain and the β chain each independently comprise a variable region; e.g., the α chain comprises TRAV1, and the β chain comprises TRBV1. In some embodiments, the α chain and the β chain each independently comprise a variable region and a constant region; e.g., the α chain comprises TRAV1 and TRAC, and the β chain comprises TRBV1 and TRBC.

In some embodiments of the multi-specific polypeptide molecule of the third aspect of the present disclosure, the polypeptide molecule comprises a first polypeptide chain, a second polypeptide chain, and a third polypeptide chain; wherein:
the first polypeptide chain comprises, from N-terminus to C-terminus: TRBV1- optional linker -TRBC, the second polypeptide chain comprises, from N-terminus to C-terminus: VL1- linker -VH1- optional linker - hinge region -CH2-CH3, and the third polypeptide chain comprises, from N-terminus to C-terminus: TRAV1-optional linker -TRAC- optional linker -hinge region -CH2-CH3 (format 59);
In some embodiments, the first polypeptide chain comprises, from N-terminus to C-terminus: TRBV1-optional linker -TRBC, the second polypeptide chain comprises, from N-terminus to C-terminus: VH1- linker -VL1- optional linker -hinge region -CH2-CH3, and the third polypeptide chain comprises, from N-terminus to C-terminus: TRAV1- optional linker -TRAC- optional linker -hinge region -CH2-CH3;
In some embodiments, the first polypeptide chain comprises, from N-terminus to C-terminus: TRBV1-optional linker -TRBC, the second polypeptide chain comprises, from N-terminus to C-terminus: VHH1-optional linker -hinge region -CH2-CH3, and the third polypeptide chain comprises, from N-terminus to C-terminus: TRAV1- optional linker -TRAC- optional linker -hinge region -CH2-CH3;
In some embodiments, the first polypeptide chain comprises, from N-terminus to C-terminus: TRBV1-optional linker -TRBC- optional linker -hinge region -CH2-CH3, the second polypeptide chain comprises, from N-terminus to C-terminus: VL1- linker -VH1- optional linker -hinge region -CH2-CH3, and the third polypeptide chain comprises, from N-terminus to C-terminus: TRAV1- optional linker -TRAC;
In some embodiments, the first polypeptide chain comprises, from N-terminus to C-terminus: TRBV1-optional linker -TRBC- optional linker -hinge region -CH2-CH3, the second polypeptide chain comprises, from N-terminus to C-terminus: VH1- linker -VL1- optional linker -hinge region -CH2-CH3, and the third polypeptide chain comprises, from N-terminus to C-terminus: TRAV1- optional linker -TRAC;
In some embodiments, the first polypeptide chain comprises, from N-terminus to C-terminus: TRBV1-optional linker -TRBC- optional linker -hinge region -CH2-CH3, the second polypeptide chain comprises, from N-terminus to C-terminus: VHH1- optional linker -hinge region -CH2-CH3, and the third polypeptide chain comprises, from N-terminus to C-terminus: TRAV1- optional linker -TRAC.

In some embodiments, the first polypeptide chain comprises, from the N-terminus to the C-terminus: TRBV1-optional linker-TRBC-optional linker-hinge region-CH2-CH3; the second polypeptide chain comprises, from the N-terminus to the C-terminus: VHH1-optional linker-hinge region-CH2-CH3; and the third polypeptide chain comprises, from the N-terminus to the C-terminus: TRAV1-optional linker-TRAC.

In a fourth aspect, the present disclosure provides a multi-specific polypeptide molecule, wherein the multi-specific polypeptide molecule comprises, on three polypeptide chains, a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises a heavy chain variable region VH1 and a light chain variable region VL1 derived from an antibody that binds the second antigen;
wherein the first antigen is selected from the group consisting of tumor-associated antigens (TAA), tumor-specific antigens (TSA), viral antigens and self-antigens, and the second antigen is an immune cell surface molecule;
wherein TRAV1 and TRBV1 are respectively distributed on two different polypeptide chains, VH1 and VL1 are respectively distributed on two different polypeptide chains, and the polypeptide chain comprising VH1 or VL1 is connected to the C-terminus of the polypeptide chain comprising TRAV1 or TRBV1.

In some embodiments, the multi-specific polypeptide molecule further comprises at least one functional domain capable of prolonging the half-life of the multi-specific polypeptide molecule. In some embodiments, the functional domain capable of prolonging the half-life of the multi-specific polypeptide molecule is selected from the group consisting of the following 1)-5):
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the multi-specific polypeptide molecule further comprises at least one of the following functional domains:
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the N-terminus of the polypeptide chain comprising VH1 or VL1 is connected to the C-terminus of the polypeptide chain comprising TRAV1 or TRBV1.

In some embodiments, the polypeptide chain comprising VL1 or VH1 is connected to the C-terminus of the polypeptide chain comprising TRBV1 or TRAV1 via a linker.

In some embodiments, the three polypeptide chains of the multi-specific polypeptide molecule respectively comprise:
TRBV1-VL1, VH1, and TRAV1; or
TRBV1-VH1, VL1, and TRAV1; or
TRAV1-VL1, VH1, and TRBV1; or
TRAV1-VH1, VL1, and TRBV1.

In some embodiments of the multi-specific polypeptide molecule of the fourth aspect of the present disclosure, the multi-specific polypeptide molecule further comprises at least one constant domain selected from the group consisting of the following (1)-(3), which is connected to the C-terminus of TRAV1 and/or TRBV1 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments, the multi-specific polypeptide molecule comprises a TCR constant region or a fragment thereof connected to the C terminus of TRAV1 and TRBV1 via an optional linker. In some embodiments, the multi-specific polypeptide molecule comprises TRAC and TRBC respectively connected to the C terminus of TRAV1 and TRBV1 via an optional linker. In some embodiments, the three polypeptide chains of the multi-specific polypeptide molecule respectively comprise:
TRBV1-TRBC-VL1, VH1, and TRAV1-TRAC; or
TRBV1-TRBC-VH1, VL1, and TRAV1-TRAC; or
TRAV1-TRAC-VL1, VH1, and TRBV1-TRBC; or
TRAV1-TRAC-VH1, VL1, and TRBV1-TRBC.

In some embodiments of the multi-specific polypeptide molecule of the fourth aspect of the present disclosure, the multi-specific polypeptide molecule further comprises antibody-derived CH1 and CL respectively connected to VH1 and VL1. In some embodiments, the three polypeptide chains of the multi-specific polypeptide molecule respectively comprise:
TRBV1-TRBC-VL1-CL, VH1-CH, and TRAV1-TRAC; or
TRBV1-TRBC-VH1-CH, VL1-CL, and TRAV1-TRAC; or
TRAV1-TRAC-VL1-CL, VH1-CH, and TRBV1-TRBC; or
TRAV1-TRAC-VH1-CH, VL1-CL, and TRBV1-TRBC.

In some embodiments of the multi-specific polypeptide molecule of the fourth aspect of the present disclosure, the multi-specific polypeptide molecule further comprises a third binding region that binds a third antigen, wherein the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens and self-antigens, and the third antigen is the same as or different from the first antigen. In some embodiments, TRAV2 is the same as TRAV1. In some embodiments, TRBV2 is the same as TRBV1.

In some embodiments, TRAV2 or TRBV2 is connected to the C-terminus of VH1 or VL1 via an optional linker. In some embodiments, TRAV2 and TRBV2 are tandemly linked on one chain which is connected to the C-terminus of VH1 or the C-terminus of VL1 via an optional linker. In some embodiments, one of TRAV2 and TRBV2 is connected to the C-terminus of VH1, and the other is connected to the C-terminus of VL1.

In some embodiments, the multi-specific polypeptide molecule of the third aspect of the present disclosure comprises a first binding region, a second binding region and a third binding region, and the three polypeptide chains of the multi-specific polypeptide molecule respectively comprise:
TRBV1-TRBC-VL1-TRBV2-TRAV2, VH1, and TRAV1-TRAC; or
TRBV1-TRBC-VL1-TRAV2-TRBV2, VH1, and TRAV1-TRAC; or
TRBV1-TRBC-VL1, VH1-TRBV2-TRAV2, and TRAV1-TRAC; or
TRBV1-TRBC-VL1, VH1-TRAV2-TRBV2, and TRAV1-TRAC; or
TRBV1-TRBC-VH1-TRBV2-TRAV2, VL1, and TRAV1-TRAC; or
TRBV1-TRBC-VH1-TRAV2-TRBV2, VL1, and TRAV1-TRAC; or
TRBV1-TRBC-VH1, VL1-TRBV2-TRAV2, and TRAV1-TRAC; or
TRBV1-TRBC-VH1, VL1-TRAV2-TRBV2, and TRAV1-TRAC; or
TRAV1-TRAC-VL1-TRBV2-TRAV2, VH1, and TRBV1-TRBC; or
TRAV1-TRAC-VL1-TRAV2-TRBV2, VH1, and TRBV1-TRBC; or
TRAV1-TRAC-VL1, VH1-TRBV2-TRAV2, and TRBV1-TRBC; or
TRAV1-TRAC-VL1, VH1-TRAV2-TRBV2, and TRBV1-TRBC; or
TRAV1-TRAC-VH1-TRBV2-TRAV2, VL1, and TRBV1-TRBC; or
TRAV1-TRAC-VH1-TRAV2-TRBV2, VL1, and TRBV1-TRBC; or
TRAV1-TRAC-VH1, VL1-TRBV2-TRAV2, and TRBV1-TRBC; or
TRAV1-TRAC-VH1, VL1-TRAV2-TRBV2, and TRBV1-TRBC; or
TRBV1-TRBC-VL1-TRBV2, VH1-TRAV2, and TRAV1-TRAC; or
TRBV1-TRBC-VL1-TRAV2, VH1-TRBV2, and TRAV1-TRAC; or
TRBV1-TRBC-VH1-TRBV2, VL1-TRAV2, and TRAV1-TRAC; or
TRBV1-TRBC-VH1-TRAV2, VL1-TRBV2, and TRAV1-TRAC; or
TRAV1-TRAC-VL1-TRBV2, VH1-TRAV2, and TRBV1-TRBC; or
TRAV1-TRAC-VL1-TRAV2, VH1-TRBV2, and TRBV1-TRBC; or
TRAV1-TRAC-VH1-TRBV2, VL1-TRAV2, and TRBV1-TRBC; or
TRAV1-TRAC-VH1-TRAV2, VL1-TRBV2, and TRBV1-TRBC.

In some embodiments, the multi-specific polypeptide molecule further comprises at least one constant domain selected from the group consisting of the following (1)-(3), which is connected to the C-terminus of TRAV2 and/or TRBV2 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments of the multi-specific polypeptide molecule of the fourth aspect of the present disclosure, the multi-specific polypeptide molecule further comprises a fourth binding region that binds a fourth antigen, wherein the fourth binding region comprises a heavy chain variable region VH2 and a light chain variable region VL2 derived from an antibody that binds the fourth antigen, or a single heavy chain variable region (VHH) derived from a single-domain antibody or nanobody that binds the fourth antigen; wherein the fourth antigen is an immune cell surface molecule, and the fourth antigen is the same as or different from the second antigen. In some embodiments, the VH2 is the same as the VH1. In some embodiments, the VL2 is the same as the VL1.

In some embodiments, the VHH or an scFv formed by the VH2 and VL2 is connected to the N-terminus or C-terminus of the TRAV1 or the TRBV1, or one of the VH2 and VL2 is connected to the N-terminus or C-terminus of the TRAV1 and the other is connected to the N-terminus or C-terminus of the TRBV1. In some embodiments, the VHH or an scFv formed by the VH2 and VL2 is connected to the N-terminus of the TRAV1 or the TRBV1, or one of the VH2 and VL2 is connected to the N-terminus of the TRAV1 and the other is connected to the N-terminus of the TRBV1. In some embodiments, the scFv comprises VH2-VL2 or VL2-VH2.

In some embodiments, the three polypeptide chains of the multi-specific polypeptide molecule of the third aspect of the present disclosure each comprise, respectively:
scFv-TRBV1-TRBC-VL1, VH1, and TRAV1-TRAC; or
TRBV1-TRBC-VL1, VH1, and scFv-TRAV1-TRAC; or
VHH-TRBV1-TRBC-VL1, VH1, and TRAV1-TRAC; or
TRBV1-TRBC-VL1, VH1, and VHH-TRAV1-TRAC; or
VH2-TRBV1-TRBC-VL1, VH1, and VL2-TRAV1-TRAC; or
VL2-TRBV1-TRBC-VL1, VH1, and VH2-TRAV1-TRAC; or
scFv-TRBV1-TRBC-VH1, VL1, and TRAV1-TRAC; or
TRBV1-TRBC-VH1, VL1, and scFv-TRAV1-TRAC; or
VHH-TRBV1-TRBC-VH1, VL1, and TRAV1-TRAC; or
TRBV1-TRBC-VH1, VL1, and VHH-TRAV1-TRAC; or
VH2-TRBV1-TRBC-VH1, VL1, and VL2-TRAV1-TRAC; or
VL2-TRBV1-TRBC-VH1, VL1, and VH2-TRAV1-TRAC; or
scFv-TRAV1-TRAC-VL1, VH1, and TRBV1-TRBC; or
TRAV1-TRAC-VL1, VH1, and scFv-TRBV1-TRBC; or
VHH-TRAV1-TRAC-VL1, VH1, and TRBV1-TRBC; or
TRAV1-TRAC-VL1, VH1, and VHH-TRBV1-TRBC; or
VH2-TRAV1-TRAC-VL1, VH1, and VL2-TRBV1-TRBC; or
VL2-TRAV1-TRAC-VL1, VH1, and VH2-TRBV1-TRBC; or
scFv-TRAV1-TRAC-VH1, VL1, and TRBV1-TRBC; or
TRAV1-TRAC-VH1, VL1, and scFv-TRBV1-TRBC; or
VHH-TRAV1-TRAC-VH1, VL1, and TRBV1-TRBC; or
TRAV1-TRAC-VH1, VL1, and VHH-TRBV1-TRBC; or
VH2-TRAV1-TRAC-VH1, VL1, and VL2-TRBV1-TRBC; or
VL2-TRAV1-TRAC-VH1, VL1, and VH2-TRBV1-TRBC.

In some embodiments of the multi-specific polypeptide molecule of the fourth aspect of the present disclosure, the multi-specific polypeptide molecule further comprises antibody-derived CH1 and CL that are respectively connected to the VH2 and VL2, wherein the VH2, VL2, CH1 and CL form a single-chain Fab which is connected to the N-terminus of the functional domain via an optional linker. In some embodiments, the Fab comprises VL1-CL-VH1-CH1, VL1-CH1-VH1-CL, VH1-CH1-VL1-CL or VH1-CL-VL1-CH1.

In some embodiments, the multi-specific polypeptide molecule of the fourth aspect of the present disclosure comprises a first binding region, a second binding region, a third binding region and a fourth binding region, and the three polypeptide chains of the polypeptide molecule each comprise, respectively:
scFv-TRBV1-TRBC-VL1-TRBV2-TRAV2, VH1, and TRAV1-TRAC; or
scFv-TRBV1-TRBC-VL1-TRAV2-TRBV2, VH1, and TRAV1-TRAC; or
scFv-TRBV1-TRBC-VL1, VH1-TRAV2-TRBV2, and TRAV1-TRAC; or
scFv-TRBV1-TRBC-VL1, VH1-TRAV2-TRBV2, and TRAV1-TRAC; or
TRBV1-TRBC-VL1-TRBV2-TRAV2, VH1, and scFv-TRAV1-TRAC; or
TRBV1-TRBC-VL1-TRAV2-TRBV2, VH1, and scFv-TRAV1-TRAC; or
TRBV1-TRBC-VL1, VH1-TRBV2-TRAV2, and scFv-TRAV1-TRAC; or
TRBV1-TRBC-VL1, VH1-TRAV2-TRBV2, and scFv-TRAV1-TRAC; or
VHH-TRBV1-TRBC-VL1-TRBV2-TRAV2, VH1, and TRAV1-TRAC; or
VHH-TRBV1-TRBC-VL1-TRAV2-TRBV2, VH1, and TRAV1-TRAC; or
VHH-TRBV1-TRBC-VL1, VH1-TRBV2-TRAV2, and TRAV1-TRAC; or
VHH-TRBV1-TRBC-VL1, VH1-TRAV2-TRBV2, and TRAV1-TRAC; or
TRBV1-TRBC-VL1-TRBV2-TRAV2, VH1, and VHH-TRAV1-TRAC; or
TRBV1-TRBC-VL1-TRAV2-TRBV2, VH1, and VHH-TRAV1-TRAC; or
TRBV1-TRBC-VL1, VH1-TRBV2-TRAV2, and VHH-TRAV1-TRAC; or
TRBV1-TRBC-VL1, VH1-TRAV2-TRBV2, and VHH-TRAV1-TRAC; or
VH2-TRBV1-TRBC-VL1-TRBV2-TRAV2, VH1, and VL2-TRAV1-TRAC; or
VH2-TRBV1-TRBC-VL1-TRAV2-TRBV2, VH1, and VL2-TRAV1-TRAC; or
VH2-TRBV1-TRBC-VL1, VH1-TRBV2-TRAV2, and VL2-TRAV1-TRAC; or
VH2-TRBV1-TRBC-VL1, VH1-TRAV2-TRBV2, and VL2-TRAV1-TRAC; or
VL2-TRBV1-TRBC-VL1-TRBV2-TRAV2, VH1, and VH2-TRAV1-TRAC; or
VL2-TRBV1-TRBC-VL1-TRAV2-TRBV2, VH1, and VH2-TRAV1-TRAC; or
VL2-TRBV1-TRBC-VL1, VH1-TRBV2-TRAV2, and VH2-TRAV1-TRAC; or
VL2-TRBV1-TRBC-VL1, VH1-TRAV2-TRBV2, and VH2-TRAV1-TRAC; or
scFv-TRBV1-TRBC-VH1-TRBV2-TRAV2, VL1, and TRAV1-TRAC; or
scFv-TRBV1-TRBC-VH1-TRAV2-TRBV2, VL1, and TRAV1-TRAC; or
scFv-TRBV1-TRBC-VH1, VL1-TRBV2-TRAV2, and TRAV1-TRAC; or
scFv-TRBV1-TRBC-VH1, VL1-TRAV2-TRBV2, and TRAV1-TRAC; or
TRBV1-TRBC-VH1-TRBV2-TRAV2, VL1, and scFv-TRAV1-TRAC; or
TRBV1-TRBC-VH1-TRAV2-TRBV2, VL1, and scFv-TRAV1-TRAC; or
TRBV1-TRBC-VH1, VL1-TRBV2-TRAV2, and scFv-TRAV1-TRAC; or
TRBV1-TRBC-VH1, VL1-TRAV2-TRBV2, and scFv-TRAV1-TRAC; or
VHH-TRBV1-TRBC-VH1-TRBV2-TRAV2, VL1, and TRAV1-TRAC; or
VHH-TRBV1-TRBC-VH1-TRAV2-TRBV2, VL1, and TRAV1-TRAC; or
VHH-TRBV1-TRBC-VH1, VL1-TRBV2-TRAV2, and TRAV1-TRAC; or
VHH-TRBV1-TRBC-VH1, VL1-TRAV2-TRBV2, and TRAV1-TRAC; or
TRBV1-TRBC-VH1-TRBV2-TRAV2, VL1, and VHH-TRAV1-TRAC; or
TRBV1-TRBC-VH1-TRAV2-TRBV2, VL1, and VHH-TRAV1-TRAC; or
TRBV1-TRBC-VH1, VL1-TRBV2-TRAV2, and VHH-TRAV1-TRAC; or
TRBV1-TRBC-VH1, VL1-TRAV2-TRBV2, and VHH-TRAV1-TRAC; or
VH2-TRBV1-TRBC-VH1-TRBV2-TRAV2, VL1, and VL2-TRAV1-TRAC; or
VH2-TRBV1-TRBC-VH1-TRAV2-TRBV2, VL1, and VL2-TRAV1-TRAC; or
VH2-TRBV1-TRBC-VH1, VL1-TRBV2-TRAV2, and VL2-TRAV1-TRAC; or
VH2-TRBV1-TRBC-VH1, VL1-TRAV2-TRBV2, and VL2-TRAV1-TRAC; or
VL2-TRBV1-TRBC-VH1-TRBV2-TRAV2, VL1, and VH2-TRAV1-TRAC; or
VL2-TRBV1-TRBC-VH1-TRAV2-TRBV2, VL1, and VH2-TRAV1-TRAC; or
VL2-TRBV1-TRBC-VH1, VL1-TRBV2-TRAV2, and VH2-TRAV1-TRAC; or
VL2-TRBV1-TRBC-VH1, VL1-TRAV2-TRBV2, and VH2-TRAV1-TRAC; or
scFv-TRAV1-TRAC-VL1-TRBV2-TRAV2, VH1, and TRBV1-TRBC; or
scFv-TRAV1-TRAC-VL1-TRAV2-TRBV2, VH1, and TRBV1-TRBC; or
scFv-TRAV1-TRAC-VL1, VH1-TRBV2-TRAV2, and TRBV1-TRBC; or
scFv-TRAV1-TRAC-VL1, VH1-TRAV2-TRBV2, and TRBV1-TRBC; or
TRAV1-TRAC-VL1-TRBV2-TRAV2, VH1, and scFv-TRBV1-TRBC; or
TRAV1-TRAC-VL1-TRAV2-TRBV2, VH1, and scFv-TRBV1-TRBC; or
TRAV1-TRAC-VL1, VH1-TRBV2-TRAV2, and scFv-TRBV1-TRBC; or
TRAV1-TRAC-VL1, VH1-TRAV2-TRBV2, and scFv-TRBV1-TRBC; or
VHH-TRAV1-TRAC-VL1-TRBV2-TRAV2, VH1, and TRBV1-TRBC; or
VHH-TRAV1-TRAC-VL1-TRAV2-TRBV2, VH1, and TRBV1-TRBC; or
VHH-TRAV1-TRAC-VL1, VH1-TRBV2-TRAV2, and TRBV1-TRBC; or
VHH-TRAV1-TRAC-VL1, VH1-TRAV2-TRBV2, and TRBV1-TRBC; or
TRAV1-TRAC-VL1-TRBV2-TRAV2, VH1, and VHH-TRBV1-TRBC; or
TRAV1-TRAC-VL1-TRAV2-TRBV2, VH1, and VHH-TRBV1-TRBC; or
TRAV1-TRAC-VL1, VH1-TRBV2-TRAV2, and VHH-TRBV1-TRBC; or
TRAV1-TRAC-VL1, VH1-TRAV2-TRBV2, and VHH-TRBV1-TRBC; or
VH2-TRAV1-TRAC-VL1-TRBV2-TRAV2, VH1, and VL2-TRBV1-TRBC; or
VH2-TRAV1-TRAC-VL1-TRAV2-TRBV2, VH1, and VL2-TRBV1-TRBC; or
VH2-TRAV1-TRAC-VL1, VH1-TRBV2-TRAV2, and VL2-TRBV1-TRBC; or
VH2-TRAV1-TRAC-VL1, VH1-TRAV2-TRBV2, and VL2-TRBV1-TRBC; or
VL2-TRAV1-TRAC-VL1-TRBV2-TRAV2, VH1, and VH2-TRBV1-TRBC; or
VL2-TRAV1-TRAC-VL1-TRAV2-TRBV2, VH1, and VH2-TRBV1-TRBC; or
VL2-TRAV1-TRAC-VL1, VH1-TRBV2-TRAV2, and VH2-TRBV1-TRBC; or
VL2-TRAV1-TRAC-VL1, VH1-TRAV2-TRBV2, and VH2-TRBV1-TRBC; or
scFv-TRAV1-TRAC-VH1-TRBV2-TRAV2, VL1, and TRBV1-TRBC; or
scFv-TRAV1-TRAC-VH1-TRAV2-TRBV2, VL1, and TRBV1-TRBC; or
scFv-TRAV1-TRAC-VH1, VL1-TRBV2-TRAV2, and TRBV1-TRBC; or
scFv-TRAV1-TRAC-VH1, VL1-TRAV2-TRBV2, and TRBV1-TRBC; or
TRAV1-TRAC-VH1-TRBV2-TRAV2, VL1, and scFv-TRBV1-TRBC; or
TRAV1-TRAC-VH1-TRAV2-TRBV2, VL1, and scFv-TRBV1-TRBC; or
TRAV1-TRAC-VH1, VL1-TRBV2-TRAV2, and scFv-TRBV1-TRBC; or
TRAV1-TRAC-VH1, VL1-TRAV2-TRBV2, and scFv-TRBV1-TRBC; or
VHH-TRAV1-TRAC-VH1-TRBV2-TRAV2, VL1, and TRBV1-TRBC; or
VHH-TRAV1-TRAC-VH1-TRAV2-TRBV2, VL1, and TRBV1-TRBC; or
VHH-TRAV1-TRAC-VH1, VL1-TRBV2-TRAV2, and TRBV1-TRBC; or
VHH-TRAV1-TRAC-VH1, VL1-TRAV2-TRBV2, and TRBV1-TRBC; or
TRAV1-TRAC-VH1-TRBV2-TRAV2, VL1, and VHH-TRBV1-TRBC; or
TRAV1-TRAC-VH1-TRAV2-TRBV2, VL1, and VHH-TRBV1-TRBC; or
TRAV1-TRAC-VH1, VL1-TRBV2-TRAV2, and VHH-TRBV1-TRBC; or
TRAV1-TRAC-VH1, VL1-TRAV2-TRBV2, and VHH-TRBV1-TRBC; or
VH2-TRAV1-TRAC-VH1-TRBV2-TRAV2, VL1, and VL2-TRBV1-TRBC; or
VH2-TRAV1-TRAC-VH1-TRAV2-TRBV2, VL1, and VL2-TRBV1-TRBC; or
VH2-TRAV1-TRAC-VH1, VL1-TRBV2-TRAV2, and VL2-TRBV1-TRBC; or
VH2-TRAV1-TRAC-VH1, VL1-TRAV2-TRBV2, and VL2-TRBV1-TRBC; or
VL2-TRAV1-TRAC-VH1-TRBV2-TRAV2, VL1, and VH2-TRBV1-TRBC; or
VL2-TRAV1-TRAC-VH1-TRAV2-TRBV2, VL1, and VH2-TRBV1-TRBC; or
VL2-TRAV1-TRAC-VH1, VL1-TRBV2-TRAV2, and VH2-TRBV1-TRBC; or
VL2-TRAV1-TRAC-VH1, VL1-TRAV2-TRBV2, and VH2-TRBV1-TRBC.

In some embodiments of the multi-specific polypeptide molecule of the fourth aspect of the present disclosure, the functional domain is derived from albumin or a binding domain thereof and an optional hinge region, and is connected to the C-terminus or N-terminus (preferably the C-terminus) of any one polypeptide chain via an optional linker.

In some embodiments of the multi-specific polypeptide molecule of the fourth aspect of the present disclosure, the functional domain is derived from an antibody hinge region and/or an antibody Fc domain or a dimerizing portion thereof; and the functional domain is connected to the C-terminus or N-terminus (preferably the C-terminus) of any two polypeptide chains of the multi-specific polypeptide molecule via an optional linker.

In some embodiments of the multi-specific polypeptide molecule of the fourth aspect of the present disclosure, the functional domain is derived from an antibody CH3 domain and an optional antibody hinge region; and the functional domain is connected to the C-terminus or N-terminus (preferably the C-terminus) of any two polypeptide chains of the multi-specific polypeptide molecule via an optional linker.

In some embodiments of the multi-specific polypeptide molecule of the fourth aspect of the present disclosure, the functional domain is derived from a TCR constant region or a fragment thereof and an optional antibody hinge region; and the functional domain is connected to the C-terminus or N-terminus (preferably the C-terminus) of any two polypeptide chains of the multi-specific polypeptide molecule via an optional linker.

In some embodiments, the constant region of the TCR or a fragment thereof is selected from the group consisting of a TCR α chain constant region (TRAC) and a TCR β chain constant region (TRBC).

In some embodiments, the functional domain comprises two polypeptide chains, and any two chains in the multi-specific polypeptide molecule are connected via covalent bonds or non-covalent bonds between the two chains of the functional domain; e.g., the first binding region and the second binding region are connected via covalent bonds or non-covalent bonds between the two chains of the functional domain.

In some embodiments of the multi-specific polypeptide molecule of the fourth aspect of the present disclosure, the polypeptide molecule comprises a first polypeptide chain, a second polypeptide chain, and a third polypeptide chain; wherein:
the first polypeptide chain comprises, from N-terminus to C-terminus: TRBV1- optional linker -TRBC-linker -VL1, the second polypeptide chain comprises, from N-terminus to C-terminus: VH1- optional linker - hinge region -CH2-CH3, and the third polypeptide chain comprises, from N-terminus to C-terminus: TRAV1-optional linker -TRAC- optional linker -hinge region -CH2-CH3 (format 62-1).

In some embodiments, the first polypeptide chain comprises, from N-terminus to C-terminus: TRBV1-optional linker -TRBC- linker -VH1, the second polypeptide chain comprises, from N-terminus to C-terminus: VL1- optional linker -hinge region -CH2-CH3, and the third polypeptide chain comprises, from N-terminus to C-terminus: TRAV1- optional linker -TRAC- optional linker -hinge region -CH2-CH3.

In some embodiments, the first polypeptide chain comprises, from N-terminus to C-terminus: TRAV1-optional linker -TRAC- linker -VL1, the second polypeptide chain comprises, from N-terminus to C-terminus: VH1- optional linker -hinge region -CH2-CH3, and the third polypeptide chain comprises, from N-terminus to C-terminus: TRBV1- optional linker -TRBC- optional linker -hinge region -CH2-CH3.

In some embodiments, the first polypeptide chain comprises, from N-terminus to C-terminus: TRAV1-optional linker -TRAC- linker -VH1, the second polypeptide chain comprises, from N-terminus to C-terminus: VL1- optional linker -hinge region -CH2-CH3, and the third polypeptide chain comprises, from N-terminus to C-terminus: TRBV1- optional linker -TRBC- optional linker -hinge region -CH2-CH3.
the first polypeptide chain comprises, from N-terminus to C-terminus: TRBV1- optional linker -TRBC-linker -VL1- optional linker -hinge region -CH2-CH3, the second polypeptide chain comprises, from N-terminus to C-terminus: VH1- optional linker -hinge region -CH2-CH3, and the third polypeptide chain comprises, from N-terminus to C-terminus: TRAV1- optional linker -TRAC.

In some embodiments, the first polypeptide chain comprises, from N-terminus to C-terminus: TRBV1-optional linker -TRBC- linker -VH1- optional linker -hinge region -CH2-CH3, the second polypeptide chain comprises, from N-terminus to C-terminus: VL1- optional linker -hinge region -CH2-CH3, and the third polypeptide chain comprises, from N-terminus to C-terminus: TRAV1- optional linker -TRAC.

In some embodiments, the first polypeptide chain comprises, from N-terminus to C-terminus: TRAV1-optional linker -TRAC- linker -VL1- optional linker -hinge region -CH2-CH3, the second polypeptide chain comprises, from N-terminus to C-terminus: VH1- optional linker -hinge region -CH2-CH3, and the third polypeptide chain comprises, from N-terminus to C-terminus: TRBV1- optional linker -TRBC.

In some embodiments, the first polypeptide chain comprises, from N-terminus to C-terminus: TRAV1-optional linker -TRAC- linker -VH1- optional linker -hinge region -CH2-CH3, the second polypeptide chain comprises, from N-terminus to C-terminus: VL1- optional linker -hinge region -CH2-CH3, and the third polypeptide chain comprises, from N-terminus to C-terminus: TRBV1- optional linker -TRBC.

In some embodiments, the first polypeptide chain comprises, from N-terminus to C-terminus: TRBV1-optional linker -TRBC- linker -VL1, the second polypeptide chain comprises, from N-terminus to C-terminus: VH1- optional linker - optional hinge region - optional linker -Alb, and the third polypeptide chain comprises, from N-terminus to C-terminus: TRAV1- optional linker -TRAC- optional linker - optional hinge region.
the first polypeptide chain comprises, from N-terminus to C-terminus: TRBV1- optional linker -TRBC-linker -VL1- optional linker -Alb, the second polypeptide chain comprises, from N-terminus to C-terminus: VH1- optional linker - optional hinge region, and the third polypeptide chain comprises, from N-terminus to C-terminus: TRAV1- optional linker -TRAC- optional linker - optional hinge region.
the first polypeptide chain comprises, from N-terminus to C-terminus: TRBV1- optional linker -TRBC-linker -VL1, the second polypeptide chain comprises, from N-terminus to C-terminus: VH1- optional linker - optional hinge region, and the third polypeptide chain comprises, from N-terminus to C-terminus: TRAV1-optional linker -TRAC- optional linker - optional hinge region - optional linker -Alb.

In some embodiments, the first polypeptide chain comprises, from N-terminus to C-terminus: TRBV1-optional linker -TRBC- linker -VH1, the second polypeptide chain comprises, from N-terminus to C-terminus: VL1- optional linker - optional hinge region - optional linker -Alb, and the third polypeptide chain comprises, from N-terminus to C-terminus: TRAV1- optional linker -TRAC- optional linker - optional hinge region.

In some embodiments, the first polypeptide chain comprises, from N-terminus to C-terminus: TRBV1-optional linker -TRBC- linker -VH1- optional linker -Alb, the second polypeptide chain comprises, from N-terminus to C-terminus: VL1- optional linker - optional hinge region, and the third polypeptide chain comprises, from N-terminus to C-terminus: TRAV1- optional linker -TRAC- optional linker - optional hinge region.

In some embodiments, the first polypeptide chain comprises, from N-terminus to C-terminus: TRBV1-optional linker -TRBC- linker -VH1, the second polypeptide chain comprises, from N-terminus to C-terminus: VL1- optional linker - optional hinge region, and the third polypeptide chain comprises, from N-terminus to C-terminus: TRAV1- optional linker -TRAC- optional linker - optional hinge region - optional linker -Alb.

In some embodiments, the first polypeptide chain comprises, from N-terminus to C-terminus: TRAV1-optional linker -TRAC- linker -VL1, the second polypeptide chain comprises, from N-terminus to C-terminus: VH1- optional linker - optional hinge region - optional linker -Alb, and the third polypeptide chain comprises, from N-terminus to C-terminus: TRBV1- optional linker -TRBC- optional linker - optional hinge region.

In some embodiments, the first polypeptide chain comprises, from N-terminus to C-terminus: TRAV1-optional linker -TRAC- linker -VL1- optional linker -Alb, the second polypeptide chain comprises, from N-terminus to C-terminus: VH1- optional linker - optional hinge region, and the third polypeptide chain comprises, from N-terminus to C-terminus: TRBV1- optional linker -TRBC- optional linker - optional hinge region.

In some embodiments, the first polypeptide chain comprises, from N-terminus to C-terminus: TRAV1-optional linker -TRAC- linker -VL1, the second polypeptide chain comprises, from N-terminus to C-terminus: VH1- optional linker - optional hinge region, and the third polypeptide chain comprises, from N-terminus to C-terminus: TRBV1- optional linker -TRBC- optional linker - optional hinge region - optional linker -Alb.

In some embodiments, the first polypeptide chain comprises, from N-terminus to C-terminus: TRAV1-optional linker -TRAC- linker -VH1, the second polypeptide chain comprises, from N-terminus to C-terminus: VL1- optional linker - optional hinge region - optional linker -Alb, and the third polypeptide chain comprises, from N-terminus to C-terminus: TRBV1- optional linker -TRBC- optional linker - optional hinge region.

In some embodiments, the first polypeptide chain comprises, from N-terminus to C-terminus: TRAV1-optional linker -TRAC- linker -VH1- optional linker -Alb, the second polypeptide chain comprises, from N-terminus to C-terminus: VL1- optional linker - optional hinge region, and the third polypeptide chain comprises, from N-terminus to C-terminus: TRBV1- optional linker -TRBC- optional linker - optional hinge region.

In some embodiments, the first polypeptide chain comprises, from N-terminus to C-terminus: TRAV1-optional linker -TRAC- linker -VH1, the second polypeptide chain comprises, from N-terminus to C-terminus: VL1- optional linker - optional hinge region, and the third polypeptide chain comprises, from N-terminus to C-terminus: TRBV1- optional linker -TRBC- optional linker - optional hinge region - optional linker -Alb.
the first polypeptide chain comprises, from N-terminus to C-terminus: TRBV1- optional linker -TRBC-linker -VL1- optional linker - optional hinge region - optional linker -Alb, the second polypeptide chain comprises, from N-terminus to C-terminus: VH1- optional linker - optional hinge region, and the third polypeptide chain comprises, from N-terminus to C-terminus: TRAV1- optional linker -TRAC.
the first polypeptide chain comprises, from N-terminus to C-terminus: TRBV1- optional linker -TRBC-linker -VL1- optional linker - optional hinge region , the second polypeptide chain comprises, from N-terminus to C-terminus: VH1- optional linker - optional hinge region - optional linker -Alb, and the third polypeptide chain comprises, from N-terminus to C-terminus: TRAV1- optional linker -TRAC.
the first polypeptide chain comprises, from N-terminus to C-terminus: TRBV1- optional linker -TRBC-linker -VL1- optional linker - optional hinge region , the second polypeptide chain comprises, from N-terminus to C-terminus: VH1- optional linker - optional hinge region, and the third polypeptide chain comprises, from N-terminus to C-terminus: TRAV1- optional linker -TRAC- optional linker -Alb.

In some embodiments, the first polypeptide chain comprises, from N-terminus to C-terminus: TRBV1-optional linker -TRBC- linker -VH1- optional linker - optional hinge region - optional linker -Alb, the second polypeptide chain comprises, from N-terminus to C-terminus: VL1- optional linker - optional hinge region, and the third polypeptide chain comprises, from N-terminus to C-terminus: TRAV1- optional linker -TRAC.

In some embodiments, the first polypeptide chain comprises, from N-terminus to C-terminus: TRBV1-optional linker -TRBC- linker -VH1- optional linker - optional hinge region , the second polypeptide chain comprises, from N-terminus to C-terminus: VL1- optional linker - optional hinge region - optional linker -Alb, and the third polypeptide chain comprises, from N-terminus to C-terminus: TRAV1- optional linker -TRAC.

In some embodiments, the first polypeptide chain comprises, from N-terminus to C-terminus: TRBV1-optional linker -TRBC- linker -VH1- optional linker - optional hinge region , the second polypeptide chain comprises, from N-terminus to C-terminus: VL1- optional linker - optional hinge region, and the third polypeptide chain comprises, from N-terminus to C-terminus: TRAV1- optional linker -TRAC- optional linker -Alb.

In some embodiments, the first polypeptide chain comprises, from N-terminus to C-terminus: TRAV1-optional linker -TRAC- linker -VL1- optional linker - optional hinge region - optional linker -Alb, the second polypeptide chain comprises, from N-terminus to C-terminus: VH1- optional linker - optional hinge region, and the third polypeptide chain comprises, from N-terminus to C-terminus: TRBV1- optional linker -TRBC.

In some embodiments, the first polypeptide chain comprises, from N-terminus to C-terminus: TRAV1-optional linker -TRAC- linker -VL1- optional linker - optional hinge region , the second polypeptide chain comprises, from N-terminus to C-terminus: VH1- optional linker - optional hinge region - optional linker -Alb, and the third polypeptide chain comprises, from N-terminus to C-terminus: TRBV1- optional linker -TRBC.

In some embodiments, the first polypeptide chain comprises, from N-terminus to C-terminus: TRAV1-optional linker -TRAC- linker -VL1- optional linker - optional hinge region , the second polypeptide chain comprises, from N-terminus to C-terminus: VH1- optional linker - optional hinge region, and the third polypeptide chain comprises, from N-terminus to C-terminus: TRBV1- optional linker -TRBC- optional linker -Alb.

In some embodiments, the first polypeptide chain comprises, from N-terminus to C-terminus: TRAV1-optional linker -TRAC- linker -VH1- optional linker - optional hinge region - optional linker -Alb, the second polypeptide chain comprises, from N-terminus to C-terminus: VL1- optional linker - optional hinge region, and the third polypeptide chain comprises, from N-terminus to C-terminus: TRBV1- optional linker -TRBC.

In some embodiments, the first polypeptide chain comprises, from N-terminus to C-terminus: TRAV1-optional linker -TRAC- linker -VH1- optional linker - optional hinge region , the second polypeptide chain comprises, from N-terminus to C-terminus: VL1- optional linker - optional hinge region - optional linker -Alb, and the third polypeptide chain comprises, from N-terminus to C-terminus: TRBV1- optional linker -TRBC.

In some embodiments, the first polypeptide chain comprises, from N-terminus to C-terminus: TRAV1-optional linker -TRAC- linker -VH1- optional linker - optional hinge region , the second polypeptide chain comprises, from N-terminus to C-terminus: VL1- optional linker - optional hinge region, and the third polypeptide chain comprises, from N-terminus to C-terminus: TRBV1- optional linker -TRBC- optional linker -Alb.

In a fifth aspect, the present disclosure provides a multi-specific polypeptide molecule, wherein the multi-specific polypeptide molecule comprises, on two polypeptide chains, a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises heavy chain variable region VH1 and light chain variable region VL1 derived from an antibody that binds the second antigen;
wherein the first antigen is selected from the group consisting of tumor-associated antigens (TAA), tumor-specific antigens (TSA), viral antigens, and self-antigens, and the second antigen is an immune cell surface molecule;
wherein VH1 and VL1 are respectively distributed on two different polypeptide chains of the multi-specific polypeptide molecule, and the C-terminus of the polypeptide chain comprising VH1 or VL1 is connected to the N-terminus of the TCR;
wherein the multi-specific polypeptide molecule further comprises at least one of the following functional domains:
   1) a hinge region derived from an antibody;
   2) an Fc domain derived from an antibody or a dimerization portion thereof;
   3) a CH3 domain derived from an antibody;
   4) albumin (Alb) or a binding domain thereof;
   5) a constant region derived from a TCR or a fragment thereof.

In some embodiments, TRAV1 and TRBV1 are linked in tandem on one polypeptide chain. In some embodiments, VH1 and VL1 are respectively distributed on two different polypeptide chains of the multi-specific polypeptide molecule, and TRAV1 and TRBV1 are linked in tandem on one polypeptide chain, and the N-terminus thereof is connected to the C-terminus of the polypeptide chain comprising VH1 or VL1.

In some embodiments, adjacent variable regions in the multi-specific polypeptide molecule are connected by a linker.

In some embodiments, the multi-specific polypeptide molecule comprises:
VH1-TRBV1-TRAV1 and VL1; or
VL1-TRBV1-TRAV1 and VH1; or
VH1-TRAV1-TRBV1 and VL1; or
VL1-TRAV1-TRBV1 and VH1.

In some embodiments of the multi-specific polypeptide molecule according to the fifth aspect of the present disclosure, the multi-specific polypeptide molecule further comprises a constant domain selected from the group consisting of the following (1)-(3), which is connected to the C-terminus of TRAV1 and/or TRBV1 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments of the multi-specific polypeptide molecule according to the fifth aspect of the present disclosure, the multi-specific polypeptide molecule further comprises an antibody-derived CH1 and an antibody-derived CL which are respectively connected to the VH1 and VL1.

In embodiments of the multi-specific polypeptide molecule according to the fifth aspect of the present disclosure, the multi-specific polypeptide molecule further comprises a third binding region that binds a third antigen, wherein the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens, and self-antigens, and the third antigen is the same as or different from the first antigen. In some embodiments, the TRAV2 is the same as the TRAV1. In some embodiments, the TRBV2 is the same as the TRBV1.

In some embodiments, the TRAV2 and TRBV2 are linked in tandem on one polypeptide chain. In some embodiments, TRAV2 and TRBV2 are linked in tandem on one polypeptide chain and are distributed between TRAV1 and TRBV1. In some embodiments, the TRAV2 and TRBV2 are respectively distributed on two different polypeptide chains, and one of them is connected to the N-terminus of VH1 and the other is connected to the N-terminus of VL1.

In some embodiments of the multi-specific polypeptide molecule according to the fifth aspect of the present disclosure, the two polypeptide chains of the multi-specific polypeptide molecule respectively comprise:
VH1-TRBV1-TRAV2-TRBV2-TRAV1 and VL1; or
VH1-TRBV1-TRBV2-TRAV2-TRAV1 and VL1; or
VL1-TRBV1-TRAV2-TRBV2-TRAV1 and VH1; or
VL1-TRBV1-TRBV2-TRAV2-TRAV1 and VH1; or
VH1-TRAV1-TRAV2-TRBV2-TRBV1 and VL1; or
VH1-TRAV1-TRBV2-TRAV2-TRBV1 and VL1; or
VL1-TRAV1-TRBV2-TRAV2-TRBV1 and VH1; or
VL1-TRAV1-TRAV2-TRBV2-TRBV1 and VH1; or
TRBV2-VH1-TRBV1-TRAV1 and TRAV2-VL1; or
TRBV2-VL1-TRBV1-TRAV1 and TRAV2-VH1; or
TRBV2-VH1-TRAV1-TRBV1 and TRAV2-VL1; or
TRBV2-VL1-TRAV1-TRBV1 and TRAV2-VH1; or
TRAV2-VH1-TRBV1-TRAV1 and TRBV2-VL1; or
TRAV2-VL1-TRBV1-TRAV1 and TRBV2-VH1; or
TRAV2-VH1-TRAV1-TRBV1 and TRBV2-VL1; or
TRAV2-VL1-TRAV1-TRBV1 and TRBV2-VH1.

In some embodiments of the multi-specific polypeptide molecule according to the fifth aspect of the present disclosure, the multi-specific polypeptide molecule further comprises a constant domain selected from the group consisting of the following (1)-(3), which is connected to the C-terminus of TRAV2 and/or TRBV2 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments of the multi-specific polypeptide molecule according to the fifth aspect of the present disclosure, the multi-specific polypeptide molecule further comprises a fourth binding region that binds a fourth antigen, wherein the fourth binding region comprises a heavy chain variable region VH2 and a light chain variable region VL2 derived from an antibody that binds the fourth antigen, or a single heavy chain variable region (VHH) derived from a single-domain antibody or nanobody that binds the fourth antigen; wherein the fourth antigen is an immune cell surface molecule, and the fourth antigen is the same as or different from the second antigen. In some embodiments, the VH2 is the same as the VH1. In some embodiments, the VL2 is the same as the VL1.

In some embodiments, the VHH, or the scFv2 formed by VH2 and VL2 is connected to the N-terminus of the VH1 or the VL1 via a linker, or is distributed between TRAV1 and TRBV1, or one of VH2 and VL2 is connected to the N-terminus of the VH1 via a linker and the other is connected to the N-terminus of the VL1 via a linker.

In some embodiments of the multi-specific polypeptide molecule according to the fifth aspect of the present disclosure, the two polypeptide chains of the multi-specific polypeptide molecule respectively comprise:
scFv2-VH1-TRBV1-TRAV1 and VL1; or
scFv2-VL1-TRBV1-TRAV1 and VH1; or
scFv2-VH1-TRAV1-TRBV1 and VL1; or
scFv2-VL1-TRAV1-TRBV1 and VH1; or
VH1-TRBV1-scFv2-TRAV1 and VL1; or
VL1-TRBV1-scFv2-TRAV1 and VH1; or
VH1-TRAV1-scFv2-TRBV1 and VL1; or
VL1-TRAV1-scFv2-TRBV1 and VH1; or
VH1-TRBV1-VHH-TRAV1 and VL1; or
VL1-TRBV1-VHH-TRAV1 and VH1; or
VH1-TRAV1-VHH-TRBV1 and VL1; or
VL1-TRAV1-VHH-TRBV1 and VH1; or
VH1-TRBV1-TRAV1 and scFv2-VL1; or
VL1-TRBV1-TRAV1 and scFv2-VH1; or
VH1-TRAV1-TRBV1 and scFv2-VL1; or
VL1-TRAV1-TRBV1 and scFv2-VH1; or
VHH-VH1-TRBV1-TRAV1 and VL1; or
VHH-VL1-TRBV1-TRAV1 and VH1; or
VHH-VH1-TRAV1-TRBV1 and VL1; or
VHH-VL1-TRAV1-TRBV1 and VH1; or
VH1-TRBV1-TRAV1 and VHH-VL1; or
VL1-TRBV1-TRAV1 and VHH-VH1; or
VH1-TRAV1-TRBV1 and VHH-VL1; or
VL1-TRAV1-TRBV1 and VHH-VH1; or
VH2-VH1-TRBV1-TRAV1 and VL2-VL1; or
VL2-VH1-TRBV1-TRAV1 and VH2-VL1; or
VH2-VL1-TRBV1-TRAV1 and VL2-VH1; or
VL2-VL1-TRBV1-TRAV1 and VH2-VH1; or
VH2-VH1-TRAV1-TRBV1 and VL2-VL1; or
VL2-VH1-TRAV1-TRBV1 and VH2-VL1; or
VH2-VL1-TRAV1-TRBV1 and VL2-VH1; or
VL2-VL1-TRAV1-TRBV1 and VH2-VH1.

In some embodiments, the functional domain comprises two chains, and the two chains of the multi-specific polypeptide molecule are connected via covalent bonds or non-covalent bonds between the two chains of the functional domain.

In some embodiments, the functional domain comprises two chains, and the first binding region and the second binding region of the multi-specific polypeptide molecule are connected via covalent bonds or non-covalent bonds between the two chains of the functional domain.

In some embodiments, the functional domain comprises two chains, which are respectively connected to the C-terminus of the two polypeptide chains of the multi-specific polypeptide molecule.

In some embodiments, the functional domain comprises two chains, which are respectively connected to the N-terminus of the two polypeptide chains of the multi-specific polypeptide molecule.

In some embodiments, VH1 and VL1 are respectively distributed on two different polypeptide chains of the multi-specific polypeptide molecule, and TRAV1 and TRBV1 are linked in tandem on one polypeptide chain, and the N-terminus thereof is connected to the C-terminus of the polypeptide chain comprising VH1 or VL1, the functional domain comprises two chains, and the first binding region and the second binding region of the multi-specific polypeptide molecule are connected via covalent bonds or non-covalent bonds between the two chains of the functional domain, e.g., the C-terminus of one polypeptide chain of the multi-specific polypeptide molecule is connected to one chain of the functional domain, and the C-terminus of the other polypeptide chain of the multi-specific polypeptide molecule is connected to the other chain of the functional domain.

In some embodiments of the multi-specific polypeptide molecule of the fifth aspect of the present disclosure, the functional domain is derived from albumin or a binding domain thereof and an optional antibody hinge region; and the functional domain is connected to the C-terminus or N-terminus (preferably the C-terminus) of any one polypeptide chain of the multi-specific polypeptide molecule via an optional linker. In some embodiments, the functional domain is derived from albumin or a binding domain thereof, and the albumin or binding domain thereof is connected to the C-terminus or N-terminus (preferably the C-terminus) of any one polypeptide chain of the multi-specific polypeptide molecule via an optional linker. In some embodiments, the functional domain is derived from albumin or a binding domain thereof and an antibody hinge region, the functional domain is connected to the C-terminus of any one polypeptide chain of the multi-specific polypeptide molecule via an optional linker, and the albumin or binding domain thereof is connected to the C-terminus of any one chain of the hinge region via an optional linker.

In some embodiments, the polypeptide molecule comprises a first polypeptide chain and a second polypeptide chain; wherein the first polypeptide chain comprises, from N-terminus to C-terminus: VH1- linker -TRBV1- linker -TRAV1- optional linker - optional hinge region - optional linker -Alb, and the second polypeptide chain comprises, from N-terminus to C-terminus: VL1- optional linker - optional hinge region.

In some embodiments, the first polypeptide chain comprises, from N-terminus to C-terminus: VH1- linker -TRBV1- linker -TRAV1- optional linker - optional hinge region, and the second polypeptide chain comprises, from N-terminus to C-terminus: VL1- optional linker - optional hinge region - optional linker -Alb.

In some embodiments, the first polypeptide chain comprises, from N-terminus to C-terminus: VL1- linker -TRBV1- linker -TRAV1- optional linker - optional hinge region - optional linker -Alb, and the second polypeptide chain comprises, from N-terminus to C-terminus: VH1- optional linker - optional hinge region.

In some embodiments, the first polypeptide chain comprises, from N-terminus to C-terminus: VL1- linker -TRBV1- linker -TRAV1- optional linker - optional hinge region, and the second polypeptide chain comprises, from N-terminus to C-terminus: VH1- optional linker - optional hinge region - optional linker -Alb.

In some embodiments, the first polypeptide chain comprises, from N-terminus to C-terminus: VH1- linker -TRAV1- linker -TRBV1- optional linker - optional hinge region - optional linker -Alb, and the second polypeptide chain comprises, from N-terminus to C-terminus: VL1- optional linker - optional hinge region.

In some embodiments, the first polypeptide chain comprises, from N-terminus to C-terminus: VH1- linker -TRAV1- linker -TRBV1- optional linker - optional hinge region, and the second polypeptide chain comprises, from N-terminus to C-terminus: VL1- optional linker - optional hinge region - optional linker -Alb.

In some embodiments, the first polypeptide chain comprises, from N-terminus to C-terminus: VL1- linker -TRAV1- linker -TRBV1- optional linker - optional hinge region - optional linker -Alb, and the second polypeptide chain comprises, from N-terminus to C-terminus: VH1- optional linker - optional hinge region.

In some embodiments, the first polypeptide chain comprises, from N-terminus to C-terminus: VL1- linker -TRAV1- linker -TRBV1- optional linker - optional hinge region, and the second polypeptide chain comprises, from N-terminus to C-terminus: VH1- optional linker - optional hinge region - optional linker -Alb.

In some embodiments, the first polypeptide chain comprises, from N-terminus to C-terminus: scFv2- linker -VH1- linker -TRBV1- linker -TRAV1- optional linker - optional hinge region - optional linker -Alb, and the second polypeptide chain comprises, from N-terminus to C-terminus: VL1- optional linker - optional hinge region.

In some embodiments, the first polypeptide chain comprises, from N-terminus to C-terminus: scFv2- linker -VH1- linker -TRBV1- linker -TRAV1- optional linker - optional hinge region, and the second polypeptide chain comprises, from N-terminus to C-terminus: VL1- optional linker - optional hinge region - optional linker -Alb.

In some embodiments, the first polypeptide chain comprises, from N-terminus to C-terminus: scFv2- linker -VL1- linker -TRBV1- linker -TRAV1- optional linker - optional hinge region - optional linker -Alb, and the second polypeptide chain comprises, from N-terminus to C-terminus: VH1- optional linker - optional hinge region.

In some embodiments, the first polypeptide chain comprises, from N-terminus to C-terminus: scFv2- linker -VL1- linker -TRBV1- linker -TRAV1- optional linker - optional hinge region, and the second polypeptide chain comprises, from N-terminus to C-terminus: VH1- optional linker - optional hinge region - optional linker -Alb.

In some embodiments, the first polypeptide chain comprises, from N-terminus to C-terminus: scFv2- linker -VH1- linker -TRAV1- linker -TRBV1- optional linker - optional hinge region - optional linker -Alb, and the second polypeptide chain comprises, from N-terminus to C-terminus: VL1- optional linker - optional hinge region.

In some embodiments, the first polypeptide chain comprises, from N-terminus to C-terminus: scFv2- linker -VH1- linker -TRAV1- linker -TRBV1- optional linker - optional hinge region, and the second polypeptide chain comprises, from N-terminus to C-terminus: VL1- optional linker - optional hinge region - optional linker -Alb.

In some embodiments, the first polypeptide chain comprises, from N-terminus to C-terminus: scFv2- linker -VL1- linker -TRAV1- linker -TRBV1- optional linker - optional hinge region - optional linker -Alb, and the second polypeptide chain comprises, from N-terminus to C-terminus: VH1- optional linker - optional hinge region.

In some embodiments, the first polypeptide chain comprises, from N-terminus to C-terminus: scFv2- linker -VL1- linker -TRAV1- linker -TRBV1- optional linker - optional hinge region, and the second polypeptide chain comprises, from N-terminus to C-terminus: VH1- optional linker - optional hinge region - optional linker -Alb.

In some embodiments, the first polypeptide chain comprises, from N-terminus to C-terminus: VH1- linker -TRBV1- linker -TRAV1- optional linker - optional hinge region - optional linker -Alb, and the second polypeptide chain comprises, from N-terminus to C-terminus: scFv2- linker -VL1- optional linker - optional hinge region.

In some embodiments, the first polypeptide chain comprises, from N-terminus to C-terminus: VH1- linker -TRBV1- linker -TRAV1- optional linker - optional hinge region, and the second polypeptide chain comprises, from N-terminus to C-terminus: scFv2- linker -VL1- optional linker - optional hinge region - optional linker - Alb.

In some embodiments, the first polypeptide chain comprises, from N-terminus to C-terminus: VL1- linker -TRBV1- linker -TRAV1- optional linker - optional hinge region - optional linker -Alb, and the second polypeptide chain comprises, from N-terminus to C-terminus: scFv2- linker -VH1- optional linker - optional hinge region.

In some embodiments, the first polypeptide chain comprises, from N-terminus to C-terminus: VL1- linker -TRBV1- linker -TRAV1- optional linker - optional hinge region, and the second polypeptide chain comprises, from N-terminus to C-terminus: scFv2- linker -VH1- optional linker - optional hinge region - optional linker - Alb.

In some embodiments, the first polypeptide chain comprises, from N-terminus to C-terminus: VH1- linker -TRAV1- linker -TRBV1- optional linker - optional hinge region - optional linker -Alb, and the second polypeptide chain comprises, from N-terminus to C-terminus: scFv2- linker -VL1- optional linker - optional hinge region.

In some embodiments, the first polypeptide chain comprises, from N-terminus to C-terminus: VH1- linker -TRAV1- linker -TRBV1- optional linker - optional hinge region, and the second polypeptide chain comprises, from N-terminus to C-terminus: scFv2- linker -VL1- optional linker - optional hinge region - optional linker - Alb.

In some embodiments, the first polypeptide chain comprises, from N-terminus to C-terminus: VL1- linker -TRAV1- linker -TRBV1- optional linker - optional hinge region - optional linker -Alb, and the second polypeptide chain comprises, from N-terminus to C-terminus: scFv2- linker -VH1- optional linker - optional hinge region.

In some embodiments, the first polypeptide chain comprises, from N-terminus to C-terminus: VL1- linker -TRAV1- linker -TRBV1- optional linker - optional hinge region, and the second polypeptide chain comprises, from N-terminus to C-terminus: scFv2- linker -VH1- optional linker - optional hinge region - optional linker - Alb.

In some embodiments, the first polypeptide chain comprises, from N-terminus to C-terminus: VHH- linker -VH1- linker -TRBV1- linker -TRAV1- optional linker - optional hinge region - optional linker -Alb, and the second polypeptide chain comprises, from N-terminus to C-terminus: VL1- optional linker - optional hinge region.

In some embodiments, the first polypeptide chain comprises, from N-terminus to C-terminus: VHH- linker -VH1- linker -TRBV1- linker -TRAV1- optional linker - optional hinge region, and the second polypeptide chain comprises, from N-terminus to C-terminus: VL1- optional linker - optional hinge region - optional linker -Alb.

In some embodiments, the first polypeptide chain comprises, from N-terminus to C-terminus: VHH- linker -VL1- linker -TRBV1- linker -TRAV1- optional linker - optional hinge region - optional linker -Alb, and the second polypeptide chain comprises, from N-terminus to C-terminus: VH1- optional linker - optional hinge region.

In some embodiments, the first polypeptide chain comprises, from N-terminus to C-terminus: VHH- linker -VL1- linker -TRBV1- linker -TRAV1- optional linker - optional hinge region, and the second polypeptide chain comprises, from N-terminus to C-terminus: VH1- optional linker - optional hinge region - optional linker -Alb.

In some embodiments, the first polypeptide chain comprises, from N-terminus to C-terminus: VHH- linker -VH1- linker -TRAV1- linker -TRBV1- optional linker - optional hinge region - optional linker -Alb, and the second polypeptide chain comprises, from N-terminus to C-terminus: VL1- optional linker - optional hinge region.

In some embodiments, the first polypeptide chain comprises, from N-terminus to C-terminus: VHH- linker -VH1- linker -TRAV1- linker -TRBV1- optional linker - optional hinge region, and the second polypeptide chain comprises, from N-terminus to C-terminus: VL1- optional linker - optional hinge region - optional linker -Alb.

In some embodiments, the first polypeptide chain comprises, from N-terminus to C-terminus: VHH- linker -VL1- linker -TRAV1- linker -TRBV1- optional linker - optional hinge region - optional linker -Alb, and the second polypeptide chain comprises, from N-terminus to C-terminus: VH1- optional linker - optional hinge region.

In some embodiments, the first polypeptide chain comprises, from N-terminus to C-terminus: VHH- linker -VL1- linker -TRAV1- linker -TRBV1- optional linker - optional hinge region, and the second polypeptide chain comprises, from N-terminus to C-terminus: VH1- optional linker - optional hinge region - optional linker -Alb.

In some embodiments, the first polypeptide chain comprises, from N-terminus to C-terminus: VH1- linker -TRBV1- linker -TRAV1- optional linker - optional hinge region - optional linker -Alb, and the second polypeptide chain comprises, from N-terminus to C-terminus: VHH- linker -VL1- optional linker - optional hinge region.

In some embodiments, the first polypeptide chain comprises, from N-terminus to C-terminus: VH1- linker -TRBV1- linker -TRAV1- optional linker - optional hinge region, and the second polypeptide chain comprises, from N-terminus to C-terminus: VHH- linker -VL1- optional linker - optional hinge region - optional linker - Alb.

In some embodiments, the first polypeptide chain comprises, from N-terminus to C-terminus: VL1- linker -TRBV1- linker -TRAV1- optional linker - optional hinge region - optional linker -Alb, and the second polypeptide chain comprises, from N-terminus to C-terminus: VHH- linker -VH1- optional linker - optional hinge region.

In some embodiments, the first polypeptide chain comprises, from N-terminus to C-terminus: VL1- linker -TRBV1- linker -TRAV1- optional linker - optional hinge region, and the second polypeptide chain comprises, from N-terminus to C-terminus: VHH- linker -VH1- optional linker - optional hinge region - optional linker - Alb.

In some embodiments, the first polypeptide chain comprises, from N-terminus to C-terminus: VH1- linker -TRAV1- linker -TRBV1- optional linker - optional hinge region - optional linker -Alb, and the second polypeptide chain comprises, from N-terminus to C-terminus: VHH- linker -VL1- optional linker - optional hinge region.

In some embodiments, the first polypeptide chain comprises, from N-terminus to C-terminus: VH1- linker -TRAV1- linker -TRBV1- optional linker - optional hinge region, and the second polypeptide chain comprises, from N-terminus to C-terminus: VHH- linker -VL1- optional linker - optional hinge region - optional linker - Alb.

In some embodiments, the first polypeptide chain comprises, from N-terminus to C-terminus: VL1- linker -TRAV1- linker -TRBV1- optional linker - optional hinge region - optional linker -Alb, and the second polypeptide chain comprises, from N-terminus to C-terminus: VHH- linker -VH1- optional linker - optional hinge region.

In some embodiments, the first polypeptide chain comprises, from N-terminus to C-terminus: VL1- linker -TRAV1- linker -TRBV1- optional linker - optional hinge region, and the second polypeptide chain comprises, from N-terminus to C-terminus: VHH- linker -VH1- optional linker - optional hinge region - optional linker - Alb.

In some embodiments, the first polypeptide chain comprises, from N-terminus to C-terminus: VH2- linker -VH1- linker -TRBV1- linker -TRAV1- optional linker - optional hinge region - optional linker -Alb, and the second polypeptide chain comprises, from N-terminus to C-terminus: VL2-VL1- optional linker - optional hinge region.

In some embodiments, the first polypeptide chain comprises, from N-terminus to C-terminus: VH2- linker -VH1- linker -TRBV1- linker -TRAV1- optional linker - optional hinge region, and the second polypeptide chain comprises, from N-terminus to C-terminus: VL2-VL1- optional linker - optional hinge region - optional linker -Alb.

In some embodiments, the first polypeptide chain comprises, from N-terminus to C-terminus: VL2- linker -VH1- linker -TRBV1- linker -TRAV1- optional linker - optional hinge region - optional linker -Alb, and the second polypeptide chain comprises, from N-terminus to C-terminus: VH2-VL1- optional linker - optional hinge region.

In some embodiments, the first polypeptide chain comprises, from N-terminus to C-terminus: VL2- linker -VH1- linker -TRBV1- linker -TRAV1- optional linker - optional hinge region, and the second polypeptide chain comprises, from N-terminus to C-terminus: VH2-VL1- optional linker - optional hinge region - optional linker -Alb.

In some embodiments, the first polypeptide chain comprises, from N-terminus to C-terminus: VH2- linker -VL1- linker -TRBV1- linker -TRAV1- optional linker - optional hinge region - optional linker -Alb, and the second polypeptide chain comprises, from N-terminus to C-terminus: VL2-VH1- optional linker - optional hinge region.

In some embodiments, the first polypeptide chain comprises, from N-terminus to C-terminus: VH2- linker -VL1- linker -TRBV1- linker -TRAV1- optional linker - optional hinge region, and the second polypeptide chain comprises, from N-terminus to C-terminus: VL2-VH1- optional linker - optional hinge region - optional linker -Alb.

In some embodiments, the first polypeptide chain comprises, from N-terminus to C-terminus: VL2- linker -VL1- linker -TRBV1- linker -TRAV1- optional linker - optional hinge region - optional linker -Alb, and the second polypeptide chain comprises, from N-terminus to C-terminus: VH2-VH1- optional linker - optional hinge region.

In some embodiments, the first polypeptide chain comprises, from N-terminus to C-terminus: VL2- linker -VL1- linker -TRBV1- linker -TRAV1- optional linker - optional hinge region, and the second polypeptide chain comprises, from N-terminus to C-terminus: VH2-VH1- optional linker - optional hinge region - optional linker -Alb.

In some embodiments, the first polypeptide chain comprises, from N-terminus to C-terminus: VH2- linker -VH1- linker -TRAV1- linker -TRBV1- optional linker - optional hinge region - optional linker -Alb, and the second polypeptide chain comprises, from N-terminus to C-terminus: VL2-VL1- optional linker - optional hinge region.

In some embodiments, the first polypeptide chain comprises, from N-terminus to C-terminus: VH2- linker -VH1- linker -TRAV1- linker -TRBV1- optional linker - optional hinge region, and the second polypeptide chain comprises, from N-terminus to C-terminus: VL2-VL1- optional linker - optional hinge region - optional linker -Alb.

In some embodiments, the first polypeptide chain comprises, from N-terminus to C-terminus: VL2- linker -VH1- linker -TRAV1- linker -TRBV1- optional linker - optional hinge region - optional linker -Alb, and the second polypeptide chain comprises, from N-terminus to C-terminus: VH2-VL1- optional linker - optional hinge region.

In some embodiments, the first polypeptide chain comprises, from N-terminus to C-terminus: VL2- linker -VH1- linker -TRAV1- linker -TRBV1- optional linker - optional hinge region, and the second polypeptide chain comprises, from N-terminus to C-terminus: VH2-VL1- optional linker - optional hinge region - optional linker -Alb.

In some embodiments, the first polypeptide chain comprises, from N-terminus to C-terminus: VH2- linker -VL1- linker -TRAV1- linker -TRBV1- optional linker - optional hinge region - optional linker -Alb, and the second polypeptide chain comprises, from N-terminus to C-terminus: VL2-VH1- optional linker - optional hinge region.

In some embodiments, the first polypeptide chain comprises, from N-terminus to C-terminus: VH2- linker -VL1- linker -TRAV1- linker -TRBV1- optional linker - optional hinge region, and the second polypeptide chain comprises, from N-terminus to C-terminus: VL2-VH1- optional linker - optional hinge region - optional linker -Alb.

In some embodiments, the first polypeptide chain comprises, from N-terminus to C-terminus: VL2- linker -VL1- linker -TRAV1- linker -TRBV1- optional linker - optional hinge region - optional linker -Alb, and the second polypeptide chain comprises, from N-terminus to C-terminus: VH2-VH1- optional linker - optional hinge region.

In some embodiments, the first polypeptide chain comprises, from N-terminus to C-terminus: VL2- linker -VL1- linker -TRAV1- linker -TRBV1- optional linker - optional hinge region, and the second polypeptide chain comprises, from N-terminus to C-terminus: VH2-VH1- optional linker - optional hinge region - optional linker -Alb.

In some embodiments of the multi-specific polypeptide molecule according to the fifth aspect of the present disclosure, the functional domain is derived from an antibody hinge region and/or an antibody Fc domain or a dimerization portion thereof and/or an antibody CH3 domain; and the functional domain is connected to the C-terminus or N-terminus (preferably the C-terminus) of the two polypeptide chains of the multi-specific polypeptide molecule via an optional linker.

In some embodiments, the functional domain is derived from an antibody hinge region and an antibody Fc domain or a dimerization portion thereof.

In some embodiments, the polypeptide molecule comprises a first polypeptide chain and a second polypeptide chain; wherein the first polypeptide chain comprises, from N-terminus to C-terminus: VH1- linker -TRBV1- linker -TRAV1- optional linker -hinge region -CH2-CH3, and the second polypeptide chain comprises, from N-terminus to C-terminus: VL1- optional linker -hinge region -CH2-CH3 (format 62-2).

In some embodiments, the polypeptide molecule comprises a first polypeptide chain and a second polypeptide chain; wherein the first polypeptide chain comprises, from N-terminus to C-terminus: CH3-CH2-hinge region - optional linker -VH1- linker -TRBV1- linker -TRAV1, and the second polypeptide chain comprises, from N-terminus to C-terminus: CH3-CH2-hinge region - optional linker -VL1.

In some embodiments, the first polypeptide chain comprises, from N-terminus to C-terminus: VL1- linker -TRBV1- linker -TRAV1- optional linker -hinge region -CH2-CH3, and the second polypeptide chain comprises, from N-terminus to C-terminus: VH1- optional linker -hinge region -CH2-CH3.

In some embodiments, the first polypeptide chain comprises, from N-terminus to C-terminus: VH1- linker -TRAV1- linker -TRBV1- optional linker -hinge region -CH2-CH3, and the second polypeptide chain comprises, from N-terminus to C-terminus: VL1- optional linker -hinge region -CH2-CH3.

In some embodiments, the first polypeptide chain comprises, from N-terminus to C-terminus: VL1- linker -TRAV1- linker -TRBV1- optional linker -hinge region -CH2-CH3, and the second polypeptide chain comprises, from N-terminus to C-terminus: VH1- optional linker -hinge region -CH2-CH3.

In some embodiments, the polypeptide molecule comprises a first polypeptide chain and a second polypeptide chain; wherein; wherein the first polypeptide chain comprises, from N-terminus to C-terminus: CH3-CH2-hinge region - optional linker -VH1- linker -TRBV1- linker -TRAV1, and the second polypeptide chain comprises, from N-terminus to C-terminus: CH3-CH2-hinge region - optional linker -VL1- optional linker.

In some embodiments, the first polypeptide chain comprises, from N-terminus to C-terminus: CH3-CH2-hinge region - optional linker -VL1- linker -TRBV1- linker -TRAV1, and the second polypeptide chain comprises, from N-terminus to C-terminus: CH3-CH2-hinge region - optional linker -VH1.

In some embodiments, the first polypeptide chain comprises, from N-terminus to C-terminus: CH3-CH2-hinge region - optional linker -VH1- linker -TRAV1- linker -TRBV1, and the second polypeptide chain comprises, from N-terminus to C-terminus: CH3-CH2-hinge region - optional linker -VL1.

In some embodiments, the first polypeptide chain comprises, from N-terminus to C-terminus: CH3-CH2-hinge region - optional linker -VL1- linker -TRAV1- linker -TRBV1, and the second polypeptide chain comprises, from N-terminus to C-terminus: CH3-CH2-hinge region - optional linker -VH1.

In some embodiments, the first polypeptide chain comprises, from N-terminus to C-terminus: scFv2- linker -VH1- linker -TRBV1- linker -TRAV1- optional linker -hinge region -CH2-CH3, and the second polypeptide chain comprises, from N-terminus to C-terminus: VL1- optional linker -hinge region -CH2-CH3.

In some embodiments, the first polypeptide chain comprises, from N-terminus to C-terminus: scFv2- linker -VL1- linker -TRBV1- linker -TRAV1- optional linker -hinge region -CH2-CH3, and the second polypeptide chain comprises, from N-terminus to C-terminus: VH1- optional linker -hinge region -CH2-CH3.

In some embodiments, the first polypeptide chain comprises, from N-terminus to C-terminus: scFv2- linker -VH1- linker -TRAV1- linker -TRBV1- optional linker -hinge region -CH2-CH3, and the second polypeptide chain comprises, from N-terminus to C-terminus: VL1- optional linker -hinge region -CH2-CH3.

In some embodiments, the first polypeptide chain comprises, from N-terminus to C-terminus: scFv2- linker -VL1- linker -TRAV1- linker -TRBV1- optional linker -hinge region -CH2-CH3, and the second polypeptide chain comprises, from N-terminus to C-terminus: VH1- optional linker -hinge region -CH2-CH3.

In some embodiments, the first polypeptide chain comprises, from N-terminus to C-terminus: VH1- linker -TRBV1- linker -TRAV1- optional linker -hinge region -CH2-CH3, and the second polypeptide chain comprises, from N-terminus to C-terminus: scFv2- linker -VL1- optional linker -hinge region -CH2-CH3 (format 72-1).

In some embodiments, the first polypeptide chain comprises, from N-terminus to C-terminus: VL1- linker -TRBV1- linker -TRAV1- optional linker -hinge region -CH2-CH3, and the second polypeptide chain comprises, from N-terminus to C-terminus: scFv2- linker -VH1- optional linker -hinge region -CH2-CH3.

In some embodiments, the first polypeptide chain comprises, from N-terminus to C-terminus: VH1- linker -TRAV1- linker -TRBV1- optional linker -hinge region -CH2-CH3, and the second polypeptide chain comprises, from N-terminus to C-terminus: scFv2- linker -VL1- optional linker -hinge region -CH2-CH3.

In some embodiments, the first polypeptide chain comprises, from N-terminus to C-terminus: VL1- linker -TRAV1- linker -TRBV1- optional linker -hinge region -CH2-CH3, and the second polypeptide chain comprises, from N-terminus to C-terminus: scFv2- linker -VH1- optional linker -hinge region -CH2-CH3.

In some embodiments, the first polypeptide chain comprises, from N-terminus to C-terminus: VHH- linker -VH1- linker -TRBV1- linker -TRAV1- optional linker -hinge region -CH2-CH3, and the second polypeptide chain comprises, from N-terminus to C-terminus: VL1- optional linker -hinge region -CH2-CH3.

In some embodiments, the first polypeptide chain comprises, from N-terminus to C-terminus: VHH- linker -VL1- linker -TRBV1- linker -TRAV1- optional linker -hinge region -CH2-CH3, and the second polypeptide chain comprises, from N-terminus to C-terminus: VH1- optional linker -hinge region -CH2-CH3.

In some embodiments, the first polypeptide chain comprises, from N-terminus to C-terminus: VHH- linker -VH1- linker -TRAV1- linker -TRBV1- optional linker -hinge region -CH2-CH3, and the second polypeptide chain comprises, from N-terminus to C-terminus: VL1- optional linker -hinge region -CH2-CH3.

In some embodiments, the first polypeptide chain comprises, from N-terminus to C-terminus: VHH- linker -VL1- linker -TRAV1- linker -TRBV1- optional linker -hinge region -CH2-CH3, and the second polypeptide chain comprises, from N-terminus to C-terminus: VH1- optional linker -hinge region -CH2-CH3.

In some embodiments, the first polypeptide chain comprises, from N-terminus to C-terminus: VH1- linker -TRBV1- linker -TRAV1- optional linker -hinge region -CH2-CH3, and the second polypeptide chain comprises, from N-terminus to C-terminus: VHH- linker -VL1- optional linker -hinge region -CH2-CH3.

In some embodiments, the first polypeptide chain comprises, from N-terminus to C-terminus: VL1- linker -TRBV1- linker -TRAV1- optional linker -hinge region -CH2-CH3, and the second polypeptide chain comprises, from N-terminus to C-terminus: VHH- linker -VH1- optional linker -hinge region -CH2-CH3.

In some embodiments, the first polypeptide chain comprises, from N-terminus to C-terminus: VH1- linker -TRAV1- linker -TRBV1- optional linker -hinge region -CH2-CH3, and the second polypeptide chain comprises, from N-terminus to C-terminus: VHH- linker -VL1- optional linker -hinge region -CH2-CH3.

In some embodiments, the first polypeptide chain comprises, from N-terminus to C-terminus: VL1- linker -TRAV1- linker -TRBV1- optional linker -hinge region -CH2-CH3, and the second polypeptide chain comprises, from N-terminus to C-terminus: VHH- linker -VH1- optional linker -hinge region -CH2-CH3.

In some embodiments, the first polypeptide chain comprises, from N-terminus to C-terminus: VH2- linker -VH1- linker -TRBV1- linker -TRAV1- optional linker -hinge region -CH2-CH3, and the second polypeptide chain comprises, from N-terminus to C-terminus: VL2-VL1- optional linker -hinge region -CH2-CH3.

In some embodiments, the first polypeptide chain comprises, from N-terminus to C-terminus: VL2- linker -VH1- linker -TRBV1- linker -TRAV1- optional linker -hinge region -CH2-CH3, and the second polypeptide chain comprises, from N-terminus to C-terminus: VH2-VL1- optional linker -hinge region -CH2-CH3.

In some embodiments, the first polypeptide chain comprises, from N-terminus to C-terminus: VH2- linker -VL1- linker -TRBV1- linker -TRAV1- optional linker -hinge region -CH2-CH3, and the second polypeptide chain comprises, from N-terminus to C-terminus: VL2-VH1- optional linker -hinge region -CH2-CH3.

In some embodiments, the first polypeptide chain comprises, from N-terminus to C-terminus: VL2- linker -VL1- linker -TRBV1- linker -TRAV1- optional linker -hinge region -CH2-CH3, and the second polypeptide chain comprises, from N-terminus to C-terminus: VH2-VH1- optional linker -hinge region -CH2-CH3.

In some embodiments, the first polypeptide chain comprises, from N-terminus to C-terminus: VH2- linker -VH1- linker -TRAV1- linker -TRBV1- optional linker -hinge region -CH2-CH3, and the second polypeptide chain comprises, from N-terminus to C-terminus: VL2-VL1- optional linker -hinge region -CH2-CH3.

In some embodiments, the first polypeptide chain comprises, from N-terminus to C-terminus: VL2- linker -VH1- linker -TRAV1- linker -TRBV1- optional linker -hinge region -CH2-CH3, and the second polypeptide chain comprises, from N-terminus to C-terminus: VH2-VL1- optional linker -hinge region -CH2-CH3.

In some embodiments, the first polypeptide chain comprises, from N-terminus to C-terminus: VH2- linker -VL1- linker -TRAV1- linker -TRBV1- optional linker -hinge region -CH2-CH3, and the second polypeptide chain comprises, from N-terminus to C-terminus: VL2-VH1- optional linker -hinge region -CH2-CH3.

In some embodiments, the first polypeptide chain comprises, from N-terminus to C-terminus: VL2- linker -VL1- linker -TRAV1- linker -TRBV1- optional linker -hinge region -CH2-CH3, and the second polypeptide chain comprises, from N-terminus to C-terminus: VH2-VH1- optional linker -hinge region -CH2-CH3.

In some embodiments, the functional domain is derived from an antibody CH3 domain.

In some embodiments, the polypeptide molecule comprises a first polypeptide chain and a second polypeptide chain; wherein the first polypeptide chain comprises, from N-terminus to C-terminus: VH1- linker -TRBV1- linker -TRAV1- optional linker -CH3, and the second polypeptide chain comprises, from N-terminus to C-terminus: VL1- optional linker -CH3 (format 22-3).

In some embodiments, the polypeptide molecule comprises a first polypeptide chain and a second polypeptide chain; wherein the first polypeptide chain comprises, from N-terminus to C-terminus: CH3-optional linker -VH1- linker -TRBV1- linker -TRAV1, and the second polypeptide chain comprises, from N-terminus to C-terminus: CH3- optional linker -VL1.

In some embodiments, the first polypeptide chain comprises, from N-terminus to C-terminus: VL1- linker -TRBV1- linker -TRAV1- optional linker -CH3, and the second polypeptide chain comprises, from N-terminus to C-terminus: VH1- optional linker -CH3.

In some embodiments, the first polypeptide chain comprises, from N-terminus to C-terminus: VH1- linker -TRAV1- linker -TRBV1- optional linker -CH3, and the second polypeptide chain comprises, from N-terminus to C-terminus: VL1- optional linker -CH3.

In some embodiments, the first polypeptide chain comprises, from N-terminus to C-terminus: VL1- linker -TRAV1- linker -TRBV1- optional linker -CH3, and the second polypeptide chain comprises, from N-terminus to C-terminus: VH1- optional linker -CH3.

In some embodiments, the polypeptide molecule comprises a first polypeptide chain and a second polypeptide chain; wherein the first polypeptide chain comprises, from N-terminus to C-terminus: CH3-optional linker -VH1- linker -TRBV1- linker -TRAV1, and the second polypeptide chain comprises, from N-terminus to C-terminus: CH3- optional linker -VL1- optional linker.

In some embodiments, the first polypeptide chain comprises, from N-terminus to C-terminus: CH3-optional linker -VL1- linker -TRBV1- linker -TRAV1, and the second polypeptide chain comprises, from N-terminus to C-terminus: CH3 - optional linker -VH1.

In some embodiments, the first polypeptide chain comprises, from N-terminus to C-terminus: CH3 - optional linker -VH1- linker -TRAV1- linker -TRBV1, and the second polypeptide chain comprises, from N-terminus to C-terminus: CH3 - optional linker -VL1.

In some embodiments, the first polypeptide chain comprises, from N-terminus to C-terminus: CH3 - optional linker -VL1- linker -TRAV1- linker -TRBV1, and the second polypeptide chain comprises, from N-terminus to C-terminus: CH3 - optional linker -VH1.

In some embodiments, the first polypeptide chain comprises, from N-terminus to C-terminus: scFv2- linker -VH1- linker -TRBV1- linker -TRAV1- optional linker -CH3, and the second polypeptide chain comprises, from N-terminus to C-terminus: VL1- optional linker -CH3.

In some embodiments, the first polypeptide chain comprises, from N-terminus to C-terminus: scFv2- linker -VL1- linker -TRBV1- linker -TRAV1- optional linker -CH3, and the second polypeptide chain comprises, from N-terminus to C-terminus: VH1- optional linker -CH3.

In some embodiments, the first polypeptide chain comprises, from N-terminus to C-terminus: scFv2- linker -VH1- linker -TRAV1- linker -TRBV1- optional linker -CH3, and the second polypeptide chain comprises, from N-terminus to C-terminus: VL1- optional linker -CH3.

In some embodiments, the first polypeptide chain comprises, from N-terminus to C-terminus: scFv2- linker -VL1- linker -TRAV1- linker -TRBV1- optional linker -CH3, and the second polypeptide chain comprises, from N-terminus to C-terminus: VH1- optional linker -CH3.

In some embodiments, the first polypeptide chain comprises, from N-terminus to C-terminus: VH1- linker -TRBV1- linker -TRAV1- optional linker -CH3, and the second polypeptide chain comprises, from N-terminus to C-terminus: scFv2- linker -VL1- optional linker -CH3.

In some embodiments, the first polypeptide chain comprises, from N-terminus to C-terminus: VL1- linker -TRBV1- linker -TRAV1- optional linker - CH3, and the second polypeptide chain comprises, from N-terminus to C-terminus: scFv2- linker -VH1- optional linker -CH3.

In some embodiments, the first polypeptide chain comprises, from N-terminus to C-terminus: VH1- linker -TRAV1- linker -TRBV1- optional linker - CH3, and the second polypeptide chain comprises, from N-terminus to C-terminus: scFv2- linker -VL1- optional linker -CH3.

In some embodiments, the first polypeptide chain comprises, from N-terminus to C-terminus: VL1- linker -TRAV1- linker -TRBV1- optional linker -CH3, and the second polypeptide chain comprises, from N-terminus to C-terminus: scFv2- linker -VH1- optional linker -CH3.

In some embodiments, the first polypeptide chain comprises, from N-terminus to C-terminus: VHH- linker -VH1- linker -TRBV1- linker -TRAV1- optional linker -CH3, and the second polypeptide chain comprises, from N-terminus to C-terminus: VL1- optional linker -CH3.

In some embodiments, the first polypeptide chain comprises, from N-terminus to C-terminus: VHH- linker -VL1- linker -TRBV1- linker -TRAV1- optional linker -CH3, and the second polypeptide chain comprises, from N-terminus to C-terminus: VH1- optional linker -CH3.

In some embodiments, the first polypeptide chain comprises, from N-terminus to C-terminus: VHH- linker -VH1- linker -TRAV1- linker -TRBV1- optional linker -CH3, and the second polypeptide chain comprises, from N-terminus to C-terminus: VL1- optional linker -CH3.

In some embodiments, the first polypeptide chain comprises, from N-terminus to C-terminus: VHH- linker -VL1- linker -TRAV1- linker -TRBV1- optional linker -CH3, and the second polypeptide chain comprises, from N-terminus to C-terminus: VH1- optional linker -CH3.

In some embodiments, the first polypeptide chain comprises, from N-terminus to C-terminus: VH1- linker -TRBV1- linker -TRAV1- optional linker -CH3, and the second polypeptide chain comprises, from N-terminus to C-terminus: VHH- linker -VL1- optional linker -CH3.

In some embodiments, the first polypeptide chain comprises, from N-terminus to C-terminus: VL1- linker -TRBV1- linker -TRAV1- optional linker -CH3, and the second polypeptide chain comprises, from N-terminus to C-terminus: VHH- linker -VH1- optional linker -CH3.

In some embodiments, the first polypeptide chain comprises, from N-terminus to C-terminus: VH1- linker -TRAV1- linker -TRBV1- optional linker -CH3, and the second polypeptide chain comprises, from N-terminus to C-terminus: VHH- linker -VL1- optional linker -CH3.

In some embodiments, the first polypeptide chain comprises, from N-terminus to C-terminus: VL1- linker -TRAV1- linker -TRBV1- optional linker -CH3, and the second polypeptide chain comprises, from N-terminus to C-terminus: VHH- linker -VH1- optional linker -CH3.

In some embodiments, the first polypeptide chain comprises, from N-terminus to C-terminus: VH2- linker -VH1- linker -TRBV1- linker -TRAV1- optional linker -CH3, and the second polypeptide chain comprises, from N-terminus to C-terminus: VL2-VL1- optional linker -CH3.

In some embodiments, the first polypeptide chain comprises, from N-terminus to C-terminus: VL2- linker -VH1- linker -TRBV1- linker -TRAV1- optional linker -CH3, and the second polypeptide chain comprises, from N-terminus to C-terminus: VH2-VL1- optional linker -CH3.

In some embodiments, the first polypeptide chain comprises, from N-terminus to C-terminus: VH2- linker -VL1- linker -TRBV1- linker -TRAV1- optional linker -CH3, and the second polypeptide chain comprises, from N-terminus to C-terminus: VL2-VH1- optional linker -CH3.

In some embodiments, the first polypeptide chain comprises, from N-terminus to C-terminus: VL2- linker -VL1- linker -TRBV1- linker -TRAV1- optional linker -CH3, and the second polypeptide chain comprises, from N-terminus to C-terminus: VH2-VH1- optional linker -CH3.

In some embodiments, the first polypeptide chain comprises, from N-terminus to C-terminus: VH2- linker -VH1- linker -TRAV1- linker -TRBV1- optional linker -CH3, and the second polypeptide chain comprises, from N-terminus to C-terminus: VL2-VL1- optional linker -CH3.

In some embodiments, the first polypeptide chain comprises, from N-terminus to C-terminus: VL2- linker -VH1- linker -TRAV1- linker -TRBV1- optional linker -CH3, and the second polypeptide chain comprises, from N-terminus to C-terminus: VH2-VL1- optional linker -CH3.

In some embodiments, the first polypeptide chain comprises, from N-terminus to C-terminus: VH2- linker -VL1- linker -TRAV1- linker -TRBV1- optional linker -CH3, and the second polypeptide chain comprises, from N-terminus to C-terminus: VL2-VH1- optional linker -CH3.

In some embodiments, the first polypeptide chain comprises, from N-terminus to C-terminus: VL2- linker -VL1- linker -TRAV1- linker -TRBV1- optional linker -CH3, and the second polypeptide chain comprises, from N-terminus to C-terminus: VH2-VH1- optional linker -CH3.

In some embodiments of the multi-specific polypeptide molecule of the fifth aspect of the present disclosure, the functional domain is derived from a TCR constant region or a fragment thereof and an optional antibody hinge region; and the functional domain is connected to the C-terminus or N-terminus (preferably the C-terminus) of the two polypeptide chains of the multi-specific polypeptide molecule via an optional linker.

In some embodiments, the polypeptide molecule comprises a first polypeptide chain and a second polypeptide chain; wherein the first polypeptide chain comprises, from N-terminus to C-terminus: VH1- linker -TRBV1- linker -TRAV1- optional linker -TRAC, and the second polypeptide chain comprises, from N-terminus to C-terminus: VL1- optional linker -TRBC (format 22-4).

In some embodiments, the polypeptide molecule comprises a first polypeptide chain and a second polypeptide chain; wherein the first polypeptide chain comprises, from N-terminus to C-terminus: TRAC-optional linker -VH1- linker -TRBV1- linker -TRAV1, and the second polypeptide chain comprises, from N-terminus to C-terminus: TRBC- optional linker -VL1.

In some embodiments, the first polypeptide chain comprises, from N-terminus to C-terminus: VL1- linker -TRBV1- linker -TRAV1- optional linker -TRAC, and the second polypeptide chain comprises, from N-terminus to C-terminus: VH1- optional linker - TRBC.

In some embodiments, the first polypeptide chain comprises, from N-terminus to C-terminus: VH1- linker -TRAV1- linker -TRBV1- optional linker - TRBC, and the second polypeptide chain comprises, from N-terminus to C-terminus: VL1- optional linker - TRAC.

In some embodiments, the first polypeptide chain comprises, from N-terminus to C-terminus: VL1- linker -TRAV1- linker -TRBV1- optional linker - TRBC, and the second polypeptide chain comprises, from N-terminus to C-terminus: VH1- optional linker - TRAC.

In some embodiments, the first polypeptide chain comprises, from N-terminus to C-terminus: scFv2- linker -VH1- linker -TRBV1- linker -TRAV1- optional linker - TRAC, and the second polypeptide chain comprises, from N-terminus to C-terminus: VL1- optional linker - TRBC.

In some embodiments, the first polypeptide chain comprises, from N-terminus to C-terminus: scFv2- linker -VL1- linker -TRBV1- linker -TRAV1- optional linker - TRAC, and the second polypeptide chain comprises, from N-terminus to C-terminus: VH1- optional linker - TRBC.

In some embodiments, the first polypeptide chain comprises, from N-terminus to C-terminus: scFv2- linker -VH1- linker -TRAV1- linker -TRBV1- optional linker - TRBC, and the second polypeptide chain comprises, from N-terminus to C-terminus: VL1- optional linker - TRAC.

In some embodiments, the first polypeptide chain comprises, from N-terminus to C-terminus: scFv2- linker -VL1- linker -TRAV1- linker -TRBV1- optional linker - TRBC, and the second polypeptide chain comprises, from N-terminus to C-terminus: VH1- optional linker - TRAC.

In some embodiments, the first polypeptide chain comprises, from N-terminus to C-terminus: VH1- linker -TRBV1- linker -TRAV1- optional linker - TRAC, and the second polypeptide chain comprises, from N-terminus to C-terminus: scFv2- linker -VL1- optional linker - TRBC.

In some embodiments, the first polypeptide chain comprises, from N-terminus to C-terminus: VL1- linker -TRBV1- linker -TRAV1- optional linker - TRAC, and the second polypeptide chain comprises, from N-terminus to C-terminus: scFv2- linker -VH1- optional linker - TRBC.

In some embodiments, the first polypeptide chain comprises, from N-terminus to C-terminus: VH1- linker -TRAV1- linker -TRBV1- optional linker - TRBC, and the second polypeptide chain comprises, from N-terminus to C-terminus: scFv2- linker -VL1- optional linker - TRAC.

In some embodiments, the first polypeptide chain comprises, from N-terminus to C-terminus: VL1- linker -TRAV1- linker -TRBV1- optional linker - TRBC, and the second polypeptide chain comprises, from N-terminus to C-terminus: scFv2- linker -VH1- optional linker - TRAC.

In some embodiments, the first polypeptide chain comprises, from N-terminus to C-terminus: VHH- linker -VH1- linker -TRBV1- linker -TRAV1- optional linker - TRAC, and the second polypeptide chain comprises, from N-terminus to C-terminus: VL1- optional linker - TRBC.

In some embodiments, the first polypeptide chain comprises, from N-terminus to C-terminus: VHH- linker -VL1- linker -TRBV1- linker -TRAV1- optional linker - TRAC, and the second polypeptide chain comprises, from N-terminus to C-terminus: VH1- optional linker - TRBC.

In some embodiments, the first polypeptide chain comprises, from N-terminus to C-terminus: VHH- linker -VH1- linker -TRAV1- linker -TRBV1- optional linker - TRBC, and the second polypeptide chain comprises, from N-terminus to C-terminus: VL1- optional linker - TRAC.

In some embodiments, the first polypeptide chain comprises, from N-terminus to C-terminus: VHH- linker -VL1- linker -TRAV1- linker -TRBV1- optional linker - TRBC, and the second polypeptide chain comprises, from N-terminus to C-terminus: VH1- optional linker - TRAC.

In some embodiments, the first polypeptide chain comprises, from N-terminus to C-terminus: VH1- linker -TRBV1- linker -TRAV1- optional linker - TRAC, and the second polypeptide chain comprises, from N-terminus to C-terminus: VHH- linker -VL1- optional linker - TRBC.

In some embodiments, the first polypeptide chain comprises, from N-terminus to C-terminus: VL1- linker -TRBV1- linker -TRAV1- optional linker - TRAC, and the second polypeptide chain comprises, from N-terminus to C-terminus: VHH- linker -VH1- optional linker - TRBC.

In some embodiments, the first polypeptide chain comprises, from N-terminus to C-terminus: VH1- linker -TRAV1- linker -TRBV1- optional linker - TRBC, and the second polypeptide chain comprises, from N-terminus to C-terminus: VHH- linker -VL1- optional linker - TRAC.

In some embodiments, the first polypeptide chain comprises, from N-terminus to C-terminus: VL1- linker -TRAV1- linker -TRBV1- optional linker - TRBC, and the second polypeptide chain comprises, from N-terminus to C-terminus: VHH- linker -VH1- optional linker - TRAC.

In some embodiments, the first polypeptide chain comprises, from N-terminus to C-terminus: VH2- linker -VH1- linker -TRBV1- linker -TRAV1- optional linker - TRAC, and the second polypeptide chain comprises, from N-terminus to C-terminus: VL2-VL1- optional linker - TRBC.

In some embodiments, the first polypeptide chain comprises, from N-terminus to C-terminus: VL2- linker -VH1- linker -TRBV1- linker -TRAV1- optional linker - TRAC, and the second polypeptide chain comprises, from N-terminus to C-terminus: VH2-VL1- optional linker - TRBC.

In some embodiments, the first polypeptide chain comprises, from N-terminus to C-terminus: VH2- linker -VL1- linker -TRBV1- linker -TRAV1- optional linker - TRAC, and the second polypeptide chain comprises, from N-terminus to C-terminus: VL2-VH1- optional linker - TRBC.

In some embodiments, the first polypeptide chain comprises, from N-terminus to C-terminus: VL2- linker -VL1- linker -TRBV1- linker -TRAV1- optional linker - TRAC, and the second polypeptide chain comprises, from N-terminus to C-terminus: VH2-VH1- optional linker - TRBC.

In some embodiments, the first polypeptide chain comprises, from N-terminus to C-terminus: VH2- linker -VH1- linker -TRAV1- linker -TRBV1- optional linker - TRBC, and the second polypeptide chain comprises, from N-terminus to C-terminus: VL2-VL1- optional linker - TRAC.

In some embodiments, the first polypeptide chain comprises, from N-terminus to C-terminus: VL2- linker -VH1- linker -TRAV1- linker -TRBV1- optional linker - TRBC, and the second polypeptide chain comprises, from N-terminus to C-terminus: VH2-VL1- optional linker - TRAC.

In some embodiments, the first polypeptide chain comprises, from N-terminus to C-terminus: VH2- linker -VL1- linker -TRAV1- linker -TRBV1- optional linker - TRBC, and the second polypeptide chain comprises, from N-terminus to C-terminus: VL2-VH1- optional linker - TRAC.

In some embodiments, the first polypeptide chain comprises, from N-terminus to C-terminus: VL2- linker -VL1- linker -TRAV1- linker -TRBV1- optional linker - TRBC, and the second polypeptide chain comprises, from N-terminus to C-terminus: VH2-VH1- optional linker - TRAC.

In a sixth aspect, the present disclosure provides a multi-specific polypeptide molecule, wherein the multi-specific polypeptide molecule comprises, on three polypeptide chains, a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises a heavy chain variable region VH1 and a light chain variable region VL1 derived from an antibody that binds the second antigen;
wherein the first antigen is selected from the group consisting of tumor-associated antigens (TAA), tumor-specific antigens (TSA), viral antigens and self-antigens, and the second antigen is an immune cell surface molecule;
and wherein the C-terminus of the polypeptide chain comprising VH1 or VL1 is connected to the N-terminus of the TCR.

In some embodiments, the multi-specific polypeptide molecule further comprises at least one functional domain capable of extending the half-life of the multi-specific polypeptide molecule. In some embodiments, the functional domain capable of prolonging the half-life of the multi-specific polypeptide molecule is selected from the group consisting of the following 1)-5):
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, the multi-specific polypeptide molecule further comprises at least one of the following functional domains:
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

In some embodiments, VH1 and VL1 are respectively distributed on two polypeptide chains of the multi-specific polypeptide molecule.

In some embodiments, TRAV1 and TRBV1 are respectively distributed on two polypeptide chains of the multi-specific polypeptide molecule.

In some embodiments, the C-terminus of the polypeptide chain comprising VH1 or VL1 is connected to the N-terminus of the polypeptide chain comprising TRAV1 or TRBV1 via a linker.

In some embodiments, VH1 and VL1 are respectively distributed on two polypeptide chains of the multi-specific polypeptide molecule, TRAV1 and TRBV1 are respectively distributed on two polypeptide chains of the multi-specific polypeptide molecule, and the C-terminus of the polypeptide chain comprising VH1 or VL1 is connected to the N-terminus of the polypeptide chain comprising TRAV1 or TRBV1 via a linker.

In some embodiments, the three polypeptide chains of the multi-specific polypeptide molecule respectively comprise:
VL1-TRBV1, VH1, and TRAV1; or
VH1-TRBV1, VL1, and TRAV1; or
VL1-TRAV1, VH1, and TRBV1; or
VH1-TRAV1, VL1, and TRBV1.

In some embodiments of the multi-specific polypeptide molecule of the sixth aspect of the present disclosure, the multi-specific polypeptide molecule further comprises a constant domain selected from the group consisting of the following (1)-(3), which is connected to the C-terminus of TRAV1 and/or TRBV1 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments, the multi-specific polypeptide molecule comprises a TCR constant region or a fragment thereof connected to the C-terminus of TRAV1 and TRBV1 via an optional linker. In some embodiments, the multi-specific polypeptide molecule comprises TRAC and TRBC which are respectively connected to the C-terminus of TRAV1 and TRBV1 via an optional linker. In some embodiments, the three polypeptide chains of the multi-specific polypeptide molecule respectively comprise:
VL1-TRBV1-TRBC, VH1, and TRAV1-TRAC; or
VH1-TRBV1-TRBC, VL1, and TRAV1-TRAC; or
VL1-TRAV1-TRAC, VH1, and TRBV1-TRBC; or
VH1-TRAV1-TRAC, VL1, and TRBV1-TRBC.

In some embodiments of the multi-specific polypeptide molecule of the sixth aspect of the present disclosure, the multi-specific polypeptide molecule further comprises an antibody-derived CH1 and an antibody-derived CL which are respectively connected to the C-terminus of VH1 and VL1.

In some embodiments of the multi-specific polypeptide molecule of the sixth aspect of the present disclosure, the multi-specific polypeptide molecule further comprises a third binding region that binds a third antigen, wherein the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens and self-antigens, and the third antigen is the same as or different from the first antigen. In some embodiments, TRAV2 is the same as TRAV1. In some embodiments, TRBV2 is the same as TRBV1.

In some embodiments of the multi-specific polypeptide molecule of the sixth aspect of the present disclosure, the multi-specific polypeptide molecule further comprises a constant domain selected from the group consisting of the following (1)-(3), which is connected to the C-terminus of TRAV1 and/or TRBV1 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments of the multi-specific polypeptide molecule of the sixth aspect of the present disclosure, the multi-specific polypeptide molecule further comprises a fourth binding region that binds a fourth antigen, wherein the fourth binding region comprises a heavy chain variable region VH2 and a light chain variable region VL2 derived from an antibody that binds the fourth antigen, or a single heavy chain variable region (VHH) derived from a single-domain antibody or nanobody that binds the fourth antigen; wherein the fourth antigen is an immune cell surface molecule, and the fourth antigen is the same as or different from the second antigen. In some embodiments, VH2 is the same as VH1. In some embodiments, VL2 is the same as VL1.

In some embodiments of the multi-specific polypeptide molecule of the sixth aspect of the present disclosure, the functional domain is derived from an albumin or a binding domain thereof and an optional antibody hinge region; and the functional domain is connected to the C-terminus or N-terminus (preferably the C-terminus) of any one polypeptide chain of the multi-specific polypeptide molecule via an optional linker. In some embodiments, the functional domain is derived from an albumin or a binding domain thereof; and the albumin or binding domain thereof is connected to the C-terminus or N-terminus (preferably the C-terminus) of any one polypeptide chain of the multi-specific polypeptide molecule via an optional linker. In some embodiments, the functional domain is derived from an albumin or a binding domain thereof and an antibody hinge region, the functional domain is connected to the C-terminus or N-terminus (preferably the C-terminus) of any one polypeptide chain of the multi-specific polypeptide molecule via an optional linker, and albumin or a binding domain thereof is connected to the C-terminus of any one chain of the hinge region via an optional linker.

In some embodiments of the multi-specific polypeptide molecule in the sixth aspect of the present disclosure, the functional domain is derived from an antibody hinge region and/or an antibody Fc domain or a dimerization portion thereof and/or an antibody CH3 domain; and the functional domain is connected to the C-terminus or N-terminus (preferably the C-terminus) of any two polypeptide chains of the multi-specific polypeptide molecule via an optional linker.

In some embodiments of the multi-specific polypeptide molecule in the sixth aspect of the present disclosure, the functional domain is derived from a TCR constant region or a fragment thereof and an optional antibody hinge region; and the functional domain is connected to the C-terminus or N-terminus (preferably the C-terminus) of any two polypeptide chains of the multi-specific polypeptide molecule via an optional linker.

In some embodiments, the functional domain comprises two chains, and any two chains of the multi-specific polypeptide molecule are connected via covalent bonds or non-covalent bonds between the two chains of the functional domain.

In some embodiments, the functional domain comprises two chains, and the first binding region and the second binding region are connected via covalent bonds or non-covalent bonds between the two chains of the functional domain.

In some embodiments, the functional domain comprises two chains, wherein one chain is connected to the C-terminus or N-terminus (preferably the C-terminus) of the polypeptide chain comprising VH1 or VL1, and the other chain is connected to the C-terminus or N-terminus (preferably the C-terminus) of another polypeptide chain comprising TRAV1 or TRBV1.

In some embodiments of the multi-specific polypeptide molecule in the sixth aspect of the present disclosure, the polypeptide molecule comprises a first polypeptide chain, a second polypeptide chain and a third polypeptide chain; wherein:
the first polypeptide chain comprises, from N-terminus to C-terminus: VL1- linker -TRBV1- optional linker -TRBC, the second polypeptide chain comprises, from N-terminus to C-terminus: VH1- optional linker -hinge region -CH2-CH3, and the third polypeptide chain comprises, from N-terminus to C-terminus: TRAV1-optional linker -TRAC- optional linker -hinge region -CH2-CH3 (format 62-9).

In some embodiments, the first polypeptide chain comprises, from N-terminus to C-terminus: CH3-CH2-hinge region - optional linker -VL1- linker -TRBV1- optional linker -TRBC, the second polypeptide chain comprises, from N-terminus to C-terminus: CH3-CH2-hinge region - optional linker -VH1, and the third polypeptide chain comprises, from N-terminus to C-terminus: TRAV1- optional linker -TRAC.

In some embodiments, the first polypeptide chain comprises, from N-terminus to C-terminus: VH1- linker -TRBV1- optional linker -TRBC, the second polypeptide chain comprises, from N-terminus to C-terminus: VL1- optional linker -hinge region -CH2-CH3, and the third polypeptide chain comprises, from N-terminus to C-terminus: TRAV1- optional linker -TRAC- optional linker -hinge region -CH2-CH3.

In some embodiments, the first polypeptide chain comprises, from N-terminus to C-terminus: VL1- linker -TRAV1- optional linker -TRAC, the second polypeptide chain comprises, from N-terminus to C-terminus: VH1- optional linker -hinge region -CH2-CH3, and the third polypeptide chain comprises, from N-terminus to C-terminus: TRBV1- optional linker -TRBC- optional linker -hinge region -CH2-CH3.

In some embodiments, the first polypeptide chain comprises, from N-terminus to C-terminus: VH1- linker -TRAV1- optional linker -TRAC, the second polypeptide chain comprises, from N-terminus to C-terminus: VL1- optional linker -hinge region -CH2-CH3, and the third polypeptide chain comprises, from N-terminus to C-terminus: TRBV1- optional linker -TRBC- optional linker -hinge region -CH2-CH3.

In some embodiments, the first polypeptide chain comprises, from N-terminus to C-terminus: VL1- linker -TRBV1- optional linker -TRBC- optional linker -hinge region -CH2-CH3, the second polypeptide chain comprises, from N-terminus to C-terminus: VH1- optional linker -hinge region -CH2-CH3, and the third polypeptide chain comprises, from N-terminus to C-terminus: TRAV1- optional linker -TRAC.

In some embodiments, the first polypeptide chain comprises, from N-terminus to C-terminus: VH1- linker -TRBV1- optional linker -TRBC- optional linker -hinge region -CH2-CH3, the second polypeptide chain comprises, from N-terminus to C-terminus: VL1- optional linker -hinge region -CH2-CH3, and the third polypeptide chain comprises, from N-terminus to C-terminus: TRAV1- optional linker -TRAC.

In some embodiments, the first polypeptide chain comprises, from N-terminus to C-terminus: VL1- linker -TRAV1- optional linker -TRAC- optional linker -hinge region -CH2-CH3, the second polypeptide chain comprises, from N-terminus to C-terminus: VH1- optional linker -hinge region -CH2-CH3, and the third polypeptide chain comprises, from N-terminus to C-terminus: TRBV1- optional linker -TRBC.

In some embodiments, the first polypeptide chain comprises, from N-terminus to C-terminus: VH1- linker -TRAV1- optional linker -TRAC- optional linker -hinge region -CH2-CH3, the second polypeptide chain comprises, from N-terminus to C-terminus: VL1- optional linker -hinge region -CH2-CH3, and the third polypeptide chain comprises, from N-terminus to C-terminus: TRBV1- optional linker -TRBC.

In some embodiments, the first polypeptide chain comprises, from N-terminus to C-terminus: VL1- linker -TRBV1- optional linker -TRBC, the second polypeptide chain comprises, from N-terminus to C-terminus: VH1- optional linker - optional hinge region - optional linker -Alb, and the third polypeptide chain comprises, from N-terminus to C-terminus: TRAV1- optional linker -TRAC- optional linker - optional hinge region.

In some embodiments, the first polypeptide chain comprises, from N-terminus to C-terminus: VL1- linker -TRBV1- optional linker -TRBC- optional linker -Alb, the second polypeptide chain comprises, from N-terminus to C-terminus: VH1- optional linker - optional hinge region, and the third polypeptide chain comprises, from N-terminus to C-terminus: TRAV1- optional linker -TRAC- optional linker - optional hinge region.

In some embodiments, the first polypeptide chain comprises, from N-terminus to C-terminus: VL1- linker -TRBV1- optional linker -TRBC, the second polypeptide chain comprises, from N-terminus to C-terminus: VH1- optional linker - optional hinge region, and the third polypeptide chain comprises, from N-terminus to C-terminus: TRAV1- optional linker -TRAC- optional linker - optional hinge region - optional linker -Alb.

In some embodiments, the first polypeptide chain comprises, from N-terminus to C-terminus: VH1- linker -TRBV1- optional linker -TRBC, the second polypeptide chain comprises, from N-terminus to C-terminus: VL1- optional linker - optional hinge region - optional linker -Alb, and the third polypeptide chain comprises, from N-terminus to C-terminus: TRAV1- optional linker -TRAC- optional linker - optional hinge region.

In some embodiments, the first polypeptide chain comprises, from N-terminus to C-terminus: VH1- linker -TRBV1- optional linker -TRBC- optional linker -Alb, the second polypeptide chain comprises, from N-terminus to C-terminus: VL1- optional linker - optional hinge region, and the third polypeptide chain comprises, from N-terminus to C-terminus: TRAV1- optional linker -TRAC- optional linker - optional hinge region.

In some embodiments, the first polypeptide chain comprises, from N-terminus to C-terminus: VH1- linker -TRBV1- optional linker -TRBC, the second polypeptide chain comprises, from N-terminus to C-terminus: VL1- optional linker - optional hinge region, and the third polypeptide chain comprises, from N-terminus to C-terminus: TRAV1- optional linker -TRAC- optional linker - optional hinge region - optional linker -Alb.

In some embodiments, the first polypeptide chain comprises, from N-terminus to C-terminus: VL1- linker -TRAV1- optional linker -TRAC, the second polypeptide chain comprises, from N-terminus to C-terminus: VH1- optional linker - optional hinge region - optional linker -Alb, and the third polypeptide chain comprises, from N-terminus to C-terminus: TRBV1- optional linker -TRBC- optional linker - optional hinge region.

In some embodiments, the first polypeptide chain comprises, from N-terminus to C-terminus: VL1- linker -TRAV1- optional linker -TRAC- optional linker -Alb, the second polypeptide chain comprises, from N-terminus to C-terminus: VH1- optional linker - optional hinge region, and the third polypeptide chain comprises, from N-terminus to C-terminus: TRBV1- optional linker -TRBC- optional linker - optional hinge region.

In some embodiments, the first polypeptide chain comprises, from N-terminus to C-terminus: VL1- linker -TRAV1- optional linker -TRAC, the second polypeptide chain comprises, from N-terminus to C-terminus: VH1- optional linker - optional hinge region, and the third polypeptide chain comprises, from N-terminus to C-terminus: TRBV1- optional linker -TRBC- optional linker - optional hinge region -Alb.

In some embodiments, the first polypeptide chain comprises, from N-terminus to C-terminus: VH1- linker -TRAV1- optional linker -TRAC, the second polypeptide chain comprises, from N-terminus to C-terminus: VL1- optional linker - optional hinge region - optional linker -Alb, and the third polypeptide chain comprises, from N-terminus to C-terminus: TRBV1- optional linker -TRBC- optional linker - optional hinge region.

In some embodiments, the first polypeptide chain comprises, from N-terminus to C-terminus: VH1- linker -TRAV1- optional linker -TRAC- optional linker -Alb, the second polypeptide chain comprises, from N-terminus to C-terminus: VL1- optional linker - optional hinge region, and the third polypeptide chain comprises, from N-terminus to C-terminus: TRBV1- optional linker -TRBC- optional linker - optional hinge region.

In some embodiments, the first polypeptide chain comprises, from N-terminus to C-terminus: VH1- linker -TRAV1- optional linker -TRAC, the second polypeptide chain comprises, from N-terminus to C-terminus: VL1- optional linker - optional hinge region, and the third polypeptide chain comprises, from N-terminus to C-terminus: TRBV1- optional linker -TRBC- optional linker - optional hinge region - optional linker -Alb.

In some embodiments, the first polypeptide chain comprises, from N-terminus to C-terminus: VL1- linker -TRBV1- optional linker -TRBC- optional linker - optional hinge region - optional linker -Alb, the second polypeptide chain comprises, from N-terminus to C-terminus: VH1- optional linker - optional hinge region, and the third polypeptide chain comprises, from N-terminus to C-terminus: TRAV1- optional linker -TRAC.

In some embodiments, the first polypeptide chain comprises, from N-terminus to C-terminus: VL1- linker -TRBV1- optional linker -TRBC- optional linker - optional hinge region , the second polypeptide chain comprises, from N-terminus to C-terminus: VH1- optional linker - optional hinge region - optional linker -Alb, and the third polypeptide chain comprises, from N-terminus to C-terminus: TRAV1- optional linker -TRAC.

In some embodiments, the first polypeptide chain comprises, from N-terminus to C-terminus: VL1- linker -TRBV1- optional linker -TRBC- optional linker - optional hinge region , the second polypeptide chain comprises, from N-terminus to C-terminus: VH1- optional linker - optional hinge region, and the third polypeptide chain comprises, from N-terminus to C-terminus: TRAV1- optional linker -TRAC- optional linker -Alb.

In some embodiments, the first polypeptide chain comprises, from N-terminus to C-terminus: VH1- linker -TRBV1- optional linker -TRBC- optional linker - optional hinge region - optional linker -Alb, the second polypeptide chain comprises, from N-terminus to C-terminus: VL1- optional linker - optional hinge region, and the third polypeptide chain comprises, from N-terminus to C-terminus: TRAV1- optional linker -TRAC.

In some embodiments, the first polypeptide chain comprises, from N-terminus to C-terminus: VH1- linker -TRBV1- optional linker -TRBC- optional linker - optional hinge region , the second polypeptide chain comprises, from N-terminus to C-terminus: VL1- optional linker - optional hinge region - optional linker -Alb, and the third polypeptide chain comprises, from N-terminus to C-terminus: TRAV1- optional linker -TRAC.

In some embodiments, the first polypeptide chain comprises, from N-terminus to C-terminus: VH1- linker -TRBV1- optional linker -TRBC- optional linker - optional hinge region , the second polypeptide chain comprises, from N-terminus to C-terminus: VL1- optional linker - optional hinge region, and the third polypeptide chain comprises, from N-terminus to C-terminus: TRAV1- optional linker -TRAC- optional linker -Alb.

In some embodiments, the first polypeptide chain comprises, from N-terminus to C-terminus: VL1- linker -TRAV1- optional linker -TRAC- optional linker - optional hinge region - optional linker -Alb, the second polypeptide chain comprises, from N-terminus to C-terminus: VH1- optional linker - optional hinge region, and the third polypeptide chain comprises, from N-terminus to C-terminus: TRBV1- optional linker -TRBC.

In some embodiments, the first polypeptide chain comprises, from N-terminus to C-terminus: VL1- linker -TRAV1- optional linker -TRAC- optional linker - optional hinge region , the second polypeptide chain comprises, from N-terminus to C-terminus: VH1- optional linker - optional hinge region - optional linker -Alb, and the third polypeptide chain comprises, from N-terminus to C-terminus: TRBV1- optional linker -TRBC.

In some embodiments, the first polypeptide chain comprises, from N-terminus to C-terminus: VL1- linker -TRAV1- optional linker -TRAC- optional linker - optional hinge region , the second polypeptide chain comprises, from N-terminus to C-terminus: VH1- optional linker - optional hinge region, and the third polypeptide chain comprises, from N-terminus to C-terminus: TRBV1- optional linker -TRBC- optional linker -Alb.

In some embodiments, the first polypeptide chain comprises, from N-terminus to C-terminus: VH1- linker -TRAV1- optional linker -TRAC- optional linker - optional hinge region - optional linker -Alb, the second polypeptide chain comprises, from N-terminus to C-terminus: VL1- optional linker - optional hinge region, and the third polypeptide chain comprises, from N-terminus to C-terminus: TRBV1- optional linker -TRBC.

In some embodiments, the first polypeptide chain comprises, from N-terminus to C-terminus: VH1- linker -TRAV1- optional linker -TRAC- optional linker - optional hinge region , the second polypeptide chain comprises, from N-terminus to C-terminus: VL1- optional linker - optional hinge region - optional linker -Alb, and the third polypeptide chain comprises, from N-terminus to C-terminus: TRBV1- optional linker -TRBC.

In some embodiments, the first polypeptide chain comprises, from N-terminus to C-terminus: VH1- linker -TRAV1- optional linker -TRAC- optional linker - optional hinge region , the second polypeptide chain comprises, from N-terminus to C-terminus: VL1- optional linker - optional hinge region, and the third polypeptide chain comprises, from N-terminus to C-terminus: TRBV1- optional linker -TRBC- optional linker -Alb.

In some embodiments of the multi-specific polypeptide molecule of the sixth aspect of the present disclosure, the functional domain is derived from a TCR constant region or a fragment thereof and an optional antibody hinge region; and the functional domain is connected to the C-terminus or N-terminus, preferably the C-terminus, of the two polypeptide chains of the multi-specific polypeptide molecule via an optional linker. In this case, the antibody hinge region and the antibody Fc domain or a dimerization portion thereof in the first polypeptide chain and the second polypeptide chain of the above multi-specific polypeptide molecule are replaced with the TCR constant region or a fragment thereof and an optional antibody hinge region.

In some embodiments of the multi-specific polypeptide molecule of the sixth aspect of the present disclosure, the functional domain is derived from CH3 or a fragment thereof and an optional antibody hinge region; and the functional domain is connected to the C-terminus or N-terminus, preferably the C-terminus, of the two polypeptide chains of the multi-specific polypeptide molecule via an optional linker. In this case, the antibody hinge region and the antibody Fc domain or a dimerization portion thereof in the first polypeptide chain and the second polypeptide chain of the above multi-specific polypeptide molecule are replaced with the CH3 or a fragment thereof and an optional antibody hinge region.

In a seventh aspect, the present disclosure provides a multi-specific polypeptide molecule, wherein the multi-specific polypeptide molecule comprises, on two polypeptide chains, a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises a heavy chain variable region VH1 and a light chain variable region VL1 derived from an antibody that binds the second antigen, or a single heavy chain variable region (VHH1) derived from a single-domain antibody or a nanobody that binds the second antigen;
wherein the first antigen is selected from the group consisting of tumor-associated antigens (TAA), tumor-specific antigens (TSA), viral antigens and self-antigens, and the second antigen is an immune cell surface molecule;
wherein VH1 and VL1 are linked in tandem on one polypeptide chain, and TRAV1 and TRBV1 are linked in tandem on the other polypeptide chain.

In some embodiments, the multi-specific polypeptide molecule further comprises at least one functional domain capable of extending the half-life of the multi-specific polypeptide molecule, and the functional domain comprises two polypeptide chains, and the two chains of the multi-specific polypeptide molecule are connected via covalent bonds or non-covalent bonds between the two chains of the functional domain.

In some embodiments, the multi-specific polypeptide molecule further comprises at least one of the following functional domains:
1) a hinge region derived from an antibody;
2) an Fc domain derived from an antibody or a dimerization portion thereof;
3) a CH3 domain derived from an antibody;
4) a constant region derived from a TCR or a fragment thereof;
and wherein the two chains of the multi-specific polypeptide molecule are connected via covalent bonds or non-covalent bonds between the two chains of the functional domain.

In some embodiments, the polypeptide chain formed by tandemly linking VH1 and VL1 is not directly connected to the polypeptide chain formed by tandemly linking TRAV1 and TRBV1. The term "not directly connected" means that the two polypeptide chains are not connected in any form to form one polypeptide chain.

In some embodiments, the two polypeptide chains of the multi-specific polypeptide molecule respectively comprise:
TRBV1-TRAV1 and VL1-VH1; or
TRBV1-TRAV1 and VH1-VL1; or
TRAV1-TRBV1 and VL1-VH1; or
TRAV1-TRBV1 and VH1-VL1; or
TRBV1-TRAV1 and VHH1; or
TRAV1-TRBV1 and VHH1.

In some embodiments of the multi-specific polypeptide molecule of the seventh aspect of the present disclosure, adjacent variable regions are linked via a linker.

In some embodiments of the multi-specific polypeptide molecule of the seventh aspect of the present disclosure, the multi-specific polypeptide molecule further comprises a constant domain selected from the group consisting of the following (1)-(3), which is connected to the C-terminus of TRAV1 and/or TRBV1 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments of the multi-specific polypeptide molecule of the seventh aspect of the present disclosure, the multi-specific polypeptide molecule further comprises a TCR constant region or a fragment thereof connected to the C-terminus of TRAV1 and TRBV1 via an optional linker, wherein the TCR constant region comprises a TCR α chain constant region (TRAC) and a β chain constant region (TRBC).

In some embodiments of the multi-specific polypeptide molecule of the seventh aspect of the present disclosure, the two polypeptide chains of the multi-specific polypeptide molecule respectively comprise:
TRBV1-TRBC-TRAV1-TRAC and VL1-VH1; or
TRBV1-TRBC-TRAV1-TRAC and VH1-VL1; or
TRAV1-TRAC-TRBV1-TRBC and VL1-VH1; or
TRAV1-TRAC-TRBV1-TRBC and VH1-VL1; or
TRBV1-TRBC-TRAV1-TRAC and VHH1; or
TRAV1-TRAC-TRBV1-TRBC and VHH1.

In some embodiments of the multi-specific polypeptide molecule of the seventh aspect of the present disclosure, the multi-specific polypeptide molecule further comprises a TCR constant region or a fragment thereof connected to the C-terminus of TRAV1 or TRBV1 via an optional linker, e.g. TRAC or TRBC.

In some embodiments of the multi-specific polypeptide molecule of the seventh aspect of the present disclosure, the two polypeptide chains of the multi-specific polypeptide molecule respectively comprise:
TRBV1-TRAV1-TRAC and VL1-VH1; or
TRBV1-TRAV1-TRAC and VH1-VL1; or
TRAV1-TRBV1-TRBC and VL1-VH1; or
TRAV1-TRBV1-TRBC and VH1-VL1; or
TRBV1-TRAV1-TRAC and VHH1; or
TRAV1-TRBV1-TRBC and VHH1.

In some embodiments of the multi-specific polypeptide molecule of the seventh aspect of the present disclosure, the multi-specific polypeptide molecule further comprises an antibody-derived CH1 and an antibody-derived CL which are respectively connected to VH1 and VL1, wherein VH1, VL1, CH1 and CL form a single-chain Fab1.

In embodiments of the multi-specific polypeptide molecule of the seventh aspect of the present disclosure, the multi-specific polypeptide molecule further comprises a third binding region that binds a third antigen, wherein the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens and self-antigens, and the third antigen is the same as or different from the first antigen. In some embodiments, TRAV2 is the same as TRAV1. In some embodiments, TRBV2 is the same as TRBV1.

In some embodiments of the multi-specific polypeptide molecule of the seventh aspect of the present disclosure, the multi-specific polypeptide molecule further comprises a constant domain selected from the group consisting of the following (1)-(3), which is connected to the C-terminus of TRAV2 and/or TRBV2 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments of the multi-specific polypeptide molecule of the seventh aspect of the present disclosure, the multi-specific polypeptide molecule further comprises a fourth binding region that binds a fourth antigen, wherein the fourth binding region comprises a heavy chain variable region VH2 and a light chain variable region VL2 derived from an antibody that binds the fourth antigen, or a single heavy chain variable region (VHH2) derived from a single-domain antibody or nanobody that binds the fourth antigen; wherein the fourth antigen is an immune cell surface molecule, and the fourth antigen is the same as or different from the second antigen. In some embodiments, VH2 is the same as VH1. In some embodiments, VL2 is the same as VL1. In some embodiments, VHH2 is the same as VHH1.

In some embodiments of the multi-specific polypeptide molecule of the seventh aspect of the present disclosure, the multi-specific polypeptide molecule further comprises an antibody-derived CH1 and an antibody-derived CL that are respectively connected to VH2 and VL2, wherein VH2, VL2, CH1 and CL form a single-chain Fab2.

In some embodiments of the multi-specific polypeptide molecule of the seventh aspect of the present disclosure, the functional domain is derived from an antibody hinge region and/or an antibody Fc domain or a dimerization portion thereof and/or an antibody CH3 domain; and the functional domain is connected to the C-terminus or N-terminus (preferably to the C-terminus) of the two polypeptide chains of the multi-specific polypeptide molecule via an optional linker.

In some embodiments, the functional domain is derived from an antibody hinge region and an antibody Fc domain or a dimerization portion thereof.

In some embodiments, the polypeptide molecule comprises a first polypeptide chain and a second polypeptide chain; wherein the first polypeptide chain comprises, from N-terminus to C-terminus: TRBV1-optional linker -TRBC- linker -TRAV1- optional linker -TRAC- optional linker -hinge region -CH2-CH3, and the second polypeptide chain comprises, from N-terminus to C-terminus: VL1- linker -VH1- optional linker - hinge region -CH2-CH3 (format 60).

In some embodiments, the first polypeptide chain comprises, from N-terminus to C-terminus: TRBV1-optional linker -TRBC- linker -TRAV1- optional linker -TRAC- optional linker -hinge region -CH2-CH3, and the second polypeptide chain comprises, from N-terminus to C-terminus: VH1- linker -VL1- optional linker - hinge region -CH2-CH3.

In some embodiments, the first polypeptide chain comprises, from N-terminus to C-terminus: TRAV1-optional linker -TRAC- linker -TRBV1- optional linker -TRBC- optional linker -hinge region -CH2-CH3, and the second polypeptide chain comprises, from N-terminus to C-terminus: VL1- linker -VH1- optional linker - hinge region -CH2-CH3.

In some embodiments, the first polypeptide chain comprises, from N-terminus to C-terminus: TRAV1-optional linker -TRAC- linker -TRBV1- optional linker -TRBC- optional linker -hinge region -CH2-CH3, and the second polypeptide chain comprises, from N-terminus to C-terminus: VH1- linker -VL1- optional linker - hinge region -CH2-CH3.

In some embodiments, the first polypeptide chain comprises, from N-terminus to C-terminus: TRBV1-optional linker -TRBC- linker -TRAV1- optional linker -TRAC- optional linker -hinge region -CH2-CH3, and the second polypeptide chain comprises, from N-terminus to C-terminus: VHH1- optional linker -hinge region -CH2-CH3.

In some embodiments, the first polypeptide chain comprises, from N-terminus to C-terminus: TRAV1-optional linker -TRAC- linker -TRBV1- optional linker -TRBC- optional linker -hinge region -CH2-CH3, and the second polypeptide chain comprises, from N-terminus to C-terminus: VHH1- optional linker -hinge region -CH2-CH3.

In some embodiments, the polypeptide molecule comprises a first polypeptide chain and a second polypeptide chain; wherein the first polypeptide chain comprises, from N-terminus to C-terminus: TRBV1-linker -TRAV1- optional linker -TRAC- optional linker -hinge region -CH2-CH3, and the second polypeptide chain comprises, from N-terminus to C-terminus: VL1- linker -VH1- optional linker -hinge region -CH2-CH3 (format 62-11).

In some embodiments, the first polypeptide chain comprises, from N-terminus to C-terminus: TRBV1-linker -TRAV1- optional linker -TRAC- optional linker -hinge region -CH2-CH3, and the second polypeptide chain comprises, from N-terminus to C-terminus: VH1- linker -VL1- optional linker -hinge region -CH2-CH3.

In some embodiments, the first polypeptide chain comprises, from N-terminus to C-terminus: TRAV1-linker -TRBV1- optional linker -TRBC- optional linker -hinge region -CH2-CH3, and the second polypeptide chain comprises, from N-terminus to C-terminus: VL1- linker -VH1- optional linker -hinge region -CH2-CH3.

In some embodiments, the first polypeptide chain comprises, from N-terminus to C-terminus: TRAV1-linker -TRBV1- optional linker -TRBC- optional linker -hinge region -CH2-CH3, and the second polypeptide chain comprises, from N-terminus to C-terminus: VH1- linker -VL1- optional linker -hinge region -CH2-CH3.

In some embodiments, the first polypeptide chain comprises, from N-terminus to C-terminus: TRBV1-linker -TRAV1- optional linker -TRAC- optional linker -hinge region -CH2-CH3, and the second polypeptide chain comprises, from N-terminus to C-terminus: VHH1- optional linker -hinge region -CH2-CH3.

In some embodiments, the first polypeptide chain comprises, from N-terminus to C-terminus: TRAV1-linker -TRBV1- optional linker -TRBC- optional linker -hinge region -CH2-CH3, and the second polypeptide chain comprises, from N-terminus to C-terminus: VHH1- optional linker -hinge region -CH2-CH3.

In some embodiments of the multi-specific polypeptide molecule of the seventh aspect of the present disclosure, the functional domain is derived from a TCR constant region or a fragment thereof and an optional antibody hinge region; and the functional domain is connected to the C-terminus or N-terminus (preferably the C-terminus) of the two polypeptide chains of the multi-specific polypeptide molecule via an optional linker. In this case, the antibody hinge region and the antibody Fc domain or a dimerizing part thereof in the first polypeptide chain and the second polypeptide chain of the above multi-specific polypeptide molecule are replaced with the TCR constant region or a fragment thereof and an optional antibody hinge region.

In some embodiments of the multi-specific polypeptide molecule of the seventh aspect of the present disclosure, the functional domain is derived from CH3 or a fragment thereof and an optional antibody hinge region; and the functional domain is connected to the C-terminus or N-terminus (preferably the C-terminus) of the two polypeptide chains of the multi-specific polypeptide molecule via an optional linker. In this case, the antibody hinge region and the antibody Fc domain or a dimerizing part thereof in the first polypeptide chain and the second polypeptide chain of the above multi-specific polypeptide molecule are replaced with the CH3 or a fragment thereof and an optional antibody hinge region.

In an eighth aspect, the present disclosure provides a multi-specific polypeptide molecule, wherein the multi-specific polypeptide molecule comprises, on four polypeptide chains, a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises a heavy chain variable region VH1 and a light chain variable region VL1 derived from an antibody that binds the second antigen;
wherein TRAV1, TRBV1, VH1 and VL1 are respectively located on different polypeptide chains;
wherein the first antigen is selected from the group consisting of tumor-associated antigens (TAA), tumor-specific antigens (TSA), viral antigens and self-antigens, and the second antigen is an immune cell surface molecule.

In some embodiments, the multi-specific polypeptide molecule further comprises at least one functional domain capable of extending the half-life of the multi-specific polypeptide molecule, and the functional domain comprises two polypeptide chains, and the first binding region and the second binding region of the multi-specific polypeptide molecule are connected via covalent bonds or non-covalent bonds between the two chains of the functional domain.

In some embodiments, the multi-specific polypeptide molecule further comprises at least one of the following functional domains:
1) a hinge region derived from an antibody;
2) an Fc domain derived from an antibody or a dimerization portion thereof;
3) a CH3 domain derived from an antibody;
4) a constant region derived from a TCR or a fragment thereof;
and wherein the first binding region and the second binding region of the multi-specific polypeptide molecule are connected via covalent bonds or non-covalent bonds between the two chains of the functional domain.

In some embodiments, one chain of the functional domain is connected to the C-terminus or N-terminus of TRAV1 or TRBV1, preferably the C-terminus, and the other chain is connected to the C-terminus or N-terminus of VH1 or VL1, preferably the C-terminus.

In some embodiments of the multi-specific polypeptide molecule of the eighth aspect of the present disclosure, the multi-specific polypeptide molecule further comprises a constant domain selected from the group consisting of the following (1)-(3), which is connected to the C-terminus of TRAV1 and/or TRBV1 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In embodiments of the multi-specific polypeptide molecule of the eighth aspect of the present disclosure, the four chains of the multi-specific polypeptide molecule comprise:
TRBV1-TRBC, TRAV1-TRAC, VL1, VH1.

In some embodiments of the multi-specific polypeptide molecule of the eighth aspect of the present disclosure, the multi-specific polypeptide molecule further comprises an antibody-derived CH1 and an antibody-derived CL which are respectively connected to the VH1 and VL1.

In embodiments of the multi-specific polypeptide molecule of the eighth aspect of the present disclosure, the four chains of the multi-specific polypeptide molecule comprise:
TRBV1, TRAV1, VL1-CL, VH1-CH1.

In embodiments of the multi-specific polypeptide molecule of the eighth aspect of the present disclosure, the four chains of the multi-specific polypeptide molecule comprise:
TRBV1-TRBC, TRAV1-TRAC, VL1-CL, VH1-CH1.

In embodiments of the multi-specific polypeptide molecule of the eighth aspect of the present disclosure, the multi-specific polypeptide molecule further comprises a third binding region that binds a third antigen, wherein the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens and self-antigens, and the third antigen is the same as or different from the first antigen. In some embodiments, the TRAV2 is the same as the TRAV1. In some embodiments, the TRBV2 is the same as the TRBV1.

In some embodiments of the multi-specific polypeptide molecule of the eighth aspect of the present disclosure, the multi-specific polypeptide molecule further comprises a constant domain selected from the group consisting of the following (1)-(3), which is connected to the C-terminus of TRAV2 and/or TRBV2 via an optional linker:
(1) a TCR constant region or a fragment thereof, wherein the TCR constant region, e.g., comprises a TCR α chain constant region (TRAC) and/or a β chain constant region (TRBC);
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
(3) CL and CH1, wherein the CL is derived from an antibody light chain CL domain, and the CH1 is derived from an antibody heavy chain CH1 domain.

In some embodiments of the multi-specific polypeptide molecule of the eighth aspect of the present disclosure, the multi-specific polypeptide molecule further comprises a fourth binding region that binds a fourth antigen, wherein the fourth binding region comprises a heavy chain variable region VH2 and a light chain variable region VL2 derived from an antibody that binds the fourth antigen, or a single heavy chain variable region (VHH) derived from a single-domain antibody or a nanobody that binds the fourth antigen; wherein the fourth antigen is an immune cell surface molecule, and the fourth antigen is the same as or different from the second antigen. In some embodiments, the VH2 is the same as the VH1. In some embodiments, the VL2 is the same as the VL1.

In some embodiments of the multi-specific polypeptide molecule of the eighth aspect of the present disclosure, the multi-specific polypeptide molecule further comprises an antibody-derived CH1 and an antibody-derived CL that are respectively connected to the VH2 and VL2; optionally, the VH2, VL2, CH1 and CL form a single-chain Fab2.

In some embodiments of the multi-specific polypeptide molecule of the eighth aspect of the present disclosure, the functional domain is derived from an antibody hinge region and/or an antibody Fc domain or a dimerization portion thereof and/or an antibody CH3 domain; and the functional domain is connected to the C-terminus or N-terminus (preferably the C-terminus) of any two polypeptide chains of the multi-specific polypeptide molecule via an optional linker.

In some embodiments, the functional domain is derived from an antibody hinge region and an antibody Fc domain or a dimerization portion thereof.

In some embodiments, the polypeptide molecule comprises a first polypeptide chain, a second polypeptide chain, a third polypeptide chain and a fourth polypeptide chain; wherein:
the first polypeptide chain comprises, from N-terminus to C-terminus: TRBV1- optional linker -TRBC, the second polypeptide chain comprises, from N-terminus to C-terminus: VL1- optional linker -CL, the third polypeptide chain comprises, from N-terminus to C-terminus: VH1- optional linker -CH1- optional linker - hinge region -CH2-CH3, and the fourth polypeptide chain comprises, from N-terminus to C-terminus: TRAV1-optional linker -TRAC- optional linker -hinge region -CH2-CH3 (format 62-13).

In some embodiments, the first polypeptide chain comprises, from N-terminus to C-terminus: TRBV1-optional linker -TRBC, the second polypeptide chain comprises, from N-terminus to C-terminus: VL1- optional linker -CL- optional linker -hinge region -CH2-CH3, the third polypeptide chain comprises, from N-terminus to C-terminus: VH1- optional linker -CH1, and the fourth polypeptide chain comprises, from N-terminus to C-terminus: TRAV1- optional linker -TRAC- optional linker -hinge region -CH2-CH3.

In some embodiments, the first polypeptide chain comprises, from N-terminus to C-terminus: TRBV1-optional linker -TRBC- optional linker -hinge region -CH2-CH3, the second polypeptide chain comprises, from N-terminus to C-terminus: VL1- optional linker -CL- optional linker -hinge region -CH2-CH3, the third polypeptide chain comprises, from N-terminus to C-terminus: VH1- optional linker -CH1, and the fourth polypeptide chain comprises, from N-terminus to C-terminus: TRAV1- optional linker -TRAC.

In some embodiments, the first polypeptide chain comprises, from N-terminus to C-terminus: TRBV1-optional linker -TRBC- optional linker -hinge region -CH2-CH3, the second polypeptide chain comprises, from N-terminus to C-terminus: VL1- optional linker -CL, the third polypeptide chain comprises, from N-terminus to C-terminus: VH1- optional linker -CH1- optional linker -hinge region -CH2-CH3, and the fourth polypeptide chain comprises, from N-terminus to C-terminus: TRAV1- optional linker -TRAC.

In some embodiments, the polypeptide molecule comprises a first polypeptide chain, a second polypeptide chain, a third polypeptide chain and a fourth polypeptide chain; wherein the first polypeptide chain comprises, from N-terminus to C-terminus: TRBV1, the second polypeptide chain comprises, from N-terminus to C-terminus: VL1, the third polypeptide chain comprises, from N-terminus to C-terminus: VH1- optional linker - hinge region -CH2-CH3, and the fourth polypeptide chain comprises, from N-terminus to C-terminus: TRAV1-optional linker -hinge region -CH2-CH3.

In some embodiments, the first polypeptide chain comprises, from N-terminus to C-terminus: TRBV1, the second polypeptide chain comprises, from N-terminus to C-terminus: VL1- optional linker -hinge region -CH2-CH3, the third polypeptide chain comprises, from N-terminus to C-terminus: VH1, and the fourth polypeptide chain comprises, from N-terminus to C-terminus: TRAV1- optional linker -hinge region -CH2-CH3.

In some embodiments, the first polypeptide chain comprises, from N-terminus to C-terminus: TRBV1-optional linker -hinge region -CH2-CH3, the second polypeptide chain comprises, from N-terminus to C-terminus: VL1- optional linker -hinge region -CH2-CH3, the third polypeptide chain comprises, from N-terminus to C-terminus: VH1, and the fourth polypeptide chain comprises, from N-terminus to C-terminus: TRAV1.

In some embodiments, the first polypeptide chain comprises, from N-terminus to C-terminus: TRBV1-optional linker -hinge region -CH2-CH3, the second polypeptide chain comprises, from N-terminus to C-terminus: VL1, the third polypeptide chain comprises, from N-terminus to C-terminus: VH1- optional linker - hinge region -CH2-CH3, and the fourth polypeptide chain comprises, from N-terminus to C-terminus: TRAV1.

In some embodiments, the polypeptide molecule comprises a first polypeptide chain, a second polypeptide chain, a third polypeptide chain and a fourth polypeptide chain; wherein the first polypeptide chain comprises, from N-terminus to C-terminus: TRBV1, the second polypeptide chain comprises, from N-terminus to C-terminus: VL1- optional linker -CL, the third polypeptide chain comprises, from N-terminus to C-terminus: VH1- optional linker -CH1- optional linker -hinge region -CH2-CH3, and the fourth polypeptide chain comprises, from N-terminus to C-terminus: TRAV1 - optional linker -hinge region -CH2-CH3.

In some embodiments, the first polypeptide chain comprises, from N-terminus to C-terminus: TRBV1, the second polypeptide chain comprises, from N-terminus to C-terminus: VL1- optional linker -CL- optional linker -hinge region -CH2-CH3, the third polypeptide chain comprises, from N-terminus to C-terminus: VH1- optional linker -CH1, and the fourth polypeptide chain comprises, from N-terminus to C-terminus: TRAV1- optional linker -hinge region -CH2-CH3.

In some embodiments, the first polypeptide chain comprises, from N-terminus to C-terminus: TRBV1-optional linker -hinge region -CH2-CH3, the second polypeptide chain comprises, from N-terminus to C-terminus: VL1- optional linker -CL- optional linker -hinge region -CH2-CH3, the third polypeptide chain comprises, from N-terminus to C-terminus: VH1- optional linker -CH1, and the fourth polypeptide chain comprises, from N-terminus to C-terminus: TRAV1.

In some embodiments, the first polypeptide chain comprises, from N-terminus to C-terminus: TRBV1-optional linker -hinge region -CH2-CH3, the second polypeptide chain comprises, from N-terminus to C-terminus: VL1- optional linker -CL, the third polypeptide chain comprises, from N-terminus to C-terminus: VH1- optional linker -CH1- optional linker -hinge region -CH2-CH3, and the fourth polypeptide chain comprises, from N-terminus to C-terminus: TRAV1.

In some embodiments, the polypeptide molecule comprises a first polypeptide chain, a second polypeptide chain, a third polypeptide chain and a fourth polypeptide chain; wherein the first polypeptide chain comprises, from N-terminus to C-terminus: TRBV1- optional linker -TRBC, the second polypeptide chain comprises, from N-terminus to C-terminus: VL1, the third polypeptide chain comprises, from N-terminus to C-terminus: VH1-optional linker -hinge region -CH2-CH3, and the fourth polypeptide chain comprises, from N-terminus to C-terminus: TRAV1- optional linker -TRAC- optional linker -hinge region -CH2-CH3.

In some embodiments, the first polypeptide chain comprises, from N-terminus to C-terminus: TRBV1-optional linker -TRBC, the second polypeptide chain comprises, from N-terminus to C-terminus: VL1- optional linker -hinge region -CH2-CH3, the third polypeptide chain comprises, from N-terminus to C-terminus: VH1, and the fourth polypeptide chain comprises, from N-terminus to C-terminus: TRAV1- optional linker -TRAC-optional linker -hinge region -CH2-CH3.

In some embodiments, the first polypeptide chain comprises, from N-terminus to C-terminus: TRBV1-optional linker -TRBC- optional linker -hinge region -CH2-CH3, the second polypeptide chain comprises, from N-terminus to C-terminus: VL1- optional linker -hinge region -CH2-CH3, the third polypeptide chain comprises, from N-terminus to C-terminus: VH1, and the fourth polypeptide chain comprises, from N-terminus to C-terminus: TRAV1- optional linker -TRAC.

In some embodiments, the first polypeptide chain comprises, from N-terminus to C-terminus: TRBV1-optional linker -TRBC- optional linker -hinge region -CH2-CH3, the second polypeptide chain comprises, from N-terminus to C-terminus: VL1, the third polypeptide chain comprises, from N-terminus to C-terminus: VH1-optional linker -hinge region -CH2-CH3, and the fourth polypeptide chain comprises, from N-terminus to C-terminus: TRAV1- optional linker -TRAC.

In some embodiments of the multi-specific polypeptide molecule of the eighth aspect of the present disclosure, the functional domain is derived from a TCR constant region or a fragment thereof and an optional antibody hinge region; and the functional domain is connected to the C-terminus or N-terminus (preferably the C-terminus) of any two polypeptide chains of the multi-specific polypeptide molecule via an optional linker.

In some embodiments of the multi-specific polypeptide molecule of the eighth aspect of the present disclosure, the functional domain is derived from a TCR constant region or a fragment thereof and an optional antibody hinge region; and the functional domain is connected to the C-terminus or N-terminus (preferably the C-terminus) of two polypeptide chains of the multi-specific polypeptide molecule via an optional linker. In this case, the antibody hinge region and the antibody Fc domain or a dimerizing part thereof in the first polypeptide chain and the second polypeptide chain of the above multi-specific polypeptide molecule are replaced with the TCR constant region or a fragment thereof and an optional antibody hinge region.

In some embodiments of the multi-specific polypeptide molecule of the eighth aspect of the present disclosure, the functional domain is derived from CH3 or a fragment thereof and an optional antibody hinge region; and the functional domain is connected to the C-terminus or N-terminus (preferably the C-terminus) of two polypeptide chains of the multi-specific polypeptide molecule via an optional linker. In this case, the antibody hinge region and the antibody Fc domain or a dimerizing part thereof in the first polypeptide chain and the second polypeptide chain of the above multi-specific polypeptide molecule are replaced with the CH3 or a fragment thereof and an optional antibody hinge region.

In the embodiments of the multi-specific polypeptide molecule of the first to eighth aspects of the present disclosure, the albumin may be an albumin derived from any mammal, such as human albumin, bovine albumin, mouse albumin, and the like. In some embodiments, the albumin is serum albumin, such as human serum albumin or bovine serum albumin. As used herein, the albumin includes wild-type albumin or a fragment thereof. In some embodiments, the fragment of albumin may be, e.g., a fragment having a length of 100-550, 150-550, 200-550, 250-550, 300-550, 350-550, 400-550, 450-550, 500-550, 100-500, 150-500, 200-500, 250-500, 300-500, 350-500, 400-500, 450-500, 100-450, 150-450, 200-450, 250-450, 300-450, 350-450, 400-450, 100-400, 150-400, 200-400, 250-400, 300-400, 350-400, 100-350, 150-350, 200-350, 250-350, 300-350, 100-300, 150-300, 200-300, 250-300, 100-250, 150-250, 200-250, 100-200, 150-200, 100-150 amino acids. In preferred embodiments, the albumin is human serum albumin, and the amino acid sequence thereof is as shown in SEQ ID NO: 2.

In the embodiments of the multi-specific polypeptide molecule of the first to eighth aspects of the present disclosure, the antibody hinge region may comprise part or all of the wild-type hinge sequence of the antibody, or a variant thereof having one or more substitutions. In some embodiments, the antibody hinge region is a hinge region of a human antibody. The antibody hinge region may be of any isotype, including but not limited to IgG1, IgG2, IgG3 and IgG4. For example, the antibody hinge region may be a hinge domain or a portion thereof derived from human IgG1, IgG2 or IgG4. In some embodiments, the hinge region comprises amino acids at positions 216-236 according to the EU numbering system, wherein the amino acid sequence of the hinge region of an IgG1 molecule comprises EPKSCDKTHTCPPCPAPELLG (SEQ ID NO: 124); the amino acid sequence of the hinge region of an IgG2 molecule comprises ERKCCVECPPCPAPPVAGP (SEQ ID NO: 125); and the amino acid sequence of the hinge region of an IgG4 molecule comprises ESKYGPPCPSCPAPEFLG (SEQ ID NO: 126).

In the embodiments of the multi-specific polypeptide molecule of the first to eighth aspects of the present disclosure, the antibody Fc domain comprises CH2 and CH3. In some embodiments, the antibody Fc domain or a dimerization portion thereof is an antibody Fc domain or a dimerization portion thereof derived from human. The antibody Fc domain may be of any isotype, including but not limited to IgG1, IgG2, IgG3 and IgG4, and may comprise one or more mutations or modifications. In some embodiments, the antibody Fc domain or a dimerization portion thereof is an Fc domain or a dimerization portion thereof derived from human IgG1, IgG2 or IgG4. In one embodiment, the Fc domain is an IgG1 isotype or derived therefrom, optionally having one or more mutations or modifications. In another embodiment, the Fc domain is an IgG4 isotype or derived therefrom, optionally having one or more mutations or modifications. In one embodiment, the Fc domain is human IgG1 Fc or human IgG4 Fc.

In some embodiments, the CH2 domain of the Fc domain comprises amino acids at positions 237-340 according to the EU numbering system, wherein
the amino acid sequence of the CH2 domain of the Fc domain of an IgG1 molecule comprises the following amino acids at positions 237-340: 
GPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAK (SEQ ID NO: 127);
the amino acid sequence of the CH2 domain of the Fc domain of an IgG2 molecule comprises the following amino acids at positions 237-340: 
GPSVFLFPPKPKDTLMISRTPEVTCVVVDVSHEDPEVQFNWYVDGVEVHNAKTKPREEQFNSTFRVV
SVLTVVHQDWLNGKEYKCKVSNKGLPAPIEKTISKTK (SEQ ID NO: 128);
the amino acid sequence of the CH2 domain of the Fc domain of an IgG4 molecule comprises the following amino acids at positions 237-340:

GPSVFLFPPKPKDTLMISRTPEVTCVVVDVSQEDPEVQFNWYVDGVEVHNAKTKPREEQFNSTYRVV
SVLTVLHQDWLNGKEYKCKVSNKGLPSSIEKTISKAK (SEQ ID NO: 129).

In some embodiments, the CH3 domain of the Fc domain comprises amino acids at positions 341-447 according to the EU numbering system, wherein
the amino acid sequence of the CH3 domain of the Fc domain of an IgG1 molecule comprises the following amino acids at positions 341-447: 
GQPREPQVYTLPPSRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK (SEQ ID NO: 130);
the amino acid sequence of the CH3 domain of the Fc domain of an IgG2 molecule comprises the following amino acids at positions 341-447 
GQPREPQVYTLPPSREEMTKNQVSLTCLVKGFYPSDISVEWESNGQPENNYKTTPPMLDSDGSFFLYS
KLTVDKSRWQQGNVFSCSVMHEALHNHYTQKSLSLSPGK (SEQ ID NO: 131);
the amino acid sequence of the CH3 domain of the Fc domain of an IgG4 molecule comprises the following amino acids at positions 341-447:

GQPREPQVYTLPPSQEEMTKNQVSLTCLVKGFYPSDIAVEWESNGQPENNYKTTPPVLDSDGSFFLYS
RLTVDKSRWQEGNVFSCSVMHEALHNHYTQKSLSLSLGK (SEQ ID NO: 132).

In some embodiments, the functional domain comprises an antibody hinge region-CH2-CH3. In some embodiments, both polypeptide chains of the multi-specific polypeptide molecule comprise a hinge region-CH2-CH3.

The potency of the multi-specific polypeptide molecule is at least in part due to the ability of the hinge region and/or Fc region to mediate one or more effector functions. These effector functions are due to interactions of antibodies and antibody-antigen complexes with cells of the immune system to stimulate multiple responses, including antibody-dependent cell-mediated cytotoxicity (ADCC) and complement-dependent cytotoxicity (CDC) (Daeron, Annu.Rev.Immunol.15:203-234 (1997); Ward and Ghetie, Therapeutic Immunol.2:77-94 (1995); and Ravetch and Kinet, Annu.Rev.Immunol.9:457-492 (1991)). Multiple effector functions are mediated by Fc receptors (FcR). FcR are defined by their specificity for immunoglobulin isotypes; the Fc receptor for IgG antibodies is referred to as FcγR, the Fc receptor for IgE antibodies is referred to as FcεR, the Fc receptor for IgA antibodies is referred to as FcαR, and so forth. The hinge region and/or Fc region of an antibody also mediates functions that act independently of antigen binding and that confer persistence in circulation and the ability to transcytose across cell barriers via endocytosis, e.g., binding to FcRn (Ward and Ghetie, TherapeuticImmunology 2:77-94 (1995)). However, the effector functions mediated by the hinge region and/or Fc region of an antibody may in some cases cause undesirable side effects; therefore, efforts have been made to design antibodies with reduced or silenced effector functions.

In some embodiments, the antibody hinge region comprises at least one amino acid mutation capable of reducing or eliminating binding of the multi-specific molecule to the FcR receptor and/or C1q; e.g., the mutation site is selected from the group consisting of positions 233, 234, 235 and 236. In some embodiments, the mutation is selected from the group consisting of 233P, 234A, 234V, 235A, a 236 deletion mutation, and 236G. In some embodiments, the mutation is 234A235A. In some embodiments, the mutation sites are 233, 234, 235 and 236, preferably 233P, 234V, 235A, and no residue or G at position 236.

In some embodiments, in order to eliminate a disulfide bond formed between the hinge region and the non-hinge region, e.g., when the multi-specific polypeptide molecules according to the first to eighth aspects of the present disclosure comprise CL and/or CH1 structures, in order to reduce or eliminate a disulfide bond formed between the hinge region and CL and/or CH1, the hinge region further comprises a mutation at position 220, e.g., C220S.

In some embodiments, the amino acid sequence of the hinge region is selected from the group consisting of EPKSSDKTHTCPPCPAPPVAG (SEQ ID NO: 133), EPKSSDKTHTCPPCPAPPVA (SEQ ID NO: 4), EPKSSDKTHTCPPCP (SEQ ID NO: 134), PKSSDKTHTCPPCPAPPVAG (SEQ ID NO: 135), PKSSDKTHTCPPCPAPPVA (SEQ ID NO: 136), KSSDKTHTCPPCPAPPVAG (SEQ ID NO: 137), KSSDKTHTCPPCPAPPVA (SEQ ID NO: 138), SSDKTHTCPPCPAPPVAG (SEQ ID NO: 139), SSDKTHTCPPCPAPPVA (SEQ ID NO: 140), SDKTHTCPPCPAPPVAG (SEQ ID NO: 141), SDKTHTCPPCPAPPVA (SEQ ID NO: 142), DKTHTCPPCPAPPVAG (SEQ ID NO: 143), DKTHTCPPCPAPPVA (SEQ ID NO: 144), KTHTCPPCPAPPVAG (SEQ ID NO: 145), KTHTCPPCPAPPVA (SEQ ID NO: 146), THTCPPCPAPPVAG (SEQ ID NO: 147), THTCPPCPAPPV (SEQ ID NO: 148), HTCPPCPAPPVAG (SEQ ID NO: 149), HTCPPCPAPPVA (SEQ ID NO: 150), TCPPCPAPPVAG (SEQ ID NO: 151), TCPPCPAPPVA (SEQ ID NO: 152), CPPCPAPPVAG (SEQ ID NO: 153), CPPCPAPPVA (SEQ ID NO: 154), PPCPAPPVAG (SEQ ID NO: 155), PPCPAPPVA (SEQ ID NO: 156), CPAPPVAG (SEQ ID NO: 157), CPAPPVA (SEQ ID NO: 158), CPAPPVAG (SEQ ID NO: 159) or PAPPVA (SEQ ID NO: 160); or a variant thereof having one or more substitutions (e.g., 1-6 substitutions, such as 1-5, 1-4, 1, 2, 3, 4, 5 or 6 substitutions). In preferred embodiments, the amino acid sequence of the hinge region comprises EPKSSDKTHTCPPCPAPPVA (SEQ ID NO: 4).

In some embodiments, the CH2 domain in the Fc domain comprises at least one amino acid mutation capable of reducing or eliminating binding of the multi-specific molecule to the FcR receptor and/or C1q; e.g., the mutation site is selected from the group consisting of positions 237, 250, 252, 254, 256, 270, 322, 327, 330 and 331, and preferably, the mutation is selected from the group consisting of 237A, 250R, 252Y, 254T, 256E, 270, 322, 327G, 327A, 330A, 330S and 331S. In some embodiments, the mutation site is selected from the group consisting of 327, 330 and 331. In some embodiments, the mutation sites are 327G, 330S and 331S. In some embodiments, the mutation site is 331S. In some embodiments, the amino acids at positions 327, 330 and 331 are A, A and S, respectively.

In addition, the ability of IgG binding C1q and activate the complement cascade also depends on the presence, absence, or modification of a carbohydrate moiety located between the two CH2 domains (which is typically anchored at Asn297). (Ward and Ghetie, Therapeutic Immunology 2:77-94 (1995) at page 81.). In some embodiments, the N297 mutation is selected from the group consisting of 297A, 297G and 297Q. In preferred embodiments, the N297 mutation is 297Q.

In some embodiments, the amino acid sequence of the CH2 domain in the Fc domain comprises amino acids at positions 237-340 selected from the group consisting of SEQ ID NOs: 161-163 and 5:

GPSVFLFPPKPKDTLMISRTPEVTCVVVDVSHEDPEVKFNWYVDGVEVHNAKTKPREEQYQST
YRVVSVLTVLHQDWLNGKEYKCKVSNKALPASIEKTISKAK (SEQ ID NO: 161);

GPSVFLFPPKPKDTLMISRTPEVTCVVVDVSHEDPEVKFNWYVDGVEVHNAKTKPREEQYNST
YRVVSVLTVLHQDWLNGKEYKCKVSNKALPASIEKTISKAK (SEQ ID NO: 162);

GPSVFLFPPKPKDTLMISRTPEVTCVVVDVSHEDPEVKFNWYVDGVEVHNAKTKPREEQYNST
YRVVSVLTVLHQDWLNGKEYKCKVSNKGLPSSIEKTISKAK (SEQ ID NO: 163); or

GPSVFLFPPKPKDTLMISRTPEVTCVVVDVSHEDPEVKFNWYVDGVEVHNAKTKPREEQYQST
YRVVSVLTVLHQDWLNGKEYKCKVSNKGLPSSIEKTISKAK (SEQ ID NO: 5).

In a preferred embodiment, the amino acid sequence of the CH2 domain in the Fc domain comprises SEQ ID NO: 5. In another preferred embodiment, the amino acid sequence of the CH2 domain in the Fc domain comprises SEQ ID NO: 161.

In some embodiments, the effector function silencing mutation is at one or more of the following positions in the hinge region and the CH2 domain: 233, 234, 235, 236, 237, 297, 327, 330 and 331; preferably, the effector function silencing mutation is generated by replacing at least one residue at positions 233, 234, 235, 236 and 331 with the corresponding residues derived from IgG2 or IgG4. In some embodiments, the effector function silencing mutation comprises, e.g., one or more of the following mutations: 233P, 234V, a deletion mutation at position 236 or 236G, 327G, 327A, 330A, 330S, 331S. In some embodiments, the effector function silencing mutation is 233P, 234V, 235A and no residue or G at position 236, 297Q, 331S. In some embodiments, the effector function silencing mutation is 233P, 234V, 235A and no residue or G at position 236, and 297Q, 327G, 330S and 331S. In some embodiments, the effector function silencing mutation is 233P, 234V, 235A and no residue or G at position 236, 331S. In some embodiments, the effector function silencing mutation is 233P, 234V, 235A and no residue or G at position 236, and 327G, 330S and 331S.

In some embodiments, the CH3 domain in the Fc domain comprises at least one amino acid mutations capable of reducing or eliminating binding of the multi-specific molecule to an FcR receptor and/or C1q, e.g., the mutation site is selected from the group consisting of 428 and 434. In some embodiments, the mutation is selected from the group consisting of 428L, 434S, 434A.

In some specific embodiments, the Fc domain comprises amino acid mutations capable of reducing or eliminating binding of the multi-specific molecule to an FcR receptor and/or C1q, wherein the mutations are 252Y254T256E, 428L434S and 250R428L434A.

In some embodiments, the CH3 in the Fc domain of any one polypeptide chain or two polypeptide chains of the polypeptide molecule further comprises mutations for promoting the polypeptide molecule to form a heterodimer.

In some embodiments, the CH3 in the Fc domain of any one polypeptide chain or two polypeptide chains of the polypeptide molecule comprises at least two cysteine residue mutations to form an inter-chain disulfide bond, e.g., 354C and 349C, or 242C and 334C.

In some embodiments, the CH3 in the Fc domain of any one polypeptide chain or two polypeptide chains of the polypeptide molecule comprises mutations capable of allowing the two CH3 chains to form a knob-into-hole structure, e.g., each CH3 independently comprises a mutation at one or more positions selected from the group consisting of positions 366, 368, 405 and 407, and the two CH3 chains do not comprise the same mutation. In some embodiments, the knob-into-hole structure mutations are T366W as the knob and T366'S, L368'A and Y407'V as the hole; in other embodiments, the knob-into-hole structure mutations are T366Y as the knob and Y407'T as the hole.

In some embodiments, the amino acid sequence of the CH3 domain in the Fc domain comprises:
the hole structure shown in GQPREPQVCTLPPSRDELTKNQVSLSCAVKGFYPSDIAVEWESNGQPENNYKTTPPVLDSDGSFFLVS KLTVDKSRWQQGNVFSCSVMHEALHNHYTQKSLSLSPGK (Hole: comprising the T366S, L368A and Y407V mutations and the Y349C mutation, SEQ ID NO: 6) and the knob structure shown in GQPREPQVYTLPPCRDELTKNQVSLWCLVKGFYPSDIAVEWESNGQPENNYKTTPPVLDSDGSFFLY SKLTVDKSRWQQGNVFSCSVMHEALHNHYTQKSLSLSPGK (Knob: comprising the T366W mutation and the S354C mutation, SEQ ID NO: 7).

In some embodiments, the CH3 in the Fc domain of any one polypeptide chain or two polypeptide chains of the polypeptide molecule comprises mutations capable of altering the charge property of the amino acid at the interaction interface of the CH3 region to promote heterodimer formation, e.g., the mutation positions of the two CH3 chains are each independently selected from at least 1, at least 2, or at least 3 of the group consisting of Y391, K392, T393, T394, P395, P396, V397, L398, D399, S400, Q347, V348, Y349, T350, S354, R355, E356, E357, F405, Y407 and K409. In some embodiments, mutations on one CH3 chain include P395K, P396K, V397K; P395K, P396K, V397C; or P395R, P396R, V397R; mutations on the other CH3 chain include T394D, P395D, P396D; T394C, P395D, P396D; or T394E, P395E, P396E. In other embodiments, the mutation positions on one CH3 chain are selected from the group consisting of K409, K392, K439 and K370, and are substituted negatively charged amino acids, e.g., aspartic acid (D) or glutamic acid (E); the mutation positions on the other CH3 chain are selected from the group consisting of D399, D356 and E357, and are substituted with positively charged amino acids, e.g., lysine (K), histidine (H) or arginine (R).

In embodiments of the multi-specific polypeptide molecule of the first aspect to the eighth aspect of the present disclosure, the antibody CH3 domain is derived from an antibody Fc domain. In some embodiments, the antibody CH3 domain is a CH3 domain derived from a human antibody, e.g., a CH3 domain derived from human IgG1, IgG2 or IgG4.

In some embodiments, the mutations of the antibody CH3 domain refer to the mutations of the CH3 domain in the Fc domain, and to avoid unnecessary repetition, details are not repeated herein.

Unless otherwise stated, the mutation position numbering of the amino acid positions on the hinge region disclosed in the present disclosure, the amino acid positions on the Fc domain, the amino acid positions on CH3 in the Fc domain, or the amino acid positions on the antibody CH3 domain are all according to the EU numbering system.

In embodiments of the multi-specific polypeptide molecule of the first aspect to the eighth aspect of the present disclosure, CL is derived from the CL domain of an antibody light chain, and CH1 is derived from the CH1 domain of an antibody heavy chain. In some embodiments, CL may comprise a C-terminal NEC deletion mutation relative to the wild-type sequence. In some embodiments, CH1 may comprise a C-terminal VPRDC deletion mutation relative to the wild-type sequence.

In embodiments of the multi-specific polypeptide molecule of the first aspect to the eighth aspect of the present disclosure, the TCR constant region or a fragment thereof is human or murine. In some embodiments, TRAC and TRBC are human or murine.

In the present disclosure, TRAC and TRBC may be wild-type or variants thereof, e.g., a truncated variant, an extended variant, an insertion variant, a deletion variant, and a mutant. For example, a TRAC variant may comprise one or more of T48C, N113K, a PESS deletion mutation, and an FFPSPESS deletion mutation relative to the wild-type sequence. For example, a TRBC variant may comprise one or more of S57C, C187A, N210D, and an FG loop deletion mutation relative to the wild-type sequence. In a preferred embodiment, TRAC and/or TRBC comprise at least one cysteine mutation relative to the wild-type sequence to form a disulfide bond between TRAC and TRBC; more preferably, the cysteine mutation is at the following positions: position 48 of the wild-type TCR α chain constant region and position 57 of the wild-type TCR β chain constant region. In some embodiments, 1-5 amino acids at the N-terminus of TRAC may be substituted. In some embodiments, 1-5 amino acids at the N-terminus of TRBC may be substituted.

In some embodiments, TRAC comprises one of the following amino acid sequences:
>TRAC comprises T48C, N113K (IMGT numbering system), and an FFPSPESS deletion mutation 
NIQKPDPAVYQLRDSKSSDKSVCLFTDFDSQTNVSQSKDSDVYITDKCVLDMRSMDFKSNSAV
AWSNKSDFACANAFNNSIIPEDT (SEQ ID NO: 8)
>TRAC comprises T48C, N113K (IMGT numbering system), and a PESS deletion mutation 
NIQKPDPAVYQLRDSKSSDKSVCLFTDFDSQTNVSQSKDSDVYITDKCVLDMRSMDFKSNSAV
AWSNKSDFACANAFNNSIIPEDTFFPS (SEQ ID NO: 9)
>TRAC comprises T48C and N113K (IMGT numbering system) mutations

NIQKPDPAVYQLRDSKSSDKSVCLFTDFDSQTNVSQSKDSDVYITDKCVLDMRSMDFKSNSAV
AWSNKSDFACANAFNNSIIPEDTFFPSPESS (SEQ ID NO: 10)

In some embodiments, TRBC comprises one of the following amino acid sequences:
>TRBC2, comprising S57C, C187A, and N210D mutations 
EDLKNVFPPEVAVFEPSEAEISHTQKATLVCLATGFYPDHVELSWWVNGKEVHSGVCTDPQPLK
EQPALNDSRYALSSRLRVSATFWQDPRNHFRCQVQFYGLSENDEWTQDRAKPVTQIVSAEAWGRAD
(SEQ ID NO: 11)
>TRBC2, comprising S57C, C187A, N210D, and an FG loop deletion mutation 
EDLKNVFPPEVAVFEPSEAEISHTQKATLVCLATGFYPDHVELSWWVNGKEVHSGVCTDPQPLK
EQPALNDSRYALSSRLRVSATFWQDPRNHFRCQVQFQIVSAEAWGRAD (SEQ ID NO: 12)
>TRBC1, comprising S57C, C187A, and N210D mutations 
EDLNKVFPPEVAVFEPSEAEISHTQKATLVCLATGFFPDHVELSWWVNGKEVHSGVCTDPQPLK
EQPALNDSRYALSSRLRVSATFWQDPRNHFRCQVQFYGLSENDEWTQDRAKPVTQIVSAEAWGRAD
(SEQ ID NO: 13)
>TRBC1, comprising S57C, C187A, N210D, and an FG loop deletion mutation

EDLNKVFPPEVAVFEPSEAEISHTQKATLVCLATGFFPDHVELSWWVNGKEVHSGVCTDPQPLK
EQPALNDSRYALSSRLRVSATFWQDPRNHFRCQVQFQIVSAEAWGRAD (SEQ ID NO: 14)

In some embodiments, the polypeptide molecule comprises TRAC or a fragment thereof, which is in cis connected to TRAV1 without a linker. Preferably, 1-5 amino acids at the N-terminus of TRAC may be substituted. In some embodiments, the polypeptide molecule comprises TRBC or a fragment thereof, which is in cis connected to TRBV1 without a linker. Preferably, 1-5 amino acids at the N-terminus of TRBC may be substituted.

In the present disclosure, CL and CH1 may be wild-type or variants thereof, e.g., a truncated variant, an extended variant, an insertion variant, a deletion variant, and a mutant. For example, a CL variant may comprise an NEC deletion mutation relative to the wild-type sequence. For example, a CH1 variant may comprise a VPRDC deletion mutation relative to the wild-type sequence.

In some embodiments, CL comprises one of the following amino acid sequences:
>CL 
RADAAPTVSIFPPSSEQLTSGGASVVCFLNNFYPKDINVKWKIDGSERQNGVLNSWTDQDSKDS
TYSMSSTLTLTKDEYERHNSYTCEATHKTSTSPIVKSFNRNEC (SEQ ID NO: 15)
>CL (comprising an NEC deletion mutation)

RADAAPTVSIFPPSSEQLTSGGASVVCFLNNFYPKDINVKWKIDGSERQNGVLNSWTDQDSKDS
TYSMSSTLTLTKDEYERHNSYTCEATHKTSTSPIVKSFNR (SEQ ID NO: 16)

In some embodiments, CH1 comprises one of the following amino acid sequences:
>CH1 
AKTTPPSVYPLAPGSAAQTNSMVTLGCLVKGYFPEPVTVTWNSGSLSSGVHTFPAVLQSDLYTLS
SSVTVPSSPRPSETVTCNVAHPASSTKVDKKIVPRDC (SEQ ID NO: 17)
>CH1 (comprising a VPRDC deletion mutation)

AKTTPPSVYPLAPGSAAQTNSMVTLGCLVKGYFPEPVTVTWNSGSLSSGVHTFPAVLQSDLYTLS
SSVTVPSSPRPSETVTCNVAHPASSTKVDKKI (SEQ ID NO: 18)

In embodiments of the multi-specific polypeptide molecule of the first to eighth aspects of the present disclosure, the polypeptide molecule comprises CL and CH1 or fragments thereof, which are in cis connected to TRAV1 and TRBV1, TRAV2 and TRBV2, VH1 and VL1, and VH2 and VL2 with or without a linker,.

In the absence of any contrary statement, the term "in cis connection" refers to a connection form between a TCR constant region and a variable region in the native state.

In embodiments of the multi-specific polypeptide molecule of the first to eighth aspects of the present disclosure, TRAV1 and/or TRAV2 may be a native TRAV or a functional derivative form thereof, provided that the functional derivative form retains the ability to bind the target antigen. For example, TRAV1 and/or TRAV2 may be a truncated derivative form of a native TRAV, such as a derivative form truncated by 8 amino acids at the N-terminus or the C-terminus. In some embodiments, TRBV1 and/or TRBV2 may be a native TRBV or a functional derivative form thereof, provided that the functional derivative form retains the ability to bind the target antigen. In some embodiments, TRAV1 and/or TRAV2 are TRAV of an αβ TCR; and TRBV1 and/or TRBV2 are TRBV of an αβ TCR. In some embodiments, TRAV1 and/or TRAV2 are TRAV of an scTCR;and TRBV1 and/or TRBV2 are TRBV of an scTCR.

In embodiments of the multi-specific polypeptide molecule of the first to eighth aspects of the present disclosure, VH1 and VL1 comprise at least one cysteine mutation to form a disulfide bond between VH1 and VL1, and the cysteine is introduced into FR4 in the case of VL and introduced into FR2 in the case of VH. In some embodiments, VH2 and VL2 comprise at least one cysteine mutation to form a disulfide bond between VH2 and VL2, and the cysteine is introduced into FR4 in the case of VL and introduced into FR2 in the case of VH.

In some embodiments, VH1 and VL1 comprise at least one cysteine mutation to form a disulfide bond between VH1 and VL1, preferably, the cysteine mutations are at position 44 of VH1 and position 100 of VL1.

In some embodiments, VH2 and VL2 comprise at least one cysteine mutation to form a disulfide bond between VH2 and VL2, preferably, the cysteine mutations are at position 44 of VH2 and position 100 of VL2.

In some embodiments, the TRAV1 and VL1 or VL2 comprise at least one cysteine mutation to form a disulfide bond between TRAV1 and VL1 or VL2, preferably, the cysteine mutations are at position 104 of TRAV1 and position 80 of VL1 or VL2.

In some embodiments, the TRAV2 and VL1 or VL2 comprise at least one cysteine mutation to form a disulfide bond between TRAV2 and VL1 or VL2, preferably, the cysteine mutations are at position 104 of TRAV2 and position 80 of VL1 or VL2.

In some embodiments, the TRBV1 and VL1 or VL2 comprise at least one cysteine mutation to form a disulfide bond between TRBV1 and VL1 or VL2, preferably, the cysteine mutations are at position 84 of TRBV1 and position 80 of VL1 or VL2.

In some embodiments, the TRBV2 and VL1 or VL2 comprise at least one cysteine mutation to form a disulfide bond between TRBV2 and VL1 or VL2, preferably, the cysteine mutations are at position 84 of TRBV2 and position 80 of VL1 or VL2.

In some embodiments, the TRAV1 and VH1 or VH2 comprise at least one cysteine mutation to form a disulfide bond between TRAV1 and VH1 or VH2, preferably, the cysteine mutations are at position 104 of TRAV1 and position 89 of VH1 or VH2.

In some embodiments, the TRAV2 and VH1 or VH2 comprise at least one cysteine mutation to form a disulfide bond between TRAV2 and VH1 or VH2, preferably, the cysteine mutations are at position 104 of TRAV2 and position 89 of VH1 or VH2.

In embodiments of the multi-specific polypeptide molecule of the first to eighth aspects of the present disclosure, the polypeptide molecule further comprises one or more amino acid mutations, insertions or deletions capable of enhancing its affinity or biological activity (e.g., enhancing its solubility, enhancing its aggregation, enhancing its stability, extending its half-life, reducing its immunogenicity) or reducing its post-translational modifications (e.g., glycosylation modification), e.g., 1, 2, 3, 4, 5 or more amino acid mutations, insertions or deletions. The amino acids of the mutations, insertions or deletions include but are not limited to: (a) neutral amino acids: Ser, Gln; (b) acidic amino acids: Asp; (c) long-chain aliphatic amino acids: Ile; (d) short aliphatic amino acids: Ala, Gly. In some embodiments, the amino acid mutations, insertions or deletions are located at the following amino acid positions: the beginning of TRAV, TRBV, VL, or VH, the TRAV/TRAC junction amino acid(s), the TRBV/TRBC junction amino acid(s), the VL/CL junction amino acid(s), the VH/CH1 junction amino acid(s), the hinge region or amino acid(s) adjacent thereto, the CH2/CH3 junction amino acid(s), the C-terminus of CH3, or amino acid(s) near the linker. In some embodiments, the amino acid mutations include mutations capable of enhancing binding to FcRn and/or effector function silencing mutations.

In some embodiments, the effector function silencing mutations are in the Fc region, e.g., the CH2 and CH3 domains. In some embodiments, the CH2 domain in the Fc region comprises at least one amino acid mutation capable of reducing or eliminating binding of the multi-specific molecule to FcR receptor and/or C1q, e.g., the mutations are selected from the group consisting of positions 237, 250, 252, 254, 256, 270, 322, 327, 330 and 331, preferably, the mutations are selected from the group consisting of 237A, 250R, 252Y, 254T, 256E, 270, 322, 327G, 327A, 330A, 330S and 331S. In some embodiments, the mutations are selected from the group consisting of positions 327, 330, 331. In some embodiments, the mutation positions are 327G, 330S, 331S. In some embodiments, the mutation position is 331S. In some embodiments, the amino acids at positions 327, 330, 331 are A, A, S, respectively. In some embodiments, the CH3 domain in the Fc region comprises at least one amino acid mutation capable of reducing or eliminating binding of the multi-specific molecule to FcR receptor and/or C1q, e.g., the mutation positions are selected from the group consisting of 428 and 434. In some embodiments, the mutations are selected from the group consisting of 428L, 434S, 434A.

In some embodiments, the effector function silencing mutations are in the hinge region. In some embodiments, the antibody hinge region comprises at least one amino acid mutation capable of reducing or eliminating binding of the multi-specific molecule to FcR receptor and/or C1q, e.g., the mutations are selected from the group consisting of positions 233, 234, 235 and 236. In some embodiments, the mutations are selected from the group consisting of 233P, 234A, 234V, 235A, 236 deletion mutation and 236G. In some embodiments, the mutations are 234A235A. In some embodiments, the mutation positions are 233, 234, 235 and 236, preferably 233P, 234V, 235A, and no residue or G at position 236.

In some embodiments, the effector function silencing mutations are at one or more of the following positions of the hinge region and CH2 domain: 233, 234, 235, 236, 237, 297, 327, 330 and 331; preferably, the effector function silencing mutations are generated by substituting at least one residue at positions 233, 234, 235, 236 and 331 with corresponding residues derived from IgG2 or IgG4. In some embodiments, the effector function silencing mutations include, e.g., one or more of the following mutations: 233P, 234V, 236 deletion mutation or 236G, 327G, 327A, 330A, 330S, 331S. In some embodiments, the effector function silencing mutations are 233P, 234V, 235A and no residue or G at position 236, 297Q, 331S. In some embodiments, the effector function silencing mutations are 233P, 234V, 235A and no residue or G at position 236 and 297Q, 327G, 330S and 331S. In some embodiments, the effector function silencing mutations are 233P, 234V, 235A and no residue or G at position 236, 331S. In some embodiments, the effector function silencing mutations are 233P, 234V, 235A and no residue or G at position 236 and 327G, 330S and 331S.

In some specific embodiments, the Fc domain comprises amino acid mutations capable of reducing or eliminating binding of the multi-specific molecule to FcR receptor and/or C1q, wherein the mutations are 252Y254T256E, 428L434S or 250R428L434A.

In some embodiments, the amino acid mutations capable of reducing post-translational modifications of the polypeptide molecule include, e.g., N113K in TRAC and N210D in TRBC.

In embodiments of the multi-specific polypeptide molecule of the first to eighth aspects of the present disclosure, when the polypeptide molecule comprises more than one linker, the linkers may each be identical or different. In some embodiments, the linkers in the polypeptide molecule are each independently selected from linkers consisting of 1-35 amino acids.

In some embodiments, the linkers in the polypeptide molecule are each independently selected from the group consisting of S (SEQ ID NO: 19), GS (LM3, SEQ ID NO: 20), GGGS (SEQ ID NO: 21), GGGGS (SEQ ID NO: 22), GGGSGGGG (SEQ ID NO: 23), GGSGGS (LM1, SEQ ID NO: 24), GGSGGSGGS (SEQ ID NO: 25), GGGGSGGGGS (SEQ ID NO: 26), GGGGSGGGGSGGGGS (LM2, SEQ ID NO: 27), GGGGSGGGGSGGGGSGGGGSGGGS (SEQ ID NO: 28), GGGGSGGGGSGGGGSGGGGSGGGGSGGGGS (SEQ ID NO: 29), GQPKAAP (SEQ ID NO: 30), TVLRT (SEQ ID NO: 31), TVSSAS (SEQ ID NO: 32), GGEGG (SEQ ID NO: 33), GSEGGGS (SEQ ID NO: 34), RTSGPGDGGKGGPGKGPGGEGTKGTGPGG (SEQ ID NO: 35), GKGPGGEGTKGTGPGG (SEQ ID NO: 36), TVLSSAS (SEQ ID NO: 37) , EDLKN (SEQ ID NO: 38) or a variant thereof, EDLNK (SEQ ID NO: 39) or a variant thereof, and ANIQK (SEQ ID NO: 40) or a variant thereof, or any combination thereof.

In some embodiments, a variant of EDLKN is an amino acid sequence formed by substituting, deleting or adding one or more amino acids in EDLKN; and/or a variant of EDLNK is an amino acid sequence formed by substituting, deleting or adding one or more amino acids in EDLNK; and/or a variant of ANIQK is an amino acid sequence formed by substituting, deleting or adding one or more amino acids in ANIQK.

Preferably, a variant of EDLKN is an amino acid sequence formed by substituting one or more amino acids in EDLKN; and/or a variant of EDLNK is an amino acid sequence formed by substituting one or more amino acids in EDLNK; and/or a variant of ANIQK is an amino acid sequence formed by substituting one or more amino acids in ANIQK.

Preferably, a variant of EDLKN is an amino acid sequence consisting of 1-3 amino acids formed by deleting one or more amino acids from EDLKN; and/or a variant of EDLNK is an amino acid sequence consisting of 1-3 amino acids formed by deleting one or more amino acids from EDLNK; and/or a variant of ANIQK is an amino acid sequence consisting of 1-3 amino acids formed by deleting one or more amino acids from ANIQK.

Preferably, variants of EDLKN are EDL, DL, L, ED, E, D; and/or variants of ANIQK are ANI, NI, I, AN, N, A, PNI, P, PN.

Preferably, a variant of EDLKN is an amino acid sequence consisting of 6-35 amino acids formed by adding (preferably at the N-terminus) one or more amino acids to EDLKN; and/or a variant of EDLNK is an amino acid sequence consisting of 6-35 amino acids formed by adding (preferably at the N-terminus) one or more amino acids to EDLNK; and/or a variant of ANIQK is an amino acid sequence consisting of 6-35 amino acids formed by adding (preferably at the N-terminus) one or more amino acids to ANIQK.

In some embodiments, the linkers in the polypeptide molecule are each independently selected from linkers consisting of no more than 15 amino acids. Preferably, the linkers are each independently selected from the group consisting of S, GS, GGGS, GGGGS, GGGSGGGG, GGSGGS, GGSGGSGGS, GGGGSGGGGS, GGGGSGGGGSGGGGS, EDLKN or a variant thereof, EDLNK or a variant thereof, and ANIQK or a variant thereof. In some embodiments, a variant of EDLKN is an amino acid sequence formed by substituting, deleting or adding one or more amino acids in EDLKN; and/or a variant of EDLNK is an amino acid sequence formed by substituting, deleting or adding one or more amino acids in EDLNK; and/or a variant of ANIQK is an amino acid sequence formed by substituting, deleting or adding one or more amino acids in ANIQK. Preferably, a variant of EDLKN is an amino acid sequence formed by substituting one or more amino acids in EDLKN; and/or a variant of EDLNK is an amino acid sequence formed by substituting one or more amino acids in EDLNK; and/or a variant of ANIQK is an amino acid sequence formed by substituting one or more amino acids in ANIQK. Preferably, a variant of EDLKN is an amino acid sequence consisting of 1-3 amino acids formed by deleting one or more amino acids from EDLKN; and/or a variant of EDLNK is an amino acid sequence consisting of 1-3 amino acids formed by deleting one or more amino acids from EDLNK; and/or a variant of ANIQK is an amino acid sequence consisting of 1-3 amino acids formed by deleting one or more amino acids from ANIQK. Preferably, a variant of EDLKN is an amino acid sequence consisting of 6-15 amino acids formed by adding (preferably at the N-terminus) one or more amino acids to EDLKN; and/or a variant of EDLNK is an amino acid sequence consisting of 6-15 amino acids formed by adding (preferably at the N-terminus) one or more amino acids to EDLNK; and/or a variant of ANIQK is an amino acid sequence consisting of 6-15 amino acids formed by adding (preferably at the N-terminus) one or more amino acids to ANIQK. Preferably, variants of EDLKN are EDL, DL, L, ED, E, D; and/or variants of ANIQK are ANI, NI, I, AN, N, A, PNI, P, PN.

In some embodiments, the linkers in the polypeptide molecule are each independently one or a combination of 2-3 or more of the above linkers. In some embodiments, the linkers in the polypeptide molecule are each independently one or more of the above linkers. In some embodiments, the linkers in the polypeptide molecule are each independently a combination of two or more of the above linkers. In some embodiments, the linkers in the polypeptide molecule are each independently a combination of three or more of the above linkers.

In embodiments of the multi-specific polypeptide molecule of the first to eighth aspects of the present disclosure, the polypeptide molecule further comprises a signal peptide sequence at the N-terminus of one or more polypeptide chains, preferably comprises a signal peptide sequence at the N-terminus of each chain, e.g. a signal peptide sequence derived from albumin or immunoglobulin, preferably MGWSLILLFLVAVATRVHS (SEQ ID NO: 164).

In embodiments of the multi-specific polypeptide molecule of the first to eighth aspects of the present disclosure, the first antigen and the third antigen are each independently a TAA selected from the group consisting of melanoma-associated antigens (e.g., gp100, MAGEA1, MAGEA3, MAGEA6, MAGEA4, MAGEA2, MAGEA12, MAGEA2B, MAGEA9B, MAGEA10, MAGEA11, MAGEB2, MAGEC1, MAGEC2), IGF2BP1, GNGT1, PI4K2B, CCR8, NPSR1, COX7B2, ONECUT3, SMC1B, FOXI3, GAGE2A, FBXO43, BRDT, PAGE2, GAGE13, POU5F1B, CTAG1A and endogenous reverse transcriptase antigens.

In some embodiments, the first antigen and the third antigen are each independently a TSA selected from the group consisting of KRAS (e.g., KRAS G12 mutant antigens, such as G12D, G12V, G12C, G12R, G12A; G13D; Q61H; G125), TP53 (e.g., R175H, R173H, R273C, R248W, R248Q, R282W, Y220C, V157F, G245S, Y163C, R249S), PIK3CA (e.g., E542K, E545K, H1047R), CTNNB1 (e.g., S45P, T41A), EGFR (e.g., L858R, T790M), BRAF (e.g., V600E) and GNAS (e.g., R201C, R201H).

In some embodiments, the first antigen and the third antigen are each independently a viral antigen selected from the group consisting of HPV antigen (e.g., HPV E6 or E7 antigen), CMV antigen, HBV antigen, EBV antigen, herpesvirus antigen, human immunodeficiency virus (HIV) antigen, influenza virus antigen, and coronavirus antigen.

In some embodiments, the first antigen and the third antigen are each independently an self-antigen selected from the group consisting of AFP, CEA, CD19, CD20, BCMA, CD22, CD30, SLAM, CLDN18.2, GD2, mesothelin, CD38, Her2, GPC3, MUC1, Ro52, Ro60, La, Jo-1, SRP, IFIH1, CENPA, CENPB, SNRPA1, SNRNP70, SNR-PD3, RNAP3, TOPO1, Insulin, GAD65, IA2, Znt8, PL7, TARS, ARS, MI2, Insulin, EXOSC9, EXOSC107, POLR3A, POLR3K, PTRN, GAD2, SLC30A8, AchR, MUSK, LRP4, PLA2R, THSD7A, TSHR, IFN-γ, CHRNA1, MUSK, LRP4, AQP4, MOG, GRIN1, COL4A3, PLA2R, GM-SCF, PR3 and MPO.

In some embodiments, the second antigen and the fourth antigen are each independently selected from the group consisting of CD3 (e.g., CD3γ, CD3δ, and CD3ε chains), CD4, CD8, CD10, CD11b, CD11c, CD14, CD16, CD18, CD25, CD32a, CD32b, CD41, CD41b, CD42a, CD42b, CD44, CD45RA, CD49, CD61, CD64, CD68, CD94, CD90, CD117, Nkp46, NKG2D, FcεRI, TCRα/β, TCR γ/δ, HLA-DR, CD28, 4-1BB(CD137), OX40(CD134), ICOS(CD278), 2B4 (CD244), HVEM, LAG3, DAP10, DAP12, CD27, CD40, GITR, LFA-1, MyD88, CD2, CD7, LIGHT, B7-H3, CTLA-4, PD-1, PD-L1, CD80, BTLA, TIM3, TIGIT and LAG-3.

In some embodiments, the first antigen and the third antigen are each independently a TAA selected from the group consisting of melanoma-associated antigens (e.g., gp100, MAGEA1, MAGEA3, MAGEA6, MAGEA4, MAGEA2, MAGEA12, MAGEA2B, MAGEA9B, MAGEA10, MAGEA11, MAGEB2, MAGEC1, MAGEC2), IGF2BP1, GNGT1, PI4K2B, CCR8, NPSR1, COX7B2, ONECUT3, SMC1B, FOXI3, GAGE2A, FBXO43, BRDT, PAGE2, GAGE13, POU5F1B, CTAG1A, and endogenous reverse transcriptase antigen; and the second antigen and the fourth antigen are each independently selected from the group consisting of CD3 (e.g., CD3γ, CD3δ, and CD3ε chains), CD4, CD8, CD10, CD11b, CD11c, CD14, CD16, CD18, CD25, CD32a, CD32b, CD41, CD41b, CD42a, CD42b, CD44, CD45RA, CD49, CD61, CD64, CD68, CD94, CD90, CD117, Nkp46, NKG2D, FcεRI, TCRα/β, TCR γ/δ, HLA-DR, CD28, 4-1BB(CD137), OX40(CD134), ICOS(CD278), 2B4 (CD244), HVEM, LAG3, DAP10, DAP12, CD27, CD40, GITR, LFA-1, MyD88, CD2, CD7, LIGHT and B7-H3.

In some embodiments, the first antigen and the third antigen are each independently a TSA selected from the group consisting of KRAS (e.g., G12D, G12V, G12C, G12R, G12A, G13D, Q61H, and G125), TP53 (e.g., R175H, R173H, R273C, R248W, R248Q, R282W, Y220C, V157F, G245S, Y163C, and R249S), PIK3CA (e.g., E542K, E545K, and H1047R), CTNNB1 (e.g., S45P and T41A), EGFR (e.g., L858R and T790M), BRAF (e.g., V600E), and GNAS (e.g., R201C and R201H); and the second antigen and the fourth antigen are each independently selected from the group consisting of CD3 (e.g., CD3γ, CD3δ, and CD3ε chains), CD4, CD8, CD10, CD11b, CD11c, CD14, CD16, CD18, CD25, CD32a, CD32b, CD41, CD41b, CD42a, CD42b, CD44, CD45RA, CD49, CD61, CD64, CD68, CD94, CD90, CD117, Nkp46, NKG2D, FcεRI, TCRα/β, TCR γ/δ, HLA-DR, CD28, 4-1BB(CD137), OX40(CD134), ICOS(CD278), 2B4 (CD244), HVEM, LAG3, DAP10, DAP12, CD27, CD40, GITR, LFA-1, MyD88, CD2, CD7, LIGHT and B7-H3.

In some embodiments, the first antigen and the third antigen are each independently a viral antigen selected from the group consisting of HPV antigen (e.g., HPV E6 or E7 antigen), CMV antigen, HBV antigen, EBV antigen, herpesvirus antigen, human immunodeficiency virus (HIV) antigen, influenza virus antigen, and coronavirus antigen; and the second antigen and the fourth antigen are each independently selected from the group consisting of CD3 (e.g., CD3γ, CD3δ, and CD3ε chains), CD4, CD8, CD10, CD11b, CD11c, CD14, CD16, CD18, CD25, CD32a, CD32b, CD41, CD41b, CD42a, CD42b, CD44, CD45RA, CD49, CD61, CD64, CD68, CD94, CD90, CD117, Nkp46, NKG2D, FcεRI, TCRα/β, TCR γ/δ, HLA-DR, CD28, 4-1BB(CD137), OX40(CD134), ICOS(CD278), 2B4 (CD244), HVEM, LAG3, DAP10, DAP12, CD27, CD40, GITR, LFA-1, MyD88, CD2, CD7, LIGHT and B7-H3.

In some embodiments, the first antigen and the third antigen are each independently a self-antigen selected from group consisting of AFP, CEA, CD19, CD20, BCMA, CD22, CD30, SLAM, CLDN18.2, GD2, mesothelin, GAD65, IA2, Znt8, PL7, TARS, ARS, MI2, Topoisomerase 1, EXOSC9, EXOSC107, POLR3A, POLR3K, PTRN, GAD2, SLC30A8, AchR, MUSK, LRP4, PLA2R, THSD7A, TSHR, IFN-γ, CHRNA1, MUSK, LRP4, AQP4, MOG, GRIN1, COL4A3, PLA2R, GM-SCF, PR3 and MPO; and the second antigen and the fourth antigen are each independently selected from the group consisting of CTLA-4, PD-1, CD80, BTLA, TIM3, TIGIT, and LAG-3.

In some embodiments, the first antigen and the third antigen are each independently selected from the group consisting of melanoma-associated antigens (e.g., gp100, MAGEA1), KRAS, and HPV antigens (e.g., the HPV E7 antigens).

In some embodiments, the first antigen and the third antigen are each independently MAGE-A1.

In some embodiments, in the polypeptide molecule, TRAV1 and TRAV2 targeting MAGE-A1 each independently comprise RGEDVEQSLFLSVREGDSSVINCTYTDSSSTYLYWYKQEPGAGLQLLTYIFSNMDMKQDQRLTVLLN KKDKHLSLRIADTQTGDSAIYFCAGSGGGTDKLIFGTGTRLQVFPN (SEQ ID NO: 41). In some embodiments, in the polypeptide molecule, TRBV1 and TRBV2 targeting MAGE-A1 each independently comprise

GAVVSQHPSWVICKSGTSVKIECRSLDFQATTMFWYRQFPKQSLMLMATSDYQTCVTYEQGVEKDK
FLINHASLTLSTLTVTSAHPEDSSFYICSAREPGQGPFEQYFGPGTRLTVTE (SEQ ID NO: 42).

In some embodiments, the first antigen and the third antigen are each independently an HPV antigen, e.g., HPV E7.

In some embodiments, in the polypeptide molecule, TRAV1 and TRAV2 targeting the HPV E7 antigen each independently comprise CDR1 as shown in NSASQS (SEQ ID NO:43), CDR2 as shown in VYSSGN (SEQ ID NO:44), and CDR3 as shown in AVISAGTALI (SEQ ID NO:45); and TRBV1 and TRBV2 targeting the HPV E7 antigen each independently comprise CDR1 as shown in SGHDT (SEQ ID NO:46), CDR2 as shown in YYEEEE (SEQ ID NO:47), and CDR3 as shown in ASSLGWRGGLYTEAF (SEQ ID NO:48). In some embodiments, TRAV1 and TRAV2 targeting the HPV E7 antigen each independently comprise the amino acid sequence of RKEVEQDPGPFNVPEGATVAFNCTYSNSASQSFFWYRQDCRKEPKLLMSVYSSGNEDGRFTAQLNR ASQYISLLIRDSKLSDSATYLCAVISAGTALIFGKGTTLSVSS (SEQ ID NO:49). In some embodiments, TRBV1 and TRBV2 targeting the HPV E7 antigen each independently comprise the amino acid sequence of

DAGVTQSPTHLIKTRGQQVTLRCSPKSGHDTVSWYQQALGQGPQFIFQYYEEEERQRGNFPDRFSGH
QFPNYSSELNVNALLLGDSALYLCASSLGWRGGLYTEAFFGQGTRLTVV (SEQ ID NO:50).

In some embodiments, the first antigen and the third antigen are each independently KRAS, e.g., G12V, G12D.

In some embodiments, in the polypeptide molecule, TRAV1 and TRAV2 targeting KRAS each independently comprise CDR1 as shown in YGATPY(SEQ ID NO:51), CDR2 as shown in YFSGDTLV(SEQ ID NO:52), and CDR3 as shown in AVGVPGGYNKLI(SEQ ID NO:53); and TRBV1 and TRBV2 targeting KRAS each independently comprise CDR1 as shown in SGHVS(SEQ ID NO:54), CDR2 as shown in FQNEAQ(SEQ ID NO:55), and CDR3 as shown in ASSLDRGNTIY(SEQ ID NO:56). In some embodiments, in the polypeptide molecule, TRAV1 and TRAV2 targeting KRAS each independently comprise the amino acid sequence of AQSVTQPDIHITVSEGASLELRCNYSYGATPYLFWYVQSPGQGLQLLLKYFSGDTLVQGIKGFEAEFK RSQSSFNLRKPSVHWSDAAEYFCAVGVPGGYNKLIFGAGTRLAVHP (SEQ ID NO:57). In some embodiments, TRBV1 and TRBV2 targeting KRAS each independently comprise the amino acid sequence of GAGVSQSPRYKVAKRGQDVALRCDPISGHVSLFWYQQALGQGPEFLTYFQNEAQLDKSGLPSDRFF AERPEGSVSTLKIQRTQQEDSAVYLCASSLDRGNTIYFGEGSWLTVV (SEQ ID NO:58).

In some embodiments, in the polypeptide molecule, TRAV1 and TRAV2 targeting KRAS each independently comprise CDR1 as shown in VSGNPY(SEQ ID NO:59), CDR2 as shown in YITGDNLV(SEQ ID NO:60), and CDR3 as shown in AVRDIEGAGNNRKLI(SEQ ID NO:61); and TRBV1 and TRBV2 targeting KRAS each independently comprise CDR1 as shown in SGHNS(SEQ ID NO:62), CDR2 as shown in FNNNVP(SEQ ID NO:63), and CDR3 as shown in ASSEGFYTEAF(SEQ ID NO:64). In some embodiments, in the polypeptide molecule, TRAV1 and TRAV2 targeting KRAS each independently comprise the amino acid sequence of AQSVAQPEDQVNVAEGNPLTVKCTYSVSGNPYLFWYVQYPNRGLQFLLKYITGDNLVKGSYGFEAE FNKSQTSFHLKKPSALVSDSALYFCAVRDIEGAGNNRKLIWGLGTSLAVNP (SEQ ID NO:65). In some embodiments, in the polypeptide molecule, TRBV1 and TRBV2 targeting KRAS each independently comprise the amino acid sequence of

DAGVIQSPRHEVTEMGQEVTLRCKPISGHNSLFWYRQTMMRGLELLIYFNNNVPIDDSGMPEDRFSA
KMPNASFSTLKIQPSEPRDSAVYFCASSEGFYTEAFFGQGTRLTVV (SEQ ID NO:66).

In some embodiments, in the polypeptide molecule, TRAV1 and TRAV2 targeting KRAS each independently comprise CDR1 as shown in NSASDY(SEQ ID NO:67), CDR2 as shown in IRSNMDK(SEQ ID NO:68), and CDR3 as shown in AENSDGTSYGKLT(SEQ ID NO:69); and TRBV1 and TRBV2 targeting KRAS each independently comprise CDR1 as shown in SGHAT(SEQ ID NO:70), CDR2 as shown in FQNNGV(SEQ ID NO:71), and CDR3 as shown in ASSLVGSPDYEQY(SEQ ID NO:72). In some embodiments, in the polypeptide molecule, TRAV1 and TRAV2 targeting KRAS each independently comprise the amino acid sequence of GESVGLHLPTLGVQEGDNSIINCAYSNSASDYFIWYKQESGKGPQFIIDIRSNMDKRQGQRVTVLLNK TVKHLSLQIAATQPGDSAVYFCAENSDGTSYGKLTFGQGTILTVHP (SEQ ID NO:73). In some embodiments, in the polypeptide molecule, TRBV1 and TRBV2 targeting KRAS each independently comprise the amino acid sequence of

EAGVAQSPRYKIIEKRQSVAFWCNPISGHATLYWYQQILGQGPKLLIQFQNNGVVDDSQLPKDRFSA
ERLKGVDSTLKIQPAKLEDSAVYLCASSLVGSPDYEQYFGPGTRLTVT (SEQ ID NO:74).

In some embodiments, in the polypeptide molecule, TRAV1 and TRAV2 targeting KRAS each independently comprise a CDR1 as shown in DRGSQS(SEQ ID NO:75), a CDR2 as shown in IYSNGD(SEQ ID NO:76), and a CDR3 as shown in AVNLGAAGNKLT(SEQ ID NO:77); and TRBV1 and TRBV2 targeting KRAS each independently comprise a CDR1 as shown in SGHAT(SEQ ID NO:78), a CDR2 as shown in FQNNGV(SEQ ID NO:79), and a CDR3 as shown in ASSFSDSYNSPLH(SEQ ID NO:80). In some embodiments, in the polypeptide molecule, the TRAV1 and TRAV2 targeting KRAS each independently comprise the amino acid sequence of QKEVEQNSGPLSVPEGAIASLNCTYSDRGSQSFFWYRQYSGKSPELIMSIYSNGDKEDGRFTAQLNKA SQYVSLLIRDSQPSDSATYLCAVNLGAAGNKLTFGGGTRVLVKP (SEQ ID NO:81). In some embodiments, in the polypeptide molecule, the TRBV1 and TRBV2 targeting KRAS each independently comprise the amino acid sequence of

EAGVAQSPRYKIIEKRQSVAFWCNPISGHATLYWYQQILGQGPKLLIQFQNNGVVDDSQLPKDRFSA
ERLKGVDSTLKIQPAKLEDSAVYLCASSFSDSYNSPLHFGNGTRLTVT (SEQ ID NO:82).

In some embodiments, in the polypeptide molecule, the TRAV1 and TRAV2 targeting KRAS each independently comprise a CDR1 as shown in NSASQS(SEQ ID NO:83), a CDR2 as shown in VYSSGN(SEQ ID NO:84), and a CDR3 as shown in VVTNARLM(SEQ ID NO:85); and the TRBV1 and TRBV2 targeting KRAS each independently comprise a CDR1 as shown in MNHEY(SEQ ID NO:86), a CDR2 as shown in SMNVEV(SEQ ID NO:87), and a CDR3 as shown in ASTSIVSSGRGEQF(SEQ ID NO:88). In some embodiments, in the polypeptide molecule, the TRAV1 and TRAV2 targeting KRAS each independently comprise the amino acid sequence of RKEVEQDPGPFNVPEGATVAFNCTYSNSASQSFFWYRQDCRKEPKLLMSVYSSGNEDGRFTAQLNR ASQYISLLIRDSKLSDSATYLCVVTNARLMFGDGTQLVVKP (SEQ ID NO:89). In some embodiments, in the polypeptide molecule, the TRBV1 and TRBV2 targeting KRAS each independently comprise the amino acid sequence of

EAQVTQNPRYLITVTGKKLTVTCSQNMNHEYMSWYRQDPGLGLRQIYYSMNVEVTDKGDVPEGYK
VSRKEKRNFPLILESPSPNQTSLYFCASTSIVSSGRGEQFFGPGTRLTVL (SEQ ID NO:90).

In some embodiments, the first antigen and the third antigen are each independently gp100.

In some embodiments, in the polypeptide molecule, the TRAV1 and TRAV2 targeting gp100 each independently comprise the amino acid sequence of SQQGEEDPQALSIQEGENATMNCSYKTSINNLQWYRQNSGRGLVHLILIRSNEREKHSGRLRVTLDTS KKSSSLLITASRAADTASYFCATDGSTPMQFGKGTRLSVI (SEQ ID NO:91), and the TRBV1 and TRBV2 targeting gp100 each independently comprise the amino acid sequence of DGGITQSPKYLFRKEGQNVTLSCEQNLNHDAMYWYRQDPGQGLRLIYYSWAQGDFQKGDIAEGYS VSREKKESFPLTVTSAQKNPTAFYLCASSWGAPYEQYFGPGTRLTVT (SEQ ID NO:92). In some embodiments, the TRAV1 and TRAV2 targeting gp100 each independently comprise the amino acid sequence of SQQGEEDPQALSIQEGENATMNCSYKTSINNLQWYRQNSGRGLVHLILIRSNEREKHSGRLRVTLDTS KKSSSLLITASRACDTASYFCATDGSTPMQFGKGTRLSVI (SEQ ID NO:93); and the TRBV1 and TRBV2 targeting gp100 each independently comprise the amino acid sequence of

DGGITQSPKYLFRKEGQNVTLSCEQNLNHDAMYWYRQDPGQGLRLIYYSWAQGDFQKGDIAEGYS
VSREKKESFPLTVTSAQKCPTAFYLCASSWGAPYEQYFGPGTRLTVT (SEQ ID NO:94).

In some embodiments, the second antigen and the fourth antigen are different and are each independently selected from the group consisting of CD3, CD28, 4-1BB(CD137), or PD1. In some embodiments, the second antigen is CD3. In some embodiments, the fourth antigen is CD28. In some embodiments, the second antigen or the fourth antigen is 4-1BB(CD137). In some embodiments, the second antigen is CD3, and the fourth antigen is CD28. In some embodiments, the second antigen is CD3 or CD28, and the fourth antigen is 4-1BB(CD137). In some embodiments, the second antigen is 4-1BB(CD137), and the fourth antigen is CD3 or CD28. In some embodiments, the second antigen is CD3 or CD28, and the fourth antigen is PD1.

In some embodiments, the second antigen and the fourth antigen are the same and are both CD3. In some embodiments, the second antigen and the fourth antigen are both CD3, and the second binding region and the fourth binding region each independently comprise VH and VL derived from anti-CD3 antibody. Exemplary anti-CD3 antibodies include, but are not limited to, OKT3, UCHT-1, BMA031 and 12F6.

In some embodiments, the second binding region and the fourth binding region each independently comprise VH and VL of the following antibodies:
***VH***
   >VH (anti-CD3 antibody huUCHT1, comprising a G44C mutation) 
EVQLVESGGGLVQPGGSLRLSCAASGYSFTGYTMNWVRQAPGKCLEWVALINPYKGVSTYNQK
FKDRFTISVDKSKNTAYLQMNSLRAEDTAVYYCARSGYYGDSDWYFDVWGQGTLVTVSS (SEQ ID
NO: 95)
   >VH (anti-CD3 antibody huUCHT1, comprising G44C and E89C mutations) 
EVQLVESGGGLVQPGGSLRLSCAASGYSFTGYTMNWVRQAPGKCLEWVALINPYKGVSTYNQK
FKDRFTISVDKSKNTAYLQMNSLRACDTAVYYCARSGYYGDSDWYFDVWGQGTLVTVSS (SEQ ID
NO: 96)
   >VH (anti-CD28 antibody, comprising a C50Y mutation) 
QVQLVQSGAEVKKPGASVKVSCKASGYTFTSYYIHWVRQAPGQGLEWIGYIYPGNVNTNYNEK
FKDRATLTVDTSISTAYMELSRLRSDDTAVYFCTRSHYGLDWNFDVWGQGTTVTVSS (SEQ ID NO:
97)
   >VH (anti-CD28 antibody, comprising C50Y and D89C mutations) 
QVQLVQSGAEVKKPGASVKVSCKASGYTFTSYYIHWVRQAPGQGLEWIGYIYPGNVNTNYNEK
FKDRATLTVDTSISTAYMELSRLRSCDTAVYFCTRSHYGLDWNFDVWGQGTTVTVSS (SEQ ID NO:
98)
   >VH-BB1 (anti-4-1BB antibody, monovalent) 
EVQLLESGGGLVQPGGSLRLSCAASGFTFSSYDMSWVRQAPGKCLEWVSWISYSGGSIYYADS
VKGRFTISRDNSKNTLYLQMNSLRAEDTAVYYCARDAQRNSMREFDYWGQGTLVTVSS (SEQ ID
NO: 99)
   >VH-BB2 (anti-4-1BB antibody, bivalent) 
EVQLVESGGGLVQPGRSLRLSCTASGFSLNDYWMSWVRQAPGKGLEWVGYIDVGGSLYYAAS
VKGRFTISRDDSKSIAYLQMNSLKTEDTAVYYCARGGLTYGFDLWGQGTLVTVSS (SEQ ID NO: 100)
***VL***
   >VL (anti-CD3 antibody huUCHT1, comprising an E100C mutation) 
DIQMTQSPSSLSASVGDRVTITCRASQDIRNYLNWYQQKPGKAPKLLIYYTSRLESGVPSRFSGS
GSGTDYTLTISSLQPEDFATYYCQQGNTLPWTFGCGTKVEIK (SEQ ID NO: 101)
   >VL (anti-CD3 antibody huUCHT1, comprising E80C and E100C mutations) 
DIQMTQSPSSLSASVGDRVTITCRASQDIRNYLNWYQQKPGKAPKLLIYYTSRLESGVPSRFSGS
GSGTDYTLTISSLQPCDFATYYCQQGNTLPWTFGCGTKVEIK (SEQ ID NO: 102)
   >VL (anti-CD28 antibody) 
DIQMTQSPSSLSASVGDRVTITCHASQNIYVWLNWYQQKPGKAPKLLIYKASNLHTGVPSRFSG
SGSGTDFTLTISSLQPEDFATYYCQQGQTYPYTFGGGTKVEIK (SEQ ID NO: 103)
   >VL (anti-CD28 antibody, comprising an E80C mutation) 
DIQMTQSPSSLSASVGDRVTITCHASQNIYVWLNWYQQKPGKAPKLLIYKASNLHTGVPSRFSG
SGSGTDFTLTISSLQPCDFATYYCQQGQTYPYTFGGGTKVEIK (SEQ ID NO: 104)
   >VL-BB1(anti-4-1BB antibody, monovalent) 
QSVLTQPPSASGTPGQRVTISCSGSSSNIGNNYVTWYQQLPGTAPKLLIYADSHRPSGVPDRFSGS
KSGTSASLAISGLRSEDEADYYCATWDYSLSGYVFGCGTKLTVL (SEQ ID NO: 105)
   >VL- BB2(anti-4-1BB antibody, bivalent)

DIVMTQSPSSLSASVGDRVTITCQASEDISSYLAWYQQKPGKAPKRLIYGASDLASGVPSRFSAS
GSGTDYTFTISSLQPEDIATYYCHYYATISGLGVAFGGGTKVEIK (SEQ ID NO: 106).

In preferred embodiments, the multi-specific polypeptide molecule provided by the present disclosure comprises:
a first polypeptide chain: TRBV1-TRBC-the first linker-VL1-the second linker-VH1-hinge region -CH2-CH3, and a second polypeptide chain: TRAV1- TRAC-hinge region -CH2-CH3 (format 62); or
a first polypeptide chain: TRAV1-TRAC-the first linker-VL1-the second linker-VH1-hinge region -CH2-CH3, and a second polypeptide chain: TRBV1-TRBC-hinge region -CH2-CH3 (format 62-LJH1); or
a first polypeptide chain: TRBV1-TRBC-the first linker-VH1-the second linker-VL1-hinge region -CH2-CH3, and a second polypeptide chain: TRAV1-TRAC-hinge region -CH2-CH3 (format 62-LJH2); or
a first polypeptide chain: TRAV1-TRAC-the first linker-VH1-the second linker-VL1-hinge region -CH2-CH3, and a second polypeptide chain: TRBV1-TRBC- linker -hinge region -CH2-CH3 (format 62-LJH3); or
a first polypeptide chain: VH1-the first linker-TRAV1-the eleventh linker -TRBV1-hinge region -CH2-CH3, and a second polypeptide chain: VL1-hinge region -CH2-CH3 (format 62-2); or
a first polypeptide chain: TRBV1-TRBC-the first linker-VL1-the second linker-VH1-the third linker-CH2-CH3, and a second polypeptide chain: TRAV1- TRAC-the fourth linker-CH2-CH3 (format 62-3); or
a first polypeptide chain: TRBV1-TRBC-the first linker-VL1-the second linker-VH1-hinge region -TRBC, and a second polypeptide chain: TRAV1- TRAC-hinge region -TRAC (format 62-5); or
a first polypeptide chain: TRBV1-TRBC-the first linker-VL1-the second linker-VH1-hinge region, and a second polypeptide chain: TRAV1- TRAC-hinge region -Alb (format 62-6); or
a first polypeptide chain: TRBV1-TRBC-the first linker-VL1-the second linker-VH1-the fifth linker-Alb, and a second polypeptide chain: TRAV1- TRAC (format 62-7); or
a first polypeptide chain: TRBV1-CH3-the first linker-VL1-the second linker-VH1-the third linker-CH2-CH3, and a second polypeptide chain: TRAV1-CH3-the fourth linker-CH2-CH3 (format 62-8); or
a first polypeptide chain: TRBV1-TRBC-the first linker-VL1, a second polypeptide chain: VH1-hinge region -CH2-CH3, and a third polypeptide chain: TRAV1-TRAC-hinge region -CH2-CH3 (format 62-1); or
a first polypeptide chain: TRBV1-TRBC, a second polypeptide chain: VL1-the second linker-VH1-hinge region -CH2-CH3, and a third polypeptide chain: TRAV1-TRAC-hinge region -CH2-CH3 (format 59); or
a first polypeptide chain: TRBV1-TRBC-the first linker-VL1-the sixth linker-TRAV2-the seventh linker-TRBV2-the eighth linker-VH1-hinge region -CH2-CH3, and a second polypeptide chain: TRAV1- TRAC-hinge region -CH2-CH3 (format 58-2); or
a first polypeptide chain: VL2-the ninth linker-VH2-the tenth linker-TRBV1-TRBC-the first linker-VL1-the second linker-VH1-hinge region -CH2-CH3, and a second polypeptide chain: TRAV1- TRAC-hinge region -CH2-CH3 (format 72); or
a first polypeptide chain: VL2-the ninth linker-VH2-the tenth linker-TRBV1-TRBC-the first linker-VL1-the sixth linker-TRAV2-the seventh linker-TRBV2-the eighth linker-VH1-hinge region -CH2-CH3, and a second polypeptide chain: TRAV1- TRAC-hinge region -CH2-CH3 (format 50-2).

In some embodiments, TRAC comprises the amino acid sequence as shown in SEQ ID NO: 8. In some embodiments, TRBC comprises the amino acid sequence as shown in SEQ ID NO: 11.

In some embodiments, the TRAV1 and TRBV1 are TRAV1 and TRBV1 of an anti-MAGE-A1 TCR, TRAV1 and TRBV1 of an anti-HPV E7 TCR, TRAV1 and TRBV1 of an anti-KRAS TCR, or TRAV1 and TRBV1 of an anti-gp100 TCR. In some embodiments, the TRAV1 and TRBV1 comprise the amino acid sequences as shown in SEQ ID NO: 41 and 42, respectively; the amino acid sequences as shown in SEQ ID NO: 49 and 50, respectively; the amino acid sequences as shown in SEQ ID NO: 57 and 58, respectively; the amino acid sequences as shown in SEQ ID NO: 65 and 66, respectively; the amino acid sequences as shown in SEQ ID NO: 73 and 74, respectively; the amino acid sequences as shown in SEQ ID NO: 81 and 82, respectively; the amino acid sequences as shown in SEQ ID NO: 89 and 90, respectively; or the amino acid sequences as shown in SEQ ID NO: 91 and 92, respectively.

In some embodiments, the TRAV2 and TRBV2 are TRAV2 and TRBV2 of an anti-MAGE-A1 TCR, TRAV2 and TRBV2 of an anti-HPV E7 TCR, TRAV2 and TRBV2 of an anti-KRAS TCR, or TRAV2 and TRBV2 of an anti-gp100 TCR. In some embodiments, the TRAV2 and TRBV2 comprise the amino acid sequences as shown in SEQ ID NO: 41 and 42, respectively; the amino acid sequences as shown in SEQ ID NO: 49 and 50, respectively; the amino acid sequences as shown in SEQ ID NO: 57 and 58, respectively; the amino acid sequences as shown in SEQ ID NO: 65 and 66, respectively; the amino acid sequences as shown in SEQ ID NO: 73 and 74, respectively; the amino acid sequences as shown in SEQ ID NO: 81 and 82, respectively; the amino acid sequences as shown in SEQ ID NO: 89 and 90, respectively; or the amino acid sequences as shown in SEQ ID NO: 91 and 92, respectively.

In some embodiments, the VH1 and VL1 are the VH and VL of an anti-CD3 antibody UCHT-1. In some embodiments, the VH1 comprises the amino acid sequence as shown in SEQ ID NO: 95. In some embodiments, the VL1 comprises the amino acid sequence as shown in SEQ ID NO: 101.

In some embodiments, the VH2 and VL2 are the VH and VL of an anti-CD28 antibody. In some embodiments, the VH2 comprises the amino acid sequence as shown in SEQ ID NO: 97. In some embodiments, the VL2 comprises the amino acid sequence as shown in SEQ ID NO: 103.

In some embodiments, the albumin is human serum albumin, and the amino acid sequence thereof is as shown in SEQ ID NO: 2.

In some embodiments, the hinge region comprises the amino acid sequence as shown in SEQ ID NO: 4.

In some embodiments, the CH2 domain comprises the amino acid sequence as shown in SEQ ID NO: 5 or 161. In some embodiments, the CH3 domain in one chain of the polypeptide molecule comprises the amino acid sequence as shown in SEQ ID NO: 6, and the CH3 domain in the other chain comprises the amino acid sequence as shown in SEQ ID NO: 7.

In some embodiments, the first linker is selected from the group consisting of GGSGGS (LM1, SEQ ID NO: 24), GGGGSGGGGSGGGGS (LM2, SEQ ID NO: 27), and GS (LM3, SEQ ID NO: 20). In more preferred embodiments, the first linker is GGGGSGGGGSGGGGS (LM2, SEQ ID NO: 27).

In some embodiments, the second linker, the third linker, the fourth linker, the fifth linker, the sixth linker, the seventh linker, the eighth linker, the ninth linker, the tenth linker, and the eleventh linker are each independently selected from the group consisting of GGGGS (SEQ ID NO: 22) and GGGGSGGGGSGGGGS (SEQ ID NO: 27). In a preferred embodiment, the second linker is GGGGSGGGGSGGGGS (SEQ ID NO: 27). In a preferred embodiment, the third linker is GGGGS (SEQ ID NO: 22). In a preferred embodiment, the fourth linker is GGGGS (SEQ ID NO: 22). In a preferred embodiment, the fifth linker is GGGGS (SEQ ID NO: 22). In a preferred embodiment, the sixth linker is GGGGS (SEQ ID NO: 22). In a preferred embodiment, the seventh linker is GGGGSGGGGSGGGGS (SEQ ID NO: 27). In a preferred embodiment, the eighth linker is GGGGS (SEQ ID NO: 22). In a preferred embodiment, the ninth linker is GGGGSGGGGSGGGGS (SEQ ID NO: 27). In a preferred embodiment, the tenth linker is GGGGS (SEQ ID NO: 22). In a preferred embodiment, the eleventh linker is GGGGSGGGGSGGGGS (SEQ ID NO: 27).

In a ninth aspect, the present disclosure provides a nucleic acid comprising the nucleotide sequences encoding each chain of the multi-specific polypeptide molecule according to any one of the first to eighth aspects of the present disclosure.

In a tenth aspect, the present disclosure provides a vector comprising the nucleic acid of the eighth aspect of the present disclosure.

Any suitable vector may be used in the present disclosure. Examples of vectors include but are not limited to, plasmids, viral vectors (including retroviral vectors, lentiviral vectors, adenoviral vectors, cowpox virus vectors, polyomavirus vectors and adenovirus-associated vectors (AAVs)), phages, phasmids, cosmids and artificial chromosomes (including BACs and YACs). The vector itself is usually a nucleotide sequence, usually comprises a DNA sequence comprising an insert (transgene) and a larger sequence that serves as the "backbone" of the vector. The engineered vector usually comprises an origin for self-replication in the host cell (if stable expression of polynucleotides is desired), a selection marker and a restriction enzyme cleavage site (e.g., a polyclonal site, MCS). The vector may additionally comprise a promoter, a genetic marker, a reporter gene, a targeting sequence, and/or a protein purification tag. As known to those skilled in the art, a large number of suitable vectors are known to those skilled in the art, and many are commercially available. Examples of suitable vectors are provided in J. Sambrook et al, Molecular Cloning: A Laboratory Manual (4th ed.), Cold Spring Harbor Laboratory, Cold Spring Harbor Laboratory Press, New York (2012), which is incorporated herein by reference in their entirety.

In some embodiments, the vector is selected from the group consisting of a lentiviral vector, a retroviral vector, a plasmid, a DNA vector, an mRNA vector, a transposon-based vector and an artificial chromosome.

In an eleventh aspect, the present disclosure provides a vector system comprising, on one or more vectors, the nucleotide sequences encoding each chain of the multi-specific polypeptide molecule according to any one of the first to eighth aspects of the present disclosure.

In a twelfth aspect, the present disclosure provides a host cell comprising the nucleic acid of the ninth aspect of the present disclosure, the vector of the tenth aspect of the present disclosure, or the vector system of the eleventh aspect of the present disclosure.

Any cell may be used as a host cell for the nucleic acid or vector of the present disclosure. The cell may be a eukaryotic cell, e.g., a plant cell (which does not have the potential to develop into a plant), animal cell, fungal cell, or algal cell, or may be a prokaryotic cell, e.g., a bacterium or protozoan cell. The cell may be a cultured cell or a primary cell, i.e., a cell directly isolated from an organism, e.g., a human. The cell may be an adherent cell or a suspension cell, i.e., a cell grown in suspension. A suitable host cell is known in the art and includes, for example, a DH5α E. coli cell, Chinese hamster ovary cell, monkey VERO cell, COS cell, HEK293 cell, and the like. For the purpose of producing the polypeptide molecules of the present disclosure, the cell is preferably a mammalian cell. Most preferably, the host cell is a human cell.

In some embodiments, the cell is selected from the group consisting of lymphocytes (e.g., T cells, NK cells), monocytes (e.g., PBMC) and stem cells. For example, the stem cells may be lymphoid progenitors, induced pluripotent stem cells (iPSC) or Hematopoietic Stem Cells (HSC). In some embodiments, the stem cells do not include embryonic stem cells obtained by destroying human embryos, and/or do not include totipotent stem cells used to develop and form an animal individual.

In a thirteenth aspect, the present disclosure provides a conjugate comprising the multi-specific polypeptide molecule according to any one of the first to eighth aspects of the present disclosure, and a chemical moiety conjugated to the multi-specific polypeptide molecule.

In some embodiments, the chemical moiety is selected from the group consisting of a therapeutic agent, an immunostimulatory molecule, and a detectable label.

In some embodiments, the therapeutic agents include, but are not limited to, immunomodulators, agonists, radioactive compounds, enzymes (e.g., perforin), chemotherapeutic agents (e.g., cisplatin), or toxins. In some embodiments, the therapeutic agent may be, for example, maytansine, geldanamycin, tubulin inhibitors such as tubulin-binding agents (e.g., auristatins), or minor groove binders such as calicheamicin.

Other suitable therapeutic agents include, for example, small molecule cytotoxic agents, i.e., compounds with a molecular weight of less than 700 Daltons that have the ability to kill mammalian cells. Such compounds may also comprise toxic metals capable of exerting a cytotoxic effect. In addition, it should be understood that these small molecule cytotoxic agents also include prodrugs, i.e., compounds that degrade or are converted under physiological conditions to release cytotoxic agents. Examples of such agents include cisplatin, maytansinoid derivatives, rachelmycin, calicheamicin, docetaxel, etoposide, gemcitabine, ifosfamide, irinotecan, melphalan, mitoxantrone, sorfimer sodium porphyrin II, temozolomide, topotecan, metformin, auristatin E, vinca alkaloids, and doxorubicin; peptide cytotoxins, i.e., proteins or fragments thereof that have the ability to kill mammalian cells, such as ricin, diphtheria toxin, Pseudomonas aeruginosa exotoxin A (PEA), DNases, and RNases; radionuclides, i.e., unstable isotopes of an element that decay with the concomitant emission of one or more of alpha particles, beta particles, or gamma rays, such as iodine-131, rhenium-186, indium-111, yttrium-90, bismuth-210, bismuth-213, actinium-225, and astatine-213; and chelating agents, which may be used to facilitate the binding of these radionuclides to the molecules or polymers thereof.

In some embodiments, the immunostimulatory molecule is an immune effector molecule that elicits an immune response. For example, the immunostimulatory molecule may be a cytokine such as interleukin-2 (IL-2) and interferon-gamma (IFN-γ), a chemokine such as interleukin-8 (IL-8), Platelet Factor 4 (PF4), melanoma growth stimulatory protein, a complement activator; a viral/bacterial protein domain, or a viral/bacterial peptide. In preferred embodiments, the immunostimulatory molecule is selected from the group consisting of cytokines, chemokines, platelet factors, and complement initiators.

In some embodiments, the detectable label may be selected from the group consisting of biotin, streptavidin, an enzyme or a catalytically active fragment thereof, a radionuclide, nanoparticles, paramagnetic metal ions, and fluorescent, phosphorescent, or chemiluminescent molecules. Detectable moieties for diagnostic purposes include, for example, fluorescent labels, radioactive labels, enzymes, nucleic acid probes, and contrast agents.

In a fourteenth aspect, the present disclosure provides a pharmaceutical composition comprising a multi-specific polypeptide molecule of any one of the first to eighth aspects of the present disclosure, a nucleic acid of the ninth aspect of the present disclosure, a vector of the tenth aspect of the present disclosure, a vector system of the eleventh aspect of the present disclosure, a host cell of the twelfth aspect of the present disclosure, a conjugate of the thirteenth aspect of the present disclosure, and a pharmaceutically acceptable excipient.

The pharmaceutical composition of the present disclosure is particularly suitable for administration to a human subject; however, it is also suitable for administration to a non-human animal. The composition and its components (i.e., the active agent and optional excipients) are preferably pharmaceutically acceptable, i.e., capable of eliciting the desired therapeutic effect in a recipient without causing any undesired local or systemic effects. The pharmaceutical composition of the present disclosure may be, for example, sterile.

Examples of excipients include, but are not limited to, fillers, binders, disintegrants, coating agents, adsorbents, anti-adherents, glidants, preservatives, antioxidants, flavoring agents, coloring agents, sweetening agents, solvents, co-solvents, buffers, chelating agents, viscosity-modifying agents, surfactants, diluents, wetting agents, carriers, emulsifiers, stabilizers, and tonicity agents. One of skill in the art will recognize how to select appropriate excipients for formulating the pharmaceutical compositions of the present disclosure. Exemplary carriers for use in the pharmaceutical compositions of the present disclosure include saline, buffered saline, glucose, and water. In general, the selection of appropriate excipients depends, inter alia, on the active agent(s) employed, the disease(s) to be treated, and the intended dosage form of the composition.

Depending on the active agent(s) employed, the pharmaceutical composition of the present disclosure may be formulated into a variety of forms, such as solid, liquid, gaseous, or lyophilized forms, specifically in the form of ointments, creams, transdermal patches, gels, powders, tablets, solutions, aerosols, granules, pills, suspensions, emulsions, capsules, syrups, liquid dosage forms, elixirs, extracts, tinctures, or fluid extracts, or any other form particularly suitable for the intended route of administration. Processes for preparing pharmaceutical compositions that are well known in the art are described in Remington's Pharmaceutical Sciences, 22nd Edition (Maack Publishing Co., Easton, PA, 2012), and may include, for example, conventional mixing, dissolving, granulating, sugar-coating, milling, emulsifying, encapsulating, embedding, or lyophilizing processes.

In some embodiments, the pharmaceutical composition further comprises a second therapeutic agent; preferably, the second therapeutic agent is selected from the group consisting of antibodies, chemotherapeutic agents, and small-molecule drugs.

Preferred examples of the second therapeutic agent include known anticancer drugs, such as cisplatin, maytansinoid derivatives, rachelmycin, calicheamicin, docetaxel, etoposide, gemcitabine, ifosfamide, irinotecan, melphalan, mitoxantrone, sorfimer sodium porphyrin II, temozolomide, topotecan, trimetrexate glucuronate, auristatin E, vincristine, and doxorubicin; peptide cytotoxins, such as ricin, diphtheria toxin, Pseudomonas aeruginosa exotoxin A (PEA), DNases, and RNases; radionuclides, such as iodine-131, rhenium-186, indium-111, yttrium-90, bismuth-210, bismuth-213, actinium-225, and astatine-213; prodrugs, such as antibody-directed enzyme-activated prodrugs; immunostimulants, such as interleukin-2 (IL-2); chemokines, such as interleukin-8 (IL-8), Platelet Factor 4 (PF4); antibodies or antigen-binding fragments thereof, such as an anti-CD3 antibody or an antigen-binding fragment thereof; complement activators; and viral/bacterial protein domains and viral/bacterial peptides.

In a fifteenth aspect, the present disclosure provides a method for preventing or treating a disease in a subject, comprising administering to the subject a therapeutically or prophylactically effective amount of a multi-specific polypeptide molecule of any one of the first through eighth aspects of the present disclosure, a nucleic acid of the ninth aspect of the present disclosure, a vector of the tenth aspect of the present disclosure, a vector system of the eleventh aspect of the present disclosure, a host cell of the twelfth aspect of the present disclosure, a conjugate of the thirteenth aspect of the present disclosure, or a pharmaceutical composition of the fourteenth aspect of the present disclosure, wherein the diseases are selected from the group consisting of cancer, infectious diseases, autoimmune diseases, and inflammatory diseases.

The cancer may be any type of cancer, such as cancers of the hematological system, cancers of the central and peripheral nervous systems, cancers of the lymphoid lineage, cancers of the myeloid lineage, cancers of mesenchymal origin, solid tumors, or the like. Examples of cancers include, but are not limited to, acute lymphoblastic leukemia (ALL), acute myeloid leukemia (AML), alveolar rhabdomyosarcoma, bone cancer, brain cancer, breast cancer, anal cancer, anal canal carcinoma, anorectal carcinoma, ocular cancer, intrahepatic cholangiocarcinoma, cancer of the joint, cancer of the neck, gallbladder cancer, cancer of the pleura, nasal cancer, nasal cavity carcinoma, middle ear carcinoma, oral cancer, vaginal cancer, vulvar cancer, chronic lymphocytic leukemia (CLL), chronic myeloid leukemia (CML), colon cancer, esophageal cancer, cervical cancer, gastrointestinal carcinoid tumors, glioma, Hodgkin lymphoma, hypopharyngeal cancer, renal cancer, laryngeal cancer, liver cancer, lung cancer, malignant mesothelioma, melanoma, multiple myeloma, nasopharyngeal carcinoma, non-Hodgkin lymphoma, oropharyngeal cancer, ovarian cancer, penile cancer, pancreatic cancer, peritoneal cancer, omental cancer, mesenteric cancer, pharyngeal cancer, prostate cancer, rectal cancer, skin cancer, small intestine cancer, soft tissue sarcoma, gastric cancer, testicular cancer, thyroid cancer, uterine cancer, ureteral cancer, and bladder cancer.

Infectious diseases are diseases caused by infection with a pathogen, including communicable and non-communicable infectious diseases. Pathogens that cause infectious diseases include, but are not limited to, viruses, bacteria, mycoplasma, chlamydia, rickettsia, prions, fungi, spirochetes, and parasites. Examples of viruses include, but are not limited to, human papillomavirus (HPV), cytomegalovirus (CMV), hepatitis B virus (HBV), Epstein-Barr virus (EBV), herpesviruses, human immunodeficiency virus (HIV), influenza viruses, and coronaviruses.

Common autoimmune diseases and inflammatory diseases include, but are not limited to, rheumatoid arthritis (RA), systemic lupus erythematosus (SLE), ankylosing spondylitis, psoriatic arthritis, type 1 diabetes mellitus caused by autoimmune destruction of pancreatic islets, Sjögren's syndrome, Hashimoto's thyroiditis, Graves' disease, toxic diffuse goiter, autoimmune hemolytic anemia, idiopathic thrombocytopenic purpura, inflammatory bowel disease (IBD, e.g., ulcerative colitis and Crohn's disease), psoriasis, glomerulonephritis, nephrotic syndrome, anti-glomerular basement membrane disease, membranous nephropathy, systemic sclerosis, polymyositis, myasthenia gravis, pemphigus, vitiligo, autoimmune diseases of the central nervous system, celiac disease, autoimmune gastritis, primary biliary cholangitis (PBC), autoimmune hepatitis, Goodpasture syndrome, autoimmune oophoritis, autoimmune orchitis, idiopathic leukopenia, primary adrenocortical atrophy, antiphospholipid antibody syndrome (APS), post-infection fibrosis, post-myocardial infarction fibrosis, hepatic fibrosis, and hypertrophic scarring.

In some embodiments, the dose administered to a subject may vary depending on the embodiment, the active agent employed, the route of administration, the site to be treated, and the subject. However, the dose should be sufficient to elicit a therapeutic response. A clinician may determine a therapeutically or prophylactically effective amount to administer to a human or other subject to treat or prevent a medical condition. The precise amount required for therapeutic or prophylactic efficacy may depend on a number of factors, such as the activity of the active agent and the route of administration.

The dose of the polypeptide molecule, conjugate, or pharmaceutical composition of the present disclosure may be administered to a mammal (including a human) in a single dose or as a series of divided doses over a suitable period of time, for example, as needed, once daily, twice weekly, once weekly, once every 2 weeks, twice monthly, once every 2 months, every 6 months, or annually. A dosage unit comprising a therapeutically effective amount of the polypeptide molecule, conjugate, or pharmaceutical composition may be administered as a single daily dose, or the total daily dose may, as needed, be administered as two, three, four, or more divided doses per day.

The appropriate mode of administration may be determined by a clinician. The route of administration may be parenteral administration, for example, by injection, intranasal administration, pulmonary administration, or transdermal administration. Administration may be systemic or local, delivered via intravenous (IV) injection, intramuscular (IM) injection, intraperitoneal (IP) injection, or subcutaneous (SC) injection. In some embodiments, the polypeptide molecule, conjugate, or pharmaceutical composition may be selected for parenteral delivery, inhalation, or delivery via the gastrointestinal (GI) tract, for example, oral administration. The dosage and route of administration may vary depending on the weight, age, condition, and the like of the subject, and may be appropriately selected.

In some embodiments, the method further comprises administering a second therapeutic agent to the subject. In certain embodiments, the polypeptide molecule, conjugate, or pharmaceutical composition of the present disclosure is administered before, substantially simultaneously with, or after administration of the second therapeutic agent. Preferably, the second therapeutic agent is selected from the group consisting of antibodies, chemotherapeutic agents, and small-molecule drugs. Preferred examples of the second therapeutic agent are as described above.

In a sixteenth aspect, the present disclosure provides a method for detecting (e.g., diagnosing) a disease in a subject, wherein the method comprises (i) contacting a sample obtained from the subject with the polypeptide molecule or the conjugate of the present disclosure; and (ii) detecting the presence of a target antigen in the sample, wherein the presence of the target antigen indicates that the subject has the corresponding disease.

The target antigen may be selected from the group consisting of tumor-associated antigens (TAA), tumor-specific antigens (TSA), viral antigens, and self-antigens. The diseases may be selected from the group consisting of cancer, infectious diseases, autoimmune diseases, and inflammatory diseases. The cancer, infectious diseases, autoimmune diseases, and inflammatory diseases are as defined above.

In some embodiments, the sample obtained from the subject may be a blood sample, a urine sample, a tissue sample, or a cell sample. In certain embodiments, the method is carried out in vitro. In some embodiments, the method comprises (i) contacting a sample obtained from the subject with the conjugate of the present disclosure, wherein the conjugate comprises a detectable label; and (ii) detecting the presence of the target antigen in the sample by detecting the detectable label. Examples of detectable labels include, but are not limited to, biotin, streptavidin, an enzyme or a catalytically active fragment thereof, a radionuclide, a nanoparticle, a paramagnetic metal ion, a nucleic acid probe, a contrast agent, and a fluorescent, phosphorescent, or chemiluminescent molecule; preferably an enzyme or a catalytically active fragment thereof, a radionuclide, a fluorescent, phosphorescent, or chemiluminescent molecule.

In a seventeenth aspect, the present disclosure provides a kit comprising the polypeptide molecule or the conjugate of the present disclosure, wherein the kit is used for detecting the presence of a target antigen in a test sample.

In some embodiments, the kit is a kit for detecting (e.g., diagnosing) a disease in a subject, comprising the polypeptide molecule or the conjugate of the present disclosure. The target antigen may be selected from the group consisting of tumor-associated antigens (TAA), tumor-specific antigens (TSA), viral antigens, and self-antigens. The diseases may be selected from the group consisting of cancer, infectious diseases, autoimmune diseases, and inflammatory diseases. The cancer, infectious diseases, autoimmune diseases, and inflammatory diseases are as defined above.

In some embodiments, the conjugate comprises a detectable label. Examples of detectable labels include, but are not limited to, biotin, streptavidin, an enzyme or a catalytically active fragment thereof, a radionuclide, a nanoparticle, a paramagnetic metal ion, a nucleic acid probe, a contrast agent, and a fluorescent, phosphorescent, or chemiluminescent molecule; preferably an enzyme or a catalytically active fragment thereof, a radionuclide, a fluorescent, phosphorescent, or chemiluminescent molecule. In some embodiments, the kit may further comprise instructions for use of the kit.

In an eighteenth aspect, the present disclosure provides use of the multi-specific polypeptide molecule according to any one of the first to eighth aspects of the present disclosure, the nucleic acid of the ninth aspect of the present disclosure, the vector of the tenth aspect of the present disclosure, the vector system of the eleventh aspect of the present disclosure, the host cell of the twelfth aspect of the present disclosure, the conjugate of the thirteenth aspect of the present disclosure, or the pharmaceutical composition of the fourteenth aspect of the present disclosure, in the manufacture of a medicament for treating or preventing a disease in a subject.

In a nineteenth aspect, the present disclosure provides the multi-specific polypeptide molecule according to any one of the first to eighth aspects of the present disclosure, the nucleic acid of the ninth aspect of the present disclosure, the vector of the tenth aspect of the present disclosure, the vector system of the eleventh aspect of the present disclosure, the host cell of the twelfth aspect of the present disclosure, the conjugate of the thirteenth aspect of the present disclosure, or the pharmaceutical composition of the fourteenth aspect of the present disclosure, for use in treating or preventing a disease in a subject.

In a twentieth aspect, the present disclosure provides use of the multi-specific polypeptide molecule according to any one of the first to eighth aspects of the present disclosure or the conjugate of the thirteenth aspect of the present disclosure, in the manufacture of a kit for detecting the presence of a target antigen in a test sample.

In some embodiments, the present disclosure provides use of the multi-specific polypeptide molecule according to any one of the first to eighth aspects of the present disclosure or the conjugate of the thirteenth aspect of the present disclosure, in the manufacture of a kit for detecting (e.g., diagnosing) a disease in a subject.

In a twenty-first aspect, the present disclosure provides the multi-specific polypeptide molecule according to any one of the first to eighth aspects of the present disclosure or the conjugate of the thirteenth aspect of the present disclosure, for use in detecting the presence of a target antigen in a test sample.

In some embodiments of the uses disclosed herein, the target antigen may be selected from the group consisting of tumor-associated antigens (TAA), tumor-specific antigens (TSA), viral antigens, and self-antigens. The diseases may be selected from the group consisting of cancer, infectious diseases, autoimmune diseases, and inflammatory diseases. The cancer, infectious diseases, autoimmune diseases, and inflammatory diseases are as defined above.

The present disclosure provides the following preferred embodiments:
1. A multi-specific polypeptide molecule, wherein the multi-specific polypeptide molecule comprises a first binding region that binds a first antigen and a second binding region that binds a second antigen,
   wherein the first binding region comprises an α chain variable region (TRAV1) and a β chain variable region (TRBV1) derived from a T cell receptor (TCR) that binds the first antigen-MHC complex;
   wherein the second binding region comprises a heavy chain variable region VH1 and a light chain variable region VL1 derived from an antibody that binds the second antigen, or a single heavy chain variable region (VHH1) derived from a single-domain antibody or a nanobody that binds the second antigen;
   wherein the first antigen is selected from the group consisting of tumor-associated antigens (TAA), tumor-specific antigens (TSA), viral antigens, and self-antigens, and the second antigen is an immune cell surface molecule;
   wherein the N-terminus of the antibody is connected to the C-terminus of the TCR;
   wherein the multi-specific polypeptide molecule further comprises at least one of the following functional domains:
      1) a hinge region derived from an antibody;
      2) an Fc domain derived from an antibody or a dimerization portion thereof;
      3) a CH3 domain derived from an antibody;
      4) albumin (Alb) or a binding domain thereof;
      5) a constant region derived from a TCR or a fragment thereof.
2. The multi-specific polypeptide molecule according to Embodiment 1, wherein TRAV1 and TRBV1 are distributed on different polypeptide chains; and/or
   VH1 and VL1 are distributed on one polypeptide chain (e.g., forming scFv1); and TRAV1 and TRBV1 are distributed on different polypeptide chains.
3. The multi-specific polypeptide molecule according to Embodiment 1 or 2, wherein the multi-specific polypeptide molecule comprises, on two polypeptide chains, the first binding region that binds the first antigen and the second binding region that binds the second antigen;
   preferably, TRAV1 and TRBV1 are distributed on different polypeptide chains, the VHH1 or the VH1 and VL1 are distributed on one polypeptide chain (e.g., forming scFv1) and connected to the C-terminus of the TCR; or TRAV1 and TRBV1 are distributed on different polypeptide chains, the VH1 and VL1 are distributed on different polypeptide chains, and the polypeptide chain on which one of the VH1 and VL1 is located is connected to the C-terminus of the polypeptide chain on which the TRAV1 is located, and the polypeptide chain on which the other is located is connected to the C-terminus of the polypeptide chain on which the TRBV1 is located;
      or,
   the multi-specific polypeptide molecule comprises, on three polypeptide chains, a first binding region that binds a first antigen and a second binding region that binds a second antigen;
   preferably, TRAV1 and TRBV1 are distributed on different polypeptide chains, the VH1 and VL1 are distributed on different polypeptide chains, and the polypeptide chain on which VH1 or VL1 is located is connected to the C-terminus of the polypeptide chain on which TRBV1 or TRAV1 is located.
4. The multi-specific polypeptide molecule according to any one of Embodiments 1-3, wherein the polypeptide chains of the multi-specific polypeptide molecule respectively comprise:
   TRBV1-scFv1 and TRAV1; or
   TRAV1-scFv1 and TRBV1; or
   TRBV1-VHH1 and TRAV1; or
   TRAV1-VHH1 and TRBV1; or
   TRBV1-VL1 and TRAV1-VH1; or
   TRAV1-VL1 and TRBV1-VH1; or
   TRBV1-VL1-VH1 and TRAV1; or
   TRBV1-VH1-VL1 and TRAV1; or
   TRAV1-VL1-VH1 and TRBV1; or
   TRAV1-VH1-VL1 and TRBV1; or
   TRBV1-VL1, VH1, and TRAV1; or
   TRBV1-VH1, VL1, and TRAV1; or
   TRAV1-VL1, VH1, and TRBV1; or
   TRAV1-VH1, VL1, and TRBV1.
5. The multi-specific polypeptide molecule according to any one of Embodiments 1-4, wherein the multi-specific polypeptide molecule further comprises a third binding region that binds a third antigen, and the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens and self-antigens, and the third antigen is the same as or different from the first antigen.
6. The multi-specific polypeptide molecule according to Embodiment 5, wherein the TRAV2 is the same as the TRAV1, and/or the TRBV2 is the same as the TRBV1.
7. The multi-specific polypeptide molecule according to Embodiment 5 or 6, wherein the TRAV2 and the TRBV2 are linked by a linker on the same polypeptide chain, and are preferably linked between VH1 and VL1.
8. The multi-specific polypeptide molecule according to Embodiment 7, wherein the multi-specific polypeptide molecule comprises two polypeptide chains, and the two polypeptide chains respectively comprise:
   TRBV1-VL1-TRAV2-TRBV2-VH1 and TRAV1; or
   TRBV1-VL1-TRBV2-TRAV2-VH1 and TRAV1; or
   TRBV1-VH1-TRBV2-TRAV2-VL1 and TRAV1; or
   TRBV1-VH1-TRAV2-TRBV2-VL1 and TRAV1; or
   TRAV1-VL1-TRBV2-TRAV2-VH1 and TRBV1; or
   TRAV1-VL1-TRAV2-TRBV2-VH1 and TRBV1; or
   TRAV1-VH1-TRAV2-TRBV2-VL1 and TRBV1; or
   TRAV1-VH1-TRBV2-TRAV2-VL1 and TRBV1.
9. The multi-specific polypeptide molecule according to any one of Embodiments 1-8, wherein the multi-specific polypeptide molecule further comprises a fourth binding region that binds a fourth antigen, the fourth binding region comprises a heavy chain variable region VH2 and a light chain variable region VL2 derived from an antibody that binds the fourth antigen, or a single heavy chain variable region (VHH2) derived from a single-domain antibody or nanobody that binds the fourth antigen; wherein the fourth antigen is an immune cell surface molecule, and the fourth antigen is the same as or different from the second antigen.
10. The multi-specific polypeptide molecule according to Embodiment 9, wherein the VH2 is the same as the VH1, and/or the VL2 is the same as the VL1, and/or the VHH2 is the same as the VHH1.
11. The multi-specific polypeptide molecule according to Embodiment 9 or 10, wherein the VHH2 or the VH2 and VL2 are arranged in tandem on one polypeptide chain (e.g., forming scFv2 or Fab2) and connected to the N-terminus of the TRAV1 or the TRBV1, or one of the VH2 and VL2 is connected to the N-terminus of the TRAV1 and the other is connected to the N-terminus of the TRBV1.
12. The multi-specific polypeptide molecule according to Embodiment 11, wherein the multi-specific polypeptide molecule comprises two polypeptide chains, and the two polypeptide chains respectively comprise:
   scFv2-TRBV1-scFv1 and TRAV1; or
   scFv2-TRAV1-scFv1 and TRBV1; or
   scFv2-TRBV1-VHH1 and TRAV1; or
   scFv2-TRAV1-VHH1 and TRBV1; or
   scFv2-TRBV1-VL1 and TRAV1-VH1; or
   scFv2-TRAV1-VL1 and TRBV1-VH1; or
   TRBV1-scFv1 and scFv2-TRAV1; or
   TRAV1-scFv1 and scFv2-TRBV1; or
   TRBV1-VHH1 and scFv2-TRAV1; or
   TRAV1-VHH1 and scFv2-TRBV1; or
   TRBV1-VL1 and scFv2-TRAV1-VH1; or
   TRAV1-VL1 and scFv2-TRBV1-VH1; or
   VHH2-TRBV1-scFv1 and TRAV1; or
   VHH2-TRAV1-scFv1 and TRBV1; or
   VHH2-TRBV1-VHH1 and TRAV1; or
   VHH2-TRAV1-VHH1 and TRBV1; or
   VHH2-TRBV1-VL1 and TRAV1-VH1; or
   VHH2-TRAV1-VL1 and TRBV1-VH1; or
   TRBV1-scFv1 and VHH2-TRAV1; or
   TRAV1-scFv1 and VHH2-TRBV1; or
   TRBV1-VHH1 and VHH2-TRAV1; or
   TRAV1-VHH1 and VHH2-TRBV1; or
   TRBV1-VL1 and VHH2-TRAV1-VH1; or
   TRAV1-VL1 and VHH2-TRBV1-VH1; or
   VL2-TRBV1-scFv1 and VH2-TRAV1; or
   VH2-TRBV1-scFv1 and VL2-TRAV1; or
   VL2-TRAV1-scFv1 and VH2-TRBV1; or
   VH2-TRAV1-scFv1 and VL2-TRBV1; or
   VL2-TRBV1-VHH1 and VH2-TRAV1; or
   VH2-TRBV1-VHH1 and VL2-TRAV1; or
   VL2-TRAV1-VHH1 and VH2-TRBV1; or
   VH2-TRAV1-VHH1 and VL2-TRBV1; or
   VL2-TRBV1-VL1 and VH2-TRAV1-VH1; or
   VH2-TRBV1-VL1 and VL2-TRAV1-VH1; or
   VL2-TRAV1-VL1 and VH2-TRBV1-VH1; or
   VH2-TRAV1-VL1 and VL2-TRBV1-VH1.
13. The multi-specific polypeptide molecule according to Embodiment 11, wherein the multi-specific polypeptide molecule comprises a first binding region, a second binding region, a third binding region and a fourth binding region, and the multi-specific polypeptide molecule comprises two polypeptide chains, and the two polypeptide chains respectively comprise:
   scFv2-TRBV1-VL1-TRAV2-TRBV2-VH1 and TRAV1; or
   scFv2-TRBV1-VL1-TRBV2-TRAV2-VH1 and TRAV1; or
   scFv2-TRBV1-VH1-TRBV2- TRAV2-VL1 and TRAV1; or
   scFv2-TRBV1-VH1-TRAV2- TRBV2-VL1 and TRAV1; or
   scFv2-TRAV1-VL1-TRBV2- TRAV2-VH1 and TRBV1; or
   scFv2-TRAV1-VL1-TRAV2- TRBV2-VH1 and TRBV1; or
   scFv2-TRAV1-VH1-TRAV2-TRBV2-VL1 and TRBV1; or
   scFv2-TRAV1-VH1-TRBV2-TRAV2-VL1 and TRBV1; or
   TRBV1-VL1-TRAV2-TRBV2-VH1 and scFv2-TRAV1; or
   TRBV1-VL1-TRBV2-TRAV2-VH1 and scFv2-TRAV1; or
   TRBV1-VH1-TRBV2- TRAV2-VL1 and scFv2-TRAV1; or
   TRBV1-VH1-TRAV2- TRBV2-VL1 and scFv2-TRAV1; or
   TRAV1-VL1-TRBV2- TRAV2-VH1 and scFv2-TRBV1; or
   TRAV1-VL1-TRAV2- TRBV2-VH1 and scFv2-TRBV1; or
   TRAV1-VH1-TRAV2-TRBV2-VL1 and scFv2-TRBV1; or
   TRAV1-VH1-TRBV2-TRAV2-VL1 and scFv2-TRBV1; or
   VHH2-TRBV1-VL1-TRAV2-TRBV2-VH1 and TRAV1; or
   VHH2-TRBV1-VL1-TRBV2-TRAV2-VH1 and TRAV1; or
   VHH2-TRBV1-VH1-TRBV2- TRAV2-VL1 and TRAV1; or
   VHH2-TRBV1-VH1-TRAV2- TRBV2-VL1 and TRAV1; or
   VHH2-TRAV1-VL1-TRBV2- TRAV2-VH1 and TRBV1; or
   VHH2-TRAV1-VL1-TRAV2- TRBV2-VH1 and TRBV1; or
   VHH2-TRAV1-VH1-TRAV2-TRBV2-VL1 and TRBV1; or
   VHH2-TRAV1-VH1-TRBV2-TRAV2-VL1 and TRBV1; or
   TRBV1-VL1-TRAV2-TRBV2-VH1 and VHH2-TRAV1; or
   TRBV1-VL1-TRBV2-TRAV2-VH1 and VHH2-TRAV1; or
   TRBV1-VH1-TRBV2- TRAV2-VL1 and VHH2-TRAV1; or
   TRBV1-VH1-TRAV2- TRBV2-VL1 and VHH2-TRAV1; or
   TRAV1-VL1-TRBV2- TRAV2-VH1 and VHH2-TRBV1; or
   TRAV1-VL1-TRAV2- TRBV2-VH1 and VHH2-TRBV1; or
   TRAV1-VH1-TRAV2-TRBV2-VL1 and VHH2-TRBV1; or
   TRAV1-VH1-TRBV2-TRAV2-VL1 and VHH2-TRBV1; or
   VL2-TRBV1-VL1-TRAV2-TRBV2-VH1 and VH2-TRAV1; or
   VL2-TRBV1-VL1-TRBV2-TRAV2-VH1 and VH2-TRAV1; or
   VH2-TRBV1-VL1-TRAV2-TRBV2-VH1 and VL2-TRAV1; or
   VH2-TRBV1-VL1-TRBV2-TRAV2-VH1 and VL2-TRAV1; or
   VL2-TRBV1-VH1-TRBV2- TRAV2-VL1 and VH2-TRAV1; or
   VL2-TRBV1-VH1-TRAV2- TRBV2-VL1 and VH2-TRAV1; or
   VH2-TRBV1-VH1-TRBV2- TRAV2-VL1 and VL2-TRAV1; or
   VH2-TRBV1-VH1-TRAV2- TRBV2-VL1 and VL2-TRAV1; or
   VL2-TRAV1-VL1-TRBV2- TRAV2-VH1 and VH2-TRBV1; or
   VL2-TRAV1-VL1-TRAV2- TRBV2-VH1 and VH2-TRBV1; or
   VH2-TRAV1-VL1-TRBV2- TRAV2-VH1 and VL2-TRBV1; or
   VH2-TRAV1-VL1-TRAV2- TRBV2-VH1 and VL2-TRBV1; or
   VL2-TRAV1-VH1-TRAV2-TRBV2-VL1 and VH2-TRBV1; or
   VL2-TRAV1-VH1-TRBV2-TRAV2-VL1 and VH2-TRBV1; or
   VH2-TRAV1-VH1-TRAV2-TRBV2-VL1 and VL2-TRBV1; or
   VH2-TRAV1-VH1-TRBV2-TRAV2-VL1 and VL2-TRBV1.
14. The multi-specific polypeptide molecule according to any one of Embodiments 1-13, wherein the functional domain is derived from albumin or a binding domain thereof and an optional antibody hinge region; and the functional domain is linked to the C-terminus or N-terminus, preferably the C-terminus, of any polypeptide chain of the multi-specific polypeptide molecule via an optional linker.
15. The multi-specific polypeptide molecule according to Embodiment 14, wherein the albumin is serum albumin, preferably human serum albumin; and/or
   the antibody hinge region is human-derived, e.g., a hinge domain derived from human IgG1, IgG2 or IgG4, or a portion thereof.
16. The multi-specific polypeptide molecule according to any one of Embodiments 1-13, wherein the functional domain is a hinge region derived from an antibody, and/or an Fc domain derived from an antibody or a dimerization portion thereof, and/or a CH3 domain derived from an antibody; and any two polypeptide chains of the multi-specific polypeptide molecule are connected through covalent bonds or non-covalent bonds between the two chains of the functional domain;
   preferably, the two chains of the functional domain are respectively linked to the C-terminus or N-terminus, preferably the C-terminus, of any two polypeptide chains of the multi-specific polypeptide molecule, via an optional linker; or
   the first binding region and the second binding region are connected through covalent bonds or non-covalent bonds between the two chains of the functional domain.
17. The multi-specific polypeptide molecule according to Embodiment 16, wherein the hinge region derived from an antibody is human-derived, e.g., a hinge domain derived from human IgG1, IgG2 or IgG4, or a portion thereof; and/or
   the antibody Fc domain or a dimerization portion thereof is of human-derived, e.g., an Fc domain derived from human IgG1, IgG2 or IgG4, or a portion thereof.
18. The multi-specific polypeptide molecule according to Embodiment 16 or 17, wherein the hinge region derived from an antibody comprises at least one amino acid mutation capable of reducing or eliminating binding of the multi-specific molecule to an FcR receptor and/or C1q, e.g., the mutation site is selected from the group consisting of positions 233, 234, 235 and 236; and/or
   the hinge region derived from an antibody comprises at least one amino acid mutation capable of reducing or eliminating formation of a disulfide bond between the hinge region and a non-hinge region, e.g., the mutation site is at position 220; and/or
   the CH2 domain in the Fc domain comprises at least one amino acid mutation capable of reducing or eliminating binding of the multi-specific molecule to an FcR receptor and/or C1q; e.g., the mutation site is selected from the group consisting of positions 237, 250, 252, 254, 256, 270, 297, 322, 327, 330 and 331; and/or
   the CH3 domain in the Fc domain comprises at least one amino acid mutation capable of reducing or eliminating binding of the multi-specific molecule to an FcR receptor and/or C1q; e.g., the mutation site is selected from the group consisting of positions 428 and 434;
   the CH3 domain in the Fc domain comprises at least one of the following mutations capable of promoting formation of a heterodimer of the polypeptide molecule:
      a) comprises at least two cysteine residue mutations to form an inter-chain disulfide bond, e.g. 354C and 349C, or 242C and 334C;
      b) comprises mutations capable of enabling two CH3 to form a knob-into-hole structure, e.g., each CH3 independently comprises a mutation at one or more positions selected from the group consisting of amino acid positions 366, 368, 405 and 407; preferably, one of the CH3 domains comprises a 366W mutation and the other comprises 366S, 368A and 407V mutations;
      c) comprises mutations capable of altering charge properties of amino acids at an interaction interface of the CH3 region to promote heterodimer formation, e.g., the mutation sites in the two CH3 chains are each independently selected at least one from the group consisting of Y391, K392, T393, T394, P395, P396, V397, L398, D399, S400, Q347, V348, Y349, T350, S354, R355, E356, E357, F405, Y407 and K409.
19. The multi-specific polypeptide molecule according to Embodiment 16 or 17, wherein the Antibody CH3 domain is human-derived, e.g., a CH3 domain derived from human IgG1, IgG2 or IgG4; and/or
   the CH3 domain comprises at least one of the following mutations capable of promoting formation of a heterodimer of the polypeptide molecule:
   a) comprises mutations of at least two cysteine residues to form an inter-chain disulfide bond, e.g., 354C and 349C, or 242C and 334C;
   b) comprises mutations capable of enabling two CH3 domains to form a knob-into-hole structure, e.g., each CH3 domain independently comprises a mutation at one or more positions selected from the group consisting of amino acid positions 366, 368, 405 and 407, preferably one of the CH3 domains comprises a 366W mutation and the other comprises 366S, 368A and 407V mutations;
   c) comprises mutations capable of altering charge properties of amino acids at an interaction interface of CH3 regions to promote heterodimer formation, e.g., the mutation sites of the two CH3 chains are each independently selected at least one from the group consisting of Y391, K392, T393, T394, P395, P396, V397, L398, D399, S400, Q347, V348, Y349, T350, S354, R355, E356, E357, F405, Y407 and K409.
20. The multi-specific polypeptide molecule according to any one of Embodiments 1-13, wherein the functional domain is a constant region derived from a TCR or a fragment thereof and an optional hinge region derived from an antibody; and any two polypeptide chains of the multi-specific polypeptide molecule are connected through covalent bonds or non-covalent bonds between two chains of the functional domain;
   preferably, the two chains of the functional domain are respectively connected to the C-terminus or N-terminus, preferably the C-terminus, of any two polypeptide chains of the multi-specific polypeptide molecule via an optional linker; or
   the first binding region and the second binding region are connected by covalent bonds or non-covalent bonds between the two chains of the functional domain.
21. The multi-specific polypeptide molecule according to Embodiment 20, wherein the hinge region derived from an antibody is human-derived, e.g., a hinge region derived from human IgG1, IgG2 or IgG4, or a portion thereof; and/or
   the constant region of the TCR or a fragment thereof is human-derived or murine-derived, and is selected from the group consisting of a TCR α chain constant region (TRAC) and a TCR β chain constant region (TRBC); preferably, the TRAC and/or TRBC comprises at least one cysteine mutation relative to a wild-type sequence, to form a disulfide bond between TRAC and TRBC, more preferably, the cysteine mutation is at position 48 of the wild-type TCR α chain constant region and position 57 of the wild-type TCR β chain constant region.
22. The multi-specific polypeptide molecule according to any one of Embodiments 1-21, wherein the multi-specific polypeptide molecule further comprises a constant domain connected to the C-terminus of TRAV1 and/or TRBV1 via an optional linker, the constant domain is selected from the group consisting of the following (1)-(3)
   (1) a TCR constant region or a fragment thereof, wherein the TCR constant region, e.g., comprises a TCR α chain constant region (TRAC) and/or a β chain constant region (TRBC);
      preferably, the TRAC and/or TRBC comprises at least one cysteine mutation relative to a wild-type sequence, to form a disulfide bond between TRAC and TRBC, more preferably, the cysteine mutation is at position 48 of the wild-type TCR α chain constant region and position 57 of the wild-type TCR β chain constant region;
   (2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
      preferably, each CH3 independently comprises a mutation at one or more positions selected from the group consisting of amino acid positions 366, 368, 405 and 407 and the two CH3 do not comprise the same mutation, more preferably, one of the CH3 comprises a T366W mutation and the other comprises T366S, L368A and Y407V mutations;
   (3) CL and CH1, wherein the CL is derived from an antibody light chain CL domain, and the CH1 is derived from an antibody heavy chain CH1 domain.
23. The multi-specific polypeptide molecule according to Embodiment 22, wherein the multi-specific polypeptide molecule comprises TRAC and TRBC that are respectively connected to the C-terminus of TRAV1 and TRBV1 via an optional linker.
24. The multi-specific polypeptide molecule according to any one of Embodiments 1-23, wherein the multi-specific polypeptide molecule further comprises CH1 and CL derived from an antibody that are respectively connected to the VH1 and VL1, wherein the VH1, VL1, CH1 and CL form a single-chain Fab and are connected to the C-terminus of the TRAV1 or the TRBV1 via a linker.
25. The multi-specific polypeptide molecule according to any one of Embodiments 1-4 and 14-24, wherein the polypeptide chains of the polypeptide molecule comprise, from the N-terminus to the C-terminus, respectively:
   a first polypeptide chain: TRBV1- optional linker -TRBC- linker -VL1- linker -VH1- optional linker - hinge region -CH2-CH3, and a second polypeptide chain: TRAV1- optional linker -TRAC- optional linker - hinge region -CH2-CH3; or
   a first polypeptide chain: TRAV1- optional linker -TRAC- linker -VL1- linker -VH1- optional linker - hinge region -CH2-CH3, and a second polypeptide chain: TRBV1- optional linker -TRBC- optional linker - hinge region -CH2-CH3; or
   a first polypeptide chain: TRBV1- optional linker -TRBC- linker -VH1- linker -VL1- optional linker - hinge region -CH2-CH3, and a second polypeptide chain: TRAV1- optional linker -TRAC- optional linker - hinge region -CH2-CH3; or
   a first polypeptide chain: TRAV1- optional linker -TRBC- linker -VH1- linker -VL1- optional linker - hinge region -CH2-CH3, and a second polypeptide chain: TRBV1- optional linker -TRBC- optional linker - hinge region -CH2-CH3; or
   a first polypeptide chain: TRBV1- optional linker -TRBC- linker -VL1- linker -VH1- linker -CH2-CH3, and a second polypeptide chain: TRAV1- optional linker -TRAC- linker -CH2-CH3; or
   a first polypeptide chain: TRBV1- optional linker -TRBC- linker -VL1- linker -VH1- optional linker - hinge region -CH3, and a second polypeptide chain: TRAV1- optional linker -TRAC- optional linker -hinge region -CH3; or
   a first polypeptide chain: TRBV1- optional linker -TRBC- linker -VL1- linker -VH1- optional linker - hinge region -TRBC, and a second polypeptide chain: TRAV1- optional linker -TRAC- optional linker -hinge region -TRAC; or
   a first polypeptide chain: TRBV1- optional linker -TRBC- linker -VL1- linker -VH1- optional linker - hinge region, and a second polypeptide chain: TRAV1- optional linker -TRAC- optional linker -hinge region - Alb; or
   a first polypeptide chain: TRBV1- optional linker -TRBC- linker -VL1- linker -VH1- linker -Alb, and a second polypeptide chain: TRAV1- optional linker -TRAC; or
   a first polypeptide chain: TRAV1- optional linker -CH3- linker -VL1- linker -VH1- optional linker - optional hinge region -CH2-CH3, and a second polypeptide chain: TRBV1- optional linker -CH3- optional linker - optional hinge region -CH2-CH3; or
   a first polypeptide chain: TRAV1- optional linker -CH1- linker -VL1- linker -VH1- optional linker - optional hinge region -CH2-CH3, and a second polypeptide chain: TRBV1- optional linker -CL- optional linker - optional hinge region -CH2-CH3; or
   a first polypeptide chain: TRBV1- optional linker -TRBC- linker -VHH1- optional linker -hinge region - CH2-CH3, and a second polypeptide chain: TRAV1- optional linker -TRAC- optional linker -hinge region - CH2-CH3;
   the second binding region comprises an antibody-derived Fab, and wherein a first polypeptide chain: TRBV1- optional linker -TRBC- linker -VL1-CL- linker -VH1-CH1- optional linker -hinge region -CH2-CH3, and a second polypeptide chain: TRAV1- optional linker -TRAC- optional linker -hinge region -CH2-CH3;
   a first polypeptide chain: TRBV1- optional linker -TRBC- linker -VL1, a second polypeptide chain: VH1-optional linker -hinge region -CH2-CH3, and a third polypeptide chain: TRAV1- optional linker -TRAC-optional linker -hinge region -CH2-CH3;
   a first polypeptide chain: TRBV1- optional linker -TRBC- linker -VH1, a second polypeptide chain: VL1-optional linker -hinge region -CH2-CH3, and a third polypeptide chain: TRAV1- optional linker -TRAC-optional linker -hinge region -CH2-CH3;
   a first polypeptide chain: TRAV1- optional linker -TRAC- linker -VL1, a second polypeptide chain: VH1-optional linker -hinge region -CH2-CH3, and a third polypeptide chain: TRBV1- optional linker -TRBC-optional linker -hinge region -CH2-CH3.
   a first polypeptide chain: TRAV1- optional linker -TRAC- linker -VH1, a second polypeptide chain: VL1-optional linker -hinge region -CH2-CH3, and a third polypeptide chain: TRBV1- optional linker -TRBC-optional linker -hinge region -CH2-CH3;
   a first polypeptide chain: TRBV1- optional linker -TRBC- linker -VL1- optional linker -hinge region - CH2-CH3, a second polypeptide chain: VH1- optional linker -hinge region -CH2-CH3, and a third polypeptide chain: TRAV1- optional linker -TRAC;
   a first polypeptide chain: TRBV1- optional linker -TRBC- linker -VH1- optional linker -hinge region - CH2-CH3, a second polypeptide chain: VL1- optional linker -hinge region -CH2-CH3, and a third polypeptide chain:TRAV1- optional linker -TRAC;
   a first polypeptide chain: TRAV1- optional linker -TRAC- linker -VL1- optional linker -hinge region - CH2-CH3, a second polypeptide chain: VH1- optional linker -hinge region -CH2-CH3, and a third polypeptide chain: TRBV1- optional linker -TRBC;
   a first polypeptide chain: TRAV1- optional linker -TRAC- linker -VH1- optional linker -hinge region - CH2-CH3, a second polypeptide chain: VL1- optional linker -hinge region -CH2-CH3, and a third polypeptide chain: TRBV1- optional linker -TRBC.
26. The multi-specific polypeptide molecule according to any one of Embodiments 5-8 and 14-24, wherein the polypeptide molecule comprises a first polypeptide chain and a second polypeptide chain; wherein:
   the first polypeptide chain comprises, from the N-terminus to the C-terminus: TRBV1- optional linker - TRBC- linker -VL1- linker -TRAV2- linker -TRBV2- linker -VH1- optional linker -hinge region -CH2-CH3, and the second polypeptide chain comprises, from N-terminus to C-terminus: TRAV1- optional linker -TRAC-optional linker -hinge region -CH2-CH3.
27. The multi-specific polypeptide molecule according to any one of Embodiments 9-12 and 14-24, wherein the polypeptide molecule comprises a first polypeptide chain and a second polypeptide chain; wherein:
   the first polypeptide chain comprises, from the N-terminus to the C-terminus: VL2- linker -VH2- linker - TRBV1- optional linker -TRBC- linker -VL1- linker -VH1- optional linker -hinge region -CH2-CH3, and the second polypeptide chain comprises, from the N-terminus to the C-terminus: TRAV1- optional linker -TRAC-optional linker -hinge region -CH2-CH3; or
   the first polypeptide chain comprises, from the N-terminus to the C-terminus: VL2- linker -TRBV1-optional linker -TRBC- linker -VL1- linker -VH1- optional linker -hinge region -CH2-CH3, and the second polypeptide chain comprises, from the N-terminus to the C-terminus: VH2- linker -TRAV1- optional linker - TRAC- optional linker -hinge region -CH2-CH3; or
   the first polypeptide chain comprises, from the N-terminus to the C-terminus: VL2- linker -TRBV1-optional linker -TRBC- linker -VL1- linker -VH1- optional linker -hinge region -CH2-CH3, and the second polypeptide chain comprises, from the N-terminus to the C-terminus: VH2- linker -TRAV1- optional linker - TRAC- optional linker -hinge region -CH2-CH3.
28. The multi-specific polypeptide molecule according to any one of Embodiments 13-24, wherein the polypeptide molecule comprises a first polypeptide chain and a second polypeptide chain; wherein:
   the first polypeptide chain comprises, from the N-terminus to the C-terminus: VL2- linker -VH2- linker - TRBV1- optional linker -TRBC- linker -VL1- linker -TRAV2- linker -TRBV2- linker -VH1- optional linker - hinge region -CH2-CH3, and the second polypeptide chain comprises, from the N-terminus to the C-terminus: TRAV1- optional linker -TRAC- optional linker -hinge region -CH2-CH3.
29. The multi-specific polypeptide molecule according to any one of Embodiments 1-28, wherein
   the VH1 and VL1 comprise at least one cysteine mutation to form a disulfide bond between VH1 and VL1, preferably, the cysteine mutations are at position 44 of VH1 and position 100 of VL1; and/or
   the VH2 and VL2 comprise at least one cysteine mutation to form a disulfide bond between VH2 and VL2, preferably, the cysteine mutations are at position 44 of VH2 and position 100 of VL2; and/or
   the TRAV1 and VL1 or VL2 comprise at least one cysteine mutation to form a disulfide bond between TRAV1 and VL1 or VL2, preferably, the cysteine mutations are at position 104 of TRAV1, and position 80 of VL1 or VL2; and/or
   the TRAV2 and VL1 or VL2 comprise at least one cysteine mutation to form a disulfide bond between TRAV2 and VL1 or VL2, preferably, the cysteine mutations are at position 104 of TRAV2, and position 80 of VL1 or VL2; and/or
   the TRBV1 and VL1 or VL2 comprise at least one cysteine mutation to form a disulfide bond between TRBV1 and VL1 or VL2, preferably, the cysteine mutations are at position 84 of TRBV1, and position 80 of VL1 or VL2; and/or
   the TRBV2 and VL1 or VL2 comprise at least one cysteine mutation to form a disulfide bond between TRBV2 and VL1 or VL2, preferably, the cysteine mutations are at position 84 of TRBV2, and position 80 of VL1 or VL2; and/or
   the TRAV1 and VH1 or VH2 comprise at least one cysteine mutation to form a disulfide bond between TRAV1 and VH1 or VH2, preferably, the cysteine mutations are at position 104 of TRAV1, and position 89 of VH1 or VH2; and/or
   the TRAV2 and VH1 or VH2 comprise at least one cysteine mutation to form a disulfide bond between TRAV2 and VH1 or VH2, preferably, the cysteine mutations are at position 104 of TRAV2, and position 89 of VH1 or VH2.
30. The multi-specific polypeptide molecule according to any one of Embodiments 1-29, wherein the polypeptide molecule further comprises one or more amino acid mutations, insertions, or deletions capable of enhancing the affinity or biological activity thereof, enhancing the stability thereof, prolonging the half-life thereof, reducing the immunogenicity thereof, or reducing the post-translational modifications thereof; e.g., the amino acid mutations include mutations capable of enhancing binding to FcRn and/or effector function silencing mutations.
31. The multi-specific polypeptide molecule according to any one of Embodiments 1-30, wherein the linkers in the polypeptide molecule are each independently selected from linkers consisting of 1-35 amino acids;
   preferably, the linkers are each independently selected from the group consisting of S, GS, GGGS, GGGGS, GGGSGGGG, GGSGGS, GGSGGSGGS, GGGGSGGGGS, GGGGSGGGGSGGGGS, GGGGSGGGGSGGGGSGGGGSGGGS, GGGGSGGGGSGGGGSGGGGSGGGGSGGGGS, GQPKAAP, TVLRT, TVSSAS, GGEGG, GSEGGGS, RTSGPGDGGKGGPGKGPGGEGTKGTGPGG, GKGPGGEGTKGTGPGG, TVLSSAS, EDLKN or a variant thereof, EDLNK or a variant thereof, and ANIQK or a variant thereof, or any combination thereof;
   preferably, a variant of EDLKN is an amino acid sequence formed by substitution, deletion, or addition of one or several amino acids in EDLKN; and/or a variant of EDLNK is an amino acid sequence formed by substitution, deletion, or addition of one or several amino acids in EDLNK; and/or a variant of ANIQK is an amino acid sequence formed by substitution, deletion, or addition of one or several amino acids in ANIQK.
32. The multi-specific polypeptide molecule according to any one of Embodiments 1-31, wherein the linkers in the polypeptide molecule are each independently selected from linkers consisting of no more than 15 amino acids;
   preferably, the linkers are each independently selected from the group consisting of S, GS, GGGS, GGGGS, GGGSGGGG, GGSGGS, GGSGGSGGS, GGGGSGGGGS, GGGGSGGGGSGGGGS, EDLKN or a variant thereof, EDLNK or a variant thereof, and ANIQK or a variant thereof;
   preferably, the variant of EDLKN is an amino acid sequence formed by substitution, deletion, or addition of one or several amino acids in EDLKN; and/or the variant of EDLNK is an amino acid sequence formed by substitution, deletion, or addition of one or several amino acids in EDLNK; and/or the variant of ANIQK is an amino acid sequence formed by substitution, deletion, or addition of one or several amino acids in ANIQK.
33. The multi-specific polypeptide molecule according to any one of Embodiments 1-32, wherein the polypeptide molecule further comprises a signal peptide sequence at the N-terminus of one or more polypeptide chains, preferably comprises a signal peptide sequence at the N-terminus of each polypeptide chain, e.g. a signal peptide sequence derived from albumin or an immunoglobulin, preferably MGWSLILLFLVAVATRVHS.
34. The multi-specific polypeptide molecule according to any one of Embodiments 1-33, wherein the first antigen and the third antigen are each independently a TAA selected from the group consisting of the following antigens: melanoma-associated antigens (e.g., gp100, MAGEA1, MAGEA3, MAGEA6, MAGEA4, MAGEA2, MAGEA12, MAGEA2B, MAGEA9B, MAGEA10, MAGEA11, MAGEB2, MAGEC1, MAGEC2), IGF2BP1, GNGT1, PI4K2B, CCR8, NPSR1, COX7B2, ONECUT3, SMC1B, FOXI3, GAGE2A, FBXO43, BRDT, PAGE2, GAGE13, POU5F1B, CTAG1A, and endogenous reverse transcriptase antigen; or
   wherein the first antigen and the third antigen are each independently a TSA selected from the group consisting of the following antigens: KRAS antigen (e.g., a KRAS G12 mutant antigen), TP53, PIK3CA, CTNNB1, EGFR, BRAF, and GNAS; or
   wherein the first antigen and the third antigen are each independently a viral antigen selected from the group consisting of the following antigens: HPV antigen (e.g., an HPV E6 or E7 antigen), CMV antigen, HBV antigen, EBV antigen, herpesvirus antigen, human immunodeficiency virus (HIV) antigen, influenza virus antigen, and coronavirus antigen; or
   the first antigen and the third antigen are each independently an self-antigen selected from the group consisting of the following antigens: AFP, CEA, CD19, CD20, BCMA, CD22, CD30, SLAM, CLDN18.2, GD2, mesothelin, CD38, Her2, GPC3, MUC1, Ro52, Ro60, La, Jo-1, SRP, IFIH1, CENPA, CENPB, SNRPA1, SNRNP70, SNR-PD3, RNAP3, TOPO1, Insulin, GAD65, IA2, Znt8, PL7, TARS, ARS, MI2, topoisomerase 1, EXOSC9, EXOSC107, POLR3A, POLR3K, PTRN, GAD2, SLC30A8, AchR, MUSK, LRP4, PLA2R, THSD7A, TSHR, IFN-γ, CHRNA1, MUSK, LRP4, AQP4, MOG, GRIN1, COL4A3, PLA2R, GM-SCF, PR3 and MPO.
35. The multi-specific polypeptide molecule according to any one of Embodiments 1-34, wherein the second antigen and the fourth antigen are each independently selected from the group consisting of CD3 (e.g., CD3γ, CD3δ and CD3ε chains), CD4, CD8, CD10, CD11b, CD11c, CD14, CD16, CD18, CD25, CD32a, CD32b, CD41, CD41b, CD42a, CD42b, CD44, CD45RA, CD49, CD61, CD64, CD68, CD94, CD90, CD117, Nkp46, NKG2D, Fc ε RI, TCR α / β , TCR γ / δ , HLA-DR, CD28, 4-1BB(CD137), OX40(CD134), ICOS(CD278), 2B4 (CD244), HVEM, LAG3, DAP10, DAP12, CD27, CD40, GITR, LFA-1, MyD88, CD2, CD7, LIGHT, B7-H3, CTLA-4, PD-1, PD-L1, CD80, BTLA, TIM3, TIGIT and LAG-3.
36. The multi-specific polypeptide molecule according to any one of Embodiments 1-35, wherein the first antigen and the third antigen are each independently selected from the group consisting of melanoma-associated antigens (e.g., gp100, MAGEA1), KRAS antigens (e.g., a KRAS G12 mutant antigen), and HPV antigens (e.g., an HPV E7 antigens), and/or the second antigen and the fourth antigen are each independently selected from the group consisting of CD3, CD28, 4-1BB(CD137), PD-1, and PD-L1.
37. A nucleic acid, comprising a nucleotide sequence encoding each chain of the multi-specific polypeptide molecule according to any one of Embodiments 1-36.
38. A vector, comprising the nucleic acid according to Embodiment 37.
39. A vector system, comprising, on one or more vectors, nucleotide sequences encoding each chain of the multi-specific polypeptide molecule according to any one of Embodiments 1-36.
40. A host cell, comprising the nucleic acid according to Embodiment 37, the vector according to Embodiment 38, or the vector system according to Embodiment 39.
41. A conjugate, comprising the multi-specific polypeptide molecule according to any one of Embodiments 1-36, and a chemical moiety conjugated to the multi-specific polypeptide molecule.
42. The conjugate according to Embodiment 41, wherein the chemical moiety is selected from the group consisting of a therapeutic agent, an immunostimulatory molecule and a detectable label;
   preferably, the therapeutic agent is selected from the group consisting of an immunomodulator, an agonist, a radioactive compound, an enzyme, a chemotherapeutic agent, and a toxin.
   preferably, the immunostimulatory molecule is selected from the group consisting of a cytokine, a chemokine, a platelet factor, and a complement initiator;
   preferably, the detectable label is selected from the group consisting of biotin, streptavidin, an enzyme or a catalytically active fragment thereof, a radionuclide, a nanoparticle, a paramagnetic metal ion, a nucleic acid probe, a contrast agent, and a fluorescent, phosphorescent, or chemiluminescent molecule.
43. A pharmaceutical composition, comprising the multi-specific polypeptide molecule according to any one of Embodiments 1-36, the nucleic acid according to Embodiment 37, the vector according to Embodiment 38, the vector system according to Embodiment 39, the host cell according to Embodiment 40, the conjugate according to Embodiment 41 or 42, and a pharmaceutically acceptable excipient.
44. The pharmaceutical composition according to Embodiment 43, wherein the pharmaceutical composition further comprises a second therapeutic agent, preferably, the second therapeutic agent is selected from the group consisting of an antibody, a chemotherapeutic agent, and a small-molecule drug.
45. A method for preventing or treating a disease in a subject, comprising administering to the subject an effective amount of the multi-specific polypeptide molecule according to any one of Embodiments 1-36, the nucleic acid according to Embodiment 37, the vector according to Embodiment 38, the vector system according to Embodiment 39, the host cell according to Embodiment 40, the conjugate according to Embodiment 41 or 42, or the pharmaceutical composition according to Embodiment 43 or 44, wherein the disease is selected from the group consisting of cancer, an infectious disease, an autoimmune disease, and an inflammatory disease.
46. The method according to Embodiment 45, further comprising administering a second therapeutic agent to the subject, preferably, the second therapeutic agent is selected from the group consisting of an antibody, a chemotherapeutic agent, and a small-molecule drug.

### DESCRIPTION OF THE DRAWINGS

Fig. 1 shows a structural schematic diagram of a specific form of format 62 of the present disclosure.
Fig. 2 shows a structural schematic diagram of a specific form of format 58-2 of the present disclosure.
Fig. 3 shows a structural schematic diagram of a specific form of format 72 of the present disclosure.
Fig. 4 shows a structural schematic diagram of a specific form of format 50-2 of the present disclosure.
Fig. 5 shows a structural schematic diagram of a specific form of format 62-1 of the present disclosure.
Fig. 6 shows a structural schematic diagram of a specific form of format 62-2 of the present disclosure.
Fig. 7 shows a structural schematic diagram of a specific form of format 62-3 of the present disclosure.
Fig. 8 shows a structural schematic diagram of a specific form of format 62-4 of the present disclosure.
Fig. 9 shows a structural schematic diagram of a specific form of format 62-5 of the present disclosure.
Fig. 10 shows a structural schematic diagram of a specific form of format 62-6 of the present disclosure.
Fig. 11 shows a structural schematic diagram of a specific form of format 62-7 of the present disclosure.
Fig. 12 shows a structural schematic diagram of a specific form of format 62-8 of the present disclosure.
Fig. 13 shows a structural schematic diagram of a specific form of format 62-9 of the present disclosure.
Fig. 14 shows a structural schematic diagram of a specific form of format 62-11 of the present disclosure.
Fig. 15 shows a structural schematic diagram of a specific form of format 62-12 of the present disclosure.
Fig. 16 shows a structural schematic diagram of a specific form of format 62-13 of the present disclosure.
Fig. 17 shows a structural schematic diagram of a specific form of format 72-1 of the present disclosure.
Fig. 18 shows a structural schematic diagram of a specific form of format 72-2 of the present disclosure.
Fig. 19 shows a structural schematic diagram of a specific form of format 72-3 of the present disclosure.
Fig 20 shows a structural schematic diagram of a specific form of format 59 of the present disclosure.
Fig. 21 shows a structural schematic diagram of a specific form of format 22-3 of the present disclosure.
Fig. 22 shows a structural schematic diagram of a specific form of format 22-4 of the present disclosure.
Fig. 23 shows a structural schematic diagram of a specific form of control format 14.
Fig. 24 shows a structural schematic diagram of a specific form of control format 14-1.
Fig. 25 shows a structural schematic diagram of a specific form of control format 14-2.
Fig. 26A shows the ELISPOT assay results of formats 62, 62-LJH1, 62-LJH2, 62-LJH3, 62-LM1, 62-LM2, and 62-LM3 of the present disclosure when gp100 is used as the target peptide.
Fig. 26B shows the ELISPOT assay results of formats 62, 62-1, 62-2, 62-3, 62-5, 62-6, 62-7, and 62-8 of the present disclosure when gp100 is used as the target peptide.
Fig. 26C shows the ELISPOT assay results of format 72 of the present disclosure when gp100 is used as the target peptide.
Fig. 26D shows the ELISPOT assay results of format 59 of the present disclosure when gp100 is used as the target peptide.
Fig. 26E shows the ELISPOT assay results of format 22-4 of the present disclosure when gp100 is used as the target peptide.
Fig. 26F shows the ELISPOT assay results of control format 14 (KIMMTRAK), 14-1, and 14-2 when gp100 is used as the target peptide.
Fig. 27 shows the monomer and aggregate contents and purity of format 62 of the present disclosure after being stored at 4°C, 25°C and 40°C for 15 days, as measured by SEC.

### Detailed Description of Embodiments

The present disclosure is further illustrated by the following specific examples. It should be understood that these examples are only used to illustrate the present disclosure and are not intended to limit the scope of the present disclosure. In the following examples, experimental methods for which no specific conditions are indicated are performed under conventional conditions in the art, e.g., the conditions described in Sambrook and Russeii et al., Molecular Cloning: A Laboratory Manual (Third Edition) (2001), CSHL Press, or the conditions recommended by the manufacturers. Unless otherwise stated, the experimental materials and reagents used in the following examples are commercially available.

The amino acid sequences of the elements used in the formats involved in the following examples are as follows:
***TRAV***
   >TRAV of the gp100-specific TCR 
SQQGEEDPQALSIQEGENATMNCSYKTSINNLQWYRQNSGRGLVHLILIRSNEREKHSGRLRVTL
DTSKKSSSLLITASRAADTASYFCATDGSTPMQFGKGTRLSVI (SEQ ID NO:91)
   >TRAV of the gp100-specific TCR, comprising an A104C mutation 
SQQGEEDPQALSIQEGENATMNCSYKTSINNLQWYRQNSGRGLVHLILIRSNEREKHSGRLRVTL
DTSKKSSSLLITASRACDTASYFCATDGSTPMQFGKGTRLSVI (SEQ ID NO:93)
   >TRAV of the MAGE-A1-specific TCR 
RGEDVEQSLFLSVREGDSSVINCTYTDSSSTYLYWYKQEPGAGLQLLTYIFSNMDMKQDQRLTV
LLNKKDKHLSLRIADTQTGDSAIYFCAGSGGGTDKLIFGTGTRLQVFPN (SEQ ID NO: 41)
   > TRAV of the MAGE-A1-specific TCR 
RGEDVEQSLFLSVREGDSSVINCTYTDSSSTYLYWYKQEPGAGLQLLTYTWPHMDMKQDQRLT
VLLNKKDKHLSLRIADTQTGDSAIYFCAGSGGGTDKLIFGTGTRLQVFPN (SEQ ID NO: 107)
   > TRAV of the HPV E7-specific TCR 
RKEVEQDPGPFNVPEGATVAFNCTYSNSASQSFFWYRQDCRKEPKLLMSVYSSGNEDGRFTAQ
LNRASQYISLLIRDSKLSDSATYLCAVISAGTALIFGKGTTLSVSS (SEQ ID NO:49)
   >TRAV of the KRAS G12V-specific TCR (KVA11-N01) 
AQSVTQPDIHITVSEGASLELRCNYSYGATPYLFWYVQSPGQGLQLLLKYFSGDTLVQGIKGFEA
EFKRSQSSFNLRKPSVHWSDAAEYFCAVGVPGGYNKLIFGAGTRLAVHP (SEQ ID NO:57)
   >TRAV of the KRAS G12V-specific TCR (KVA11-N02) 
AQSVAQPEDQVNVAEGNPLTVKCTYSVSGNPYLFWYVQYPNRGLQFLLKYITGDNLVKGSYGF
EAEFNKSQTSFHLKKPSALVSDSALYFCAVRDIEGAGNNRKLIWGLGTSLAVNP (SEQ ID NO:65)
   >TRAV of the KRAS G12V-specific TCR (KVA11-N04) 
GESVGLHLPTLGVQEGDNSIINCAYSNSASDYFIWYKQESGKGPQFIIDIRSNMDKRQGQRVTVL
LNKTVKHLSLQIAATQPGDSAVYFCAENSDGTSYGKLTFGQGTILTVHP (SEQ ID NO:73)
   >TRAV of the KRAS G12D-specific TCR (KDA11-N01) 
QKEVEQNSGPLSVPEGAIASLNCTYSDRGSQSFFWYRQYSGKSPELIMSIYSNGDKEDGRFTAQL
NKASQYVSLLIRDSQPSDSATYLCAVNLGAAGNKLTFGGGTRVLVKP (SEQ ID NO:81)
   >TRAV of the KRAS G12D-specific TCR (KDA11-N02) 
RKEVEQDPGPFNVPEGATVAFNCTYSNSASQSFFWYRQDCRKEPKLLMSVYSSGNEDGRFTAQ
LNRASQYISLLIRDSKLSDSATYLCVVTNARLMFGDGTQLVVKP (SEQ ID NO:89)
***TRAC***
   >TRAC comprising T48C, N113K, and an FFPSPESS deletion mutation 
NIQKPDPAVYQLRDSKSSDKSVCLFTDFDSQTNVSQSKDSDVYITDKCVLDMRSMDFKSNSAV
AWSNKSDFACANAFNNSIIPEDT (SEQ ID NO: 8)
   >TRAC comprising T48C, N113K, and a PESS deletion mutation 
NIQKPDPAVYQLRDSKSSDKSVCLFTDFDSQTNVSQSKDSDVYITDKCVLDMRSMDFKSNSAV
AWSNKSDFACANAFNNSIIPEDTFFPS (SEQ ID NO: 9)
   >TRAC comprising T48C and N113K mutations 
NIQKPDPAVYQLRDSKSSDKSVCLFTDFDSQTNVSQSKDSDVYITDKCVLDMRSMDFKSNSAV
AWSNKSDFACANAFNNSIIPEDTFFPSPESS (SEQ ID NO: 10)
***TRBV***
   >gp100-specific TCR TRBV 
DGGITQSPKYLFRKEGQNVTLSCEQNLNHDAMYWYRQDPGQGLRLIYYSWAQGDFQKGDIAE
GYSVSREKKESFPLTVTSAQKNPTAFYLCASSWGAPYEQYFGPGTRLTVT (SEQ ID NO:92)
   >gp100-specific TCR TRBV, comprising an N84C mutation 
DGGITQSPKYLFRKEGQNVTLSCEQNLNHDAMYWYRQDPGQGLRLIYYSWAQGDFQKGDIAE
GYSVSREKKESFPLTVTSAQKCPTAFYLCASSWGAPYEQYFGPGTRLTVT (SEQ ID NO:94)
   >MAGE-A1-specific TCR TRBV 
GAVVSQHPSWVICKSGTSVKIECRSLDFQATTMFWYRQFPKQSLMLMATSDYQTCVTYEQGVE
KDKFLINHASLTLSTLTVTSAHPEDSSFYICSAREPGQGPFEQYFGPGTRLTVTE (SEQ ID NO: 42)
   >MAGE-A1-specific TCR TRBV 
GAVVSQHPSWVICKSGTSVKIECRSLDFQATTMFWYRQFPKQSLMLMATSNEGSKATYEQGVE
KDKFLINHASLTLSTLTVTSAHPEDSSFYICSAREPGQGPYEQYFGPGTRLTVTE (SEQ ID NO: 108)
   > HPV E7-specific TCR TRBV 
DAGVTQSPTHLIKTRGQQVTLRCSPKSGHDTVSWYQQALGQGPQFIFQYYEEEERQRGNFPDRF
SGHQFPNYSSELNVNALLLGDSALYLCASSLGWRGGLYTEAFFGQGTRLTVV (SEQ ID NO:50)
   >KRAS G12V-specific TCR (KVA11-N01) TRBV 
GAGVSQSPRYKVAKRGQDVALRCDPISGHVSLFWYQQALGQGPEFLTYFQNEAQLDKSGLPSD
RFFAERPEGSVSTLKIQRTQQEDSAVYLCASSLDRGNTIYFGEGSWLTVV (SEQ ID NO:58)
   >KRAS G12V-specific TCR (KVA11-N02) TRBV 
DAGVIQSPRHEVTEMGQEVTLRCKPISGHNSLFWYRQTMMRGLELLIYFNNNVPIDDSGMPEDR
FSAKMPNASFSTLKIQPSEPRDSAVYFCASSEGFYTEAFFGQGTRLTVV (SEQ ID NO:66)
   >KRAS G12V-specific TCR (KVA11-N04) TRBV 
EAGVAQSPRYKIIEKRQSVAFWCNPISGHATLYWYQQILGQGPKLLIQFQNNGVVDDSQLPKDR
FSAERLKGVDSTLKIQPAKLEDSAVYLCASSLVGSPDYEQYFGPGTRLTVT (SEQ ID NO:74)
   >KRAS G12D-specific TCR (KDA11-N01) TRBV 
EAGVAQSPRYKIIEKRQSVAFWCNPISGHATLYWYQQILGQGPKLLIQFQNNGVVDDSQLPKDR
FSAERLKGVDSTLKIQPAKLEDSAVYLCASSFSDSYNSPLHFGNGTRLTVT (SEQ ID NO:82)
   >KRAS G12D-specific TCR (KDA11-N02) TRBV 
EAQVTQNPRYLITVTGKKLTVTCSQNMNHEYMSWYRQDPGLGLRQIYYSMNVEVTDKGDVPE
GYKVSRKEKRNFPLILESPSPNQTSLYFCASTSIVSSGRGEQFFGPGTRLTVL (SEQ ID NO:90)
***TRBC***
   >TRBC2, comprising S57C, C187A, and N210D mutations 
EDLKNVFPPEVAVFEPSEAEISHTQKATLVCLATGFYPDHVELSWWVNGKEVHSGVCTDPQPLK
EQPALNDSRYALSSRLRVSATFWQDPRNHFRCQVQFYGLSENDEWTQDRAKPVTQIVSAEAWGRAD
(SEQ ID NO: 11)
   >TRBC2, comprising S57C, C187A, N210D, and an FG loop deletion mutation 
EDLKNVFPPEVAVFEPSEAEISHTQKATLVCLATGFYPDHVELSWWVNGKEVHSGVCTDPQPLK
EQPALNDSRYALSSRLRVSATFWQDPRNHFRCQVQFQIVSAEAWGRAD (SEQ ID NO: 12)
   >TRBC1, comprising S57C, C187A, and N210D mutations 
EDLNKVFPPEVAVFEPSEAEISHTQKATLVCLATGFFPDHVELSWWVNGKEVHSGVCTDPQPLK
EQPALNDSRYALSSRLRVSATFWQDPRNHFRCQVQFYGLSENDEWTQDRAKPVTQIVSAEAWGRAD
(SEQ ID NO: 13)
   >TRBC1, comprising S57C, C187A, N210D, and an FG loop deletion mutation 
EDLNKVFPPEVAVFEPSEAEISHTQKATLVCLATGFFPDHVELSWWVNGKEVHSGVCTDPQPLK
EQPALNDSRYALSSRLRVSATFWQDPRNHFRCQVQFQIVSAEAWGRAD (SEQ ID NO: 14)
***VH***
   >VH1(anti-CD3 antibody huUCHT1, comprising a G44C mutation) 
EVQLVESGGGLVQPGGSLRLSCAASGYSFTGYTMNWVRQAPGKCLEWVALINPYKGVSTYNQK
FKDRFTISVDKSKNTAYLQMNSLRAEDTAVYYCARSGYYGDSDWYFDVWGQGTLVTVSS (SEQ ID
NO: 95)
   >VH1(anti-CD3 antibody huUCHT1, comprising G44C and E89C mutations) 
EVQLVESGGGLVQPGGSLRLSCAASGYSFTGYTMNWVRQAPGKCLEWVALINPYKGVSTYNQK
FKDRFTISVDKSKNTAYLQMNSLRACDTAVYYCARSGYYGDSDWYFDVWGQGTLVTVSS (SEQ ID
NO: 96)
   >VH2(anti-CD28 antibody, comprising a C50Y mutation) 
QVQLVQSGAEVKKPGASVKVSCKASGYTFTSYYIHWVRQAPGQGLEWIGYIYPGNVNTNYNEK
FKDRATLTVDTSISTAYMELSRLRSDDTAVYFCTRSHYGLDWNFDVWGQGTTVTVSS (SEQ ID NO:
97)
   >VH2(anti-CD28 antibody, comprising C50Y and D89C mutations) 
QVQLVQSGAEVKKPGASVKVSCKASGYTFTSYYIHWVRQAPGQGLEWIGYIYPGNVNTNYNEK
FKDRATLTVDTSISTAYMELSRLRSCDTAVYFCTRSHYGLDWNFDVWGQGTTVTVSS (SEQ ID NO:
98)
   >VH-BB1(anti-4-1BB antibody, monovalent) 
EVQLLESGGGLVQPGGSLRLSCAASGFTFSSYDMSWVRQAPGKCLEWVSWISYSGGSIYYADS
VKGRFTISRDNSKNTLYLQMNSLRAEDTAVYYCARDAQRNSMREFDYWGQGTLVTVSS (SEQ ID
NO: 99)
   >VH-BB2(anti-4-1BB antibody, bivalent) 
EVQLVESGGGLVQPGRSLRLSCTASGFSLNDYWMSWVRQAPGKGLEWVGYIDVGGSLYYAAS
VKGRFTISRDDSKSIAYLQMNSLKTEDTAVYYCARGGLTYGFDLWGQGTLVTVSS (SEQ ID NO: 100)
***VL***
   >VL1(anti-CD3 antibody huUCHT1, comprising an E100C mutation) 
DIQMTQSPSSLSASVGDRVTITCRASQDIRNYLNWYQQKPGKAPKLLIYYTSRLESGVPSRFSGS
GSGTDYTLTISSLQPEDFATYYCQQGNTLPWTFGCGTKVEIK (SEQ ID NO: 101)
   >VL1(anti-CD3 antibody huUCHT1, comprising E80C and E100C mutations) 
DIQMTQSPSSLSASVGDRVTITCRASQDIRNYLNWYQQKPGKAPKLLIYYTSRLESGVPSRFSGS
GSGTDYTLTISSLQPCDFATYYCQQGNTLPWTFGCGTKVEIK (SEQ ID NO: 102)
   >VL2(anti-CD28 antibody) 
DIQMTQSPSSLSASVGDRVTITCHASQNIYVWLNWYQQKPGKAPKLLIYKASNLHTGVPSRFSG
SGSGTDFTLTISSLQPEDFATYYCQQGQTYPYTFGGGTKVEIK (SEQ ID NO: 103)
   >VL2(anti-CD28 antibody, comprising an E80C mutation) 
DIQMTQSPSSLSASVGDRVTITCHASQNIYVWLNWYQQKPGKAPKLLIYKASNLHTGVPSRFSG
SGSGTDFTLTISSLQPCDFATYYCQQGQTYPYTFGGGTKVEIK (SEQ ID NO: 104)
   >VL-BB1(anti-4-1BB antibody, monovalent) 
QSVLTQPPSASGTPGQRVTISCSGSSSNIGNNYVTWYQQLPGTAPKLLIYADSHRPSGVPDRFSGS
KSGTSASLAISGLRSEDEADYYCATWDYSLSGYVFGCGTKLTVL (SEQ ID NO: 105)
   >VL- BB2(anti-4-1BB antibody, bivalent) 
DIVMTQSPSSLSASVGDRVTITCQASEDISSYLAWYQQKPGKAPKRLIYGASDLASGVPSRFSAS
GSGTDYTFTISSLQPEDIATYYCHYYATISGLGVAFGGGTKVEIK (SEQ ID NO: 106)
***Hinge-CH2-CH3***
   Hinge-CH2-CH3 A1B1C2-2 (FC0)
      >Hinge-CH2-CH3 Hole, KiH-dS PVA-AAS N297Q T366S-L368A-Y407V Y349C 
EPKSSDKTHTCPPCPAPPVAGPSVFLFPPKPKDTLMISRTPEVTCVVVDVSHEDPEVKFNWYVDG
VEVHNAKTKPREEQYQSTYRVVSVLTVLHQDWLNGKEYKCKVSNKALPASIEKTISKAKGQPREPQ
VCTLPPSRDELTKNQVSLSCAVKGFYPSDIAVEWESNGQPENNYKTTPPVLDSDGSFFLVSKLTVDKS
RWQQGNVFSCSVMHEALHNHYTQKSLSLSPGK (SEQ ID NO: 109)
      >Hinge-CH2-CH3 Knob, KiH-dS PVA-AAS N297Q T366W S354C 
EPKSSDKTHTCPPCPAPPVAGPSVFLFPPKPKDTLMISRTPEVTCVVVDVSHEDPEVKFNWYVDG
VEVHNAKTKPREEQYQSTYRVVSVLTVLHQDWLNGKEYKCKVSNKALPASIEKTISKAKGQPREPQ
VYTLPPCRDELTKNQVSLWCLVKGFYPSDIAVEWESNGQPENNYKTTPPVLDSDGSFFLYSKLTVDK
SRWQQGNVFSCSVMHEALHNHYTQKSLSLSPGK (SEQ ID NO: 110)
   Hinge-CH2-CH3 A1B2C2-2 (FC1)
      >Hinge-CH2-CH3 Hole, KiH-dS PVA-GSS N297Q T366S-L368A-Y407V Y349C 
EPKSSDKTHTCPPCPAPPVAGPSVFLFPPKPKDTLMISRTPEVTCVVVDVSHEDPEVKFNWYVDG
VEVHNAKTKPREEQYQSTYRVVSVLTVLHQDWLNGKEYKCKVSNKGLPSSIEKTISKAKGQPREPQ
VCTLPPSRDELTKNQVSLSCAVKGFYPSDIAVEWESNGQPENNYKTTPPVLDSDGSFFLVSKLTVDKS
RWQQGNVFSCSVMHEALHNHYTQKSLSLSPGK (SEQ ID NO: 111)
      >Hinge-CH2-CH3 Knob, KiH-dS PVA-GSS N297Q T366W S354C 
EPKSSDKTHTCPPCPAPPVAGPSVFLFPPKPKDTLMISRTPEVTCVVVDVSHEDPEVKFNWYVDG
VEVHNAKTKPREEQYQSTYRVVSVLTVLHQDWLNGKEYKCKVSNKGLPSSIEKTISKAKGQPREPQ
VYTLPPCRDELTKNQVSLWCLVKGFYPSDIAVEWESNGQPENNYKTTPPVLDSDGSFFLYSKLTVDK
SRWQQGNVFSCSVMHEALHNHYTQKSLSLSPGK (SEQ ID NO: 112)
   Hinge-CH2-CH3 A1B2C1 (FC2)
      >Hinge-CH2-CH3 Hole, KiH-dS PVA-GSS T366S-L368A-Y407V Y349C 
EPKSSDKTHTCPPCPAPPVAGPSVFLFPPKPKDTLMISRTPEVTCVVVDVSHEDPEVKFNWYVDG
VEVHNAKTKPREEQYNSTYRVVSVLTVLHQDWLNGKEYKCKVSNKGLPSSIEKTISKAKGQPREPQ
VCTLPPSRDELTKNQVSLSCAVKGFYPSDIAVEWESNGQPENNYKTTPPVLDSDGSFFLVSKLTVDKS
RWQQGNVFSCSVMHEALHNHYTQKSLSLSPGK (SEQ ID NO: 113)
      >Hinge-CH2-CH3 Knob, KiH-dS PVA-GSS T366W S354C 
EPKSSDKTHTCPPCPAPPVAGPSVFLFPPKPKDTLMISRTPEVTCVVVDVSHEDPEVKFNWYVDG
VEVHNAKTKPREEQYNSTYRVVSVLTVLHQDWLNGKEYKCKVSNKGLPSSIEKTISKAKGQPREPQ
VYTLPPCRDELTKNQVSLWCLVKGFYPSDIAVEWESNGQPENNYKTTPPVLDSDGSFFLYSKLTVDK
SRWQQGNVFSCSVMHEALHNHYTQKSLSLSPGK (SEQ ID NO: 114)
   Hinge-CH2-CH3 A3B2C1 (FC3)
      >Hinge-CH2-CH3, KiH-dS PVA-GSS 
EPKSSDKTHTCPPCPAPPVAGPSVFLFPPKPKDTLMISRTPEVTCVVVDVSHEDPEVKFNWYVDG
VEVHNAKTKPREEQYNSTYRVVSVLTVLHQDWLNGKEYKCKVSNKGLPSSIEKTISKAKGQPREPQ
VYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 115)
      >Hinge-CH2-CH3, KiH-dS PVA-GSS 
EPKSSDKTHTCPPCPAPPVAGPSVFLFPPKPKDTLMISRTPEVTCVVVDVSHEDPEVKFNWYVDGVEV
HNAKTKPREEQYNSTYRVVSVLTVLHQDWLNGKEYKCKVSNKGLPSSIEKTISKAKGQPREPQVYTL
PPSRDELTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 116)
   Hinge-CH2-CH3 A4-1B2C1 (FC4)
      >Hinge-CH2-CH3 FcK/O, KiH-dS PVA-GSS D356K D399K 
EPKSSDKTHTCPPCPAPPVAGPSVFLFPPKPKDTLMISRTPEVTCVVVDVSHEDPEVKFNWYVDG
VEVHNAKTKPREEQYNSTYRVVSVLTVLHQDWLNGKEYKCKVSNKGLPSSIEKTISKAKGQPREPQ
VYTLPPSRKELTKNQVSLTCLVKGFYPSDIAVEWESNGQPENNYKTTPPVLKSDGSFFLYSKLTVDKS
RWQQGNVFSCSVMHEALHNHYTQKSLSLSPGK (SEQ ID NO: 117)
      >Hinge-CH2-CH3 FcK/O, KiH-dS PVA-GSS K392D K409D 
EPKSSDKTHTCPPCPAPPVAGPSVFLFPPKPKDTLMISRTPEVTCVVVDVSHEDPEVKFNWYVDG
VEVHNAKTKPREEQYNSTYRVVSVLTVLHQDWLNGKEYKCKVSNKGLPSSIEKTISKAKGQPREPQ
VYTLPPSRDELTKNQVSLTCLVKGFYPSDIAVEWESNGQPENNYDTTPPVLDSDGSFFLYSDLTVDKS
RWQQGNVFSCSVMHEALHNHYTQKSLSLSPGK (SEQ ID NO: 118)
   Hinge-CH2-CH3 A4-2B2C1 (FC5)
      >Hinge-CH2-CH3 FcK/O, KiH-dS PVA-GSS PPV-KKK 
EPKSSDKTHTCPPCPAPPVAGPSVFLFPPKPKDTLMISRTPEVTCVVVDVSHEDPEVKFNWYVDG
VEVHNAKTKPREEQYNSTYRVVSVLTVLHQDWLNGKEYKCKVSNKGLPSSIEKTISKAKGQPREPQ
VYTLPPSRDELTKNQVSLTCLVKGFYPSDIAVEWESNGQPENNYKTTKKKLDSDGSFFLYSKLTVDK
SRWQQGNVFSCSVMHEALHNHYTQKSLSLSPGK (SEQ ID NO: 119)
      >Hinge-CH2-CH3 FcK/O, KiH-dS PVA-GSS TPP-DDD 
EPKSSDKTHTCPPCPAPPVAGPSVFLFPPKPKDTLMISRTPEVTCVVVDVSHEDPEVKFNWYVDG
VEVHNAKTKPREEQYNSTYRVVSVLTVLHQDWLNGKEYKCKVSNKGLPSSIEKTISKAKGQPREPQ
VYTLPPSRDELTKNQVSLTCLVKGFYPSDIAVEWESNGQPENNYKTDDDVLDSDGSFFLYSKLTVDK
SRWQQGNVFSCSVMHEALHNHYTQKSLSLSPGK (SEQ ID NO: 120)
***CH3***
   >CH3 Hole (comprising the T366S, L368A and Y407V hole mutations and the Y349C mutation) 
GQPREPQVCTLPPSRDELTKNQVSLSCAVKGFYPSDIAVEWESNGQPENNYKTTPPVLDSDGSFF
LVSKLTVDKSRWQQGNVFSCSVMHEALHNHYTQKSLSLSPGK (SEQ ID NO: 6)
   >CH3 Knob (comprising the T366W knob mutation and the S354C mutation) 
GQPREPQVYTLPPCRDELTKNQVSLWCLVKGFYPSDIAVEWESNGQPENNYKTTPPVLDSDGSF
FLYSKLTVDKSRWQQGNVFSCSVMHEALHNHYTQKSLSLSPGK (SEQ ID NO: 7)
***CL***
   >CL 
RADAAPTVSIFPPSSEQLTSGGASVVCFLNNFYPKDINVKWKIDGSERQNGVLNSWTDQDSKDS
TYSMSSTLTLTKDEYERHNSYTCEATHKTSTSPIVKSFNRNEC (SEQ ID NO: 15)
   >CL (comprising the NEC deletion mutation) 
RADAAPTVSIFPPSSEQLTSGGASVVCFLNNFYPKDINVKWKIDGSERQNGVLNSWTDQDSKDS
TYSMSSTLTLTKDEYERHNSYTCEATHKTSTSPIVKSFNR (SEQ ID NO: 16)
***CH1***
   >CH1 
AKTTPPSVYPLAPGSAAQTNSMVTLGCLVKGYFPEPVTVTWNSGSLSSGVHTFPAVLQSDLYTLS
SSVTVPSSPRPSETVTCNVAHPASSTKVDKKIVPRDC (SEQ ID NO: 17)
   >CH1 (comprising the VPRDC deletion mutation) 
AKTTPPSVYPLAPGSAAQTNSMVTLGCLVKGYFPEPVTVTWNSGSLSSGVHTFPAVLQSDLYTLS
SSVTVPSSPRPSETVTCNVAHPASSTKVDKKI (SEQ ID NO: 18)
***Alb***









 (SEQ ID NO: 2)

### Linkers

S (SEQ ID NO: 19)
GS (LM3, SEQ ID NO: 20)
GGGGS (the third linker, the fourth linker, the fifth linker, the sixth linker, the eighth linker, the tenth linker, the fourteenth linker, SEQ ID NO: 22)
GGGGSGGGGSGGGGSGGGGSGGGS (SEQ ID NO: 28)
GGGGSGGGGSGGGGS (the second linker, the seventh linker, the ninth linker, the eleventh linker, LM2, SEQ ID NO: 27)
GGGGSGGGGSGGGGSGGGGSGGGGSGGGGS (SEQ ID NO: 29)
GGGGSGGGGS (the first linker, SEQ ID NO: 26)
GGSGGS (LM1, SEQ ID NO: 24)
GGSGGSGGS (SEQ ID NO: 25)
GQPKAAP (SEQ ID NO: 30)
GGGSGGGG (the twelfth linker, the thirteenth linker, SEQ ID NO: 23)
RTSGPGDGGKGGPGKGPGGEGTKGTGPGG (SEQ ID NO: 35)
GGEGGGSEGGGS (SEQ ID NO: 121)
TVLRT (SEQ ID NO: 31)
TVSSAS (SEQ ID NO: 32)
GGEGG (SEQ ID NO: 33)
GSEGGGS (SEQ ID NO: 34)
GKGPGGEGTKGTGPGG (SEQ ID NO: 36)
TVLSSAS (SEQ ID NO: 37)
EDL (SEQ ID NO: 122)
ANI (SEQ ID NO: 123)

### Example 1 Construction of TCR-based multi-specific polypeptide molecules

### 1.1 Construction of gp100-targeting multi-specific polypeptide molecules

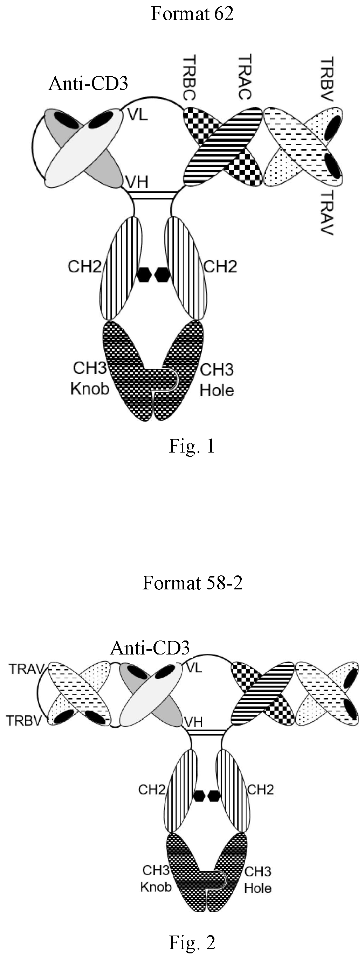

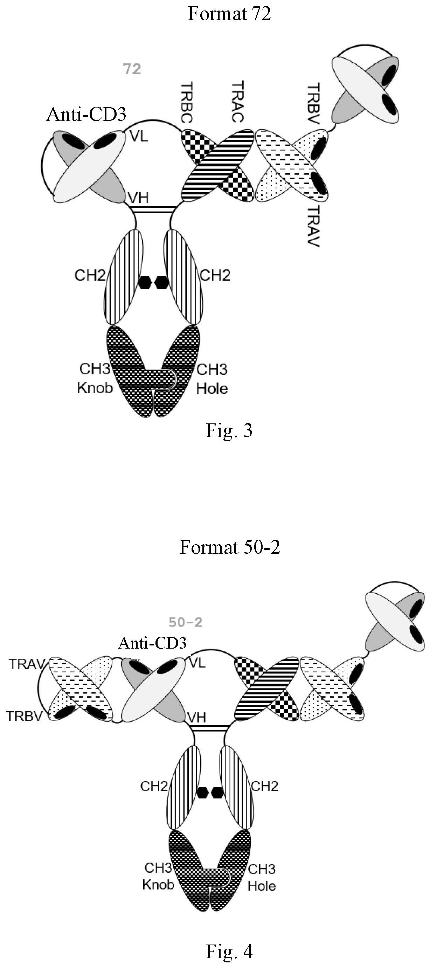

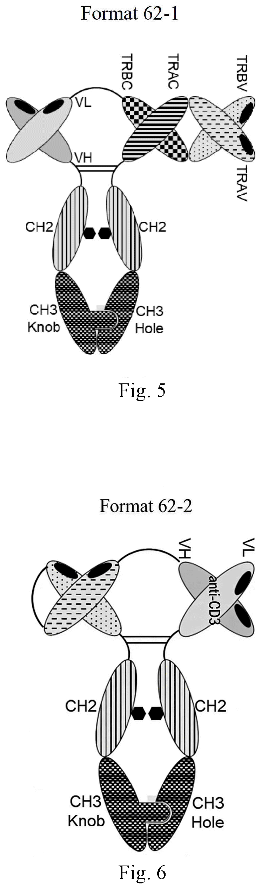

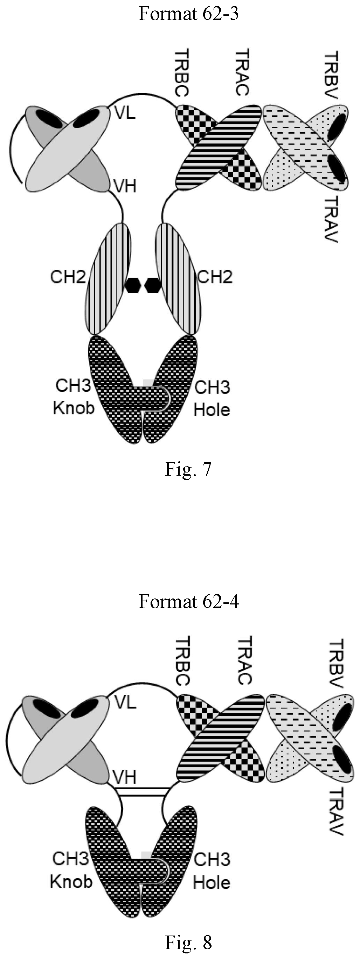

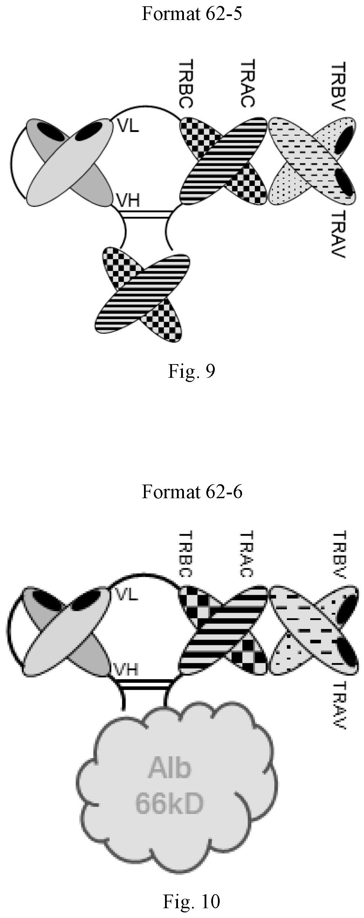

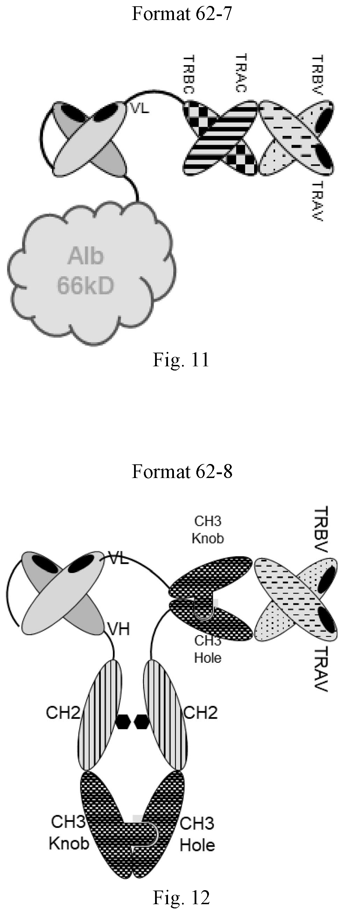

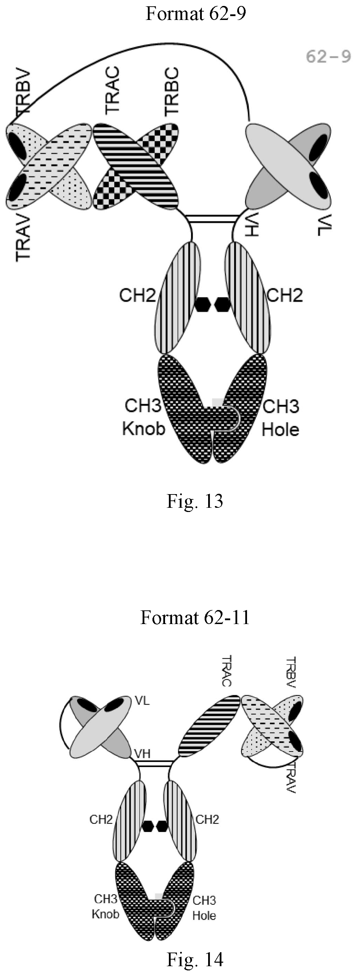

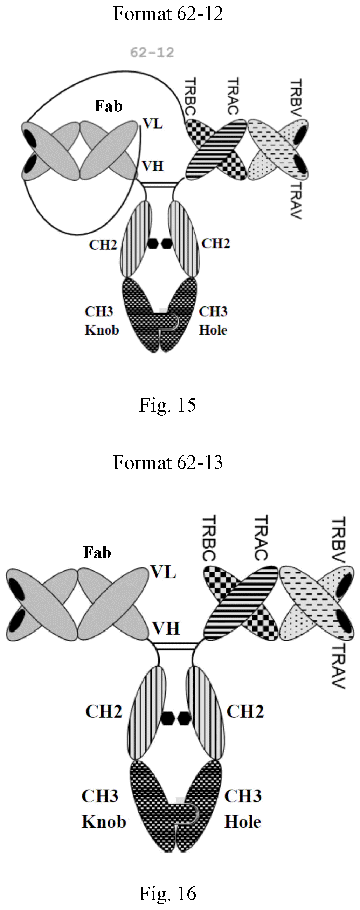

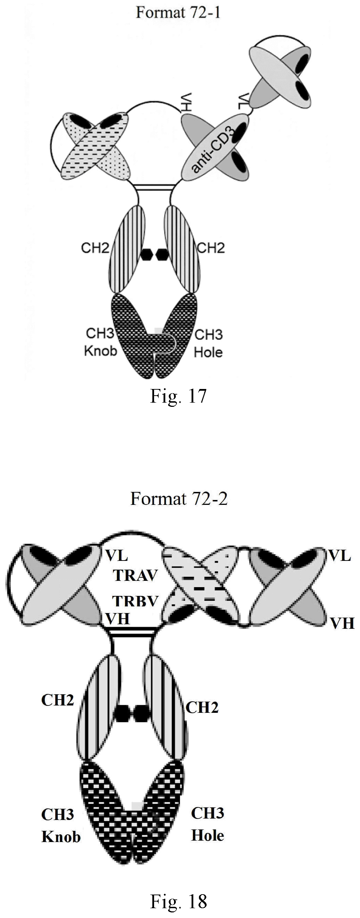

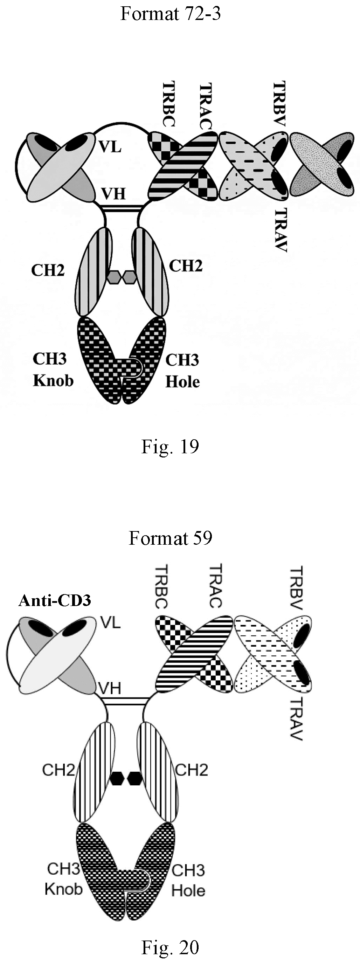

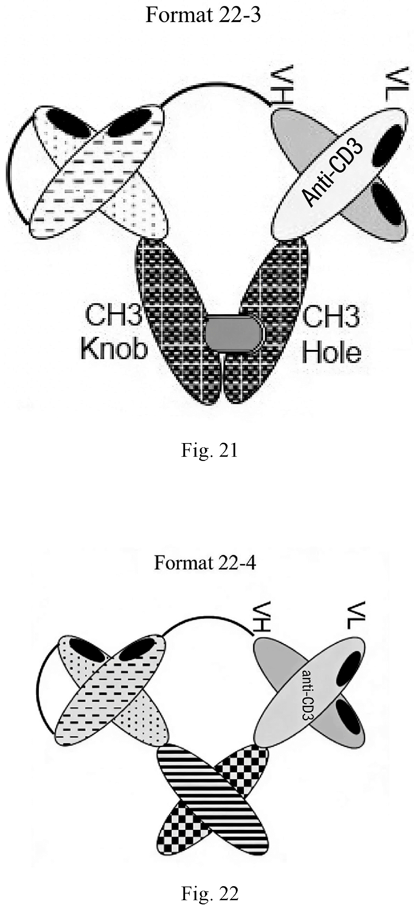

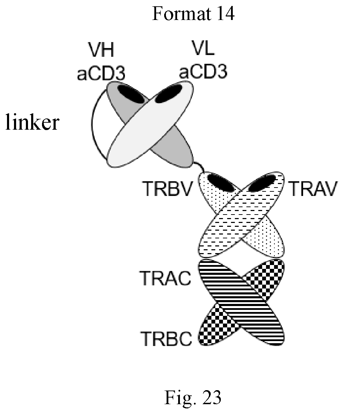

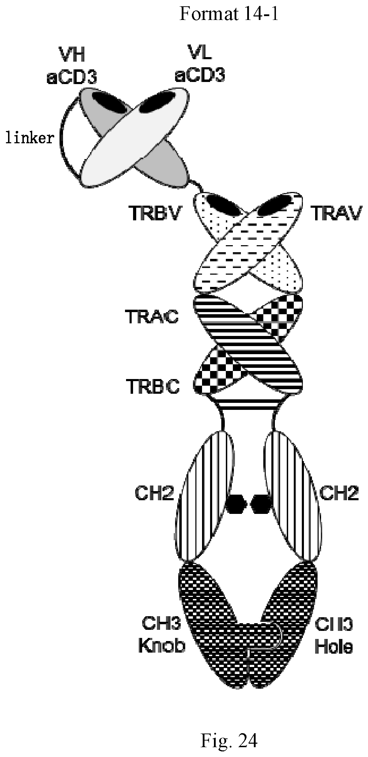

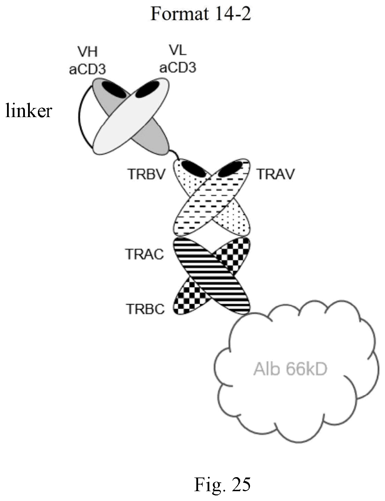

gp100 TCR-based multi-specific polypeptide molecule formats were designed and constructed, and the structures of these formats are shown in Figs. 1-25. Exemplary formats comprise:
a first polypeptide chain: TRBV1-TRBC-the first linker-VL1-the second linker-VH1-hinge region -CH2-CH3 Knob, and a second polypeptide chain: TRAV1- TRAC-hinge region -CH2-CH3 Hole (format 62); or
a first polypeptide chain: TRAV1-TRAC-the first linker-VL1-the second linker-VH1-hinge region -CH2-CH3 Knob, and a second polypeptide chain: TRBV1-TRBC-hinge region -CH2-CH3 Hole (format 62-LJH1); or
a first polypeptide chain: TRBV1-TRBC-the first linker-VH1-the second linker-VL1-hinge region -CH2-CH3 Knob, and a second polypeptide chain: TRAV1-TRAC-hinge region -CH2-CH3 Hole (format 62-LJH2); or
a first polypeptide chain: TRAV1-TRAC-the first linker-VH1-the second linker-VL1-hinge region -CH2-CH3 Knob, and a second polypeptide chain: TRBV1-TRBC- linker -hinge region -CH2-CH3 Hole (format 62-LJH3).

The Formats used for comparison comprise:
the first polypeptide chain: VL1-the second linker-VH1-the first linker-TRBV1-TRBC; and the second polypeptide chain: TRAV1-TRAC; (format 14, also referred to as KIMMTRAK);
the first polypeptide chain: VL1-the second linker-VH1-the first linker-TRBV1-TRBC-hinge region -CH2-CH3 Knob; and the second polypeptide chain: TRAV1-TRAC-hinge region-CH2-CH3 Hole; (format 14-1);
the first polypeptide chain: VL1-the second linker-VH1-the first linker-TRBV1-TRBC; and the second polypeptide chain: TRAV1-TRAC-the fourteenth linker-Alb; (format 14-2);
the first polypeptide chain: TRAV1-the twelfth linker-VH1-hinge region-CH2-CH3 Knob; and the second polypeptide chain: VL1-the thirteenth linker-TRBV1 -hinge region-CH2-CH3 Hole; (format 17).

In these formats, the amino acid sequence of TRAV1 is as shown in SEQ ID NO: 91, the amino acid sequence of TRBV1 is as shown in SEQ ID NO: 92, the amino acid sequence of TRAC is as shown in SEQ ID NO: 8, the amino acid sequence of TRBC is as shown in SEQ ID NO: 11, the amino acid sequence of VH1 is as shown in SEQ ID NO: 95, the amino acid sequence of VL1 is as shown in SEQ ID NO: 101, the amino acid sequence of hinge region-CH2-CH3 Hole is as shown in SEQ ID NO: 109, the amino acid sequence of hinge region-CH2-CH3 Knob is as shown in SEQ ID NO: 110, and the amino acid sequence of albumin is as shown in SEQ ID NO: 2.

A signal peptide sequence MGWSLILLFLVAVATRVHS (SEQ ID NO: 164) was added to the N-terminus of each polypeptide chain of these formats to increase secretion of the polypeptide molecules, and a His6 tag was added to the C-terminus of any one chain of the format for purification. The formats in this example can bind to a complex of HLA-A*02 with the gp100 antigen epitope YLEPGPVTA (SEQ ID NO: 165) or mutant form thereof (YLEPGPVTV, SEQ ID NO: 166).

### 1.2 Compatibility of the multi-specific polypeptide molecules of the present disclosure with different TCRs

### 1.2.1 Construction of MAGEA1-targeting multi-specific polypeptide molecules

MAGEA1-targeting multi-specific polypeptide molecule formats were constructed as described in Example 1.1, and the only difference between the structures of these formats and those of the formats of Example 1.1 is that TRAV1 and TRBV1 of a MAGEA1-specific TCR were used to replace TRAV1 and TRBV1 of the gp100-specific TCR in Example 1.1.

The amino acid sequence of TRAV and TRBV of the MAGEA1-specific TCR is as follows:
A1A2-WT: comprises TRAV1 as shown in SEQ ID NO: 41 and TRBV1 as shown in SEQ ID NO: 42.

### 1.2.2 Construction of KRAS-targeting multi-specific polypeptide molecules

KRAS-targeting multi-specific polypeptide molecule formats were constructed as described in Example 1.1, and the only difference between the structures of these formats and those of the formats of Example 1.1 is that TRAV1 and TRBV1 of a KRAS-specific TCR were used to replace TRAV1 and TRBV1 of the gp100-specific TCR in Example 1.1.

The amino acid sequences of TRAV and TRBV of the KRAS-specific TCR are as follows:
KVA11-N01: comprises TRAV1 as shown in SEQ ID NO: 57 and TRBV1 as shown in SEQ ID NO: 58;
KVA11-N02: comprises TRAV1 as shown in SEQ ID NO: 65 and TRBV1 as shown in SEQ ID NO: 66;
KVA11-N04: comprises TRAV1 as shown in SEQ ID NO: 73 and TRBV1 as shown in SEQ ID NO: 74;
KDA11-N01: comprises TRAV1 as shown in SEQ ID NO: 81 and TRBV1 as shown in SEQ ID NO: 82;
KDA11-N02: comprises TRAV1 as shown in SEQ ID NO: 89 and TRBV1 as shown in SEQ ID NO: 90.

### 1.2.3 Construction of a multi-specific polypeptide molecule targeting HPV E7

HPV E7-targeting multi-specific polypeptide molecule format was constructed as described in Example 1.1, and the only difference between the structure of the format and those of the formats of Example 1.1 is that TRAV1 and TRBV1 of an HPV E7-specific TCR were used to replace TRAV1 and TRBV1 of the gp100-specific TCR in Example 1.1. .

TRAV1 and TRBV1 of the HPV E7-specific TCR comprise the amino acid sequences as shown in SEQ ID NO: 49 and SEQ ID NO:50, respectively.

### 1.3 Effect of the first linker on the multi-specific polypeptide molecules of the present disclosure

gp100-targeting multi-specific polypeptide molecule formats were constructed as described in Example 1.1, and the structures of the formats are the same as that of format 62 in Example 1.1, except that the first linker was replaced with LM1 (Format 62-LM1), LM2 (Format 62-LM2), LM3 (Format 62-LM3), or the first linker sequence was removed (Format 59):
Format 62-LM1:a first polypeptide chain: TRBV1-TRBC- LM1-VL1-the second linker-VH1-hinge region -CH2-CH3 Knob, and a second polypeptide chain: TRAV1- TRAC-hinge region -CH2-CH3 Hole;
Format 62-LM2:a first polypeptide chain: TRBV1-TRBC- LM2-VL1-the second linker-VH1-hinge region -CH2-CH3 Knob, and a second polypeptide chain: TRAV1- TRAC-hinge region -CH2-CH3 Hole;
Format 62-LM3:a first polypeptide chain: TRBV1-TRBC- LM3-VL1-the second linker-VH1-hinge region -CH2-CH3 Knob, and a second polypeptide chain: TRAV1- TRAC-hinge region -CH2-CH3 Hole;
Format 59: a first polypeptide chain: TRBV1-TRBC, a second polypeptide chain: VL1-the second linker-VH1-hinge region -CH2-CH3 Knob, and a third polypeptide chain: TRAV1-TRAC-hinge region -CH2-CH3 Hole.

### 1.4 Effect of the second linker on the multi-specific polypeptide molecules of the present disclosure

gp100-targeting multi-specific polypeptide molecule format was constructed as described in Example 1.1, and the structure of the format is the same as that of format 62 in Example 1.1, except that the second linker was removed to form Format 62-1:
the first polypeptide chain: TRBV1-TRBC-first linker-VL1, the second polypeptide chain: VH1-hinge region-CH2-CH3 Knob, and the third polypeptide chain: TRAV1-TRAC-hinge region-CH2-CH3 Hole.

### 1.5 Effect of the hinge region on the multi-specific polypeptide molecules of the present disclosure

gp100-targeting multi-specific polypeptide molecule format was constructed as described in Example 1.1, and the structure of the format is the same as that of format 62 in Example 1.1, except that the hinge region of the first polypeptide chain was replaced with the third linker (SEQ ID NO: 22) and the hinge region of the second polypeptide chain was replaced with the fourth linker (SEQ ID NO: 22), thereby obtaining format 62-3:
the first polypeptide chain: TRBV1-TRBC-first linker-VL1-second linker-VH1-third linker-CH2-CH3 Knob, and the second polypeptide chain: TRAV1- TRAC-fourth linker-CH2-CH3 Hole.

### 1.6 Effect of the functional domains on the multi-specific polypeptide molecules of the present disclosure

gp100-targeting multi-specific polypeptide molecule formats were constructed as described in Example 1.1, and the structures of the formats are the same as that of format 62 in Example 1.1; the difference lies in that:
1) The CH2-CH3 domains in the first polypeptide chain and the second polypeptide chain were replaced with TRBC (SEQ ID NO: 11) and TRAC (SEQ ID NO: 8), respectively, to form Format 62-5:
   the first polypeptide chain: TRBV1-TRBC-first linker-VL1-second linker-VH1-hinge region-TRBC, and the second polypeptide chain: TRAV1-TRAC-hinge region-TRAC;
2) The CH2-CH3 domain was removed from the first polypeptide chain, and the CH2-CH3 domain in the second polypeptide chain was replaced with albumin to form Format 62-6:
   the first polypeptide chain: TRBV1-TRBC-first linker-VL1-second linker-VH1-hinge region, and the second polypeptide chain: TRAV1- TRAC-hinge region-Alb;
3) The hinge region-CH2-CH3 domain in the first polypeptide chain was replaced with the fifth linker-Alb (the amino acid sequence of the fifth linker is as shown in SEQ ID NO: 22), and the hinge region-CH2-CH3 domain in the second polypeptide chain was removed, to form Format 62-7:
   the first polypeptide chain: TRBV1-TRBC-first linker-VL1-second linker-VH1-fifth linker-Alb, and second polypeptide chain: TRAV1- TRAC.

### 1.7 Effect of TRAC, TRBC and the hinge region on the multi-specific polypeptide molecules of the present disclosure

gp100-targeting multi-specific polypeptide molecule format was constructed as described in Example 1.1, and the structure of the format is the same as that of format 62 in Example 1.1, except that: the hinge region of the first polypeptide chain was replaced with the third linker (SEQ ID NO: 22); the hinge region of the second polypeptide chain was replaced with the fourth linker (SEQ ID NO: 22); and TRAC and TRBC were replaced with CH3 knob and CH3 Hole, respectively, to form format 62-8:
the first polypeptide chain: TRBV1-CH3 Hole-first linker-VL1-second linker-VH1-third linker-CH2-CH3 Knob, and the second polypeptide chain: TRAV1-CH3 Knob-fourth linker-CH2-CH3 Hole.

### 1.8 Effect of different Hinge-CH2-CH3 on the multi-specific polypeptide molecules of the present disclosure

MAGE-A1-targeting multi-specific polypeptide molecule formats were constructed as described in Example 1.2.1, and the structures of the formats are the same as that of format 62-LJH2 in Example 1.2.1; the difference lies in that: FC0 in format 62-LJH2 was replaced with FC1, FC2, FC3, FC4, or FC5, respectively.

### 1.9 Effect of the linkage mode of the TCR on the antibody on the multi-specific polypeptide molecules of the present disclosure

gp100-targeting multi-specific polypeptide molecule format was constructed as described in Example 1.1, the difference lies in that: the C-terminus of the antibody of the format in this Example was connected to the N-terminus of the TCR, and the TCR was an scTCR, thereby obtaining Format 62-2:
the first polypeptide chain: VH1-first linker-TRAV1-eleventh linker-TRBV1-hinge region-CH2-CH3 Knob, and the second polypeptide chain: VL1-hinge region-CH2-CH3 Hole; wherein: the eleventh linker is SEQ ID NO: 27.

### 1.10 Effect of increasing the TCR valency on the multi-specific polypeptide molecules of the present disclosure

gp100-targeting multi-specific polypeptide molecule format was constructed as described in Example 1.1, and the difference between the structure of the format in this Example and that of format 62 in Example 1.1 lies in that: based on format 62, a pair of TRAV and TRBV (referred to as TRAV2 and TRBV2) was added, thereby obtaining format 58-2:
the first polypeptide chain: TRBV1-TRBC-the first linker-VL1-the sixth linker-TRAV2-the seventh linker-TRBV2-the eighth linker-VH1-hinge region -CH2-CH3 Knob, and a second polypeptide chain: TRAV1-TRAC-hinge region -CH2-CH3 Hole; wherein:
The amino acid sequence of TRAV2 is as shown in SEQ ID NO:91, and the amino acid sequence of TRBV2 is as shown in SEQ ID NO:92;
The amino acid sequence of the sixth linker is as shown in SEQ ID NO: 22, the amino acid sequence of the seventh linker is as shown in SEQ ID NO: 27, and the amino acid sequence of the eighth linker is as shown in SEQ ID NO: 22.

### 1.11 Effect of increasing antibody valency on the multi-specific polypeptide molecule of the present disclosure

gp100-targeting multi-specific polypeptide molecule format was constructed as described in Example 1.1, and the difference between the structure of the format in this Example and that of format 62 in Example 1.1 lies in that: based on format 62, a pair of VH and VL (referred to as VH2 and VL2) targeting CD28 was added, thereby obtaining format 72:
the first polypeptide chain: VL2-the ninth linker-VH2-the tenth linker-TRBV1-TRBC-the first linker-VL1-the second linker-VH1-hinge region -CH2-CH3, and the second polypeptide chain: TRAV1- TRAC-hinge region -CH2-CH3, wherein:
the amino acid sequence of VL2 is as shown in SEQ ID NO: 103, and the amino acid sequence of VH2 is as shown in SEQ ID NO: 97;
the amino acid sequence of the ninth linker is as shown in SEQ ID NO: 27, and the amino acid sequence of the tenth linker is as shown in SEQ ID NO: 22.

### 1.12 Effect of increasing TCR and antibody valency on the multi-specific polypeptide molecule of the present disclosure

gp100-targeting multi-specific polypeptide molecule format was constructed as described in Example 1.1, and the difference between the structure of the format in this Example and that of format 62 in Example 1.1 lies in that: based on format 62, a pair of of TRAV and TRBV (referred to as TRAV2 and TRBV2) and a pair of VH and VL (referred to as VH2 and VL2) targeting CD28 were added, thereby obtaining format 50-2:
a first polypeptide chain: VL2-the ninth linker-VH2-the tenth linker-TRBV1-TRBC-the first linker-VL1-the sixth linker-TRAV2-the seventh linker-TRBV2-the eighth linker-VH1-hinge region -CH2-CH3, and a second polypeptide chain: TRAV1- TRAC-hinge region -CH2-CH3, wherein:
the amino acid sequence of TRAV2 is as shown in SEQ ID NO:91, and the amino acid sequence of TRBV2 is as shown in SEQ ID NO:92;
The amino acid sequence of VL2 is as shown in SEQ ID NO: 103, and the amino acid sequence of VH2 is as shown in SEQ ID NO: 97;
the amino acid sequence of the sixth linker is as shown in SEQ ID NO: 22, and the amino acid sequence of the seventh linker is as shown in SEQ ID NO: 27, the amino acid sequence of the eighth linker is as shown in SEQ ID NO: 22, the amino acid sequence of the ninth linker is as shown in SEQ ID NO: 27, and the amino acid sequence of the tenth linker is as shown in SEQ ID NO: 22.

### Example 2 Expression and purification of TCR-based multi-specific polypeptide molecules

### 1. Vector construction and plasmid extraction

The nucleotide sequence encoding one or more polypeptide chains of the format in Example 1 was directly cloned into the expression vector pTT5, and the obtained vector clone was subjected to amplification culture and plasmid extraction after sequencing confirmation.

### 2. Transfection

The CHO cell density was adjusted to 1X10⁶ cells/ml, with a cell suspension volume of 40mL per bottle, then the bottle cap was tightened and the bottle was placed into a shaker for continued culture, and after culturing for 2-4 hours under the conditions of 36.5°C, 175rpm, and 5% CO₂, transfection was performed with plasmids. Prepare the transfection mixture (1ml): use about 800µL of 150 mM sterile NaCl solution to dilute 10µg DNA, mix well and place on the bench for 5min; add about 50µl transfection reagent to the DNA dilution and mix well, and the final total volume of the transfection mixture is 1mL. After placing on the bench for 10min, add the transfection mixture dropwise into the cell culture medium, shake to mix well, tighten the bottle cap, and return to the shaker (36.5°C, turn off 5%CO₂, 175rpm). At 20-24h after transfection, add SMS 293-I feed solution (0.7mL/bottle), thereafter feed every other day and culture for 6-10 days, and then collect the culture supernatant.

### 3. Purification

Using nickel column to purify the collected culture supernatant. Prepare a 1ml packed-bed nickel column, rinse with 10ml sterile water, and equilibrate with 10ml 1XBind Buffer. Centrifuge the culture supernatant at 3000rpm for 5min, then perform filtration in 450nm chlorine, and load the sample. After sample loading is completed, wash the column with 10ml 1XBind Buffer, wash the column with 10ml 1XWash Buffer (collect 10 tubes of eluate, with a flow rate of approximately 0.3-0.4ml/min), elute the protein with 1.2ml 1XElution Buffer, and the collected eluate is the purified protein, which is aliquoted and stored at -80.

### 4. Protein staining and immunoblotting

The purified protein was subjected to denaturing (R) or non-denaturing (NR) treatment followed by SDS-PAGE gel electrophoresis and Coomassie brilliant blue staining, to evaluate the purity of expressed protein and the degree of aggregation. Another set of parallel samples was subjected to western blot after gel electrophoresis, and mouse anti-His6 was used as the primary antibody to detect the His6 tag of the purified protein, to confirm the reliability of the Coomassie brilliant blue staining. The results show that the format provided by the present disclosure may be successfully expressed.

The yield of the target protein was predicted according to the harvested target protein yield, and the purity was determined according to the SDS-PAGE Coomassie brilliant blue staining results. The results are shown in Table 1-Table 3 below.

**Table 1. Yield and purity of format 62 polypeptide molecules constructed with TRAV and TRBV of TCRs targeting different antigens**

| | | gp100 | E7A2 | A1A2-WT | KVA11-N01 | KVA11-N02 | KVA11-N04 | KDA11-N01 | KDA11-N02 |
|---|---|---|---|---|---|---|---|---|---|
| Yield (mg/L) | Format 62 | 23.5 | 60 | 36.5 | -- | -- | -- | 214.5 | -- |
| | Format 14 | 105 | 3.45 | 3.2 | -- | -- | -- | 4.05 | -- |
| Purity (%) | Format 62 | 83 | 68 | 78 | 65 | 56 | 73 | 71 | 68 |
| | Format 14 | 77 | 17 | 46 | -- | 13 | 27 | 17 | 6.6 |

The results of Table 1 show that, after being loaded with different TCR variable regions, the format 62 of the present disclosure achieved a relatively high purity through one-step affinity purification (all above 55%, up to 83%), indicating good reproducibility among different batches; whereas the purity of the control format 14 was significantly lower than that of the format 62 of the present disclosure. In addition, after different TCR variable regions were loaded into format 62, the yield of properly assembled protein was relatively high, and individual TCRs were overexpressed at a high level; although there were inter-batch differences in protein yield, there is substantial room for process optimization. These results indicate that the format 62 of the present disclosure has good compatibility with different TCRs.

**Table 2. Yield and purity of different formats obtained from Format 62 containing anti-gp 100 TRAV1 and TRBV1 by chain exchange or replacement of the first linker.**

| | Format 62- | Format 62- | Format 62- | Format 62- | Format 62- | Format 62- |
|---|---|---|---|---|---|---|
| | LJH1 | LJH2 | LJH3 | LM1 | LM2 | LM3 |
| Yield (mg/L) | 66.3 | 80.8 | 82.8 | 91.8 | 92.3 | 95.8 |
| Purity(%) | 84 | 82 | 80 | 82 | 87 | 83 |

The results of Table 2 show that the purity of the formats obtained by chain exchange or replacement of the first linker was all above 80%, and in particular, the purity of Format 62-LM2 in which the first linker was replaced with LM2 reached 87%. These results indicate that chain exchange between TRAV1-TRAC and TRBV1-TRBC, or between VH1 and VL1, or a change in the first linker connecting TRBC and VL1, will not have a negative impact on protein yield and purity.

**Table 3. Yield and purity of Format 62-1, 62-3, 62-6 and 62-8 containing anti-gp100 TRAV1 and TRBV1**

| | Format 62-1 | Format 62-3 | Format 62-6 | Format 62-7 | Format 62-8 |
|---|---|---|---|---|---|
| Yield (mg/L) | 115 | 60 | 32.15 | 31.5 | 14.4 |
| Purity(%) | 80 | 76 | 65 | 42 | 47 |

The results of Table 3 show that these Format 62-1, 62-3, 62-6, 62-7 and 62-8 engineered on the basis of format 62 all have high yield and purity after expression.

### Example 3 ELISPOT assay

Format of Example 1 of the present disclosure was subjected to gradient serial dilution (10^-7 to 10^-13 M) using 1640 complete medium containing 10% FBS. The gp100 peptide, KRAS peptide, MAGE-A1 peptide, and HPV E7 peptide were each added into DMSO for dissolution, and then diluted with water to a working concentration of 10^-4 M. T2 cells were loaded with 10^-6 M gp100 peptide, KRAS peptide, MAGE-A1 peptide, and HPV E7 peptide, respectively. 1640 complete medium containing 10% FBS was added into an ELISPOT plate and blocked at room temperature for 30 min. The medium was discarded, and PBMC at 5x10^5 cells/mL (100 µL/well), peptide-loaded T2 cells at 5x10^5 cells/mL (100 µL/well), and format at different concentrations (10^-7, 10^-8, 10^-9, 10^-10, 10^-11, 10^-12 M) were added into the plate. For the negative control, T2 cells without peptide loading and PBMC were added, and the candidate polypeptide molecule was at the same concentration as in the experimental group; the positive control was PBMC with 10 µL of anti-CD3-2 mAb provided in the kit. After all samples were added, the plate was covered with a lid and incubated in a 37°C, 5% CO2 incubator for 20-24 h, and then IFN-γ secretion was determined by ELISPOT detection, so as to evaluate immune cell activation induced by the formats of the present disclosure through the target antigen peptide-MHC complex.

The results show that the formats of the present disclosure can activate immune cells in the presence of a target antigen.

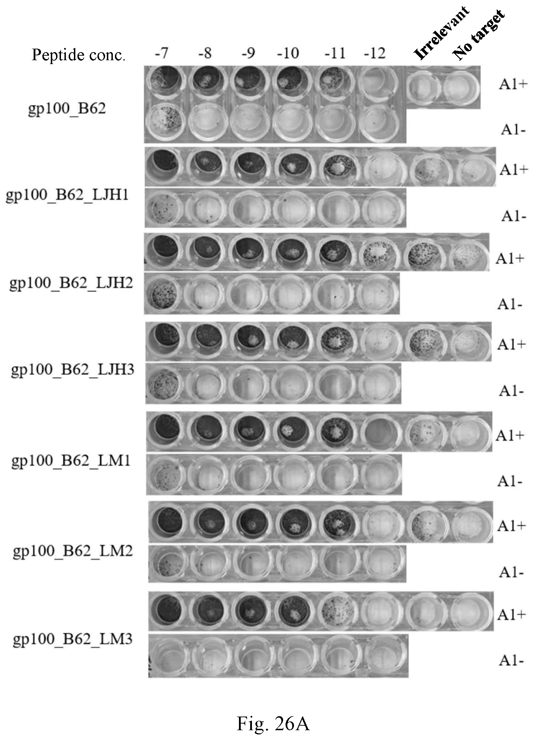

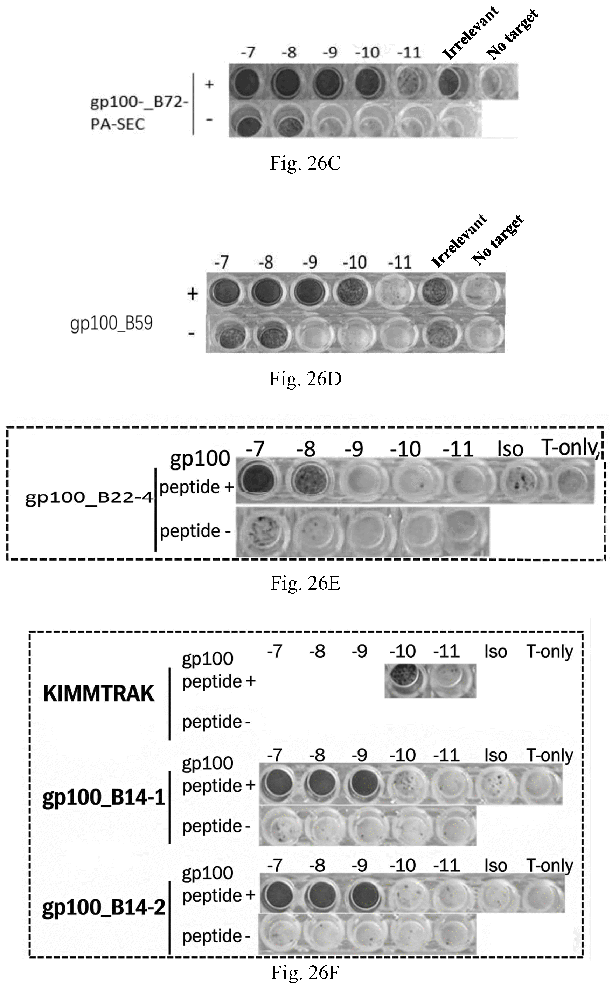

When gp100 is used as the target peptide, exemplary results of different formats of the present disclosure are shown in Figs. 26A-26F (the reference sign "+" or "A1+" indicates an experimental group to which gp100 peptide was added, "-" or "A1-" indicates a negative control group to which no gp100 peptide was added, "irrelevant" or "Iso" indicates a control group to which an irrelevant peptide was added, and "no target" or "T-only" indicates a control group in which no target peptide was added to the control containing T cells).

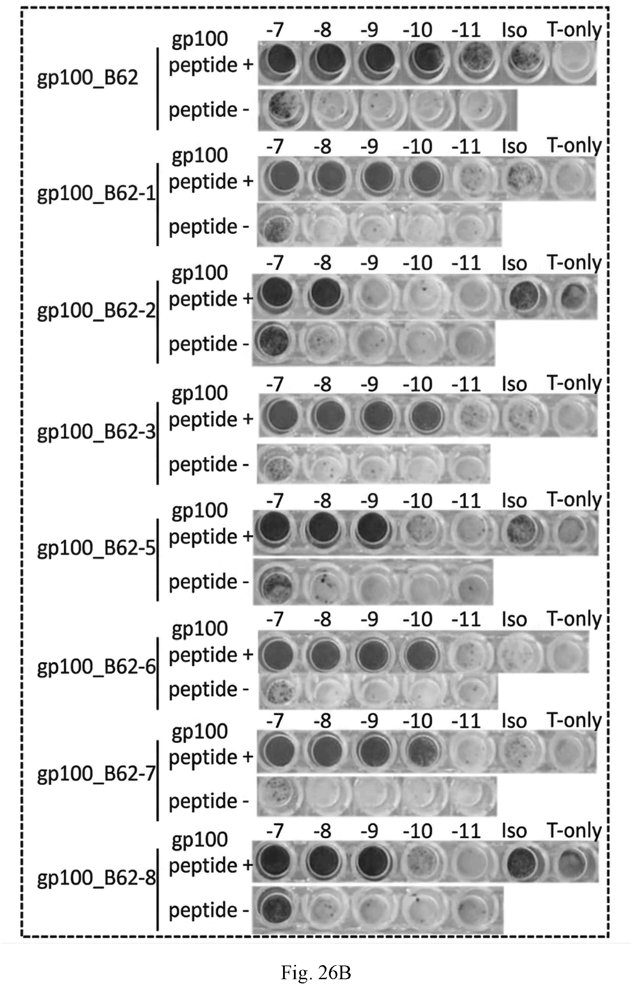

The results of Figs. 26A-26F show that the formats of the present disclosure can achieve effective activation of immune cells within the concentration range of 10^-7-10^-11 M, e.g., formats 62, 62-LJH1, 62-LJH2, 62-LJH3, 62-LM1, and 62-LM2 can effectively activate immune cells at the low concentration of 10^-11 M (Fig. 26A), formats 62-LM3, 62-1, 62-3, 62-6, 62-7, 72, and 59 can effectively activate immune cells at the low concentration of 10^-10 M (Figs. 26A, 26B, 26C, and 26D), and formats 62-5, 62-8, 72-1, 72-2, and 72-3 can activate immune cells at the concentration of 10^-9 M (Figs. 26B and 26C); whereas the control format 14 can activate immune cells at the concentration of 10^-10 M, and the control format 14-1 and 14-2 can activate immune cells at the concentration of 10^-9 M (Fig. 26F), indicating that the immune cell activation effect of the formats of the present disclosure is comparable to or better than that of the control formats.

For formats 62-6 and 62-7 of the present disclosure comprising albumin as a functional domain, the minimum concentration for effectively activating immune cells is 10^-10 M, whereas the minimum concentration for the control format 14-2 likewise comprising albumin to effectively activate immune cells is 10^-9 M, which indicates that the formats scaffold structure of the present disclosure has superior activity compared with the control format.

### Example 4 Physicochemical Properties

### 1. Thermal Stability and Colloidal Stability

To evaluate the stability of the format of the present disclosure, the format samples of the present disclosure were purified and buffer-exchanged into PBS buffer, with PBS buffer as a control.

Differential scanning fluorimetry (Differential Scanning Fluorimetry, abbreviated as DSF) was used to determine the Tm value of the TCR-based multi-specific polypeptide molecules of the present disclosure, and the heating rate was generally set to 1°C/10s. Generally, the higher the Tm, the better the structural thermal stability.

Static light scattering (SLS) was used to determine the Tagg value of the TCR-based multi-specific polypeptide molecules of the present disclosure, and the heating rate was generally set to 1°C/10s. Generally, the Tagg value reflects protein colloidal stability, and generally the higher the Tagg, the better the protein colloidal thermal stability.

The results are shown in Table 4.

**Table 4**

| Samples | Tm value (°C) | Tagg value (°C) |
|---|---|---|
| gp100_B62 | 54.1 | 64.5 |
| MAGE-A1_B62 | 56.3 | 76.5 |
| KDA11-N01_B62 | 56.9 | 76.3 |
| KVA11-N02_B62 | 48.6 | 64.6 |
| E7A2_B62 | 55 | 76.2 |

### 2. Accelerated Stability

To evaluate the accelerated stability of the format of the present disclosure, size exclusion chromatography (SEC) was used to measure monomer and aggregate contents of the TCR-based multi-specific polypeptide molecules of the present disclosure after storage at 4°C, 25°C, and 40°C for 15 days, thereby calculating sample purity.

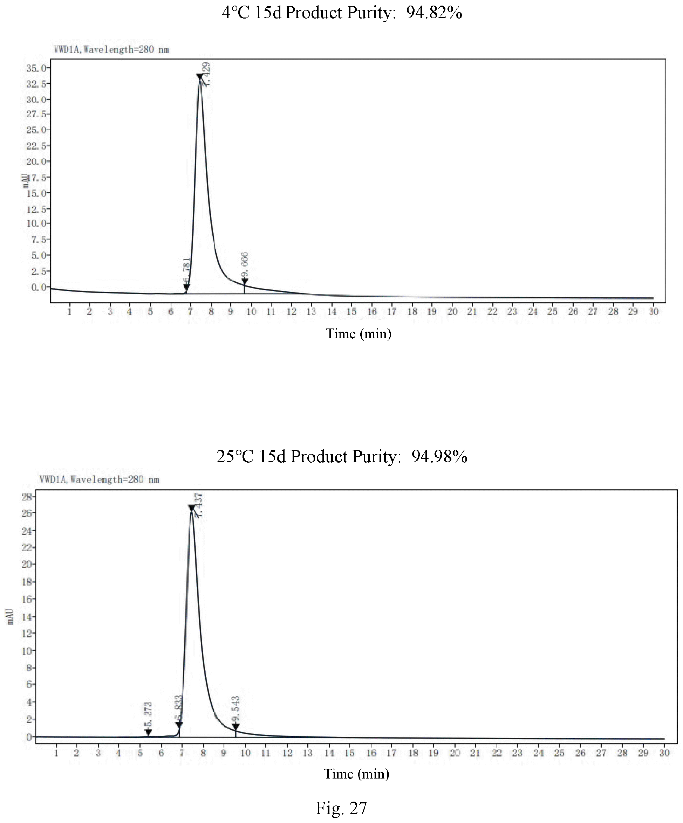

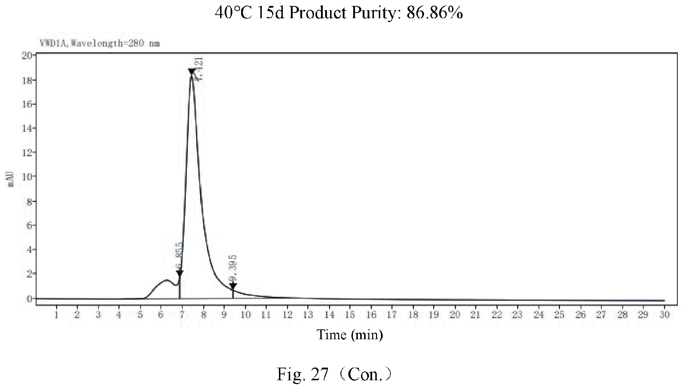

Exemplary results are shown in Fig. 27. The results show that the purity of format 62 targeting MAGE-A1 of Example 1.2.1 of the present disclosure after storage at 4°C, 25°C, and 40°C for 15 days is 94.82%, 94.98%, and 86.86%, respectively, indicating that format 62 of the present disclosure has good accelerated stability.

### Example 5 Half-life Determination

The TCR-based multi-specific polypeptide molecules of the present disclosure were administered to mice by intravenous bolus injection at a dose of 0.1 mg/kg mouse body weight, and serum samples were collected at given time points. Pharmacokinetic (PK) assessment was performed by ELISA. Specifically, serum samples were added to a streptavidin-coated plate linked with a biotinylated pHLA complex, and then a primary antibody (goat anti-Fc) and a secondary antibody (HRP-conjugated anti-goat IgG) were used, and finally colorimetric detection was performed at 450 nm, thereby determining the presence and binding activity of the formats provided by the present disclosure.

### Example 6 In vivo Antitumor Efficacy Determination

An antitumor efficacy study was conducted in immunodeficient mice using a target cell xenograft model. The killing of transplanted tumors by the TCR-based multi-specific polypeptide molecules of the present disclosure were determined to evaluate its in vivo tumor inhibition effect. The TCR-based multi-specific polypeptide molecule provided by the present disclosure has in vivo antitumor efficacy.

This application refers to various issued patents, published patent applications, journal articles, and other publications, all of which are incorporated into this application by reference. If any incorporated reference conflicts with this specification, this specification shall prevail. In addition, any specific embodiment of the present disclosure that falls within the scope of the prior art may be explicitly excluded from any one or more claims. Because such embodiments are considered to be known to those skilled in the art, they may be excluded even if such exclusion is not expressly shown in this application. Any specific embodiment of the present disclosure may be excluded from any claim for any reason, regardless of whether it is related to the existence of prior art.

Although the present disclosure has been described with reference to specific embodiments thereof, those skilled in the art should understand that various changes may be made and equivalents may be substituted without departing from the true spirit and scope of the present disclosure. In addition, many modifications may be made to adapt a particular situation, material, composition, method, or method step to the purpose, spirit, and scope of the present disclosure. All such modifications are intended to be within the scope of the claims.

## Claims

1. A multi-specific polypeptide molecule, wherein the multi-specific polypeptide molecule comprises a first binding region that binds a first antigen and a second binding region that binds a second antigen,
wherein the first binding region comprises an α chain variable region (TRAV1) and a β chain variable region (TRBV1) derived from a T cell receptor (TCR) that binds the first antigen-MHC complex;
wherein the second binding region comprises a heavy chain variable region VH1 and a light chain variable region VL1 derived from an antibody that binds the second antigen, or a single heavy chain variable region (VHH1) derived from a single-domain antibody or a nanobody that binds the second antigen;
wherein the first antigen is selected from the group consisting of tumor-associated antigens (TAA), tumor-specific antigens (TSA), viral antigens, and self-antigens, and the second antigen is an immune cell surface molecule;
wherein the N-terminus of the antibody is connected to the C-terminus of the TCR;
wherein the multi-specific polypeptide molecule further comprises at least one of the following functional domains:
1) a hinge region derived from an antibody;
2) an Fc domain derived from an antibody or a dimerization portion thereof;
3) a CH3 domain derived from an antibody;
4) albumin (Alb) or a binding domain thereof;
5) a constant region derived from a TCR or a fragment thereof.

2. The multi-specific polypeptide molecule according to claim 1, wherein TRAV1 and TRBV1 are distributed on different polypeptide chains; and/or
VH1 and VL1 are distributed on one polypeptide chain (e.g., forming scFv1); and TRAV1 and TRBV1 are distributed on different polypeptide chains.

3. The multi-specific polypeptide molecule according to claim 1 or 2, wherein the multi-specific polypeptide molecule comprises, on two polypeptide chains, the first binding region that binds the first antigen and the second binding region that binds the second antigen;
preferably, TRAV1 and TRBV1 are distributed on different polypeptide chains, the VHH1 or the VH1 and VL1 are distributed on one polypeptide chain (e.g., forming scFv1) and connected to the C-terminus of the TCR; or TRAV1 and TRBV1 are distributed on different polypeptide chains, the VH1 and VL1 are distributed on different polypeptide chains, and the polypeptide chain on which one of the VH1 and VL1 is located is connected to the C-terminus of the polypeptide chain on which the TRAV1 is located, and the polypeptide chain on which the other is located is connected to the C-terminus of the polypeptide chain on which the TRBV1 is located;
or,
the multi-specific polypeptide molecule comprises, on three polypeptide chains, a first binding region that binds a first antigen and a second binding region that binds a second antigen;
preferably, TRAV1 and TRBV1 are distributed on different polypeptide chains, the VH1 and VL1 are distributed on different polypeptide chains, and the polypeptide chain on which VH1 or VL1 is located is connected to the C-terminus of the polypeptide chain on which TRBV1 or TRAV1 is located.

4. The multi-specific polypeptide molecule according to any one of claims 1-3, wherein the polypeptide chains of the multi-specific polypeptide molecule respectively comprise:
TRBV1-scFv1 and TRAV1; or
TRAV1-scFv1 and TRBV1; or
TRBV1-VHH1 and TRAV1; or
TRAV1-VHH1 and TRBV1; or
TRBV1-VL1 and TRAV1-VH1; or
TRAV1-VL1 and TRBV1-VH1; or
TRBV1-VL1-VH1 and TRAV1; or
TRBV1-VH1-VL1 and TRAV1; or
TRAV1-VL1-VH1 and TRBV1; or
TRAV1-VH1-VL1 and TRBV1; or
TRBV1-VL1, VH1, and TRAV1; or
TRBV 1-VH1, VL1, and TRAV1; or
TRAV1-VL1, VH1, and TRBV1; or
TRAV1-VH1, VL1, and TRBV1.

5. The multi-specific polypeptide molecule according to any one of claims 1-4, wherein the multi-specific polypeptide molecule further comprises a third binding region that binds a third antigen, and the third binding region comprises an α chain variable region (TRAV2) and a β chain variable region (TRBV2) derived from a TCR that binds the third antigen-MHC complex; wherein the third antigen is selected from the group consisting of tumor-associated antigens (TAA), tumor-specific antigens (TSA), viral antigens and self-antigens, and the third antigen is the same as or different from the first antigen.

6. The multi-specific polypeptide molecule according to claim 5, wherein the TRAV2 is the same as the TRAV1, and/or the TRBV2 is the same as the TRBV1.

7. The multi-specific polypeptide molecule according to claim 5 or 6, wherein the TRAV2 and the TRBV2 are linked by a linker on the same polypeptide chain, and are preferably linked between VH1 and VL1.

8. The multi-specific polypeptide molecule according to claim 7, wherein the multi-specific polypeptide molecule comprises two polypeptide chains, and the two polypeptide chains respectively comprise:
TRBV1-VL1-TRAV2-TRBV2-VH1 and TRAV1; or
TRBV1-VL1-TRBV2-TRAV2-VH1 and TRAV1; or
TRBV1-VH1-TRBV2-TRAV2-VL1 and TRAV1; or
TRBV1-VH1-TRAV2-TRBV2-VL1 and TRAV1; or
TRAV1-VL1-TRBV2-TRAV2-VH1 and TRBV1; or
TRAV1-VL1-TRAV2-TRBV2-VH1 and TRBV1; or
TRAV1-VH1-TRAV2-TRBV2-VL1 and TRBV1; or
TRAV1-VH1-TRBV2-TRAV2-VL1 and TRBV1.

9. The multi-specific polypeptide molecule according to any one of claims 1-8, wherein the multi-specific polypeptide molecule further comprises a fourth binding region that binds a fourth antigen, the fourth binding region comprises a heavy chain variable region VH2 and a light chain variable region VL2 derived from an antibody that binds the fourth antigen, or a single heavy chain variable region (VHH2) derived from a single-domain antibody or nanobody that binds the fourth antigen; wherein the fourth antigen is an immune cell surface molecule, and the fourth antigen is the same as or different from the second antigen.

10. The multi-specific polypeptide molecule according to claim 9, wherein the VH2 is the same as the VH1, and/or the VL2 is the same as the VL1, and/or the VHH2 is the same as the VHH1.

11. The multi-specific polypeptide molecule according to claim 9 or 10, wherein the VHH2 or the VH2 and VL2 are arranged in tandem on one polypeptide chain (e.g., forming scFv2 or Fab2) and connected to the N-terminus of the TRAV1 or the TRBV1, or one of the VH2 and VL2 is connected to the N-terminus of the TRAV1 and the other is connected to the N-terminus of the TRBV1.

12. The multi-specific polypeptide molecule according to claim 11, wherein the multi-specific polypeptide molecule comprises two polypeptide chains, and the two polypeptide chains respectively comprise:
scFv2-TRBV1-scFv1 and TRAV1; or
scFv2-TRAV1-scFv1 and TRBV1; or
scFv2-TRBV1-VHH1 and TRAV1; or
scFv2-TRAV1-VHH1 and TRBV1; or
scFv2-TRBV 1-VL1 and TRAV1-VH1; or
scFv2-TRAV1-VL1 and TRBV1-VH1; or
TRBV1-scFv1 and scFv2-TRAV1; or
TRAV1-scFv1 and scFv2-TRBV1; or
TRBV1-VHH1 and scFv2-TRAV1; or
TRAV1-VHH1 and scFv2-TRBV1; or
TRBV1-VL1 and scFv2-TRAV1-VH1; or
TRAV1-VL1 and scFv2-TRBV1-VH1; or
VHH2-TRBV1-scFv1 and TRAV1; or
VHH2-TRAV1-scFv1 and TRBV1; or
VHH2-TRBV1-VHH1 and TRAV1; or
VHH2-TRAV1-VHH1 and TRBV1; or
VHH2-TRBV1-VL1 and TRAV1-VH1; or
VHH2-TRAV1-VL1 and TRBV1-VH1; or
TRBV1-scFv1 and VHH2-TRAV1; or
TRAV1-scFv1 and VHH2-TRBV1; or
TRBV1-VHH1 and VHH2-TRAV1; or
TRAV1-VHH1 and VHH2-TRBV1; or
TRBV1-VL1 and VHH2-TRAV1-VH1; or
TRAV1-VL1 and VHH2-TRBV1-VH1; or
VL2-TRBV1-scFv1 and VH2-TRAV1; or
VH2-TRBV1-scFv1 and VL2-TRAV1; or
VL2-TRAV1-scFv1 and VH2-TRBV1; or
VH2-TRAV1-scFv1 and VL2-TRBV1; or
VL2-TRBV1-VHH1 and VH2-TRAV1; or
VH2-TRBV1-VHH1 and VL2-TRAV1; or
VL2-TRAV1-VHH1 and VH2-TRBV1; or
VH2-TRAV1-VHH1 and VL2-TRBV1; or
VL2-TRBV1-VL1 and VH2-TRAV1-VH1; or
VH2-TRBV1-VL1 and VL2-TRAV1-VH1; or
VL2-TRAV1-VL1 and VH2-TRBV1-VH1; or
VH2-TRAV1-VL1 and VL2-TRBV1-VH1.

13. The multi-specific polypeptide molecule according to claim 11, wherein the multi-specific polypeptide molecule comprises a first binding region, a second binding region, a third binding region and a fourth binding region, and the multi-specific polypeptide molecule comprises two polypeptide chains, and the two polypeptide chains respectively comprise:
scFv2-TRBV1-VL1-TRAV2-TRBV2-VH1 and TRAV1; or
scFv2-TRBV1-VL1-TRBV2-TRAV2-VH1 and TRAV1; or
scFv2-TRBV1-VH1-TRBV2- TRAV2-VL1 and TRAV1; or
scFv2-TRBV1-VH1-TRAV2- TRBV2-VL1 and TRAV1; or
scFv2-TRAV1-VL1-TRBV2- TRAV2-VH1 and TRBV1; or
scFv2-TRAV1-VL1-TRAV2- TRBV2-VH1 and TRBV1; or
scFv2-TRAV1-VH1-TRAV2-TRBV2-VL1 and TRBV1; or
scFv2-TRAV1-VH1-TRBV2-TRAV2-VL1 and TRBV1; or
TRBV1-VL1-TRAV2-TRBV2-VH1 and scFv2-TRAV1; or
TRBV1-VL1-TRBV2-TRAV2-VH1 and scFv2-TRAV1; or
TRBV1-VH1-TRBV2- TRAV2-VL1 and scFv2-TRAV1; or
TRBV1-VH1-TRAV2- TRBV2-VL1 and scFv2-TRAV1; or
TRAV1-VL1-TRBV2- TRAV2-VH1 and scFv2-TRBV1; or
TRAV1-VL1-TRAV2- TRBV2-VH1 and scFv2-TRBV1; or
TRAV1-VH1-TRAV2-TRBV2-VL1 and scFv2-TRBV1; or
TRAV1-VH1-TRBV2-TRAV2-VL1 and scFv2-TRBV1; or
VHH2-TRBV1-VL1-TRAV2-TRBV2-VH1 and TRAV1; or
VHH2-TRBV1-VL1-TRBV2-TRAV2-VH1 and TRAV1; or
VHH2-TRBV1-VH1-TRBV2- TRAV2-VL1 and TRAV1; or
VHH2-TRBV1-VH1-TRAV2- TRBV2-VL1 and TRAV1; or
VHH2-TRAV1-VL1-TRBV2- TRAV2-VH1 and TRBV1; or
VHH2-TRAV1-VL1-TRAV2- TRBV2-VH1 and TRBV1; or
VHH2-TRAV1-VH1-TRAV2-TRBV2-VL1 and TRBV1; or
VHH2-TRAV1-VH1-TRBV2-TRAV2-VL1 and TRBV1; or
TRBV1-VL1-TRAV2-TRBV2-VH1 and VHH2-TRAV1; or
TRBV1-VL1-TRBV2-TRAV2-VH1 and VHH2-TRAV1; or
TRBV1-VH1-TRBV2- TRAV2-VL1 and VHH2-TRAV1; or
TRBV1-VH1-TRAV2- TRBV2-VL1 and VHH2-TRAV1; or
TRAV1-VL1-TRBV2- TRAV2-VH1 and VHH2-TRBV1; or
TRAV1-VL1-TRAV2- TRBV2-VH1 and VHH2-TRBV1; or
TRAV1-VH1-TRAV2-TRBV2-VL1 and VHH2-TRBV1; or
TRAV1-VH1-TRBV2-TRAV2-VL1 and VHH2-TRBV1; or
VL2-TRBV1-VL1-TRAV2-TRBV2-VH1 and VH2-TRAV1; or
VL2-TRBV1-VL1-TRBV2-TRAV2-VH1 and VH2-TRAV1; or
VH2-TRBV1-VL1-TRAV2-TRBV2-VH1 and VL2-TRAV1; or
VH2-TRBV1-VL1-TRBV2-TRAV2-VH1 and VL2-TRAV1; or
VL2-TRBV1-VH1-TRBV2- TRAV2-VL1 and VH2-TRAV1; or
VL2-TRBV1-VH1-TRAV2- TRBV2-VL1 and VH2-TRAV1; or
VH2-TRBV1-VH1-TRBV2- TRAV2-VL1 and VL2-TRAV1; or
VH2-TRBV1-VH1-TRAV2- TRBV2-VL1 and VL2-TRAV1; or
VL2-TRAV1-VL1-TRBV2- TRAV2-VH1 and VH2-TRBV1; or
VL2-TRAV1-VL1-TRAV2- TRBV2-VH1 and VH2-TRBV1; or
VH2-TRAV1-VL1-TRBV2- TRAV2-VH1 and VL2-TRBV1; or
VH2-TRAV1-VL1-TRAV2- TRBV2-VH1 and VL2-TRBV1; or
VL2-TRAV1-VH1-TRAV2-TRBV2-VL1 and VH2-TRBV1; or
VL2-TRAV1-VH1-TRBV2-TRAV2-VL1 and VH2-TRBV1; or
VH2-TRAV1-VH1-TRAV2-TRBV2-VL1 and VL2-TRBV1; or
VH2-TRAV1-VH1-TRBV2-TRAV2-VL1 and VL2-TRBV1.

14. The multi-specific polypeptide molecule according to any one of claims 1-13, wherein the functional domain is derived from albumin or a binding domain thereof and an optional antibody hinge region; and the functional domain is linked to the C-terminus or N-terminus, preferably the C-terminus, of any polypeptide chain of the multi-specific polypeptide molecule via an optional linker.

15. The multi-specific polypeptide molecule according to claim 14, wherein the albumin is serum albumin, preferably human serum albumin; and/or
the antibody hinge region is human-derived, e.g., a hinge domain derived from human IgG1, IgG2 or IgG4, or a portion thereof.

16. The multi-specific polypeptide molecule according to any one of claims 1-13, wherein the functional domain is a hinge region derived from an antibody, and/or an Fc domain derived from an antibody or a dimerization portion thereof, and/or a CH3 domain derived from an antibody; and any two polypeptide chains of the multi-specific polypeptide molecule are connected through covalent bonds or non-covalent bonds between the two chains of the functional domain;
preferably, the two chains of the functional domain are respectively connected to the C-terminus or N-terminus, preferably the C-terminus, of any two polypeptide chains of the multi-specific polypeptide molecule, via an optional linker; or
the first binding region and the second binding region are connected through covalent bonds or non-covalent bonds between the two chains of the functional domain.

17. The multi-specific polypeptide molecule according to claim 16, wherein the hinge region derived from an antibody is human-derived, e.g., a hinge domain derived from human IgG1, IgG2 or IgG4, or a portion thereof; and/or
the antibody Fc domain or a dimerization portion thereof is of human-derived, e.g., an Fc domain derived from human IgG1, IgG2 or IgG4, or a portion thereof.

18. The multi-specific polypeptide molecule according to claim 16 or 17, wherein the hinge region derived from an antibody comprises at least one amino acid mutation capable of reducing or eliminating binding of the multi-specific molecule to an FcR receptor and/or C1q, e.g., the mutation site is selected from the group consisting of positions 233, 234, 235 and 236; and/or
the hinge region derived from an antibody comprises at least one amino acid mutation capable of reducing or eliminating formation of a disulfide bond between the hinge region and a non-hinge region, e.g., the mutation site is at position 220; and/or
the CH2 domain in the Fc domain comprises at least one amino acid mutation capable of reducing or eliminating binding of the multi-specific molecule to an FcR receptor and/or C1q; e.g., the mutation site is selected from the group consisting of positions 237, 250, 252, 254, 256, 270, 297, 322, 327, 330 and 331; and/or
the CH3 domain in the Fc domain comprises at least one amino acid mutation capable of reducing or eliminating binding of the multi-specific molecule to an FcR receptor and/or C1q; e.g., the mutation site is selected from the group consisting of positions 428 and 434;
the CH3 domain in the Fc domain comprises at least one of the following mutations capable of promoting formation of a heterodimer of the polypeptide molecule:
a) comprises at least two cysteine residue mutations to form an inter-chain disulfide bond, e.g. 354C and 349C, or 242C and 334C;
b) comprises mutations capable of enabling two CH3 to form a knob-into-hole structure, e.g., each CH3 independently comprises a mutation at one or more positions selected from the group consisting of amino acid positions 366, 368, 405 and 407; preferably, one of the CH3 domains comprises a 366W mutation and the other comprises 366S, 368A and 407V mutations;
c) comprises mutations capable of altering charge properties of amino acids at an interaction interface of the CH3 region to promote heterodimer formation, e.g., the mutation sites in the two CH3 chains are each independently selected at least one from the group consisting of Y391, K392, T393, T394, P395, P396, V397, L398, D399, S400, Q347, V348, Y349, T350, S354, R355, E356, E357, F405, Y407 and K409.

19. The multi-specific polypeptide molecule according to claim 16 or 17, wherein the Antibody CH3 domain is human-derived, e.g., a CH3 domain derived from human IgG1, IgG2 or IgG4; and/or
the CH3 domain comprises at least one of the following mutations capable of promoting formation of a heterodimer of the polypeptide molecule:
a) comprises mutations of at least two cysteine residues to form an inter-chain disulfide bond, e.g., 354C and 349C, or 242C and 334C;
b) comprises mutations capable of enabling two CH3 domains to form a knob-into-hole structure, e.g., each CH3 domain independently comprises a mutation at one or more positions selected from the group consisting of amino acid positions 366, 368, 405 and 407, preferably one of the CH3 domains comprises a 366W mutation and the other comprises 366S, 368A and 407V mutations;
c) comprises mutations capable of altering charge properties of amino acids at an interaction interface of CH3 regions to promote heterodimer formation, e.g., the mutation sites of the two CH3 chains are each independently selected at least one from the group consisting of Y391, K392, T393, T394, P395, P396, V397, L398, D399, S400, Q347, V348, Y349, T350, S354, R355, E356, E357, F405, Y407 and K409.

20. The multi-specific polypeptide molecule according to any one of claims 1-13, wherein the functional domain is a constant region derived from a TCR or a fragment thereof and an optional hinge region derived from an antibody; and any two polypeptide chains of the multi-specific polypeptide molecule are connected through covalent bonds or non-covalent bonds between two chains of the functional domain;
preferably, the two chains of the functional domain are respectively connected to the C-terminus or N-terminus, preferably the C-terminus, of any two polypeptide chains of the multi-specific polypeptide molecule via an optional linker; or
the first binding region and the second binding region are connected by covalent bonds or non-covalent bonds between the two chains of the functional domain.

21. The multi-specific polypeptide molecule according to claim 20, wherein the hinge region derived from an antibody is human-derived, e.g., a hinge region derived from human IgG1, IgG2 or IgG4, or a portion thereof; and/or
the constant region of the TCR or a fragment thereof is human-derived or murine-derived, and is selected from the group consisting of a TCR α chain constant region (TRAC) and a TCR β chain constant region (TRBC); preferably, the TRAC and/or TRBC comprises at least one cysteine mutation relative to a wild-type sequence, to form a disulfide bond between TRAC and TRBC, more preferably, the cysteine mutation is at position 48 of the wild-type TCR α chain constant region and position 57 of the wild-type TCR β chain constant region.

22. The multi-specific polypeptide molecule according to any one of claims 1-21, wherein the multi-specific polypeptide molecule further comprises a constant domain connected to the C-terminus of TRAV1 and/or TRBV1 via an optional linker, the constant domain is selected from the group consisting of the following (1)-(3):
(1) a TCR constant region or a fragment thereof, wherein the TCR constant region, e.g., comprises a TCR α chain constant region (TRAC) and/or a β chain constant region (TRBC);
preferably, the TRAC and/or TRBC comprises at least one cysteine mutation relative to a wild-type sequence, to form a disulfide bond between TRAC and TRBC, more preferably, the cysteine mutation is at position 48 of the wild-type TCR α chain constant region and position 57 of the wild-type TCR β chain constant region;
(2) CH3, wherein the CH3 is derived from an Fc domain of an antibody, preferably the CH3 in any one polypeptide chain or in both polypeptide chains comprises at least one mutation capable of promoting formation of a heterodimer of the polypeptide molecule;
preferably, each CH3 independently comprises a mutation at one or more positions selected from the group consisting of amino acid positions 366, 368, 405 and 407 and the two CH3 do not comprise the same mutation, more preferably, one of the CH3 comprises a T366W mutation and the other comprises T366S, L368A and Y407V mutations;
(3) CL and CH1, wherein the CL is derived from an antibody light chain CL domain, and the CH1 is derived from an antibody heavy chain CH1 domain.

23. The multi-specific polypeptide molecule according to claim 22, wherein the multi-specific polypeptide molecule comprises TRAC and TRBC that are respectively connected to the C-terminus of TRAV1 and TRBV1 via an optional linker.

24. The multi-specific polypeptide molecule according to any one of claims 1-23, wherein the multi-specific polypeptide molecule further comprises CH1 and CL derived from an antibody that are respectively connected to the VH1 and VL1, wherein the VH1, VL1, CH1 and CL form a single-chain Fab and are connected to the C-terminus of the TRAV1 or the TRBV1 via a linker.

25. The multi-specific polypeptide molecule according to any one of claims 1-4 and 14-24, wherein the polypeptide chains of the polypeptide molecule comprise, from the N-terminus to the C-terminus, respectively:
a first polypeptide chain: TRBV1- optional linker -TRBC- linker -VL1- linker -VH1- optional linker - hinge region -CH2-CH3, and a second polypeptide chain: TRAV1- optional linker -TRAC- optional linker - hinge region -CH2-CH3; or
a first polypeptide chain: TRAV1- optional linker -TRAC- linker -VL1- linker -VH1- optional linker - hinge region -CH2-CH3, and a second polypeptide chain: TRBV1- optional linker -TRBC- optional linker - hinge region -CH2-CH3; or
a first polypeptide chain: TRBV1- optional linker -TRBC- linker -VH1- linker -VL1- optional linker - hinge region -CH2-CH3, and a second polypeptide chain: TRAV1- optional linker -TRAC- optional linker - hinge region -CH2-CH3; or
a first polypeptide chain: TRAV1- optional linker -TRBC- linker -VH1- linker -VL1- optional linker - hinge region -CH2-CH3, and a second polypeptide chain: TRBV1- optional linker -TRBC- optional linker - hinge region -CH2-CH3; or
a first polypeptide chain: TRBV1- optional linker -TRBC- linker -VL1- linker -VH1- linker -CH2-CH3, and a second polypeptide chain: TRAV1- optional linker -TRAC- linker -CH2-CH3; or
a first polypeptide chain: TRBV1- optional linker -TRBC- linker -VL1- linker -VH1- optional linker - hinge region -CH3, and a second polypeptide chain: TRAV1- optional linker -TRAC- optional linker -hinge region -CH3; or
a first polypeptide chain: TRBV1- optional linker -TRBC- linker -VL1- linker -VH1- optional linker - hinge region -TRBC, and a second polypeptide chain: TRAV1- optional linker -TRAC- optional linker -hinge region -TRAC; or
a first polypeptide chain: TRBV1- optional linker -TRBC- linker -VL1- linker -VH1- optional linker - hinge region, and a second polypeptide chain: TRAV1- optional linker -TRAC- optional linker -hinge region - Alb; or
a first polypeptide chain: TRBV1- optional linker -TRBC- linker -VL1- linker -VH1- linker -Alb, and a second polypeptide chain: TRAV1- optional linker -TRAC; or
a first polypeptide chain: TRAV1- optional linker -CH3- linker -VL1- linker -VH1- optional linker - optional hinge region -CH2-CH3, and a second polypeptide chain: TRBV1- optional linker -CH3- optional linker - optional hinge region -CH2-CH3; or
a first polypeptide chain: TRAV1- optional linker -CH1- linker -VL1- linker -VH1- optional linker - optional hinge region -CH2-CH3, and a second polypeptide chain: TRBV1- optional linker -CL- optional linker - optional hinge region -CH2-CH3; or
a first polypeptide chain: TRBV1- optional linker -TRBC- linker -VHH1- optional linker -hinge region - CH2-CH3, and a second polypeptide chain: TRAV1- optional linker -TRAC- optional linker -hinge region - CH2-CH3; or
the second binding region comprises an antibody-derived Fab, and wherein a first polypeptide chain: TRBV1- optional linker -TRBC- linker -VL1-CL- linker -VH1-CH1- optional linker -hinge region -CH2-CH3, and a second polypeptide chain: TRAV1- optional linker -TRAC- optional linker -hinge region -CH2-CH3; or
a first polypeptide chain: TRBV1- optional linker -TRBC- linker -VL1, a second polypeptide chain: VH1-optional linker -hinge region -CH2-CH3, and a third polypeptide chain: TRAV1- optional linker -TRAC-optional linker -hinge region -CH2-CH3; or
a first polypeptide chain: TRBV1- optional linker -TRBC- linker -VH1, a second polypeptide chain: VL1-optional linker -hinge region -CH2-CH3, and a third polypeptide chain: TRAV1- optional linker -TRAC-optional linker -hinge region -CH2-CH3; or
a first polypeptide chain: TRAV1- optional linker -TRAC- linker -VL1, a second polypeptide chain: VH1-optional linker -hinge region -CH2-CH3, and a third polypeptide chain: TRBV1- optional linker -TRBC-optional linker -hinge region -CH2-CH3; or
a first polypeptide chain: TRAV1- optional linker -TRAC- linker -VH1, a second polypeptide chain: VL1-optional linker -hinge region -CH2-CH3, and a third polypeptide chain: TRBV1- optional linker -TRBC-optional linker -hinge region -CH2-CH3; or
a first polypeptide chain: TRBV1- optional linker -TRBC- linker -VL1- optional linker -hinge region - CH2-CH3, a second polypeptide chain: VH1- optional linker -hinge region -CH2-CH3, and a third polypeptide chain: TRAV1- optional linker -TRAC; or
a first polypeptide chain: TRBV1- optional linker -TRBC- linker -VH1- optional linker -hinge region - CH2-CH3, a second polypeptide chain: VL1- optional linker -hinge region -CH2-CH3, and a third polypeptide chain:TRAV1- optional linker -TRAC; or
a first polypeptide chain: TRAV1- optional linker -TRAC- linker -VL1- optional linker -hinge region - CH2-CH3, a second polypeptide chain: VH1- optional linker -hinge region -CH2-CH3, and a third polypeptide chain: TRBV1- optional linker -TRBC; or
a first polypeptide chain: TRAV1- optional linker -TRAC- linker -VH1- optional linker -hinge region - CH2-CH3, a second polypeptide chain: VL1- optional linker -hinge region -CH2-CH3, and a third polypeptide chain: TRBV1- optional linker -TRBC.

26. The multi-specific polypeptide molecule according to any one of claims 5-8 and 14-24, wherein the polypeptide molecule comprises a first polypeptide chain and a second polypeptide chain; wherein:
the first polypeptide chain comprises, from the N-terminus to the C-terminus: TRBV1- optional linker - TRBC- linker -VL1- linker -TRAV2- linker -TRBV2- linker -VH1- optional linker -hinge region -CH2-CH3, and the second polypeptide chain comprises, from N-terminus to C-terminus: TRAV1- optional linker -TRAC-optional linker -hinge region -CH2-CH3.

27. The multi-specific polypeptide molecule according to any one of claims 9-12 and 14-24, wherein the polypeptide molecule comprises a first polypeptide chain and a second polypeptide chain; wherein:
the first polypeptide chain comprises, from the N-terminus to the C-terminus: VL2- linker -VH2- linker - TRBV1- optional linker -TRBC- linker -VL1- linker -VH1- optional linker -hinge region -CH2-CH3, and the second polypeptide chain comprises, from the N-terminus to the C-terminus: TRAV1- optional linker -TRAC-optional linker -hinge region -CH2-CH3; or
the first polypeptide chain comprises, from the N-terminus to the C-terminus: VL2- linker -TRBV1-optional linker -TRBC- linker -VL1- linker -VH1- optional linker -hinge region -CH2-CH3, and the second polypeptide chain comprises, from the N-terminus to the C-terminus: VH2- linker -TRAV1- optional linker - TRAC- optional linker -hinge region -CH2-CH3; or
the first polypeptide chain comprises, from the N-terminus to the C-terminus: VL2- linker -TRBV1-optional linker -TRBC- linker -VL1- linker -VH1- optional linker -hinge region -CH2-CH3, and the second polypeptide chain comprises, from the N-terminus to the C-terminus: VH2- linker -TRAV1- optional linker - TRAC- optional linker -hinge region -CH2-CH3.

28. The multi-specific polypeptide molecule according to any one of claims 13-24, wherein the polypeptide molecule comprises a first polypeptide chain and a second polypeptide chain; wherein:
the first polypeptide chain comprises, from the N-terminus to the C-terminus: VL2- linker -VH2- linker - TRBV1- optional linker -TRBC- linker -VL1- linker -TRAV2- linker -TRBV2- linker -VH1- optional linker - hinge region -CH2-CH3, and the second polypeptide chain comprises, from the N-terminus to the C-terminus: TRAV1- optional linker -TRAC- optional linker -hinge region -CH2-CH3.

29. The multi-specific polypeptide molecule according to any one of claims 1-28, wherein
the VH1 and VL1 comprise at least one cysteine mutation to form a disulfide bond between VH1 and VL1, preferably, the cysteine mutations are at position 44 of VH1 and position 100 of VL1; and/or
the VH2 and VL2 comprise at least one cysteine mutation to form a disulfide bond between VH2 and VL2, preferably, the cysteine mutations are at position 44 of VH2 and position 100 of VL2; and/or
the TRAV1 and VL1 or VL2 comprise at least one cysteine mutation to form a disulfide bond between TRAV1 and VL1 or VL2, preferably, the cysteine mutations are at position 104 of TRAV1, and position 80 of VL1 or VL2; and/or
the TRAV2 and VL1 or VL2 comprise at least one cysteine mutation to form a disulfide bond between TRAV2 and VL1 or VL2, preferably, the cysteine mutations are at position 104 of TRAV2, and position 80 of VL1 or VL2; and/or
the TRBV1 and VL1 or VL2 comprise at least one cysteine mutation to form a disulfide bond between TRBV1 and VL1 or VL2, preferably, the cysteine mutations are at position 84 of TRBV1, and position 80 of VL1 or VL2; and/or
the TRBV2 and VL1 or VL2 comprise at least one cysteine mutation to form a disulfide bond between TRBV2 and VL1 or VL2, preferably, the cysteine mutations are at position 84 of TRBV2, and position 80 of VL1 or VL2; and/or
the TRAV1 and VH1 or VH2 comprise at least one cysteine mutation to form a disulfide bond between TRAV1 and VH1 or VH2, preferably, the cysteine mutations are at position 104 of TRAV1, and position 89 of VH1 or VH2; and/or
the TRAV2 and VH1 or VH2 comprise at least one cysteine mutation to form a disulfide bond between TRAV2 and VH1 or VH2, preferably, the cysteine mutations are at position 104 of TRAV2, and position 89 of VH1 or VH2.

30. The multi-specific polypeptide molecule according to any one of claims 1-29, wherein the polypeptide molecule further comprises one or more amino acid mutations, insertions, or deletions capable of enhancing the affinity or biological activity thereof, enhancing the stability thereof, prolonging the half-life thereof, reducing the immunogenicity thereof, or reducing the post-translational modifications thereof; e.g., the amino acid mutations include mutations capable of enhancing binding to FcRn and/or effector function silencing mutations.

31. The multi-specific polypeptide molecule according to any one of claims 1-30, wherein the linkers in the polypeptide molecule are each independently selected from linkers consisting of 1-35 amino acids;
preferably, the linkers are each independently selected from the group consisting of S, GS, GGGS, GGGGS, GGGSGGGG, GGSGGS, GGSGGSGGS, GGGGSGGGGS, GGGGSGGGGSGGGGS, GGGGSGGGGSGGGGSGGGGSGGGS, GGGGSGGGGSGGGGSGGGGSGGGGSGGGGS, GQPKAAP, TVLRT, TVSSAS, GGEGG, GSEGGGS, RTSGPGDGGKGGPGKGPGGEGTKGTGPGG, GKGPGGEGTKGTGPGG, TVLSSAS, EDLKN or a variant thereof, EDLNK or a variant thereof, and ANIQK or a variant thereof, or any combination thereof;
preferably, a variant of EDLKN is an amino acid sequence formed by substitution, deletion, or addition of one or several amino acids in EDLKN; and/or a variant of EDLNK is an amino acid sequence formed by substitution, deletion, or addition of one or several amino acids in EDLNK; and/or a variant of ANIQK is an amino acid sequence formed by substitution, deletion, or addition of one or several amino acids in ANIQK.

32. The multi-specific polypeptide molecule according to any one of claims 1-31, wherein the linkers in the polypeptide molecule are each independently selected from linkers consisting of no more than 15 amino acids;
preferably, the linkers are each independently selected from the group consisting of S, GS, GGGS, GGGGS, GGGSGGGG, GGSGGS, GGSGGSGGS, GGGGSGGGGS, GGGGSGGGGSGGGGS, EDLKN or a variant thereof, EDLNK or a variant thereof, and ANIQK or a variant thereof;
preferably, the variant of EDLKN is an amino acid sequence formed by substitution, deletion, or addition of one or several amino acids in EDLKN; and/or the variant of EDLNK is an amino acid sequence formed by substitution, deletion, or addition of one or several amino acids in EDLNK; and/or the variant of ANIQK is an amino acid sequence formed by substitution, deletion, or addition of one or several amino acids in ANIQK.

33. The multi-specific polypeptide molecule according to any one of claims 1-32, wherein the polypeptide molecule further comprises a signal peptide sequence at the N-terminus of one or more polypeptide chains, preferably comprises a signal peptide sequence at the N-terminus of each polypeptide chain, e.g. a signal peptide sequence derived from albumin or an immunoglobulin, preferably as shown in SEQ ID NO: 164.

34. The multi-specific polypeptide molecule according to any one of claims 1-33, wherein the first antigen and the third antigen are each independently a TAA selected from the group consisting of the following antigens: melanoma-associated antigens (e.g., gp100, MAGEA1, MAGEA3, MAGEA6, MAGEA4, MAGEA2, MAGEA12, MAGEA2B, MAGEA9B, MAGEA10, MAGEA11, MAGEB2, MAGEC1, MAGEC2), IGF2BP1, GNGT1, PI4K2B, CCR8, NPSR1, COX7B2, ONECUT3, SMC1B, FOXI3, GAGE2A, FBXO43, BRDT, PAGE2, GAGE13, POU5F1B, CTAG1A, and endogenous reverse transcriptase antigen; or
wherein the first antigen and the third antigen are each independently a TSA selected from the group consisting of the following antigens: KRAS antigens (e.g., a KRAS G12 mutant antigens), TP53, PIK3CA, CTNNB1, EGFR, BRAF, and GNAS; or
wherein the first antigen and the third antigen are each independently a viral antigen selected from the group consisting of the following antigens: HPV antigens (e.g., an HPV E6 or E7 antigen), CMV antigens, HBV antigens, EBV antigens, herpesvirus antigens, human immunodeficiency virus (HIV) antigens, influenza virus antigens, and coronavirus antigens; or
the first antigen and the third antigen are each independently an self-antigen selected from the group consisting of the following antigens: AFP, CEA, CD19, CD20, BCMA, CD22, CD30, SLAM, CLDN18.2, GD2, mesothelin, CD38, Her2, GPC3, MUC1, Ro52, Ro60, La, Jo-1, SRP, IFIH1, CENPA, CENPB, SNRPA1, SNRNP70, SNR-PD3, RNAP3, TOPO1, Insulin, GAD65, IA2, Znt8, PL7, TARS, ARS, MI2, topoisomerase **1,** EXOSC9, EXOSC107, POLR3A, POLR3K, PTRN, GAD2, SLC30A8, AchR, MUSK, LRP4, PLA2R, THSD7A, TSHR, IFN-γ, CHRNA1, MUSK, LRP4, AQP4, MOG, GRIN1, COL4A3, PLA2R, GM-SCF, PR3 and MPO.

35. The multi-specific polypeptide molecule according to any one of claims 1-34, wherein the second antigen and the fourth antigen are each independently selected from the group consisting of CD3 (e.g., CD3γ, CD3δ and CD3ε chains), CD4, CD8, CD10, CD11b, CD11c, CD14, CD16, CD18, CD25, CD32a, CD32b, CD41, CD41b, CD42a, CD42b, CD44, CD45RA, CD49, CD61, CD64, CD68, CD94, CD90, CD117, Nkp46, NKG2D, FcεRI, TCRα/β, TCR γ/δ, HLA-DR, CD28, 4-1BB(CD137), OX40(CD134), ICOS(CD278), 2B4 (CD244), HVEM, LAG3, DAP10, DAP12, CD27, CD40, GITR, LFA-1, MyD88, CD2, CD7, LIGHT, B7-H3, CTLA-4, PD-1, PD-L1, CD80, BTLA, TIM3, TIGIT and LAG-3.

36. The multi-specific polypeptide molecule according to any one of claims 1-35, wherein the first antigen and the third antigen are each independently selected from the group consisting of melanoma-associated antigens (e.g., gp100, MAGEA1), KRAS antigens (e.g., a KRAS G12 mutant antigen), and HPV antigens (e.g., an HPV E7 antigen), and/or the second antigen and the fourth antigen are each independently selected from the group consisting of CD3, CD28, 4-1BB(CD137), PD-1, and PD-L1.

37. A nucleic acid, comprising a nucleotide sequence encoding each chain of the multi-specific polypeptide molecule according to any one of claims 1-36.

38. A vector, comprising the nucleic acid according to claim 37.

39. A vector system, comprising, on one or more vectors, nucleotide sequences encoding each chain of the multi-specific polypeptide molecule according to any one of claims 1-36.

40. A host cell, comprising the nucleic acid according to claim 37, the vector according to claim 38, or the vector system according to claim 39.

41. A conjugate, comprising the multi-specific polypeptide molecule according to any one of claims 1-36, and a chemical moiety conjugated to the multi-specific polypeptide molecule.

42. The conjugate according to claim 41, wherein the chemical moiety is selected from the group consisting of a therapeutic agent, an immunostimulatory molecule and a detectable label;
preferably, the therapeutic agent is selected from the group consisting of an immunomodulator, an agonist, a radioactive compound, an enzyme, a chemotherapeutic agent, and a toxin.
preferably, the immunostimulatory molecule is selected from the group consisting of a cytokine, a chemokine, a platelet factor, and a complement initiator;
preferably, the detectable label is selected from the group consisting of biotin, streptavidin, an enzyme or a catalytically active fragment thereof, a radionuclide, a nanoparticle, a paramagnetic metal ion, a nucleic acid probe, a contrast agent, and a fluorescent, phosphorescent, or chemiluminescent molecule.

43. A pharmaceutical composition, comprising the multi-specific polypeptide molecule according to any one of claims 1-36, the nucleic acid according to claim 37, the vector according to claim 38, the vector system according to claim 39, the host cell according to claim 40, the conjugate according to claim 41 or 42, and a pharmaceutically acceptable excipient.

44. The pharmaceutical composition according to claim 43, wherein the pharmaceutical composition further comprises a second therapeutic agent, preferably, the second therapeutic agent is selected from the group consisting of an antibody, a chemotherapeutic agent, and a small-molecule drug.

45. A method for preventing or treating a disease in a subject, comprising administering to the subject an effective amount of the multi-specific polypeptide molecule according to any one of claims 1-36, the nucleic acid according to claim 37, the vector according to claim 38, the vector system according to claim 39, the host cell according to claim 40, the conjugate according to claim 41 or 42, or the pharmaceutical composition according to claim 43 or 44, wherein the disease is selected from the group consisting of cancer, an infectious disease, an autoimmune disease, and an inflammatory disease.

46. The method according to claim 45, further comprising administering a second therapeutic agent to the subject, preferably, the second therapeutic agent is selected from the group consisting of an antibody, a chemotherapeutic agent, and a small-molecule drug.
